(19) Europäisches Patentamt
European Patent Office
Office européen des brevets

(11) EP 0 549 347 B1

(12) EUROPEAN PATENT SPECIFICATION

(45) Date of publication and mention of the grant of the patent: **29.10.1997 Bulletin 1997/44**

(21) Application number: **92311766.7**

(22) Date of filing: **23.12.1992**

(51) Int. Cl.$^6$: **C07C 69/757**, C07C 69/78, C07D 239/26, C09K 19/32, C09K 19/34, C09K 19/30, G02F 1/137

# (54) Novel carboxylate compounds, liquid crystal materials, liquid crystal compositions and liquid crystal elements

Karbonsäureesterverbindungen, flüssigkristalline Stoffe, flüssigkristalline Zusammensetzungen und flüssigkristalline Bauteile

Composés carboxylales, matériaux cristallins liquides, compositions cristallines liquides et éléments cristallins liquides

(84) Designated Contracting States:
**DE FR GB IT NL**

(30) Priority: **27.12.1991 JP 347122/91**
**13.03.1992 JP 55209/92**
**13.03.1992 JP 55210/92**

(43) Date of publication of application:
**30.06.1993 Bulletin 1993/26**

(73) Proprietor: **MITSUI PETROCHEMICAL INDUSTRIES, LTD.**
**Chiyoda-ku, Tokyo 100 (JP)**

(72) Inventors:
- **Tatsuki, Yuuichirou,**
  **Mitsui Petrochemical Ind. Ltd**
  **Sodegaura-shi, Chiba 299-02 (JP)**
- **Nishiyama, Shinichi,**
  **Mitsui Petrochemical Ind. Ltd**
  **Sodegaura-shi, Chiba 299-02 (JP)**
- **Kawabata, Junichi,**
  **Mitsui Petrochemical Ind. Ltd.**
  **Sodegaura-shi, Chiba 299-02 (JP)**
- **Yamanaka, Tooru,**
  **Mitsui Petrochemical Ind. Ltd.**
  **Sodegaura-shi, Chiba 299-02 (JP)**
- **Tanaka, Chiho,**
  **Mitsui Petrochemical Ind. Ltd.**
  **Sodegaura-shi, Chiba 299-02 (JP)**

(74) Representative: **Cresswell, Thomas Anthony et al**
**J.A. KEMP & CO.**
**14 South Square**
**Gray's Inn**
**London WC1R 5LX (GB)**

(56) References cited:
**EP-A- 0 332 456**
**EP-A- 0 341 922**
**EP-A- 0 401 961**
**EP-A- 0 413 585**
**EP-A- 0 431 929**
**EP-A- 0 440 134**
**EP-A- 0 467 662**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 549 347 B1

Printed by Rank Xerox (UK) Business Services
2.14.18/3.4

## Description

This invention relates to carboxylate (carboxylic acid ester) compounds, liquid crystal materials comprising said carboxylate compounds, liquid crystal compositions comprising said carboxylate compounds and liquid crystal elements filled with said liquid crystal materials or compositions.

Display devices using liquid crystal compounds, which are used widely at present, are driven by means of a TN (twisted nematic) mode.

Such display devices, however, have problems that since the position of the molecule of the liquid crystal compound present in the element must be changed in order to change the displayed images, the driving time necessary therefor becomes longer and also the voltage necessary for changing the position of the molecule of the liquid crystal compound, that is the electric power consumption, becomes greater.

Differing from switching elements utilizing the TN mode or an STN mode, those using ferroelectric liquid crystal compounds are able to function as switching elements only by changing the direction of molecular orientation of the liquid crystal compounds; hence the switching time required for operating the switching elements is shortened remarkably. Further, because a Ps x E value obtained from a spontaneous polarization (Ps) of the ferroelectric liquid crystal compound and an intensity of the electric field (E) applied thereto is the effective energy output for changing the direction of molecular orientation of said liquid crystal compound, the power consumption required therefor can also be minimized. Such ferroelectric liquid crystal compounds are particularly suitable for use in display devices for moving pictures, because they have two steady states depending upon the direction of the applied electric field, i.e. bistability, and also have very favorable switching threshold value characteristics.

Such ferroelectric liquid crystal compounds or anti-ferroelectric liquid crystal compounds are used in, for example, optical switching elements. These compounds are required to have many characteristics such that their operating temperature is in the vicinity of or below ordinary temperature, the operating temperature range is broad, the switching speed is high (fast) and the switching threshold voltage is within an appropriate range. Of these characteristics, the operating temperature range is especially important when the ferroelectric crystal compounds or anti-ferroelectric crystal compounds are put to practical use.

However, in known ferroelectric liquid crystal compounds the operating temperature range is generally narrow. Even in the case of ferroelectric liquid crystal compounds having a wide operating temperature range, said operating temperature range is in a high temperature region excluding room temperature, as disclosed, for example, in R. B. Meyer et al., J. de Phys., Vol. 36, 169 (1975) or M. Taguchi and T. Harada, "Proceedings of Eleventh Conference on Liquid Crystals," 168 (1985). Thus, no ferroelectric liquid crystal compounds which are satisfactory from the standpoint of practical use are yet obtainable.

Further, anti-ferroelectric liquid crystal compounds have the same tendency as observed in the ferroelectric liquid crystal compounds. Thus no anti-ferroelectric liquid crystal compounds which are satisfactory from the standpoint of practical use are yet obtainable.

The present invention seeks to solve the above-mentioned problems associated with the prior art by providing compounds which are usable for forming liquid crystal elements which have a low operating temperature, high (fast) switching speed, very small electric power consumption and, moreover, which are capable of giving a stabilized contrast.

The present invention provides a first carboxylic acid ester (carboxylate) compound represented by the following formula [I] or [II]

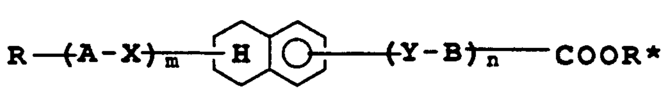

... [I]

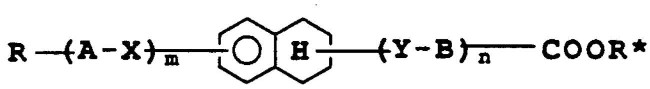

... [II]

wherein

R is a group selected from an alkyl of 3-20 carbon atoms, an alkoxy of 3-20 carbon atoms and a halogenated alkyl of 3-20 carbon atoms,

X and Y are independently a group selected from -COO-, -OCO-, $-CH_2CH_2-$, $-CH_2O-$, $-OCH_2-$, -S-S-, $-CO-CH_2-$ and $-CH_2-CO-$, or a single bond,

A is a group selected from

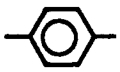 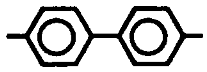 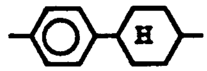 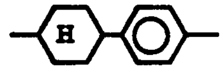 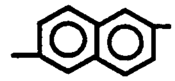

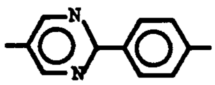 and 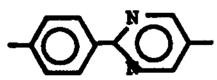 ,

B is a group selected from

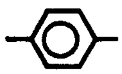 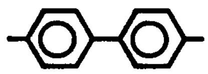 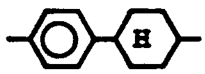

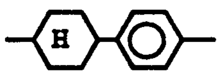 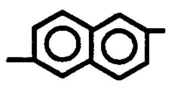 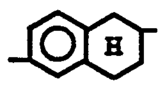 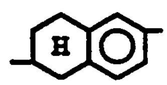 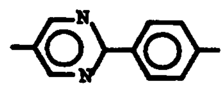

and

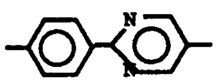 ,

R* is an optically active group of 4-20 carbon atoms containing at least one asymmetric (or chiral) carbon atom where hydrogen atoms attached to the carbon atoms constituting said optically active group may be substituted with a halogen atom,
m is 1 or 2, and n is an integer of 0-2.

The present invention also provides a second carboxylic acid ester (carboxylate) compound represented by the following formula [III] or [IV]

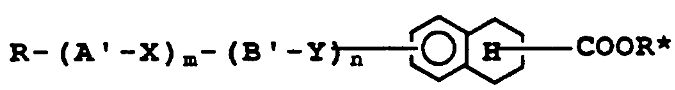 ... [III]

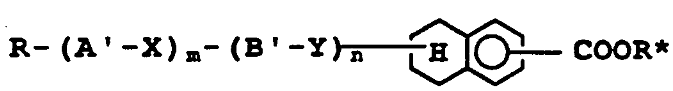 ... [IV]

wherein R, R*, X and Y are as defined in formula [I] or [II], provided that R may be a hydrocarbon group having an -OCO- group wherein at least one of the hydrogen atoms constituting said hydrocarbon group may be substituted with halogen atoms, and R may have at least one asymmetric carbon atom,

A' and B' are independently a group selected from

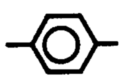 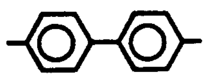 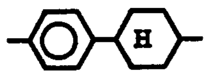 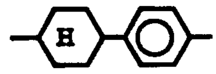

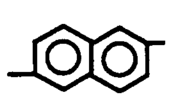 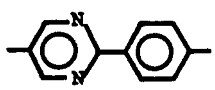 and 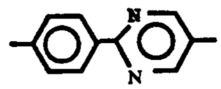

m is 1 or 2, n is 0-2, and m + n ≥ 2.

The present invention also provides a liquid crystal material comprising a carboxylate compound represented by formula [I], [II], [III] or [IV].

The present invention additionally provides a liquid crystal composition comprising a carboxylate compound represented by formula [I], [II], [III] or [IV].

The present invention further provides a liquid crystal element comprising a cell composed of two substrate opposite to each other, a gap formed by said substrates and a liquid crystal substance as defined above filled in said gap.

Thus, the present invention provides novel carboxylate compounds which are extremely useful as liquid crystal materials. Accordingly, liquid crystal elements of the invention possess excellent liquid crystal characteristics.

By the use of the carboxylate compounds of the invention as liquid crystal materials, there can be obtained various devices having excellent characteristics such as a broad operating temperature range, a fast switching speed, a very small electric power consumption and a stabilized contrast.

Fig. 1 shows the $^{1}$H-NMR spectrum of 4-[6'-(4"-decyloxybenzoyloxy)-5',6',7',8'-tetrahydro-2'-naphthoyloxy]benzoic acid R-1'''-trifluoromethylheptyl ester [Exemplified compound (4)].

Fig. 2 shows the $^{1}$H-NMR spectrum of 6-[6'-(4"-decyloxybenzoyloxy)-5',6',7',8'-tetrahydro-2'-naphthoyloxy]-1,2,3,4-tetrahydronaphthalene-2-carboxylic acid R-1'''-trifluoromethylheptyl ester [Exemplified compound (21)].

Fig. 3 shows the $^{1}$H-NMR spectrum of 6-[6'-(4"-decyloxybenzoyloxy)-5',6',7',8'-tetrahydro-2'-naphthoyloxy]-5,6,7,8-tetrahydronaphthalene-2-carboxylic acid R-1'''-trifluoromethylheptyl ester [Exemplified compound (22)].

Fig. 4 shows the $^{1}$H-NMR spectrum of 6-[4"-(5'-decyl-2'-pyrimidinyl)benzoyloxy]-5,6,7,8-tetrahydronaphthalene-2-carboxylic acid R-1'''-trifluoromethylheptyl ester [Exemplified compound (130)].

Fig. 5 shows the $^{1}$H-NMR spectrum of 4-[6'-(4"-decyloxybenzoyloxy)-1',2',3',4'-tetrahydro-2'-naphthoyloxy]benzoic acid R-1'''-trifluoromethylheptyl ester [Exemplified compound (204)].

Fig. 6 shows the $^{1}$H-NMR spectrum of 6-[6'-(4"-decyloxybenzoyloxy)-1',2',3',4'-tetrahydro-2'-naphthoyloxy]-1,2,3,4-tetrahydronaphthalene-2-carboxylic acid R-1'''-trifluoromethylheptyl ester [Exemplified compound (221)].

Fig. 7 shows the $^{1}$H-NMR spectrum of 6-[6'-(4"-decyloxybenzoyloxy)-1',2',3',4'-tetrahydro-2'-naphthoyloxy]-5,6,7,8-tetrahydronaphthalene-2-carboxylic acid R-1'''-trifluoromethylheptyl ester [Exemplified compound (222)].

Fig. 8 shows the $^{1}$H-NMR spectrum of 6-[4'-(4"-decyloxybiphenyl)carboxy]-1,2,3,4-tetrahydronaphthalene-2-carboxylic acid R-1'''-trifluoromethylheptyl ester [Exemplified compound (251)].

Fig. 9 shows the $^{1}$H-NMR spectrum of 6-[4'-(4"-decyloxyphenylcyclohexane)carboxy]-1,2,3,4-tetrahydronaphthalene-2-carboxylic acid R-1'''-trifluoromethylheptyl ester [Exemplified compound (267)].

Fig. 10 shows the $^{1}$H-NMR spectrum of 6-(6'-heptyloxy-2'-naphthoyloxy)-1,2,3,4-tetrahydronaphthalene-2-carboxylic acid R-1"-trifluoromethylheptyl ester [Exemplified compound (296)].

Fig. 11 shows the $^{1}$H-NMR spectrum of 6-(6'-decyloxy-2'-naphthoyloxy)-1,2,3,4-tetrahydronaphthalene-2-carboxylic acid R-1"-trifluoromethylheptyl ester [Exemplified compound (299)].

Fig. 12 shows the $^{1}$H-NMR spectrum of 6-[4'-(5"-decyl-2"-pyrimidinyl)benzoyloxy]-1,2,3,4-tetrahydronaphthalene-2-carboxylic acid R-1'''-trifluoromethylheptyl ester [Exemplified compound (331)].

Fig. 13 shows the $^{1}$H-NMR spectrum of 6-[4'-(4"-decyloxybenzoyloxy)benzoyloxy]-1,2,3,4-tetrahydronaphthalene-2-carboxylic acid R-1'''-trifluoromethylheptyl ester [Exemplified compound (404)].

Fig. 14 shows the $^{1}$H-NMR spectrum of 6-[4'-(4"-(R-1'''-methylheptyloxy)benzoyloxy)benzoyloxy]-1,2,3,4-tetrahydronaphthalene-2-carboxylic acid R-1''''-trifluoromethylheptyl ester [Exemplified compound (545)].

Fig. 15 shows the $^{1}$H-NMR spectrum of 6-[4'-(4"-decyloxybenzoyloxy)benzoyloxy]-5,6,7,8- tetrahydronaphthalene-2-carboxylic acid R-1'''-trifluoromethylheptyl ester [Exemplified compound (575)].

Fig. 16 shows the $^{1}$H-NMR spectrum of 6-[4'-(4"-(R-1'''-methylheptyloxy)benzoyloxy)benzoyloxy]-5,6,7,8-tetrahydronaphthalene-2-carboxylic acid R-1''''-trifluoromethylheptyl ester [Exemplified compound (716)].

Fig. 17 is a schematical sectional view of the liquid crystal element of the present invention.

Fig. 18 (a) is a sectional view of the liquid crystal element having a concentric spacer, and Fig. 18 (b) is an A-A

cross sectional view of Fig. 18 (a).

Fig. 19 (a) is a sectional view of the liquid crystal element having a comb-shaped spacer, and Fig. 19 (b) is an A-A cross sectional view of Fig. 19 (a).

Fig. 20 is a sectional view showing the structure of the liquid crystal element having a fiber spacer of the present invention.

Fig. 21 is a sectional view showing the structure of the liquid crystal element of the invention, in which a cell composed of two polarizing plates is arranged.

Figs. 22 (a) and 22 (b) show examples of a nonlinear element and a three-terminal element, respectively.

Fig. 23 shows the $^1$H-NMR spectrum of 6-(4'-decyloxybenzoyloxy)-1,2,3,4-tetrahydronaphthalene-2-carboxylic acid R-1"-trifluoromethylheptyl ester [Compound (235)].

Fig. 24 shows the $^1$H-NMR spectrum of 6-[4'-(4"-undecyloxybiphenyl)carboxy]-1,2,3,4-tetrahydronaphthalene-2-carboxylic acid R-1'"-trifluoromethylheptyl ester [Compound (252)].

Fig. 25 shows the $^1$H-NMR spectrum of 6-[4'-(4"-dodecyloxybiphenyl)carboxy]-1,2,3,4-tetrahydronaphthalene-2-carboxylic acid R-1'"-trifluoromethylheptyl ester [Compound (253)].

Fig. 26 shows the $^1$H-NMR spectrum of 6-[4'-(4"-octylbiphenyl)carboxy]-1,2,3,4-tetrahydronaphthalene-2-carboxylic acid R-1'"-trifluoromethylheptyl ester.

Fig. 27 shows the $^1$H-NMR spectrum of 6-[4'-(4"-decylbiphenyl)carboxy]-1,2,3,4-tetrahydronaphthalene-2-carboxylic acid R-1'"-trifluoromethylheptyl ester.

Fig. 28 shows the $^1$H-NMR spectrum of 6-[4'-(4"-dodecylbiphenyl)carboxy]-1,2,3,4-tetrahydronaphthalene-2-carboxylic acid R-1'"-trifluoromethylheptyl ester.

Fig. 29 shows the $^1$H-NMR spectrum of 6-[4'-(4"-octylbiphenyl)carboxy]-1,2,3,4-tetrahydronaphthalene-2-carboxylic acid R-1'"-methylheptyl ester.

Fig. 30 shows the $^1$H-NMR spectrum of 6-[4'-(4"-decylbiphenyl)carboxy]-1,2,3,4-tetrahydronaphthalene-2-carboxylic acid R-1'"-methylheptyl ester.

Fig. 31 shows the $^1$H-NMR spectrum of 6-[4'-(4"-(S-1'"-methylheptyloxy)biphenyl)carboxy]-1,2,3,4-tetrahydronaphthalene-2-carboxylic acid R-1""-methylheptyl ester.

Fig. 32 shows the $^1$H-NMR spectrum of 6-[4'-(4"-octyloxycyclohexyl)carboxy]-1,2,3,4-tetrahydronaphthalene-2-carboxylic acid R-1'"-trifluoromethylheptyl ester [Compound (265)].

Fig. 33 shows the $^1$H-NMR spectrum of 6-[4'-(4"-undecyloxyphenylcyclohexyl)carboxy]-1,2,3,4-tetrahydronaphthalene-2-carboxylic acid R-1'"-trifluoromethylheptyl ester [Compound (269)].

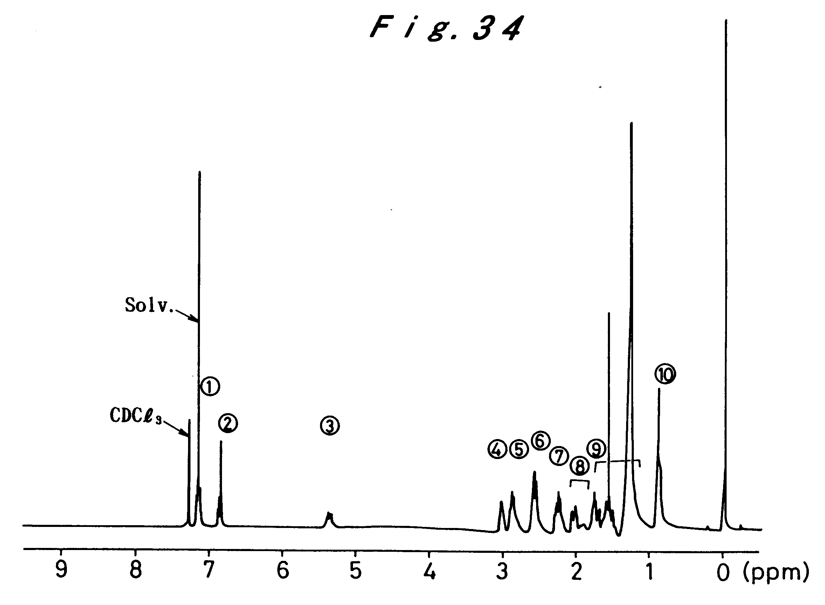

Fig. 34 shows the $^1$H-NMR spectrum of 6-[4'-(4"-decylphenyltranscyclohexyl)carboxy]-1,2,3,4-tetrahydronaphthalene-2-carboxylic acid R-1'"-trifluoromethylheptyl ester.

Numerals used in the drawings have the following meanings:

| | |
|---|---|
| 11a,11b,27a,27b,37,47,57 | Transparent substrate |
| 12,23,33,43,53 | Liquid crystal substance |
| 13,58 | Cell |
| 14 | Gap |
| 15a,15b,25a,25b,35,45,55 | Transparent electrode |
| 26 | Concentric spacer |
| 36 | Comb-shaped spacer |
| 46 | Fiber |
| 56 | Polarizing plate |

The present invention is illustrated below in more detail.

In formula [I] or [II], R is a group selected from an alkyl of 3-20 carbon atoms, an alkoxy of 3-20 carbon atoms and a halogenated alkyl of 3-20 carbon atoms.

When R is an alkyl of 2-30 carbon atoms, the alkyl may be straight, branched or alicyclic. In particular, the carboxylate compounds having a straight chain alkyl R have excellent liquid crystal properties because the molecules have a rigid linear structure. The straight chain alkyl may include hexyl, heptyl, octyl, nonyl, decyl, undecyl, dodecyl, tetradecyl, hexadecyl and octadecyl.

When R is an alkoxy of 3-20 carbon atoms, the alkoxy may include those having the alkyl moieties mentioned above. Thus, the alkoxy may include hexoxy, heptoxy, octyloxy, nonyloxy, decyloxy, undecyloxy, dodecyloxy, tetradecyloxy, heptadecyloxy, hexadecyloxy and octadecyloxy.

When R is a halogenated alkyl of 3-20 carbon atoms, the halogenated alkyl includes those in which at least a part of the hydrogen atoms of the alkyl moieties mentioned above is substituted with a halogen atom such as F, Cl, Br or I.

R may have at least one asymmetric carbon atom.

The carboxylate compounds having an alkoxy group R have particularly excellent liquid crystal properties.

In formula [I] or [II], X and Y are each independently a group selected from -COO-, -OCO-, $-CH_2CH_2-$, $-CH_2O-$, -

$OCH_2$-, -S-S-, -CO-$CH_2$- and -$CH_2$-CO-, or a single bond. In the case where the carboxylate compounds of formula [I] or [II] are used as liquid crystal materials, it is preferable that X and Y are each independently a group selected from -COO-, -OCO-, -$CH_2CH_2$-, -$CH_2$O-, and -$OCH_2$-, or a single bond. Preferably X and Y are each independently -COO-, -OCO- or a single bond. It is especially preferred that at least one of X and Y, preferably both, are -COO-.

In formulae [I] or [II], R* is an optically active group of 4-20 carbon atoms having at least one asymmetric (or chiral) carbon atom. In this case, the hydrogen atoms attached to the carbon atoms constituting this optically active group may be substituted with a halogen atom such as F, Cl, Br or I.

It is particularly preferred that R* is a group of formula [V]

$$- Q^1 - C^*H(Q^2) - Q^3 \qquad [V]$$

wherein

$Q^1$ is -$(CH_2)$q in which q is an integer of 0-6, and
$Q^2$ and $Q^3$ are different from each other, and are each independently an alkyl of 1-10 carbon atoms, a fluoroalkyl of 1-10 carbon atoms or a halogen atom. Examples of the alkyl of 1-10 carbon atoms include methyl, ethyl, propyl, butyl, pentyl, hexyl, heptyl, octyl, nonyl and decyl. Examples of the fluoroalkyl include those in which at least a part of the hydrogen atoms attached to the carbon atoms of the above-mentioned alkyl moieties is substituted with a fluorine atom. Examples of the halogen atom include F, Cl, Br or I. The groups or atoms $Q^2$ and $Q^3$ should always be different from each other, namely they should not be identical with each other. When one of $Q^2$ and $Q^3$ is a halogen atom, the other is usually alkyl or fluoroalkyl.

When $Q^1$, $Q^2$ and $Q^3$ in formula [V] have a $CH_2$ group (-$CH_2$- structure) or a $CF_2$ group (-$CF_2$- structure) in their structure, at least a part of these groups may be replaced by at least one group selected from -O-, -S-, -CO-, -CHX- (in which X is a halogen atom), -CHCN-, -O-CO-, -O-COO-, -CO-O- and -CH=CH-. In this case, however, these replacing groups are introduced into the above-mentioned structures so that the hetero atoms (eg N or O) constituting said groups are not directly bonded together. Accordingly, the replacement by these groups does not formed afresh a bond such as -O-O- or -N-O-.

In formula [I] or [II], R* is preferably -$C^*H(CF_3)$-$C_6H_{13}$, -$C^*H(CH_3)$-$C_6H_{13}$, -$C^*H(CH_3)$-$C_5H_{11}$, -$C^*H(C_2H_5)$-$C_5H_{11}$, -$C^*H(C_2H_5)$-$C_6H_{13}$, -$CH_2$-$C^*H(CH_3)$-$C_2H_5$, -$(CH_2)_3$-$C^*H(CH_3)$-$C_2H_5$ or -$C^*H(CF_3)$-$CH_2$-COO-$C_2H_5$. Thus, R* is an optically active group having at least one asymmetric carbon. As shown in the specified groups, the hydrogen atoms attached to the carbon atoms constituting the optically active group may be substituted with a halogen atom such as a fluorine atom.

Preferred groups of formula [V] are either of the following groups taking into account the characteristics of the carboxylate compound used as a liquid crystal material:

-$C^*H(CF_3)$-$C_6H_{13}$

-$C^*H(CH_3)$-$C_6H_{13}$ .

In formula [I] or [II], m is 1 or 2, and n is an integer of 0-2. Particularly, when the carboxylate compounds of formula [I] or [II] are used as a liquid crystal material, n is preferably 0 or 1.

The 5,6,7,8-tetrahydronaphthyl group constituting the main skeleton of formula [I] or [II] includes 5,6,7,8-tetrahydro-1,5-naphthyl, 5,6,7,8-tetrahydro-1,6-naphthyl, 5,6,7,8-tetrahydro-2,6-naphthyl and 5,6,7,8-tetrahydro-1,7-naphthyl.

For the use of the carboxylate compounds of the invention represented by formula [I] or [II] as liquid crystal materials, it is preferable that the molecule is linear as a whole. On that account, 5,6,7,8-tetrahydro-2,6-naphthyl is particularly preferred.

Examples of the carboxylate compounds represented by formula [I] are shown in the following Tables 1-1 to 1-7:

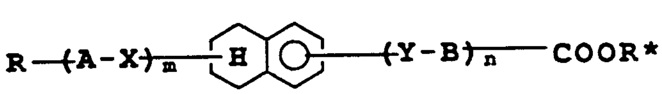 ... [I]

Table 1-1

| Compound Number | R | A | X | m | Y | B | n | R* | Exam. No. |
|---|---|---|---|---|---|---|---|---|---|
| 1 | $C_7H_{15}O-$ | 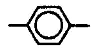 | -COO- | 1 | -COO- | 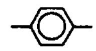 | 1 | $-C^*H(CF_3)-C_6H_{13}$ | |
| 2 | $C_8H_{17}O-$ | ditto | ditto | 1 | ditto | ditto | 1 | ditto | |
| 3 | $C_9H_{19}O-$ | ditto | ditto | 1 | ditto | ditto | 1 | ditto | |
| 4 | $C_{10}H_{21}O-$ | ditto | ditto | 1 | ditto | ditto | 1 | ditto | 1 |
| 5 | $C_{11}H_{23}O-$ | ditto | ditto | 1 | ditto | ditto | 1 | ditto | |
| 6 | $C_{12}H_{23}O-$ | ditto | ditto | 1 | ditto | ditto | 1 | ditto | |
| 7 | $C_{14}H_{29}O-$ | ditto | ditto | 1 | ditto | ditto | 1 | ditto | |
| 8 | $C_{16}H_{33}-$ | ditto | ditto | 1 | ditto | ditto | 1 | ditto | |
| 9 | $C_7H_{15}-$ | 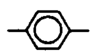 | -COO- | 1 | -COO- | 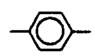 | 1 | $-C^*H(CF_3)-C_6H_{13}$ | |
| 10 | $C_8H_{17}-$ | ditto | ditto | 1 | ditto | ditto | 1 | ditto | |
| 11 | $C_9H_{19}-$ | ditto | ditto | 1 | ditto | ditto | 1 | ditto | |
| 12 | $C_{10}H_{21}-$ | ditto | ditto | 1 | ditto | ditto | 1 | ditto | |
| 13 | $C_{11}H_{23}-$ | ditto | ditto | 1 | ditto | ditto | 1 | ditto | |
| 14 | $C_{12}H_{23}-$ | ditto | ditto | 1 | ditto | ditto | 1 | ditto | |
| 15 | $C_{14}H_{29}-$ | ditto | ditto | 1 | ditto | ditto | 1 | ditto | |
| 16 | $C_{16}H_{33}-$ | ditto | ditto | 1 | ditto | ditto | 1 | ditto | |

Note) In the compounds listed in the table, a tetrahydronaphthyl group is 2,6-linked. The same shall apply hereinafter.

Table 1-2

| Compound Number | R | A | X | m | Y | B | n | R* | Exam No. |
|---|---|---|---|---|---|---|---|---|---|
| 17 | $C_{10}H_{21}O-$ | 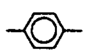 | -COO- | 1 | -COO- | 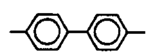 | 1 | $-C^{*}H(CF_3)-C_6H_{13}$ | |
| 18 | ditto | ditto | ditto | 1 | ditto | 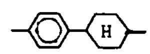 | 1 | ditto | |
| 19 | ditto | ditto | ditto | 1 | ditto | 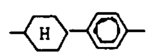 | 1 | ditto | |
| 20 | ditto | ditto | ditto | 1 | ditto | 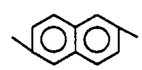 | 1 | ditto | |
| 21 | ditto | ditto | ditto | 1 | ditto | 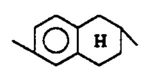 | 1 | ditto | 2 |
| 22 | ditto | ditto | ditto | 1 | ditto | 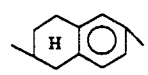 | 1 | ditto | 3 |
| 23 | ditto | ditto | ditto | 1 | ditto | 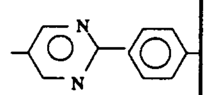 | 1 | ditto | |
| 24 | $C_{10}H_{21}O-$ | 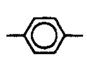 | -COO- | 1 | -COO- | 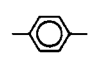 | 1 | $-C^{*}H(CH_3)-C_6H_{13}$ | |
| 25 | ditto | ditto | ditto | 1 | ditto | ditto | 1 | $-C^{*}H(CH_3)-C_5H_{11}$ | |
| 26 | ditto | ditto | ditto | 1 | ditto | ditto | 1 | $-C^{*}H(C_2H_5)-C_5H_{11}$ | |
| 27 | ditto | ditto | ditto | 1 | ditto | ditto | 1 | $-C^{*}H(C_2H_5)-C_6H_{13}$ | |
| 28 | ditto | ditto | ditto | 1 | ditto | ditto | 1 | $-CH_2-C^{*}H(CH_3)-C_2H_5$ | |
| 29 | ditto | ditto | ditto | 1 | ditto | ditto | 1 | $-(CH_2)_3-C^{*}H(CH_3)-C_2H_5$ | |
| 30 | ditto | ditto | ditto | 1 | ditto | ditto | 1 | $-C^{*}H(CF_3)-CH_2-COO-C_2H_5$ | |

Table 1-3

| Compound Number | R | A | X | m | Y | B | n | R* | Exam NO. |
|---|---|---|---|---|---|---|---|---|---|
| 31 | $C_7H_{15}O$- | 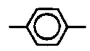 | -COO- | 1 | - | - | 0 | -C*H(CF$_3$)-$C_6H_{13}$ | |
| 32 | $C_8H_{17}O$- | ditto | ditto | 1 | - | - | 0 | ditto | |
| 33 | $C_9H_{19}O$- | ditto | ditto | 1 | - | - | 0 | ditto | |
| 34 | $C_{10}H_{21}O$- | ditto | ditto | 1 | - | - | 0 | ditto | |
| 35 | $C_{11}H_{23}O$- | ditto | ditto | 1 | - | - | 0 | ditto | |
| 36 | $C_{12}H_{23}O$- | ditto | ditto | 1 | - | - | 0 | ditto | |
| 37 | $C_{14}H_{29}O$- | ditto | ditto | 1 | - | - | 0 | ditto | |
| 38 | $C_{16}H_{33}O$- | ditto | ditto | 1 | - | - | 0 | ditto | |
| 39 | $C_7H_{15}O$- | 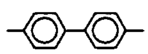 | -COO- | 1 | -COO- | 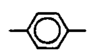 | 1 | -C*H(CF$_3$)-$C_6H_{13}$ | |
| 40 | $C_8H_{17}O$- | ditto | ditto | 1 | ditto | ditto | 1 | ditto | |
| 41 | $C_9H_{19}O$- | ditto | ditto | 1 | ditto | ditto | 1 | ditto | |
| 42 | $C_{10}H_{21}O$- | ditto | ditto | 1 | ditto | ditto | 1 | ditto | |
| 43 | $C_{11}H_{23}O$- | ditto | ditto | 1 | ditto | ditto | 1 | ditto | |
| 44 | $C_{12}H_{23}O$- | ditto | ditto | 1 | ditto | ditto | 1 | ditto | |
| 45 | $C_{14}H_{29}O$- | ditto | ditto | 1 | ditto | ditto | 1 | ditto | |
| 46 | $C_{16}H_{33}O$- | ditto | ditto | 1 | ditto | ditto | 1 | ditto | |
| 47 | $C_7H_{15}O$- | 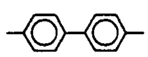 | -COO- | 1 | - | - | 0 | -C*H(CF$_3$)-$C_6H_{13}$ | |
| 48 | $C_8H_{17}O$- | ditto | ditto | 1 | - | - | 0 | ditto | |
| 49 | $C_9H_{19}O$- | ditto | ditto | 1 | - | - | 0 | ditto | |
| 50 | $C_{10}H_{21}O$- | ditto | ditto | 1 | - | - | 0 | ditto | |
| 51 | $C_{11}H_{23}O$- | ditto | ditto | 1 | - | - | 0 | ditto | |
| 52 | $C_{12}H_{23}O$- | ditto | ditto | 1 | - | - | 0 | ditto | |
| 53 | $C_{14}H_{29}O$- | ditto | ditto | 1 | - | - | 0 | ditto | |
| 54 | $C_{16}H_{33}O$- | ditto | ditto | 1 | - | - | 0 | ditto | |

Table 1-4

| Compound Number | R | A | X | m | Y | B | n | R* | Exam NO. |
|---|---|---|---|---|---|---|---|---|---|
| 55 | $C_7H_{15}O$- | 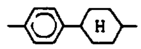 | -COO- | 1 | -COO- | 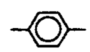 | 1 | $-C^*H(CF_3)-C_6H_{13}$ | |
| 56 | $C_8H_{17}O$- | ditto | ditto | 1 | ditto | ditto | 1 | ditto | |
| 57 | $C_9H_{19}O$- | ditto | ditto | 1 | ditto | ditto | 1 | ditto | |
| 58 | $C_{10}H_{21}O$- | ditto | ditto | 1 | ditto | ditto | 1 | ditto | |
| 59 | $C_{11}H_{23}O$- | ditto | ditto | 1 | ditto | ditto | 1 | ditto | |
| 60 | $C_{12}H_{23}O$- | ditto | ditto | 1 | ditto | ditto | 1 | ditto | |
| 61 | $C_{14}H_{29}O$- | ditto | ditto | 1 | ditto | ditto | 1 | ditto | |
| 62 | $C_{16}H_{33}O$- | ditto | ditto | 1 | ditto | ditto | 1 | ditto | |
| 63 | $C_7H_{15}O$- | 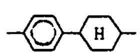 | -COO- | 1 | - | - | 0 | $-C^*H(CF_3)-C_6H_{13}$ | |
| 64 | $C_8H_{17}O$- | ditto | ditto | 1 | - | - | 0 | ditto | |
| 65 | $C_9H_{19}O$- | ditto | ditto | 1 | - | - | 0 | ditto | |
| 66 | $C_{10}H_{21}O$- | ditto | ditto | 1 | - | - | 0 | ditto | |
| 67 | $C_{11}H_{23}O$- | ditto | ditto | 1 | - | - | 0 | ditto | |
| 68 | $C_{12}H_{23}O$- | ditto | ditto | 1 | - | - | 0 | ditto | |
| 69 | $C_{14}H_{29}O$- | ditto | ditto | 1 | - | - | 0 | ditto | |
| 70 | $C_{16}H_{33}O$- | ditto | ditto | 1 | - | - | 0 | ditto | |
| 71 | $C_7H_{15}O$- | 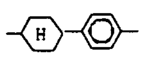 | -COO- | 1 | -COO- | 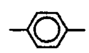 | 1 | $-C^*H(CF_3)-C_6H_{13}$ | |
| 72 | $C_8H_{17}O$- | ditto | ditto | 1 | ditto | ditto | 1 | ditto | |
| 73 | $C_9H_{19}O$- | ditto | ditto | 1 | ditto | ditto | 1 | ditto | |
| 74 | $C_{10}H_{21}O$- | ditto | ditto | 1 | ditto | ditto | 1 | ditto | |
| 75 | $C_{11}H_{23}O$- | ditto | ditto | 1 | ditto | ditto | 1 | ditto | |
| 76 | $C_{12}H_{23}O$- | ditto | ditto | 1 | ditto | ditto | 1 | ditto | |
| 77 | $C_{14}H_{29}O$- | ditto | ditto | 1 | ditto | ditto | 1 | ditto | |
| 78 | $C_{16}H_{33}O$- | ditto | ditto | 1 | ditto | ditto | 1 | ditto | |

Table 1-5

| Compound Number | R | A | X | m | Y | B | n | R* | Exam NO. |
|---|---|---|---|---|---|---|---|---|---|
| 79 | $C_7H_{15}O$- | 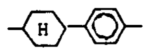 | -COO- | 1 | - | - | 0 | -C*H(CF$_3$)-C$_6$H$_{13}$ | |
| 80 | $C_8H_{17}O$- | ditto | ditto | 1 | - | - | 0 | ditto | |
| 81 | $C_9H_{19}O$- | ditto | ditto | 1 | - | - | 0 | ditto | |
| 82 | $C_{10}H_{21}O$- | ditto | ditto | 1 | - | - | 0 | ditto | |
| 83 | $C_{11}H_{23}O$- | ditto | ditto | 1 | - | - | 0 | ditto | |
| 84 | $C_{12}H_{23}O$- | ditto | ditto | 1 | - | - | 0 | ditto | |
| 85 | $C_{14}H_{29}O$- | ditto | ditto | 1 | - | - | 0 | ditto | |
| 86 | $C_{16}H_{33}O$- | ditto | ditto | 1 | - | - | 0 | ditto | |
| 87 | $C_7H_{15}O$- | 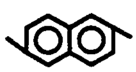 | -COO- | 1 | -COO- | 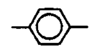 | 1 | -C*H(CF$_3$)-C$_6$H$_{13}$ | |
| 88 | $C_8H_{17}O$- | ditto | ditto | 1 | ditto | ditto | 1 | ditto | |
| 89 | $C_9H_{19}O$- | ditto | ditto | 1 | ditto | ditto | 1 | ditto | |
| 90 | $C_{10}H_{21}O$- | ditto | ditto | 1 | ditto | ditto | 1 | ditto | |
| 91 | $C_{11}H_{23}O$- | ditto | ditto | 1 | ditto | ditto | 1 | ditto | |
| 92 | $C_{12}H_{23}O$- | ditto | ditto | 1 | ditto | ditto | 1 | ditto | |
| 93 | $C_{14}H_{29}O$- | ditto | ditto | 1 | ditto | ditto | 1 | ditto | |
| 94 | $C_{16}H_{33}O$- | ditto | ditto | 1 | ditto | ditto | 1 | ditto | |
| 95 | $C_7H_{15}O$- | 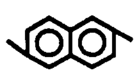 | -COO- | 1 | - | - | 0 | -C*H(CF$_3$)-C$_6$H$_{13}$ | |
| 96 | $C_8H_{17}O$- | ditto | ditto | 1 | - | - | 0 | ditto | |
| 97 | $C_9H_{19}O$- | ditto | ditto | 1 | - | - | 0 | ditto | |
| 98 | $C_{10}H_{21}O$- | ditto | ditto | 1 | - | - | 0 | ditto | |
| 99 | $C_{11}H_{23}O$- | ditto | ditto | 1 | - | - | 0 | ditto | |
| 100 | $C_{12}H_{23}O$- | ditto | ditto | 1 | - | - | 0 | ditto | |
| 101 | $C_{14}H_{29}O$- | ditto | ditto | 1 | - | - | 0 | ditto | |
| 102 | $C_{16}H_{33}O$- | ditto | ditto | 1 | - | - | 0 | ditto | |

Table 1-6

| Compound Number | R | A | X | m | Y | B | n | R* | Exam NO. |
|---|---|---|---|---|---|---|---|---|---|
| 103 | $C_7H_{15}O-$ | 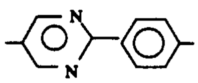 | -COO- | 1 | -COO- | 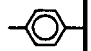 | 1 | $-C^*H(CF_3)-C_6H_{13}$ | |
| 104 | $C_8H_{17}O-$ | ditto | ditto | 1 | ditto | ditto | 1 | ditto | |
| 105 | $C_9H_{19}O-$ | ditto | ditto | 1 | ditto | ditto | 1 | ditto | |
| 106 | $C_{10}H_{21}O-$ | ditto | ditto | 1 | ditto | ditto | 1 | ditto | |
| 107 | $C_{11}H_{23}O-$ | ditto | ditto | 1 | ditto | ditto | 1 | ditto | |
| 108 | $C_{12}H_{23}O-$ | ditto | ditto | 1 | ditto | ditto | 1 | ditto | |
| 109 | $C_{14}H_{29}O-$ | ditto | ditto | 1 | ditto | ditto | 1 | ditto | |
| 110 | $C_{16}H_{33}O-$ | ditto | ditto | 1 | ditto | ditto | 1 | ditto | |
| 111 | $C_7H_{15}O-$ | 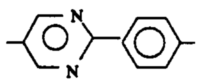 | -COO- | 1 | - | - | 0 | $-C^*H(CF_3)-C_6H_{13}$ | |
| 112 | $C_8H_{17}O-$ | ditto | ditto | 1 | - | - | 0 | ditto | |
| 113 | $C_9H_{19}O-$ | ditto | ditto | 1 | - | - | 0 | ditto | |
| 114 | $C_{10}H_{21}O-$ | ditto | ditto | 1 | - | - | 0 | ditto | |
| 115 | $C_{11}H_{23}O-$ | ditto | ditto | 1 | - | - | 0 | ditto | |
| 116 | $C_{12}H_{23}O-$ | ditto | ditto | 1 | - | - | 0 | ditto | |
| 117 | $C_{14}H_{29}O-$ | ditto | ditto | 1 | - | - | 0 | ditto | |
| 118 | $C_{16}H_{33}O-$ | ditto | ditto | 1 | - | - | 0 | ditto | |
| 119 | $C_7H_{15}-$ | 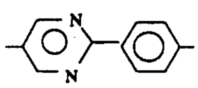 | -COO- | 1 | -COO- | 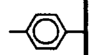 | 1 | $-C^*H(CF_3)-C_6H_{13}$ | |
| 120 | $C_8H_{17}-$ | ditto | ditto | 1 | ditto | ditto | 1 | ditto | |
| 121 | $C_9H_{19}-$ | ditto | ditto | 1 | ditto | ditto | 1 | ditto | |
| 122 | $C_{10}H_{21}-$ | ditto | ditto | 1 | ditto | ditto | 1 | ditto | |
| 123 | $C_{11}H_{23}-$ | ditto | ditto | 1 | ditto | ditto | 1 | ditto | |
| 124 | $C_{12}H_{23}-$ | ditto | ditto | 1 | ditto | ditto | 1 | ditto | |
| 125 | $C_{14}H_{29}-$ | ditto | ditto | 1 | ditto | ditto | 1 | ditto | |
| 126 | $C_{16}H_{33}-$ | ditto | ditto | 1 | ditto | ditto | 1 | ditto | |

Table 1-7

| Compound Number | R | A | X | m | Y | B | n | R* | Exam NO. |
|---|---|---|---|---|---|---|---|---|---|
| 127 | $C_7H_{15}$- | 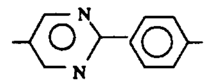 | -COO- | 1 | - | - | 0 | -C*H($CF_3$)-$C_6H_{13}$ | |
| 128 | $C_8H_{17}$- | ditto | ditto | 1 | - | - | 0 | ditto | |
| 129 | $C_9H_{19}$- | ditto | ditto | 1 | - | - | 0 | ditto | |
| 130 | $C_{10}H_{21}$- | ditto | ditto | 1 | - | - | 0 | ditto | 4 |
| 131 | $C_{11}H_{23}$- | ditto | ditto | 1 | - | - | 0 | ditto | |
| 132 | $C_{12}H_{23}$- | ditto | ditto | 1 | - | - | 0 | ditto | |
| 133 | $C_{14}H_{29}$- | ditto | ditto | 1 | - | - | 0 | ditto | |
| 134 | $C_{16}H_{33}$- | ditto | ditto | 1 | - | - | 0 | ditto | |
| 135 | $C_7H_{15}O$- | 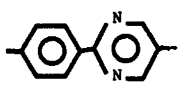 | -COO- | 1 | -COO- | 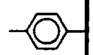 | 1 | -C*H($CF_3$)-$C_6H_{13}$ | |
| 136 | $C_8H_{17}O$- | ditto | ditto | 1 | ditto | ditto | 1 | ditto | |
| 137 | $C_9H_{19}O$- | ditto | ditto | 1 | ditto | ditto | 1 | ditto | |
| 138 | $C_{10}H_{21}O$- | ditto | ditto | 1 | ditto | ditto | 1 | ditto | |
| 139 | $C_{11}H_{23}O$- | ditto | ditto | 1 | ditto | ditto | 1 | ditto | |
| 140 | $C_{12}H_{23}O$- | ditto | ditto | 1 | ditto | ditto | 1 | ditto | |
| 141 | $C_{14}H_{29}O$- | ditto | ditto | 1 | ditto | ditto | 1 | ditto | |
| 142 | $C_{16}H_{33}O$- | ditto | ditto | 1 | ditto | ditto | 1 | ditto | |
| 143 | $C_7H_{15}O$- | 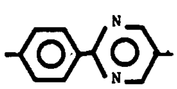 | -COO- | 1 | - | - | 0 | -C*H($CF_3$)-$C_6H_{13}$ | |
| 144 | $C_8H_{17}O$- | ditto | ditto | 1 | - | - | 0 | ditto | |
| 145 | $C_9H_{19}O$- | ditto | ditto | 1 | - | - | 0 | ditto | |
| 146 | $C_{10}H_{21}O$- | ditto | ditto | 1 | - | - | 0 | ditto | |
| 147 | $C_{11}H_{23}O$- | ditto | ditto | 1 | - | - | 0 | ditto | |
| 148 | $C_{12}H_{23}O$- | ditto | ditto | 1 | - | - | 0 | ditto | |
| 149 | $C_{14}H_{29}O$- | ditto | ditto | 1 | - | - | 0 | ditto | |
| 150 | $C_{16}H_{33}O$- | ditto | ditto | 1 | - | - | 0 | ditto | |

Examples of the carboxylate compounds represented by the formula [II] are shown in the following Tables 2-1 to 2-7.

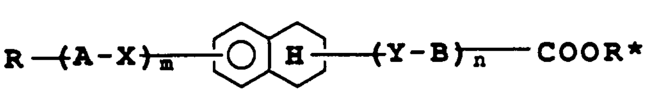 ... [II]

Table 2-1

| Compound Number | R | A | X | m | Y | B | n | R* | Exam. No. |
|---|---|---|---|---|---|---|---|---|---|
| 201 | $C_7H_{15}O-$ | 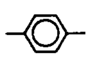 | -COO- | 1 | -COO- | 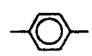 | 1 | $-C^*H(CF_3)-C_6H_{13}$ | |
| 202 | $C_8H_{17}O-$ | ditto | ditto | 1 | ditto | ditto | 1 | ditto | |
| 203 | $C_9H_{19}O-$ | ditto | ditto | 1 | ditto | ditto | 1 | ditto | |
| 204 | $C_{10}H_{21}O-$ | ditto | ditto | 1 | ditto | ditto | 1 | ditto | 8 |
| 205 | $C_{11}H_{23}O-$ | ditto | ditto | 1 | ditto | ditto | 1 | ditto | |
| 206 | $C_{12}H_{23}O-$ | ditto | ditto | 1 | ditto | ditto | 1 | ditto | |
| 207 | $C_{14}H_{29}O-$ | ditto | ditto | 1 | ditto | ditto | 1 | ditto | |
| 208 | $C_{16}H_{33}O-$ | ditto | ditto | 1 | ditto | ditto | 1 | ditto | |
| 209 | $C_7H_{15}-$ | 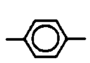 | -COO- | 1 | -COO- | 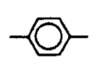 | 1 | $-C^*H(CF_3)-C_6H_{13}$ | |
| 210 | $C_8H_{17}-$ | ditto | ditto | 1 | ditto | ditto | 1 | ditto | |
| 211 | $C_9H_{19}-$ | ditto | ditto | 1 | ditto | ditto | 1 | ditto | |
| 212 | $C_{10}H_{21}-$ | ditto | ditto | 1 | ditto | ditto | 1 | ditto | |
| 213 | $C_{11}H_{23}-$ | ditto | ditto | 1 | ditto | ditto | 1 | ditto | |
| 214 | $C_{12}H_{23}-$ | ditto | ditto | 1 | ditto | ditto | 1 | ditto | |
| 215 | $C_{14}H_{29}-$ | ditto | ditto | 1 | ditto | ditto | 1 | ditto | |
| 216 | $C_{16}H_{33}-$ | ditto | ditto | 1 | ditto | ditto | 1 | ditto | |

Note) In the compounds listed in the table, a tetrahydronaphthyl group is 2,6-linked. The same shall apply hereinafter.

Table 2-2

| Compoud Number | R | A | X | m | Y | B | n | R* | Ex. No. |
|---|---|---|---|---|---|---|---|---|---|
| 217 | $C_{10}H_{21}O-$ | 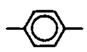 | -COO- | 1 | -COO- | 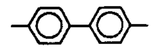 | 1 | $-C^{*}H(CF_3)-C_6H_{13}$ | |
| 218 | ditto | ditto | ditto | 1 | ditto | 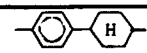 | 1 | ditto | |
| 219 | ditto | ditto | ditto | 1 | ditto | 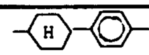 | 1 | ditto | |
| 220 | ditto | ditto | ditto | 1 | ditto | 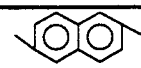 | 1 | ditto | |
| 221 | ditto | ditto | ditto | 1 | ditto | 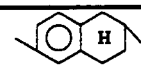 | 1 | ditto | 9 |
| 222 | ditto | ditto | ditto | 1 | ditto | 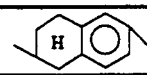 | 1 | ditto | 10 |
| 223 | ditto | ditto | ditto | 1 | ditto | 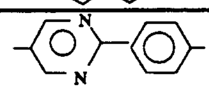 | 1 | ditto | |
| 224 | ditto | ditto | ditto | 1 | ditto | 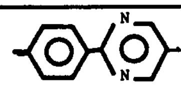 | 1 | ditto | |
| 225 | $C_{10}H_{21}O-$ | 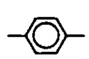 | -COO- | 1 | -COO- | 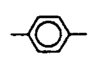 | 1 | $-C^{*}H(CH_3)-C_6H_{13}$ | |
| 226 | ditto | ditto | ditto | 1 | ditto | ditto | 1 | $-C^{*}H(CH_3)-C_5H_{11}$ | |
| 227 | ditto | ditto | ditto | 1 | ditto | ditto | 1 | $-C^{*}H(C_2H_5)-C_5H_{11}$ | |
| 228 | ditto | ditto | ditto | 1 | ditto | ditto | 1 | $-C^{*}H(C_2H_5)-C_6H_{13}$ | |
| 229 | ditto | ditto | ditto | 1 | ditto | ditto | 1 | $-CH_2-C^{*}H(CH_3)-C_2H_5$ | |
| 230 | ditto | ditto | ditto | 1 | ditto | ditto | 1 | $-(CH_2)_3-C^{*}H(CH_3)-C_2H_5-$ | |
| 231 | ditto | ditto | ditto | 1 | ditto | ditto | 1 | $-C^{*}H(CF_3)-CH_2-COO-C_2H_5$ | |
| 232 | $C_7H_{15}O-$ | 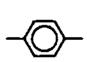 | -COO- | 1 | - | - | 0 | $-C^{*}H(CF_3)-C_6H_{13}$ | |
| 233 | $C_8H_{17}O-$ | ditto | ditto | 1 | - | - | 0 | ditto | |
| 234 | $C_9H_{19}O-$ | ditto | ditto | 1 | - | - | 0 | ditto | |
| 235 | $C_{10}H_{21}O-$ | ditto | ditto | 1 | - | - | 0 | ditto | 17 |
| 236 | $C_{11}H_{23}O-$ | ditto | ditto | 1 | - | - | 0 | ditto | |
| 237 | $C_{12}H_{23}O-$ | ditto | ditto | 1 | - | - | 0 | ditto | |
| 238 | $C_{14}H_{29}O-$ | ditto | ditto | 1 | - | - | 0 | ditto | |
| 239 | $C_{16}H_{33}O-$ | ditto | ditto | 1 | - | - | 0 | ditto | |

Table 2-3

| Compound Number | R | A | X | m | Y | B | n | R* | Exam. No. |
|---|---|---|---|---|---|---|---|---|---|
| 240 | $C_7H_{15}O-$ | 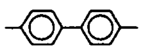 | -COO- | 1 | -COO- | 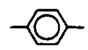 | 1 | $-C^*H(CF_3)-C_6H_{13}$ | |
| 241 | $C_8H_{17}O-$ | ditto | ditto | 1 | ditto | ditto | 1 | ditto | |
| 242 | $C_9H_{19}O-$ | ditto | ditto | 1 | ditto | ditto | 1 | ditto | |
| 243 | $C_{10}H_{21}O-$ | ditto | ditto | 1 | ditto | ditto | 1 | ditto | |
| 244 | $C_{11}H_{23}O-$ | ditto | ditto | 1 | ditto | ditto | 1 | ditto | |
| 245 | $C_{12}H_{23}O-$ | ditto | ditto | 1 | ditto | ditto | 1 | ditto | |
| 246 | $C_{14}H_{29}O-$ | ditto | ditto | 1 | ditto | ditto | 1 | ditto | |
| 247 | $C_{16}H_{33}O-$ | ditto | ditto | 1 | ditto | ditto | 1 | ditto | |
| 248 | $C_7H_{15}O-$ | 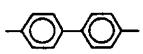 | -COO- | 1 | - | - | 0 | $-C^*H(CF_3)-C_6H_{13}$ | |
| 249 | $C_8H_{17}O-$ | ditto | ditto | 1 | - | - | 0 | ditto | |
| 250 | $C_9H_{19}O-$ | ditto | ditto | 1 | - | - | 0 | ditto | |
| 251 | $C_{10}H_{21}O-$ | ditto | ditto | 1 | - | - | 0 | ditto | 11 |
| 252 | $C_{11}H_{23}O-$ | ditto | ditto | 1 | - | - | 0 | ditto | |
| 253 | $C_{12}H_{23}O-$ | ditto | ditto | 1 | - | - | 0 | ditto | |
| 254 | $C_{14}H_{29}O-$ | ditto | ditto | 1 | - | - | 0 | ditto | |
| 255 | $C_{16}H_{33}O-$ | ditto | ditto | 1 | - | - | 0 | ditto | |
| 256 | $C_7H_{15}O-$ | 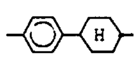 | -COO- | 1 | -COO- | 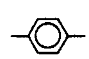 | 1 | $-C^*H(CF_3)-C_6H_{13}$ | |
| 257 | $C_8H_{17}O-$ | ditto | ditto | 1 | ditto | ditto | 1 | ditto | |
| 258 | $C_9H_{19}O-$ | ditto | ditto | 1 | ditto | ditto | 1 | ditto | |
| 259 | $C_{10}H_{21}O-$ | ditto | ditto | 1 | ditto | ditto | 1 | ditto | |
| 260 | $C_{11}H_{23}O-$ | ditto | ditto | 1 | ditto | ditto | 1 | ditto | |
| 261 | $C_{12}H_{23}O-$ | ditto | ditto | 1 | ditto | ditto | 1 | ditto | |
| 262 | $C_{14}H_{29}O-$ | ditto | ditto | 1 | ditto | ditto | 1 | ditto | |
| 263 | $C_{16}H_{33}O-$ | ditto | ditto | 1 | ditto | ditto | 1 | ditto | |

Table 2-4

| Compound Number | R | A | X | m | Y | B | n | R* | Exam. No. |
|---|---|---|---|---|---|---|---|---|---|
| 264 | $C_7H_{15}O$- | 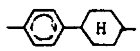 | -COO- | 1 | - | - | 0 | -C*H(CF$_3$)-C$_6$H$_{13}$ | 12 |
| 265 | $C_8H_{17}O$- | ditto | ditto | 1 | - | - | 0 | ditto | |
| 266 | $C_9H_{19}O$- | ditto | ditto | 1 | - | - | 0 | ditto | |
| 267 | $C_{10}H_{21}O$- | ditto | ditto | 1 | - | - | 0 | ditto | |
| 268 | $C_{11}H_{23}O$- | ditto | ditto | 1 | - | - | 0 | ditto | |
| 269 | $C_{12}H_{23}O$- | ditto | ditto | 1 | - | - | 0 | ditto | |
| 270 | $C_{14}H_{29}O$- | ditto | ditto | 1 | - | - | 0 | ditto | |
| 271 | $C_{16}H_{33}O$- | ditto | ditto | 1 | - | - | 0 | ditto | |
| 272 | $C_7H_{15}O$- | 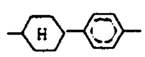 | -COO- | 1 | -COO- | 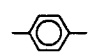 | 1 | -C*H(CF$_3$)-C$_6$H$_{13}$ | |
| 273 | $C_8H_{17}O$- | ditto | ditto | 1 | ditto | ditto | 1 | ditto | |
| 274 | $C_9H_{19}O$- | ditto | ditto | 1 | ditto | ditto | 1 | ditto | |
| 275 | $C_{10}H_{21}O$- | ditto | ditto | 1 | ditto | ditto | 1 | ditto | |
| 276 | $C_{11}H_{23}O$- | ditto | ditto | 1 | ditto | ditto | 1 | ditto | |
| 277 | $C_{12}H_{23}O$- | ditto | ditto | 1 | ditto | ditto | 1 | ditto | |
| 278 | $C_{14}H_{29}O$- | ditto | ditto | 1 | ditto | ditto | 1 | ditto | |
| 279 | $C_{16}H_{33}O$- | ditto | ditto | 1 | ditto | ditto | 1 | ditto | |
| 280 | $C_7H_{15}O$- | 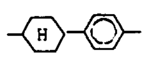 | -COO- | 1 | - | - | 0 | -C*H(CF$_3$)-C$_6$H$_{13}$ | |
| 281 | $C_8H_{17}O$- | ditto | ditto | 1 | - | - | 0 | ditto | |
| 282 | $C_9H_{19}O$- | ditto | ditto | 1 | - | - | 0 | ditto | |
| 283 | $C_{10}H_{21}O$- | ditto | ditto | 1 | - | - | 0 | ditto | |
| 284 | $C_{11}H_{23}O$- | ditto | ditto | 1 | - | - | 0 | ditto | |
| 285 | $C_{12}H_{23}O$- | ditto | ditto | 1 | - | - | 0 | ditto | |
| 286 | $C_{14}H_{29}O$- | ditto | ditto | 1 | - | - | 0 | ditto | |
| 287 | $C_{16}H_{33}O$- | ditto | ditto | 1 | - | - | 0 | ditto | |

Table 2-5

| Compound Number | R | A | X | m | Y | B | n | R* | Exam. No. |
|---|---|---|---|---|---|---|---|---|---|
| 288 | $C_7H_{15}O$- | 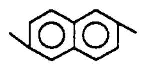 | -COO- | 1 | -COO- | 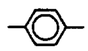 | 1 | -C*H(CF$_3$)-$C_6H_{13}$ | 13 |
| 289 | $C_8H_{17}O$- | ditto | ditto | 1 | ditto | ditto | 1 | ditto | |
| 290 | $C_9H_{19}O$- | ditto | ditto | 1 | ditto | ditto | 1 | ditto | |
| 291 | $C_{10}H_{21}O$- | ditto | ditto | 1 | ditto | ditto | 1 | ditto | |
| 292 | $C_{11}H_{23}O$- | ditto | ditto | 1 | ditto | ditto | 1 | ditto | |
| 293 | $C_{12}H_{23}O$- | ditto | ditto | 1 | ditto | ditto | 1 | ditto | |
| 294 | $C_{14}H_{29}O$- | ditto | ditto | 1 | ditto | ditto | 1 | ditto | |
| 295 | $C_{16}H_{33}O$- | ditto | ditto | 1 | ditto | ditto | 1 | ditto | |
| 296 | $C_7H_{15}O$- | 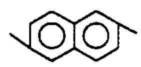 | -COO- | 1 | - | - | 0 | -C*H(CF$_3$)-$C_6H_{13}$ | 14 |
| 297 | $C_8H_{17}O$- | ditto | ditto | 1 | - | - | 0 | ditto | |
| 298 | $C_9H_{19}O$- | ditto | ditto | 1 | - | - | 0 | ditto | |
| 299 | $C_{10}H_{21}O$- | ditto | ditto | 1 | - | - | 0 | ditto | 15 |
| 300 | $C_{11}H_{23}O$- | ditto | ditto | 1 | - | - | 0 | ditto | |
| 301 | $C_{12}H_{23}O$- | ditto | ditto | 1 | - | - | 0 | ditto | |
| 302 | $C_{14}H_{29}O$- | ditto | ditto | 1 | - | - | 0 | ditto | |
| 303 | $C_{16}H_{33}O$- | ditto | ditto | 1 | - | - | 0 | ditto | |
| 304 | $C_7H_{15}O$- | 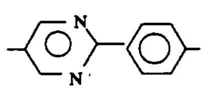 | -COO- | 1 | -COO- | 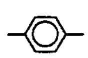 | 1 | -C*H(CF$_3$)-$C_6H_{13}$ | |
| 305 | $C_8H_{17}O$- | ditto | ditto | 1 | ditto | ditto | 1 | ditto | |
| 306 | $C_9H_{19}O$- | ditto | ditto | 1 | ditto | ditto | 1 | ditto | |
| 307 | $C_{10}H_{21}O$- | ditto | ditto | 1 | ditto | ditto | 1 | ditto | |
| 308 | $C_{11}H_{23}O$- | ditto | ditto | 1 | ditto | ditto | 1 | ditto | |
| 309 | $C_{12}H_{23}O$- | ditto | ditto | 1 | ditto | ditto | 1 | ditto | |
| 310 | $C_{14}H_{29}O$- | ditto | ditto | 1 | ditto | ditto | 1 | ditto | |
| 311 | $C_{16}H_{33}O$- | ditto | ditto | 1 | ditto | ditto | 1 | ditto | |

Table 2-6

| Compound Number | R | A | X | m | Y | B | n | R* | Exam. No. |
|---|---|---|---|---|---|---|---|---|---|
| 312 | $C_7H_{15}O$- | 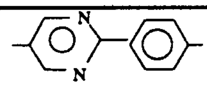 | -COO- | 1 | - | - | 0 | -C*H(CF$_3$)-$C_6H_{13}$ | |
| 313 | $C_8H_{17}O$- | ditto | ditto | 1 | - | - | 0 | ditto | |
| 314 | $C_9H_{19}O$- | ditto | ditto | 1 | - | - | 0 | ditto | |
| 315 | $C_{10}H_{21}O$- | ditto | ditto | 1 | - | - | 0 | ditto | |
| 316 | $C_{11}H_{23}O$- | ditto | ditto | 1 | - | - | 0 | ditto | |
| 317 | $C_{12}H_{23}O$- | ditto | ditto | 1 | - | - | 0 | ditto | |
| 318 | $C_{14}H_{29}O$- | ditto | ditto | 1 | - | - | 0 | ditto | |
| 319 | $C_{16}H_{33}O$- | ditto | ditto | 1 | - | - | 0 | ditto | |
| 320 | $C_7H_{15}$- | 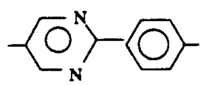 | -COO- | 1 | -COO- | 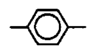 | 1 | -C*H(CF$_3$)-$C_6H_{13}$ | |
| 321 | $C_8H_{17}$- | ditto | ditto | 1 | ditto | ditto | 1 | ditto | |
| 322 | $C_9H_{19}$- | ditto | ditto | 1 | ditto | ditto | 1 | ditto | |
| 323 | $C_{10}H_{21}$- | ditto | ditto | 1 | ditto | ditto | 1 | ditto | |
| 324 | $C_{11}H_{23}$- | ditto | ditto | 1 | ditto | ditto | 1 | ditto | |
| 325 | $C_{12}H_{23}$- | ditto | ditto | 1 | ditto | ditto | 1 | ditto | |
| 326 | $C_{14}H_{29}$- | ditto | ditto | 1 | ditto | ditto | 1 | ditto | |
| 327 | $C_{16}H_{33}$- | ditto | ditto | 1 | ditto | ditto | 1 | ditto | |
| 328 | $C_7H_{15}$- | 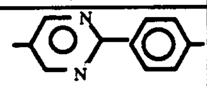 | -COO- | 1 | - | - | 0 | -C*H(CF$_3$)-$C_6H_{13}$ | |
| 329 | $C_8H_{17}$- | ditto | ditto | 1 | - | - | 0 | ditto | |
| 330 | $C_9H_{19}$- | ditto | ditto | 1 | - | - | 0 | ditto | |
| 331 | $C_{10}H_{21}$- | ditto | ditto | 1 | - | - | 0 | ditto | 16 |
| 332 | $C_{11}H_{23}$- | ditto | ditto | 1 | - | - | 0 | ditto | |
| 333 | $C_{12}H_{23}$- | ditto | ditto | 1 | - | - | 0 | ditto | |
| 334 | $C_{14}H_{29}$- | ditto | ditto | 1 | - | - | 0 | ditto | |
| 335 | $C_{16}H_{33}$- | ditto | ditto | 1 | - | - | 0 | ditto | |

Table 2-7

| Compound Number | R | A | X | m | Y | B | n | R* | Exam. No. |
|---|---|---|---|---|---|---|---|---|---|
| 336 | $C_7H_{15}O-$ | 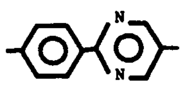 | -COO- | 1 | -COO- | 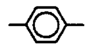 | 1 | $-C^*H(CF_3)-C_6H_{13}$ | |
| 337 | $C_8H_{17}O-$ | ditto | ditto | 1 | ditto | ditto | 1 | ditto | |
| 338 | $C_9H_{19}O-$ | ditto | ditto | 1 | ditto | ditto | 1 | ditto | |
| 339 | $C_{10}H_{21}O-$ | ditto | ditto | 1 | ditto | ditto | 1 | ditto | |
| 340 | $C_{11}H_{23}O-$ | ditto | ditto | 1 | ditto | ditto | 1 | ditto | |
| 341 | $C_{12}H_{23}O-$ | ditto | ditto | 1 | ditto | ditto | 1 | ditto | |
| 342 | $C_{14}H_{29}O-$ | ditto | ditto | 1 | ditto | ditto | 1 | ditto | |
| 343 | $C_{16}H_{33}O-$ | ditto | ditto | 1 | ditto | ditto | 1 | ditto | |
| 344 | $C_7H_{15}O-$ | 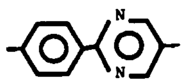 | -COO- | 1 | - | - | 0 | $-C^*H(CF_3)-C_6H_{13}$ | |
| 345 | $C_8H_{17}O-$ | ditto | ditto | 1 | - | - | 0 | ditto | |
| 346 | $C_9H_{19}O-$ | ditto | ditto | 1 | - | - | 0 | ditto | |
| 347 | $C_{10}H_{21}O-$ | ditto | ditto | 1 | - | - | 0 | ditto | |
| 348 | $C_{11}H_{23}O-$ | ditto | ditto | 1 | - | - | 0 | ditto | |
| 349 | $C_{12}H_{23}O-$ | ditto | ditto | 1 | - | - | 0 | ditto | |
| 350 | $C_{14}H_{29}O-$ | ditto | ditto | 1 | - | - | 0 | ditto | |
| 351 | $C_{16}H_{33}O-$ | ditto | ditto | 1 | - | - | 0 | ditto | |

The carboxylate compounds exemplified above may be prepared by means of a combination of known synthesis techniques.

For instance, the carboxylate compound of the above formula [I] may be synthesized in accordance with the following synthesis route.

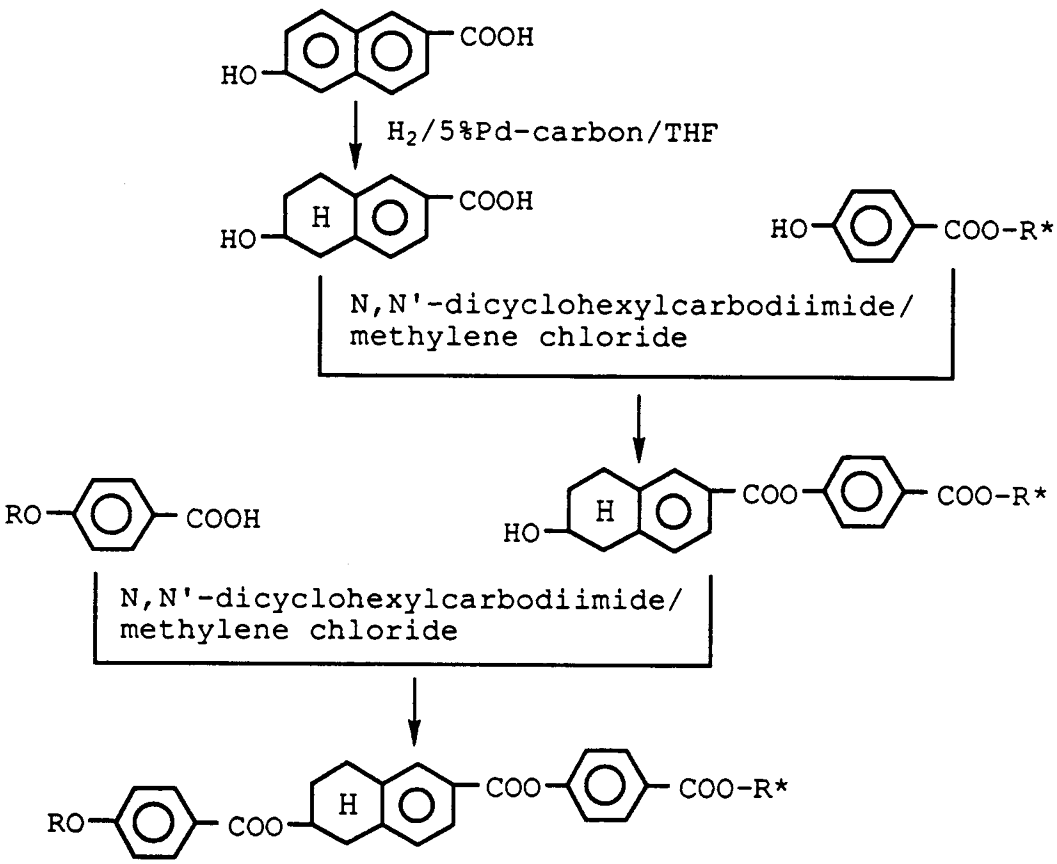


For example, 6-hydroxynaphthalene-2-carboxylic acid in a solvent such as tetrahydrofuran is reduced with hydrogen gas in the presence of a reducing catalyst such as a palladium/carbon under elevated pressure to obtain 5,6,7,8-tetrahydro-6-hydroxynaphthalene-2-carboxylic acid.

Subsequently, an ester compound obtained from hydroxybenzoic acid and an alcohol having an asymmetric carbon is reacted with the 5,6,7,8-tetrahydro-6-hydroxynaphthalene-carboxylic acid obtained by the above step in the presence of 4-N,N-dimethylaminopyridine and a solvent such as methylene chloride while adding dropwise a solution of N,N'-dicyclohexylcarbodiimide, whereby 4-(5',6',7',8'-tetrahydro-6'-hydroxy-2'-naphthoyloxy)benzoate is obtained.

Furthermore, 4-alkoxybenzoic acid prepared separately in a conventional way is reacted with the 4-(5',6',7',8'-tetrahydro-6'-hydroxy-2'-naphthoyloxy)benzoate obtained by the above step in the presence of 4-N,N-dimethylaminopyridine and a solvent such as methylene chloride while adding dropwise a solution of N,N'-dicyclohexylcarbodiimide, whereby the carboxylate compound represented by formula [I] is obtained.

The carboxylate compounds represented by formula [II] may be synthesized in accordance with the following synthesis route:

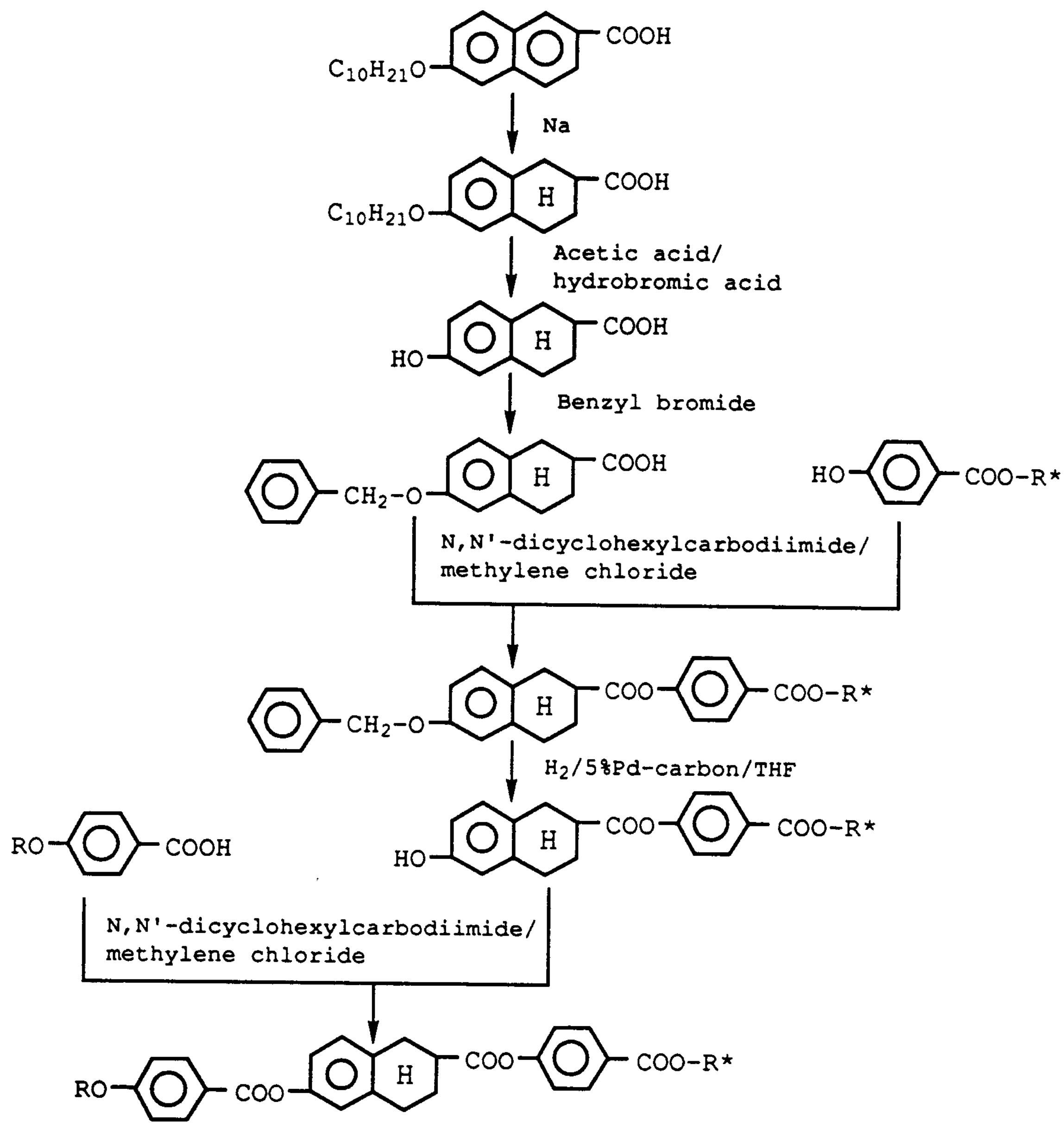


For example, a mixture of 6-n-alkoxynaphthalene-2-carboxylic acid and 1,2-ethoxyethane is refluxed in the presence of metallic sodium while adding dropwise thereto isoamyl alcohol to obtain 1,2,3,4-tetrahydro-6-n-alkoxynaphthalene-2-carboxylic acid.

The thus obtained 1,2,3,4-tetrahydro-6-n-alkoxynaphthalene-2-carboxylic acid is reacted with acetic acid and hydrobromic acid to obtain 1,2,3,4-tetrahydro-6-hydroxynaphthalene-2-carboxylic acid.

The 1,2,3,4-tetrahydro-6-hydroxynaphthalene-2-carboxylic acid obtained in the above manner is reacted with benzyl bromide in the presence of potassium hydroxide to obtain 1,2,3,4-tetrahydro-6-benzyloxynaphthalene-2-carboxylic acid.

Subsequently, an ester compound obtained from hydroxybenzoic acid and an alcohol having an asymmetiric carbon are reacted with the 1,2,3,4-tetrahydro-6-benzyloxynaphthalene-2-carboxylic acid obtained by the above step in the presence of 4-N,N-dimethylaminopyridine and a solvent such as methylene chloride while adding dropwise thereto a solution of N,N'-dicyclohexylcarbodiimide, whereby 4-(1',2',3',4'-tetrahydro-6'-benzyloxy-2'-naphthoyloxy)benzoate is obtained.

The thus obtained 4-(1',2',3',4'-tetrahydro-6'-benzyloxy-2'-naphthoyloxy)benzoate in a solvent such as tetrahydrofuran is reduced with hydrogen gas in the presence of a reducing catalyst such as palladium/carbon to obtain 4-(1',2',3',4'-tetrahydro-6'-hydroxy-2'-naphthoyloxy)benzoate.

Subsequently, 4-alkoxybenzoic acid prepared separately in a conventional way is reacted with the 4-(1',2',3',4'-tetrahydro-6'-hydroxy-2'-naphthoyloxy)benzoate obtained by the above step in the presence of 4-N,N-dimethylaminopyridine and a solvent such as methylene chloride while adding dropwise thereto N,N'-dicyclohexylcarbodiimide, whereby the carboxylate compound represented by formula [II] is obtained.

These processes for the preparation of the carboxylate compounds of the invention are provided only by way of a non-limiting illustration.

Among the carboxylate compounds of the invention prepared by the above-mentioned processes, the $^1$H-NMR of 4-[6'-(4''-decyloxybenzoyloxy)-5',6',7',8'-tetrahydro-2'-naphthoyloxy]benzoic acid R-1'''-trifluoromethylheptyl ester (Exemplified compound (4)] represented by the following formula is shown in Fig. 1.

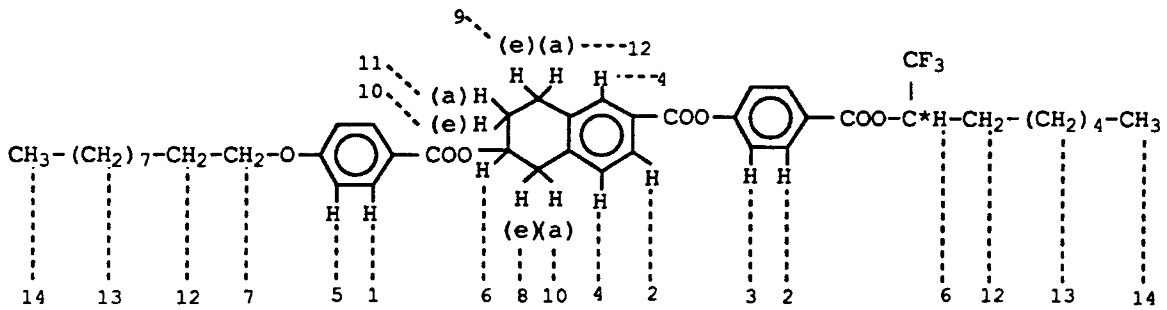

In this formula, (e) represents an equatorial steric conformation, (a) represents an axial steric conformation, and the numbers 1 to 14 show hydrogen atoms corresponding to the peaks in Fig. 1.

The $^1$H-NMR of 6-[6'-(4''-decyloxybenzoyloxy)-5',6',7',8'-tetrahydro-2'-naphthoyloxy]-1,2,3,4-tetrahydronaphthalene-2-carboxylic acid R-1'''-trifluoromethylheptyl ester [Exemplified compound (21)] represented by the following formula is shown in Fig. 2.

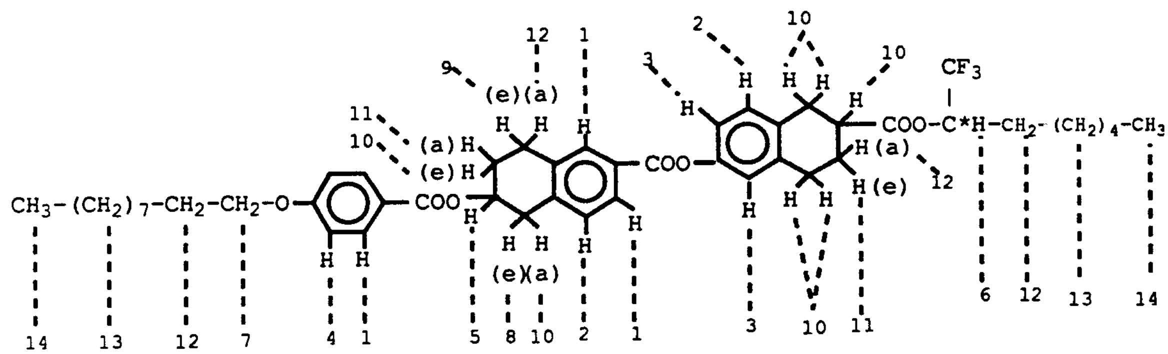

In this formula, the numbers 1 to 14 show hydrogen atoms corresponding to the peaks in Fig. 2.

Furthermore, the $^1$H-NMR of 6-[6'-(4''-decyloxybenzoyloxy)-5',6',7',8'-tetrahydro-2'-naphthoyloxy]-5,6,7,8-tetrahydronaphthalene-2-carboxylic acid R-1'''-trifluoromethylheptyl ester [Exemplified compound (22)] represented by the following formula is shown in Fig. 3.

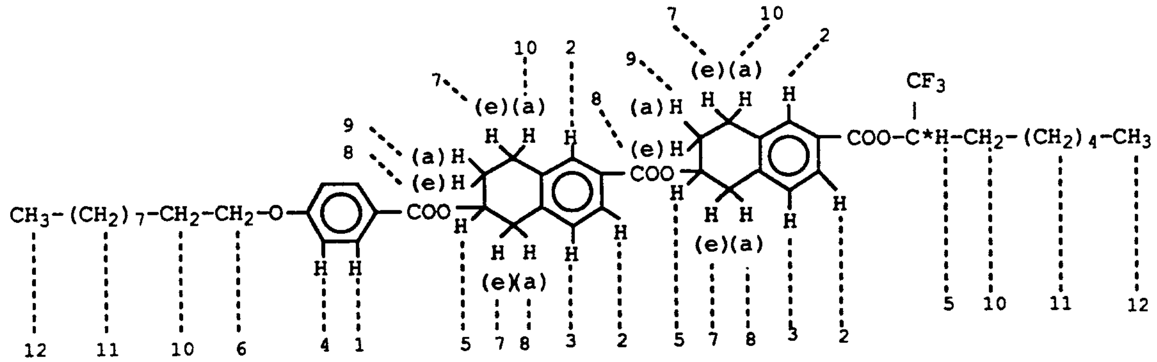

In this formula, the numbers 1 to 12 show hydrogen atoms corresponding to the peaks in Fig. 3.

Still further, the ¹H-NMR of 6-[4"-(5'-decyl-2'-pyrimidinyl)benzoyloxy]-5,6,7,8-tetrahydronaphthalene-2-carboxylic acid R-1'"-trifluoromethylheptyl ester [Exemplified compound (130)] represented by the following formula is shown in Fig. 4.

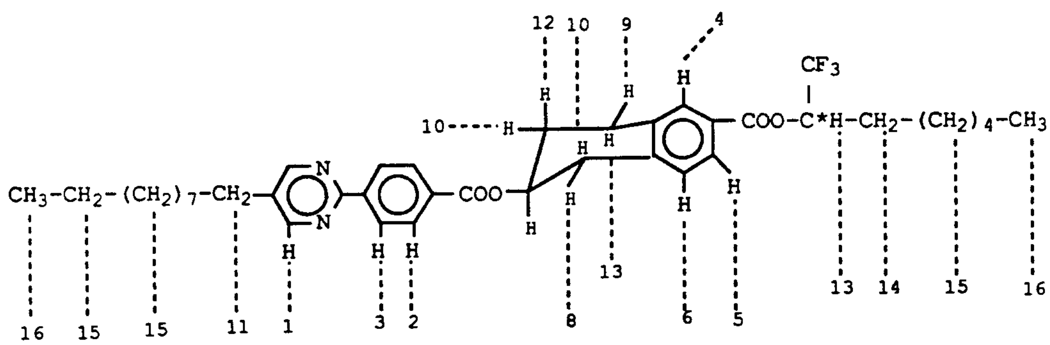

In this formula, the numbers 1 to 16 show hydrogen atoms corresponding to the peaks in Fig. 4.

Similarly, among the carboxylate compounds of the invention prepared in the manner as mentioned above, ¹H-NMR of 4-[6'-(4"-decyloxybenzoyloxy)-1',2',3',4'-tetrahydro-2'-naphthoyloxy]benzoic acid R-1'"-trifluoromethylheptyl ester [Exemplified compound (204)] represented by the following formula is shown in Fig. 5.

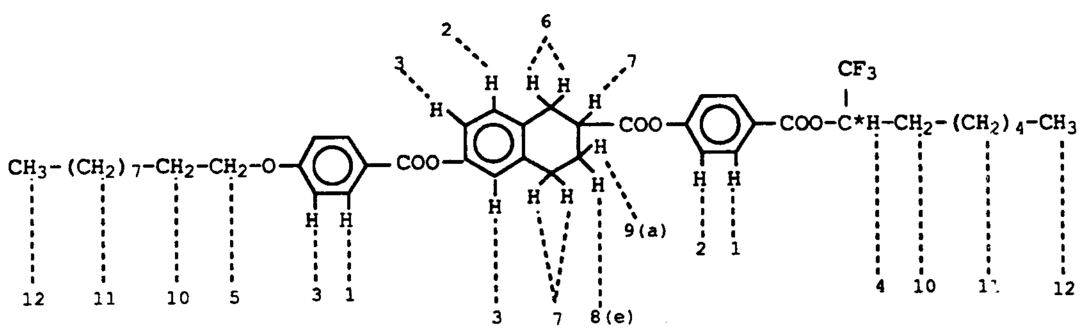

In this formula, (e) represents an equatorial steric conformation, and (a) represents an axial steric conformation, and the numbers 1 to 12 show hydrogen atoms corresponding to the peaks in Fig. 5.

The ¹H-NMR of 6-[6'-(4"-decyloxybenozyloxy)-1',2',3',4'-tetrahydro-2'-naphthoyloxy]-1,2,3,4-tetrahydronaphthalene-2-carboxylic acid R-1'"-trifluoromethylheptyl ester [Exemplified compound (221)] represented by the following formula is shown in Fig. 6.

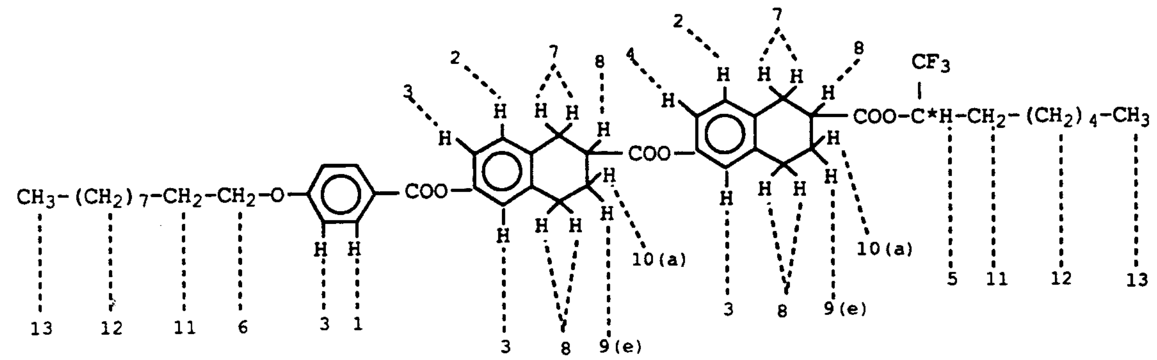

In this formula, the numbers 1 to 13 show hydrogen atoms corresponding to the peaks in Fig. 6.

Furthermore, the $^1$H-NMR of 6-[6'-(4''-decyloxybenozyloxy)-1',2',3',4'-tetrahydro-2'-naphthoyloxy]-5,6,7,8-tetrahydronaphthalene-2-carboxylic acid R-1'''-trifluoromethylheptyl ester [Exemplified compound (222)] represented by the following formula is shown in Fig. 7.

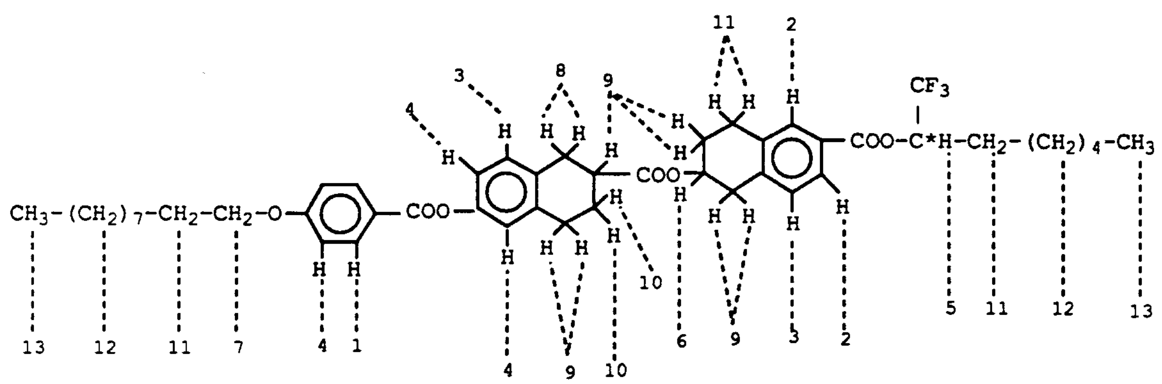

In this formula, the numbers 1 to 13 show hydrogen atoms corresponding to the peaks in Fig. 7.

Still further, the $^1$H-NMR of 6-[4'-(4''-decyloxybiphenyl)carboxy]-1,2,3,4-tetrahydronaphthalene-2-carboxylic acid R-1'''-trifluoromethylheptyl ester [Exemplified compound (251)] represented by the following formula is shown in Fig. 8.

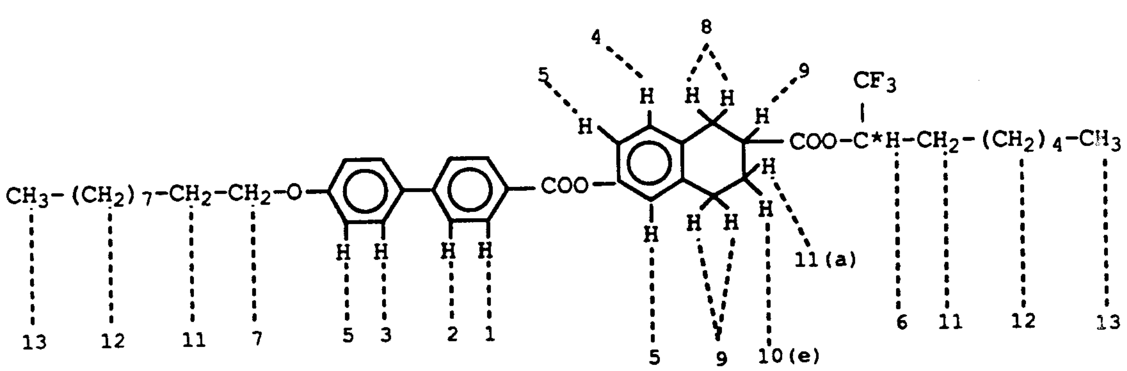

In this formula, the numbers 1 to 13 show hydrogen atoms corresponding to the peaks in Fig. 8.

Moreover, the $^1$H-NMR of 6-[4'-(4''-decyloxyphenylcyclohexane)carboxy]-1,2,3,4-tetrahydronaphthalene-2-carboxylic acid R-1'''-trifluoromethylheptyl ester [Exemplified compound (267)] represented by the formula is shown in Fig. 9.

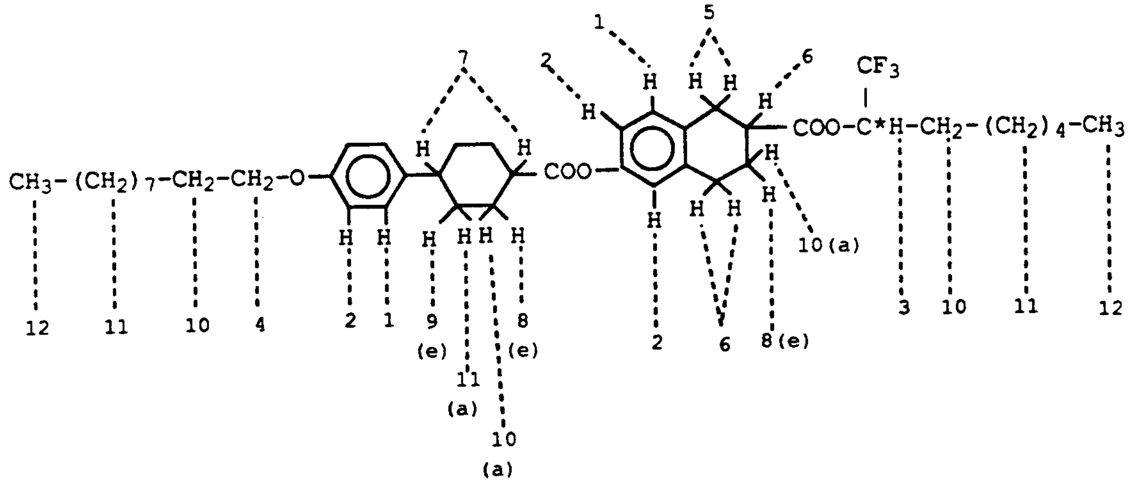

In this formula, the numbers 1 to 12 show hydrogen atoms corresponding to the peaks in Fig. 9.

The $^1$H-NMR of 6-(6'-heptyloxy-2'-naphthoyloxy)-1,2,3,4-tetrahydronaphthalene-2-carboxylic acid R-1"-trifluoromethylheptyl ester [Exemplified compound (296)] represented by the formula is shown in Fig. 10.

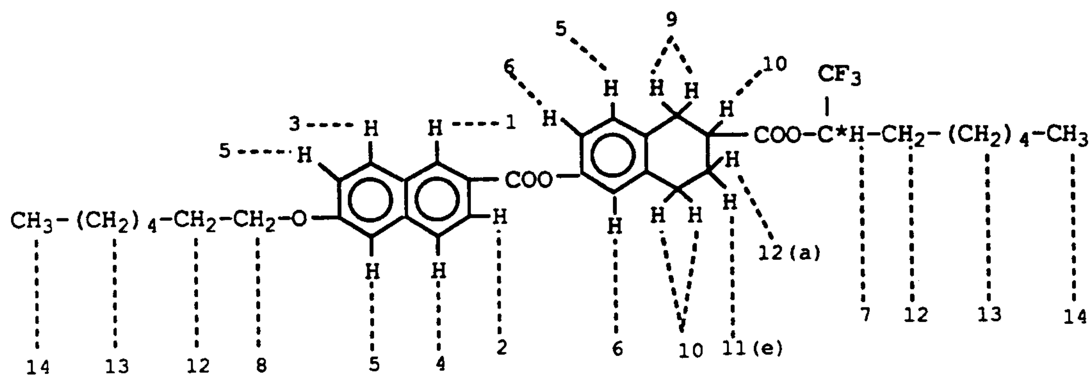

In this formula, the numbers 1 to 14 show hydrogen atoms corresponding to the peaks in Fig. 10.

The $^1$H-NMR of 6-(6'-decyloxy-2'-naphthoyloxy)-1,2,3,4-tetrahydronaphthalene-2-carboxylic acid R-1"-trifluoromethylheptyl ester [Exemplified compound (299)] represented by the following formula is shown in Fig. 11.

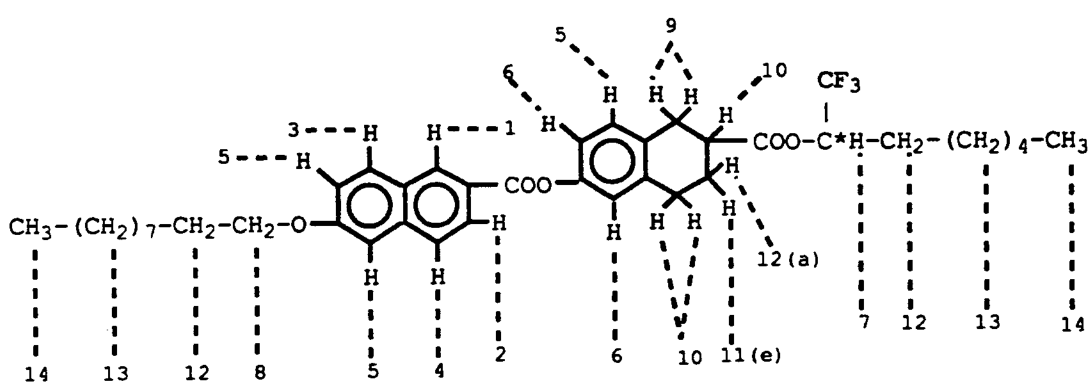

In this formula, the numbers 1 to 14 show hydrogen atoms corresponding to the peaks in Fig. 11.

Furthermore, the $^{1}$H-NMR of 6-[4'-(5"-decyl-2"-pyrimidinyl)benzoyloxy]-1,2,3,4-tetrahydronaphthalene-2-carboxylic acid R-1'"-trifluoromethylheptyl ester [Exemplified compound (331)] represented by the following formula is shown in Fig. 12.

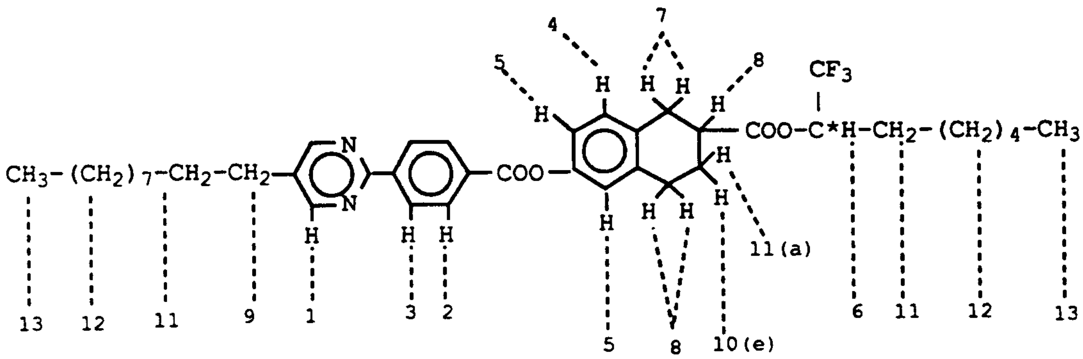

In this formula, the numbers 1 to 13 show hydrogen atoms corresponding to the peaks in Fig. 12.

Next, the second carboxylate compounds of the invention are illustrated.

The second carboxylate compounds of the invention may be represented by the following formula [III] or [IV].

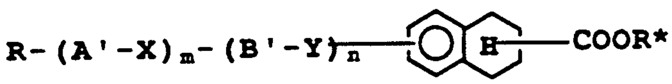

... [III]

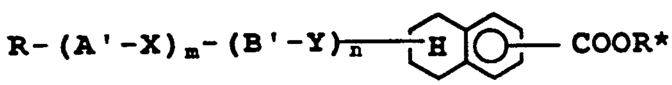

... [IV]

The 1,2,3,4-tetrahydro-naphthyl group constituting the main skeleton of formula [III] includes 1,2,3,4-tetrahydro-1,5-naphthyl, 1,2,3,4-tetrahydro-1,6-naphthyl, 1,2,3,4-tetrahydro-2,6-naphthyl and 1,2,3,4-tetrahydro-1,7-naphthyl.

Particularly, for the use of the carboxylate compounds of the invention as liquid crystal materials, it is preferable that the molecule of the compound is linear as a whole. On that account, 1,2,3,4-tetrahydro-2,6-naphthyl is particularly preferred.

In formula [III] or [IV], R, R*, X and Y are as defined in formula [I] or [II], provided that R may further be a hydrocarbon group having -OCO- wherein at least a part of hydrogen atoms constituting said hydrocarbon group may be substituted with halogen atoms. Such hydrocarbon groups include $C_2H_5$-OCO-$CH_2$-C*H($CF_3$)- and $C_2H_5$-OCO-$CH_2$-CH($CF_3$)-.

In formula [III] or [IV], m is 1 or 2, n is 0 to 2, and $m + n \geq 2$. Particularly, for the use of the carboxylate compounds of formula [III] or [IV] as liquid crystal materials, n is 0 or 1.

Examples of the compounds of formula [III] are shown in the following Tables 3-1 to 3-8:

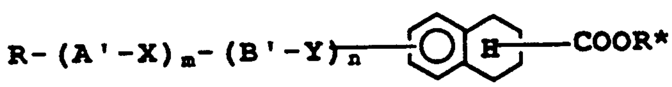

... [III]

Table 3-1

| Compound Number | R | A′ | X | m | B′ | Y | n | R* | Exam. No. |
|---|---|---|---|---|---|---|---|---|---|
| 401 | $C_7H_{15}O-$ | 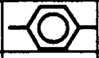 | -COO- | 2 | - | - | 0 | $-C^*H(CF_3)-C_6H_{13}$ | 23 |
| 402 | $C_8H_{17}O-$ | ditto | ditto | 2 | - | - | 0 | ditto | |
| 403 | $C_9H_{19}O-$ | ditto | ditto | 2 | - | - | 0 | ditto | |
| 404 | $C_{10}H_{21}O-$ | ditto | ditto | 2 | - | - | 0 | ditto | |
| 405 | $C_{11}H_{23}O-$ | ditto | ditto | 2 | - | - | 0 | ditto | |
| 406 | $C_{12}H_{23}O-$ | ditto | ditto | 2 | - | - | 0 | ditto | |
| 407 | $C_{14}H_{29}O-$ | ditto | ditto | 2 | - | - | 0 | ditto | |
| 408 | $C_{16}H_{33}O-$ | ditto | ditto | 2 | - | - | 0 | ditto | |
| 409 | $C_7H_{15}-$ | 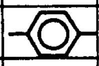 | -COO- | 2 | - | - | 0 | $-C^*H(CF_3)-C_6H_{13}$ | |
| 410 | $C_8H_{17}-$ | ditto | ditto | 2 | - | - | 0 | ditto | |
| 411 | $C_9H_{19}-$ | ditto | ditto | 2 | - | - | 0 | ditto | |
| 412 | $C_{10}H_{21}-$ | ditto | ditto | 2 | - | - | 0 | ditto | |
| 413 | $C_{11}H_{23}-$ | ditto | ditto | 2 | - | - | 0 | ditto | |
| 414 | $C_{12}H_{23}-$ | ditto | ditto | 2 | - | - | 0 | ditto | |
| 415 | $C_{14}H_{29}-$ | ditto | ditto | 2 | - | - | 0 | ditto | |
| 416 | $C_{16}H_{33}-$ | ditto | ditto | 2 | - | - | 0 | ditto | |
| 417 | $C_{10}H_{21}O-$ | 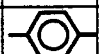 | -COO- | 2 | - | - | 0 | $-C^*H(CF_3)-C_6H_{13}$ | |
| 418 | $C_{10}H_{21}O-$ | ditto | ditto | 2 | - | - | 0 | $-C^*H(CH_3)-C_6H_{13}$ | |
| 419 | $C_{10}H_{21}O-$ | ditto | ditto | 2 | - | - | 0 | $-C^*H(C_2H_5)-C_5H_{11}$ | |
| 420 | $C_{10}H_{21}O-$ | ditto | ditto | 2 | - | - | 0 | $-C^*H(C_2H_5)-C_6H_{11}$ | |
| 421 | $C_{10}H_{21}O-$ | ditto | ditto | 2 | - | - | 0 | $-CH_2-C^*H(CH_3)-C_2H_5$ | |
| 422 | $C_{10}H_{21}O-$ | ditto | ditto | 2 | - | - | 0 | $-(CH_2)_3-C^*H(CH_3)-C_2H_5$ | |
| 423 | $C_{10}H_{21}O-$ | ditto | ditto | 2 | - | - | 0 | $-C^*H(CF_3)-CH_2-COO-C_2H_5$ | |

Table 3-2

| Compound Number | R | A′ | X | m | B′ | Y | n | R* | Exam. No. |
|---|---|---|---|---|---|---|---|---|---|
| 424 | $C_7H_{15}O-$ | 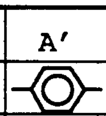 | -OCO- | 1 | 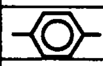 | -COO- | 1 | $-C^*H(CF_3)-C_6H_{13}$ | |
| 425 | $C_8H_{17}O-$ | ditto | ditto | 1 | ditto | ditto | 1 | ditto | |
| 426 | $C_9H_{19}O-$ | ditto | ditto | 1 | ditto | ditto | 1 | ditto | |
| 427 | $C_{10}H_{21}O-$ | ditto | ditto | 1 | ditto | ditto | 1 | ditto | |
| 428 | $C_{11}H_{23}O-$ | ditto | ditto | 1 | ditto | ditto | 1 | ditto | |
| 429 | $C_{12}H_{23}O-$ | ditto | ditto | 1 | ditto | ditto | 1 | ditto | |
| 430 | $C_{14}H_{29}O-$ | ditto | ditto | 1 | ditto | ditto | 1 | ditto | |
| 431 | $C_{16}H_{33}O-$ | ditto | ditto | 1 | ditto | ditto | 1 | ditto | |
| 432 | $C_7H_{15}O-$ | 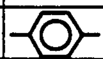 | $-CH_2O-$ | 1 | 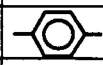 | -COO- | 1 | $-C^*H(CF_3)-C_6H_{13}$ | |
| 433 | $C_8H_{17}O-$ | ditto | ditto | 1 | ditto | ditto | 1 | ditto | |
| 434 | $C_9H_{19}O-$ | ditto | ditto | 1 | ditto | ditto | 1 | ditto | |
| 435 | $C_{10}H_{21}O-$ | ditto | ditto | 1 | ditto | ditto | 1 | ditto | |
| 436 | $C_{11}H_{23}O-$ | ditto | ditto | 1 | ditto | ditto | 1 | ditto | |
| 437 | $C_{12}H_{23}O-$ | ditto | ditto | 1 | ditto | ditto | 1 | ditto | |
| 438 | $C_{14}H_{29}O-$ | ditto | ditto | 1 | ditto | ditto | 1 | ditto | |
| 439 | $C_{16}H_{33}O-$ | ditto | ditto | 1 | ditto | ditto | 1 | ditto | |
| 440 | $C_7H_{15}O-$ | 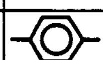 | $-OCH_2-$ | 1 | 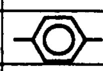 | -COO- | 1 | $-C^*H(CF_3)-C_6H_{13}$ | |
| 441 | $C_8H_{17}O-$ | ditto | ditto | 1 | ditto | ditto | 1 | ditto | |
| 442 | $C_9H_{19}O-$ | ditto | ditto | 1 | ditto | ditto | 1 | ditto | |
| 443 | $C_{10}H_{21}O-$ | ditto | ditto | 1 | ditto | ditto | 1 | ditto | |
| 444 | $C_{11}H_{23}O-$ | ditto | ditto | 1 | ditto | ditto | 1 | ditto | |
| 445 | $C_{12}H_{23}O-$ | ditto | ditto | 1 | ditto | ditto | 1 | ditto | |
| 446 | $C_{14}H_{29}O-$ | ditto | ditto | 1 | ditto | ditto | 1 | ditto | |
| 447 | $C_{16}H_{33}O-$ | ditto | ditto | 1 | ditto | ditto | 1 | ditto | |

Table 3-3

| Compound Number | R | A′ | X | m | B′ | Y | n | R* | Exam. No. |
|---|---|---|---|---|---|---|---|---|---|
| 448 | $C_7H_{15}O-$ | 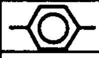 | $-CH_2-CH_2-$ | 1 | 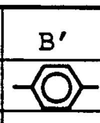 | -COO- | 1 | $-C^*H(CF_3)-C_6H_{13}$ | |
| 449 | $C_8H_{17}O-$ | ditto | ditto | 1 | ditto | ditto | 1 | ditto | |
| 450 | $C_9H_{19}O-$ | ditto | ditto | 1 | ditto | ditto | 1 | ditto | |
| 451 | $C_{10}H_{21}O-$ | ditto | ditto | 1 | ditto | ditto | 1 | ditto | |
| 452 | $C_{11}H_{23}O-$ | ditto | ditto | 1 | ditto | ditto | 1 | ditto | |
| 453 | $C_{12}H_{23}O-$ | ditto | ditto | 1 | ditto | ditto | 1 | ditto | |
| 454 | $C_{14}H_{29}O-$ | ditto | ditto | 1 | ditto | ditto | 1 | ditto | |
| 455 | $C_{16}H_{33}O-$ | ditto | ditto | 1 | ditto | ditto | 1 | ditto | |
| 456 | $C_7H_{15}O-$ | 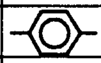 | -COO- | 1 | 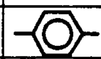 | -OCO- | 1 | $-C^*H(CF_3)-C_6H_{13}$ | |
| 457 | $C_8H_{17}O-$ | ditto | ditto | 1 | ditto | ditto | 1 | ditto | |
| 458 | $C_9H_{19}O-$ | ditto | ditto | 1 | ditto | ditto | 1 | ditto | |
| 459 | $C_{10}H_{21}O-$ | ditto | ditto | 1 | ditto | ditto | 1 | ditto | |
| 460 | $C_{11}H_{23}O-$ | ditto | ditto | 1 | ditto | ditto | 1 | ditto | |
| 461 | $C_{12}H_{23}O-$ | ditto | ditto | 1 | ditto | ditto | 1 | ditto | |
| 462 | $C_{14}H_{29}O-$ | ditto | ditto | 1 | ditto | ditto | 1 | ditto | |
| 463 | $C_{16}H_{33}O-$ | ditto | ditto | 1 | ditto | ditto | 1 | ditto | |
| 464 | $C_7H_{15}O-$ | 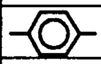 | -COO- | 1 | 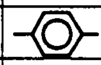 | $-CH_2O-$ | 1 | $-C^*H(CF_3)-C_6H_{13}$ | |
| 465 | $C_8H_{17}O-$ | ditto | ditto | 1 | ditto | ditto | 1 | ditto | |
| 466 | $C_9H_{19}O-$ | ditto | ditto | 1 | ditto | ditto | 1 | ditto | |
| 467 | $C_{10}H_{21}O-$ | ditto | ditto | 1 | ditto | ditto | 1 | ditto | |
| 468 | $C_{11}H_{23}O-$ | ditto | ditto | 1 | ditto | ditto | 1 | ditto | |
| 469 | $C_{12}H_{23}O-$ | ditto | ditto | 1 | ditto | ditto | 1 | ditto | |
| 470 | $C_{14}H_{29}O-$ | ditto | ditto | 1 | ditto | ditto | 1 | ditto | |
| 471 | $C_{16}H_{33}O-$ | ditto | ditto | 1 | ditto | ditto | 1 | ditto | |

Table 3-4

| Compound Number | R | A' | X | m | B' | Y | n | R* | Exam. No. |
|---|---|---|---|---|---|---|---|---|---|
| 472 | $C_7H_{15}O-$ | 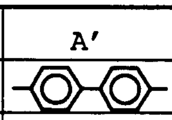 | -COO- | 1 | 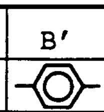 | -COO- | 1 | $-C^*H(CF_3)-C_6H_{13}$ | |
| 473 | $C_8H_{17}O-$ | ditto | ditto | 1 | ditto | ditto | 1 | ditto | |
| 474 | $C_9H_{19}O-$ | ditto | ditto | 1 | ditto | ditto | 1 | ditto | |
| 475 | $C_{10}H_{21}O-$ | ditto | ditto | 1 | ditto | ditto | 1 | ditto | |
| 476 | $C_{11}H_{23}O-$ | ditto | ditto | 1 | ditto | ditto | 1 | ditto | |
| 477 | $C_{12}H_{23}O-$ | ditto | ditto | 1 | ditto | ditto | 1 | ditto | |
| 478 | $C_{14}H_{29}O-$ | ditto | ditto | 1 | ditto | ditto | 1 | ditto | |
| 479 | $C_{16}H_{33}O-$ | ditto | ditto | 1 | ditto | ditto | 1 | ditto | |
| 480 | $C_7H_{15}O-$ | 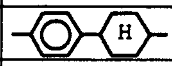 | -COO- | 1 | 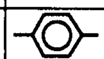 | -COO- | 1 | $-C^*H(CF_3)-C_6H_{13}$ | |
| 481 | $C_8H_{17}O-$ | ditto | ditto | 1 | ditto | ditto | 1 | ditto | |
| 482 | $C_9H_{19}O-$ | ditto | ditto | 1 | ditto | ditto | 1 | ditto | |
| 483 | $C_{10}H_{21}O-$ | ditto | ditto | 1 | ditto | ditto | 1 | ditto | |
| 484 | $C_{11}H_{23}O-$ | ditto | ditto | 1 | ditto | ditto | 1 | ditto | |
| 485 | $C_{12}H_{23}O-$ | ditto | ditto | 1 | ditto | ditto | 1 | ditto | |
| 486 | $C_{14}H_{29}O-$ | ditto | ditto | 1 | ditto | ditto | 1 | ditto | |
| 487 | $C_{16}H_{33}O-$ | ditto | ditto | 1 | ditto | ditto | 1 | ditto | |
| 488 | $C_7H_{15}O-$ | 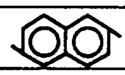 | -COO- | 1 | 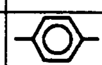 | -COO- | 1 | $-C^*H(CF_3)-C_6H_{13}$ | |
| 489 | $C_8H_{17}O-$ | ditto | ditto | 1 | ditto | ditto | 1 | ditto | |
| 490 | $C_9H_{19}O-$ | ditto | ditto | 1 | ditto | ditto | 1 | ditto | |
| 491 | $C_{10}H_{21}O-$ | ditto | ditto | 1 | ditto | ditto | 1 | ditto | |
| 492 | $C_{11}H_{23}O-$ | ditto | ditto | 1 | ditto | ditto | 1 | ditto | |
| 493 | $C_{12}H_{23}O-$ | ditto | ditto | 1 | ditto | ditto | 1 | ditto | |
| 494 | $C_{14}H_{29}O-$ | ditto | ditto | 1 | ditto | ditto | 1 | ditto | |
| 495 | $C_{16}H_{33}O-$ | ditto | ditto | 1 | ditto | ditto | 1 | ditto | |

Table 3-5

| Compound Number | R | A′ | X | m | B′ | Y | n | R* | Exam. No. |
|---|---|---|---|---|---|---|---|---|---|
| 496 | $C_7H_{15}O-$ | 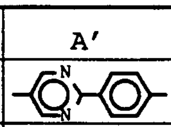 | -COO- | 1 | 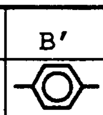 | -COO- | 1 | $-C^*H(CF_3)-C_6H_{13}$ | |
| 497 | $C_8H_{17}O-$ | ditto | ditto | 1 | ditto | ditto | 1 | ditto | |
| 498 | $C_9H_{19}O-$ | ditto | ditto | 1 | ditto | ditto | 1 | ditto | |
| 499 | $C_{10}H_{21}O-$ | ditto | ditto | 1 | ditto | ditto | 1 | ditto | |
| 500 | $C_{11}H_{23}O-$ | ditto | ditto | 1 | ditto | ditto | 1 | ditto | |
| 501 | $C_{12}H_{23}O-$ | ditto | ditto | 1 | ditto | ditto | 1 | ditto | |
| 502 | $C_{14}H_{29}O-$ | ditto | ditto | 1 | ditto | ditto | 1 | ditto | |
| 503 | $C_{16}H_{33}O-$ | ditto | ditto | 1 | ditto | ditto | 1 | ditto | |
| 504 | $C_7H_{15}-$ | 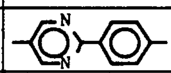 | -COO- | 1 | 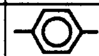 | -COO- | 1 | $-C^*H(CF_3)-C_6H_{13}$ | |
| 505 | $C_8H_{17}-$ | ditto | ditto | 1 | ditto | ditto | 1 | ditto | |
| 506 | $C_9H_{19}-$ | ditto | ditto | 1 | ditto | ditto | 1 | ditto | |
| 507 | $C_{10}H_{21}-$ | ditto | ditto | 1 | ditto | ditto | 1 | ditto | |
| 508 | $C_{11}H_{23}-$ | ditto | ditto | 1 | ditto | ditto | 1 | ditto | |
| 509 | $C_{12}H_{23}-$ | ditto | ditto | 1 | ditto | ditto | 1 | ditto | |
| 510 | $C_{14}H_{29}-$ | ditto | ditto | 1 | ditto | ditto | 1 | ditto | |
| 511 | $C_{16}H_{33}-$ | ditto | ditto | 1 | ditto | ditto | 1 | ditto | |
| 512 | $C_7H_{15}O-$ | 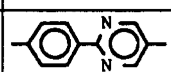 | -COO- | 1 | 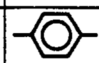 | -COO- | 1 | $-C^*H(CF_3)-C_6H_{13}$ | |
| 513 | $C_8H_{17}O-$ | ditto | ditto | 1 | ditto | ditto | 1 | ditto | |
| 514 | $C_9H_{19}O-$ | ditto | ditto | 1 | ditto | ditto | 1 | ditto | |
| 515 | $C_{10}H_{21}O-$ | ditto | ditto | 1 | ditto | ditto | 1 | ditto | |
| 516 | $C_{11}H_{23}O-$ | ditto | ditto | 1 | ditto | ditto | 1 | ditto | |
| 517 | $C_{12}H_{23}O-$ | ditto | ditto | 1 | ditto | ditto | 1 | ditto | |
| 518 | $C_{14}H_{29}O-$ | ditto | ditto | 1 | ditto | ditto | 1 | ditto | |
| 519 | $C_{16}H_{33}O-$ | ditto | ditto | 1 | ditto | ditto | 1 | ditto | |

Table 3-6

| Compound Number | R | A′ | X | m | B′ | Y | n | R* | Exam. No. |
|---|---|---|---|---|---|---|---|---|---|
| 520 | $C_7H_{15}O-$ | 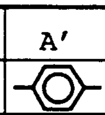 | -COO- | 1 | 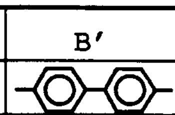 | -COO- | 1 | $-C^*H(CF_3)-C_6H_{13}$ | |
| 521 | $C_8H_{17}O-$ | ditto | ditto | 1 | ditto | ditto | 1 | ditto | |
| 522 | $C_9H_{19}O-$ | ditto | ditto | 1 | ditto | ditto | 1 | ditto | |
| 523 | $C_{10}H_{21}O-$ | ditto | ditto | 1 | ditto | ditto | 1 | ditto | |
| 524 | $C_{11}H_{23}O-$ | ditto | ditto | 1 | ditto | ditto | 1 | ditto | |
| 525 | $C_{12}H_{23}O-$ | ditto | ditto | 1 | ditto | ditto | 1 | ditto | |
| 526 | $C_{14}H_{29}O-$ | ditto | ditto | 1 | ditto | ditto | 1 | ditto | |
| 527 | $C_{16}H_{33}O-$ | ditto | ditto | 1 | ditto | ditto | 1 | ditto | |
| 528 | $C_7H_{15}O-$ | 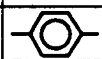 | -COO- | 1 | 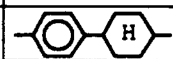 | -COO- | 1 | $-C^*H(CF_3)-C_6H_{13}$ | |
| 529 | $C_8H_{17}O-$ | ditto | ditto | 1 | ditto | ditto | 1 | ditto | |
| 530 | $C_9H_{19}O-$ | ditto | ditto | 1 | ditto | ditto | 1 | ditto | |
| 531 | $C_{10}H_{21}O-$ | ditto | ditto | 1 | ditto | ditto | 1 | ditto | |
| 532 | $C_{11}H_{23}O-$ | ditto | ditto | 1 | ditto | ditto | 1 | ditto | |
| 533 | $C_{12}H_{23}O-$ | ditto | ditto | 1 | ditto | ditto | 1 | ditto | |
| 534 | $C_{14}H_{29}O-$ | ditto | ditto | 1 | ditto | ditto | 1 | ditto | |
| 535 | $C_{16}H_{33}O-$ | ditto | ditto | 1 | ditto | ditto | 1 | ditto | |
| 536 | $C_7H_{15}O-$ | 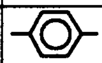 | -COO- | 1 | 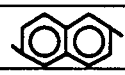 | -COO- | 1 | $-C^*H(CF_3)-C_6H_{13}$ | |
| 537 | $C_8H_{17}O-$ | ditto | ditto | 1 | ditto | ditto | 1 | ditto | |
| 538 | $C_9H_{19}O-$ | ditto | ditto | 1 | ditto | ditto | 1 | ditto | |
| 539 | $C_{10}H_{21}O-$ | ditto | ditto | 1 | ditto | ditto | 1 | ditto | |
| 540 | $C_{11}H_{23}O-$ | ditto | ditto | 1 | ditto | ditto | 1 | ditto | |
| 541 | $C_{12}H_{23}O-$ | ditto | ditto | 1 | ditto | ditto | 1 | ditto | |
| 542 | $C_{14}H_{29}O-$ | ditto | ditto | 1 | ditto | ditto | 1 | ditto | |
| 543 | $C_{16}H_{33}O-$ | ditto | ditto | 1 | ditto | ditto | 1 | ditto | |

Table 3-7

| Compound Number | R | A' | X | m | B' | Y | n | R* | Exam. No. |
|---|---|---|---|---|---|---|---|---|---|
| 544 | $C_6H_{13}$-C*H($CF_3$)O- | 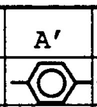 | -COO- | 2 | - | - | 0 | -C*H($CF_3$)-$C_6H_{13}$ | |
| 545 | $C_6H_{13}$-C*H($CH_3$)O- | ditto | ditto | 2 | - | - | 0 | ditto | 24 |
| 546 | $C_5H_{11}$-C*H($C_2H_5$)O- | ditto | ditto | 2 | - | - | 0 | ditto | |
| 547 | $C_6H_{13}$-C*H($C_2H_5$)O- | ditto | ditto | 2 | - | - | 0 | ditto | |
| 548 | $C_2H_5$-C*H($CH_3$)-$CH_2$O- | ditto | ditto | 2 | - | - | 0 | ditto | |
| 549 | $C_2H_5$-C*H($CH_3$)-($CH_2$)$_3$O- | ditto | ditto | 2 | - | - | 0 | ditto | |
| 550 | $C_2H_5$-OCO-$CH_2$-C*H($CF_3$)O- | ditto | ditto | 2 | - | - | 0 | ditto | |
| 551 | $C_6H_{13}$-C*H($CF_3$)O- | 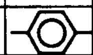 | -COO- | 2 | - | - | 0 | -C*H($CF_3$)-$C_6H_{13}$ | |
| 552 | $C_6H_{13}$-C*H($CH_3$)O- | ditto | ditto | 2 | - | - | 0 | ditto | |
| 553 | $C_5H_{11}$-C*H($C_2H_5$)O- | ditto | ditto | 2 | - | - | 0 | ditto | |
| 554 | $C_6H_{13}$-C*H($C_2H_5$)O- | ditto | ditto | 2 | - | - | 0 | ditto | |
| 555 | $C_2H_5$-CH($CH_3$)-$CH_2$O- | ditto | ditto | 2 | - | - | 0 | ditto | |
| 556 | $C_2H_5$-CH($CH_3$)-($CH_2$)$_3$O- | ditto | ditto | 2 | - | - | 0 | ditto | |
| 557 | $C_2H_5$-OCO-$CH_2$-CH($CF_3$)O- | ditto | ditto | 2 | - | - | 0 | ditto | |
| 558 | $C_6H_{13}$-C*H($CF_3$)O- | 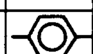 | -COO- | 2 | - | - | 0 | -C*H($CH_3$)-$C_6H_{13}$ | |
| 559 | $C_6H_{13}$-C*H($CH_3$)O- | ditto | ditto | 2 | - | - | 0 | ditto | |
| 560 | $C_5H_{11}$-C*H($C_2H_5$)O- | ditto | ditto | 2 | - | - | 0 | ditto | |
| 561 | $C_6H_{13}$-C*H($C_2H_5$)O- | ditto | ditto | 2 | - | - | 0 | ditto | |
| 562 | $C_2H_5$-C*H($CH_3$)-$CH_2$O- | ditto | ditto | 2 | - | - | 0 | ditto | |
| 563 | $C_2H_5$-C*H($CH_3$)-($CH_2$)$_3$O- | ditto | ditto | 2 | - | - | 0 | ditto | |
| 564 | $C_2H_5$-OCO-$CH_2$-C*H($CF_3$)O- | ditto | ditto | 2 | - | - | 0 | ditto | |

Table 3-8

| Compound Number | R | A' | X | m | B' | Y | n | R* | Exam. No. |
|---|---|---|---|---|---|---|---|---|---|
| 565 | $C_6H_{13}$-C*H($CF_3$)O- | 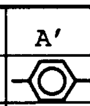 | -COO- | 2 | - | - | 0 | -C*H($CH_3$)-$C_6H_{13}$ | |
| 566 | $C_6H_{13}$-C*H($CH_3$)O- | ditto | ditto | 2 | - | - | 0 | ditto | |
| 567 | $C_5H_{11}$-CH($C_2H_5$)O- | ditto | ditto | 2 | - | - | 0 | ditto | |
| 568 | $C_6H_{13}$-CH($C_2H_5$)O- | ditto | ditto | 2 | - | - | 0 | ditto | |
| 569 | $C_2H_5$-C*H($CH_3$)-$CH_2$O- | ditto | ditto | 2 | - | - | 0 | ditto | |
| 570 | $C_2H_5$-C*H($CH_3$)-($CH_2$)$_3$O- | ditto | ditto | 2 | - | - | 0 | ditto | |
| 571 | $C_2H_5$-OCO-$CH_2$-C*H($CF_3$)O- | ditto | ditto | 2 | - | - | 0 | ditto | |

Further, examples of the compounds represented by the formula [IV] are shown in the following Tables 4-1 to 4-8.

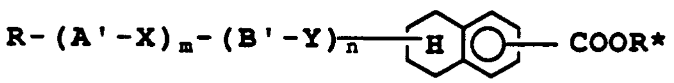 ... [IV]

Table 4-1

| Compound Number | R | A′ | X | m | B′ | Y | n | R* | Exam. No. |
|---|---|---|---|---|---|---|---|---|---|
| 572 | $C_7H_{15}O-$ | 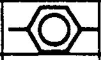 | -COO- | 2 | - | - | 0 | $-C^*H(CF_3)-C_6H_{13}$ | |
| 573 | $C_8H_{17}O-$ | ditto | ditto | 2 | - | - | 0 | ditto | |
| 574 | $C_9H_{19}O-$ | ditto | ditto | 2 | - | - | 0 | ditto | |
| 575 | $C_{10}H_{21}O-$ | ditto | ditto | 2 | - | - | 0 | ditto | 25 |
| 576 | $C_{11}H_{23}O-$ | ditto | ditto | 2 | - | - | 0 | ditto | |
| 577 | $C_{12}H_{23}O-$ | ditto | ditto | 2 | - | - | 0 | ditto | |
| 578 | $C_{14}H_{29}O-$ | ditto | ditto | 2 | - | - | 0 | ditto | |
| 579 | $C_{16}H_{33}O-$ | ditto | ditto | 2 | - | - | 0 | ditto | |
| 580 | $C_7H_{15}-$ | 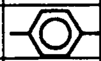 | -COO- | 2 | - | - | 0 | $-C^*H(CF_3)-C_6H_{13}$ | |
| 581 | $C_8H_{17}-$ | ditto | ditto | 2 | - | - | 0 | ditto | |
| 582 | $C_9H_{19}-$ | ditto | ditto | 2 | - | - | 0 | ditto | |
| 583 | $C_{10}H_{21}-$ | ditto | ditto | 2 | - | - | 0 | ditto | |
| 584 | $C_{11}H_{23}-$ | ditto | ditto | 2 | - | - | 0 | ditto | |
| 585 | $C_{12}H_{25}-$ | ditto | ditto | 2 | - | - | 0 | ditto | |
| 586 | $C_{14}H_{29}-$ | ditto | ditto | 2 | - | - | 0 | ditto | |
| 587 | $C_{16}H_{33}-$ | ditto | ditto | 2 | - | - | 0 | ditto | |
| 588 | $C_{10}H_{21}O-$ | 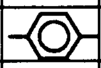 | -COO- | 2 | - | - | 0 | $-C^*H(CF_3)-C_6H_{13}$ | |
| 589 | $C_{10}H_{21}O-$ | ditto | ditto | 2 | - | - | 0 | $-C^*H(CH_3)-C_6H_{13}$ | |
| 590 | $C_{10}H_{21}O-$ | ditto | ditto | 2 | - | - | 0 | $-C^*H(C_2H_5)-C_5H_{11}$ | |
| 591 | $C_{10}H_{21}O-$ | ditto | ditto | 2 | - | - | 0 | $-C^*H(C_2H_5)-C_6H_{11}$ | |
| 592 | $C_{10}H_{21}O-$ | ditto | ditto | 2 | - | - | 0 | $-CH_2-C^*H(CH_3)-C_2H_5$ | |
| 593 | $C_{10}H_{21}O-$ | ditto | ditto | 2 | - | - | 0 | $-(CH_2)_3-C^*H(CH_3)-C_2H_5$ | |
| 594 | $C_{10}H_{21}O-$ | ditto | ditto | 2 | - | - | 0 | $-C^*H(CF_3)-CH_2-COO-C_2H_5$ | |

Table 4-2

| Compound Number | R | A′ | X | m | B′ | Y | n | R* | Exam. No. |
|---|---|---|---|---|---|---|---|---|---|
| 595 | $C_7H_{15}O-$ | 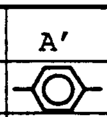 | -OCO- | 1 | 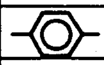 | -COO- | 1 | $-C^*H(CF_3)-C_6H_{13}$ | |
| 596 | $C_8H_{17}O-$ | ditto | ditto | 1 | ditto | ditto | 1 | ditto | |
| 597 | $C_9H_{19}O-$ | ditto | ditto | 1 | ditto | ditto | 1 | ditto | |
| 598 | $C_{10}H_{21}O-$ | ditto | ditto | 1 | ditto | ditto | 1 | ditto | |
| 599 | $C_{11}H_{23}O-$ | ditto | ditto | 1 | ditto | ditto | 1 | ditto | |
| 600 | $C_{12}H_{23}O-$ | ditto | ditto | 1 | ditto | ditto | 1 | ditto | |
| 601 | $C_{14}H_{29}O-$ | ditto | ditto | 1 | ditto | ditto | 1 | ditto | |
| 602 | $C_{16}H_{33}O-$ | ditto | ditto | 1 | ditto | ditto | 1 | ditto | |
| 603 | $C_7H_{15}O-$ | 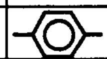 | $-CH_2O-$ | 1 | 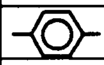 | -COO- | 1 | $-C^*H(CF_3)-C_6H_{13}$ | |
| 604 | $C_8H_{17}O-$ | ditto | ditto | 1 | ditto | ditto | 1 | ditto | |
| 605 | $C_9H_{19}O-$ | ditto | ditto | 1 | ditto | ditto | 1 | ditto | |
| 606 | $C_{10}H_{21}O-$ | ditto | ditto | 1 | ditto | ditto | 1 | ditto | |
| 607 | $C_{11}H_{23}O-$ | ditto | ditto | 1 | ditto | ditto | 1 | ditto | |
| 608 | $C_{12}H_{23}O-$ | ditto | ditto | 1 | ditto | ditto | 1 | ditto | |
| 609 | $C_{14}H_{29}O-$ | ditto | ditto | 1 | ditto | ditto | 1 | ditto | |
| 610 | $C_{16}H_{33}O-$ | ditto | ditto | 1 | ditto | ditto | 1 | ditto | |
| 611 | $C_7H_{15}O-$ | 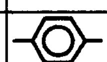 | $-OCH_2-$ | 1 | 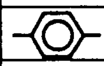 | -COO- | 1 | $-C^*H(CF_3)-C_6H_{13}$ | |
| 612 | $C_8H_{17}O-$ | ditto | ditto | 1 | ditto | ditto | 1 | ditto | |
| 613 | $C_9H_{19}O-$ | ditto | ditto | 1 | ditto | ditto | 1 | ditto | |
| 614 | $C_{10}H_{21}O-$ | ditto | ditto | 1 | ditto | ditto | 1 | ditto | |
| 615 | $C_{11}H_{23}O-$ | ditto | ditto | 1 | ditto | ditto | 1 | ditto | |
| 616 | $C_{12}H_{23}O-$ | ditto | ditto | 1 | ditto | ditto | 1 | ditto | |
| 617 | $C_{14}H_{29}O-$ | ditto | ditto | 1 | ditto | ditto | 1 | ditto | |
| 618 | $C_{16}H_{33}O-$ | ditto | ditto | 1 | ditto | ditto | 1 | ditto | |

Table 4-3

| Compound Number | R | A' | X | m | B' | Y | n | R* | Exam. No. |
|---|---|---|---|---|---|---|---|---|---|
| 619 | $C_7H_{15}O-$ | 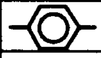 | $-CH_2-CH_2-$ | 1 | 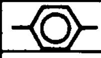 | -COO- | 1 | $-C^*H(CF_3)-C_6H_{13}$ | |
| 620 | $C_8H_{17}O-$ | ditto | ditto | 1 | ditto | ditto | 1 | ditto | |
| 621 | $C_9H_{19}O-$ | ditto | ditto | 1 | ditto | ditto | 1 | ditto | |
| 622 | $C_{10}H_{21}O-$ | ditto | ditto | 1 | ditto | ditto | 1 | ditto | |
| 623 | $C_{11}H_{23}O-$ | ditto | ditto | 1 | ditto | ditto | 1 | ditto | |
| 624 | $C_{12}H_{23}O-$ | ditto | ditto | 1 | ditto | ditto | 1 | ditto | |
| 625 | $C_{14}H_{29}O-$ | ditto | ditto | 1 | ditto | ditto | 1 | ditto | |
| 626 | $C_{16}H_{33}O-$ | ditto | ditto | 1 | ditto | ditto | 1 | ditto | |
| 627 | $C_7H_{15}O-$ | 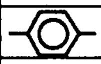 | -COO- | 1 | 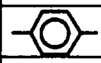 | -OCO- | 1 | $-C^*H(CF_3)-C_6H_{13}$ | |
| 628 | $C_8H_{17}O-$ | ditto | ditto | 1 | ditto | ditto | 1 | ditto | |
| 629 | $C_9H_{19}O-$ | ditto | ditto | 1 | ditto | ditto | 1 | ditto | |
| 630 | $C_{10}H_{21}O-$ | ditto | ditto | 1 | ditto | ditto | 1 | ditto | |
| 631 | $C_{11}H_{23}O-$ | ditto | ditto | 1 | ditto | ditto | 1 | ditto | |
| 632 | $C_{12}H_{23}O-$ | ditto | ditto | 1 | ditto | ditto | 1 | ditto | |
| 633 | $C_{14}H_{29}O-$ | ditto | ditto | 1 | ditto | ditto | 1 | ditto | |
| 634 | $C_{16}H_{33}O-$ | ditto | ditto | 1 | ditto | ditto | 1 | ditto | |
| 635 | $C_7H_{15}O-$ | 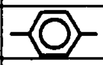 | -COO- | 1 | 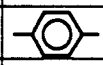 | $-CH_2O-$ | 1 | $-C^*H(CF_3)-C_6H_{13}$ | |
| 636 | $C_8H_{17}O-$ | ditto | ditto | 1 | ditto | ditto | 1 | ditto | |
| 637 | $C_9H_{19}O-$ | ditto | ditto | 1 | ditto | ditto | 1 | ditto | |
| 638 | $C_{10}H_{21}O-$ | ditto | ditto | 1 | ditto | ditto | 1 | ditto | |
| 639 | $C_{11}H_{23}O-$ | ditto | ditto | 1 | ditto | ditto | 1 | ditto | |
| 640 | $C_{12}H_{23}O-$ | ditto | ditto | 1 | ditto | ditto | 1 | ditto | |
| 641 | $C_{14}H_{29}O-$ | ditto | ditto | 1 | ditto | ditto | 1 | ditto | |
| 642 | $C_{16}H_{33}O-$ | ditto | ditto | 1 | ditto | ditto | 1 | ditto | |

Table 4-4

| Compound Number | R | A′ | X | m | B′ | Y | n | R* | Exam. No. |
|---|---|---|---|---|---|---|---|---|---|
| 643 | $C_7H_{15}O$- | 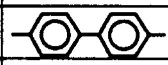 | -COO- | 1 | 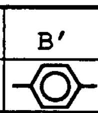 | -COO- | 1 | -C*H($CF_3$)-$C_6H_{13}$ | |
| 644 | $C_8H_{17}O$- | ditto | ditto | 1 | ditto | ditto | 1 | ditto | |
| 645 | $C_9H_{19}O$- | ditto | ditto | 1 | ditto | ditto | 1 | ditto | |
| 646 | $C_{10}H_{21}O$- | ditto | ditto | 1 | ditto | ditto | 1 | ditto | |
| 647 | $C_{11}H_{23}O$- | ditto | ditto | 1 | ditto | ditto | 1 | ditto | |
| 648 | $C_{12}H_{23}O$- | ditto | ditto | 1 | ditto | ditto | 1 | ditto | |
| 649 | $C_{14}H_{29}O$- | ditto | ditto | 1 | ditto | ditto | 1 | ditto | |
| 650 | $C_{16}H_{33}O$- | ditto | ditto | 1 | ditto | ditto | 1 | ditto | |
| 651 | $C_7H_{15}O$- | 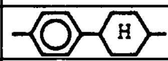 | -COO- | 1 | 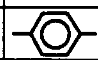 | -COO- | 1 | -C*H($CF_3$)-$C_6H_{13}$ | |
| 652 | $C_8H_{17}O$- | ditto | ditto | 1 | ditto | ditto | 1 | ditto | |
| 653 | $C_9H_{19}O$- | ditto | ditto | 1 | ditto | ditto | 1 | ditto | |
| 654 | $C_{10}H_{21}O$- | ditto | ditto | 1 | ditto | ditto | 1 | ditto | |
| 655 | $C_{11}H_{23}O$- | ditto | ditto | 1 | ditto | ditto | 1 | ditto | |
| 656 | $C_{12}H_{23}O$- | ditto | ditto | 1 | ditto | ditto | 1 | ditto | |
| 657 | $C_{14}H_{29}O$- | ditto | ditto | 1 | ditto | ditto | 1 | ditto | |
| 658 | $C_{16}H_{33}O$- | ditto | ditto | 1 | ditto | ditto | 1 | ditto | |
| 659 | $C_7H_{15}O$- | 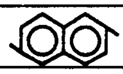 | -COO- | 1 | 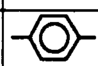 | -COO- | 1 | -C*H($CF_3$)-$C_6H_{13}$ | |
| 660 | $C_8H_{17}O$- | ditto | ditto | 1 | ditto | ditto | 1 | ditto | |
| 661 | $C_9H_{19}O$- | ditto | ditto | 1 | ditto | ditto | 1 | ditto | |
| 662 | $C_{10}H_{21}O$- | ditto | ditto | 1 | ditto | ditto | 1 | ditto | |
| 663 | $C_{11}H_{23}O$- | ditto | ditto | 1 | ditto | ditto | 1 | ditto | |
| 664 | $C_{12}H_{23}O$- | ditto | ditto | 1 | ditto | ditto | 1 | ditto | |
| 665 | $C_{14}H_{29}O$- | ditto | ditto | 1 | ditto | ditto | 1 | ditto | |
| 666 | $C_{16}H_{33}O$- | ditto | ditto | 1 | ditto | ditto | 1 | ditto | |

Table 4-5

| Compound Number | R | A' | X | m | B' | Y | n | R* | Exam. No. |
|---|---|---|---|---|---|---|---|---|---|
| 667 | $C_7H_{15}O-$ | 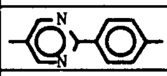 | -COO- | 1 | 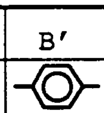 | -COO- | 1 | $-C^*H(CF_3)-C_6H_{13}$ | |
| 668 | $C_8H_{17}O-$ | ditto | ditto | 1 | ditto | ditto | 1 | ditto | |
| 669 | $C_9H_{19}O-$ | ditto | ditto | 1 | ditto | ditto | 1 | ditto | |
| 670 | $C_{10}H_{21}O-$ | ditto | ditto | 1 | ditto | ditto | 1 | ditto | |
| 671 | $C_{11}H_{23}O-$ | ditto | ditto | 1 | ditto | ditto | 1 | ditto | |
| 672 | $C_{12}H_{23}O-$ | ditto | ditto | 1 | ditto | ditto | 1 | ditto | |
| 673 | $C_{14}H_{29}O-$ | ditto | ditto | 1 | ditto | ditto | 1 | ditto | |
| 674 | $C_{16}H_{33}O-$ | ditto | ditto | 1 | ditto | ditto | 1 | ditto | |
| 675 | $C_7H_{15}O-$ | 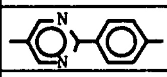 | -COO- | 1 | 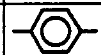 | -COO- | 1 | $-C^*H(CF_3)-C_6H_{13}$ | |
| 676 | $C_8H_{17}O-$ | ditto | ditto | 1 | ditto | ditto | 1 | ditto | |
| 677 | $C_9H_{19}O-$ | ditto | ditto | 1 | ditto | ditto | 1 | ditto | |
| 678 | $C_{10}H_{21}O-$ | ditto | ditto | 1 | ditto | ditto | 1 | ditto | |
| 679 | $C_{11}H_{23}O-$ | ditto | ditto | 1 | ditto | ditto | 1 | ditto | |
| 680 | $C_{12}H_{23}O-$ | ditto | ditto | 1 | ditto | ditto | 1 | ditto | |
| 681 | $C_{14}H_{29}O-$ | ditto | ditto | 1 | ditto | ditto | 1 | ditto | |
| 682 | $C_{16}H_{33}O-$ | ditto | ditto | 1 | ditto | ditto | 1 | ditto | |
| 683 | $C_7H_{15}O-$ | 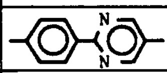 | -COO- | 1 | 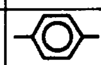 | -COO- | 1 | $-C^*H(CF_3)-C_6H_{13}$ | |
| 684 | $C_8H_{17}O-$ | ditto | ditto | 1 | ditto | ditto | 1 | ditto | |
| 685 | $C_9H_{19}O-$ | ditto | ditto | 1 | ditto | ditto | 1 | ditto | |
| 686 | $C_{10}H_{21}O-$ | ditto | ditto | 1 | ditto | ditto | 1 | ditto | |
| 687 | $C_{11}H_{23}O-$ | ditto | ditto | 1 | ditto | ditto | 1 | ditto | |
| 688 | $C_{12}H_{23}O-$ | ditto | ditto | 1 | ditto | ditto | 1 | ditto | |
| 689 | $C_{14}H_{29}O-$ | ditto | ditto | 1 | ditto | ditto | 1 | ditto | |
| 690 | $C_{16}H_{33}O-$ | ditto | ditto | 1 | ditto | ditto | 1 | ditto | |

Table 4-6

| Compound Number | R | A' | X | m | B' | Y | n | R* | Exam. No. |
|---|---|---|---|---|---|---|---|---|---|
| 691 | $C_7H_{15}O-$ | 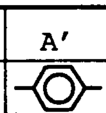 | -COO- | 1 | 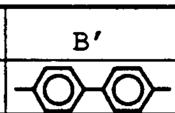 | -COO- | 1 | $-C^*H(CF_3)-C_6H_{13}$ | |
| 692 | $C_8H_{17}O-$ | ditto | ditto | 1 | ditto | ditto | 1 | ditto | |
| 693 | $C_9H_{19}O-$ | ditto | ditto | 1 | ditto | ditto | 1 | ditto | |
| 694 | $C_{10}H_{21}O-$ | ditto | ditto | 1 | ditto | ditto | 1 | ditto | |
| 695 | $C_{11}H_{23}O-$ | ditto | ditto | 1 | ditto | ditto | 1 | ditto | |
| 696 | $C_{12}H_{23}O-$ | ditto | ditto | 1 | ditto | ditto | 1 | ditto | |
| 697 | $C_{14}H_{29}O-$ | ditto | ditto | 1 | ditto | ditto | 1 | ditto | |
| 698 | $C_{16}H_{33}O-$ | ditto | ditto | 1 | ditto | ditto | 1 | ditto | |
| 699 | $C_7H_{15}O-$ | 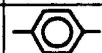 | -COO- | 1 | 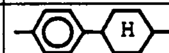 | -COO- | 1 | $-C^*H(CF_3)-C_6H_{13}$ | |
| 700 | $C_8H_{17}O-$ | ditto | ditto | 1 | ditto | ditto | 1 | ditto | |
| 701 | $C_9H_{19}O-$ | ditto | ditto | 1 | ditto | ditto | 1 | ditto | |
| 702 | $C_{10}H_{21}O-$ | ditto | ditto | 1 | ditto | ditto | 1 | ditto | |
| 703 | $C_{11}H_{23}O-$ | ditto | ditto | 1 | ditto | ditto | 1 | ditto | |
| 704 | $C_{12}H_{23}O-$ | ditto | ditto | 1 | ditto | ditto | 1 | ditto | |
| 705 | $C_{14}H_{29}O-$ | ditto | ditto | 1 | ditto | ditto | 1 | ditto | |
| 706 | $C_{16}H_{33}O-$ | ditto | ditto | 1 | ditto | ditto | 1 | ditto | |
| 707 | $C_7H_{15}O-$ | 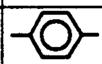 | -COO- | 1 | 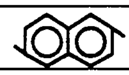 | -COO- | 1 | $-C^*H(CF_3)-C_6H_{13}$ | |
| 708 | $C_8H_{17}O-$ | ditto | ditto | 1 | ditto | ditto | 1 | ditto | |
| 709 | $C_9H_{19}O-$ | ditto | ditto | 1 | ditto | ditto | 1 | ditto | |
| 710 | $C_{10}H_{21}O-$ | ditto | ditto | 1 | ditto | ditto | 1 | ditto | |
| 711 | $C_{11}H_{23}O-$ | ditto | ditto | 1 | ditto | ditto | 1 | ditto | |
| 712 | $C_{12}H_{23}O-$ | ditto | ditto | 1 | ditto | ditto | 1 | ditto | |
| 713 | $C_{14}H_{29}O-$ | ditto | ditto | 1 | ditto | ditto | 1 | ditto | |
| 714 | $C_{16}H_{33}O-$ | ditto | ditto | 1 | ditto | ditto | 1 | ditto | |

Table 4-7

| Compound Number | R | A' | X | m | B' | Y | n | R* | Exam. No. |
|---|---|---|---|---|---|---|---|---|---|
| 715 | $C_6H_{13}$-C*H($CF_3$)O- | 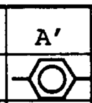 | -COO- | 2 | - | - | 0 | -C*H($CF_3$)-$C_6H_{13}$ | |
| 716 | $C_6H_{13}$-C*H($CH_3$)O- | ditto | ditto | 2 | - | - | 0 | ditto | 26 |
| 717 | $C_5H_{11}$-C*H($C_2H_5$)O- | ditto | ditto | 2 | - | - | 0 | ditto | |
| 718 | $C_6H_{13}$-C*H($C_2H_5$)O- | ditto | ditto | 2 | - | - | 0 | ditto | |
| 719 | $C_2H_5$-C*H($CH_3$)-$CH_2$O- | ditto | ditto | 2 | - | - | 0 | ditto | |
| 720 | $C_2H_5$-C*H($CH_3$)-($CH_2$)$_3$O- | ditto | ditto | 2 | - | - | 0 | ditto | |
| 721 | $C_2H_5$-OCO-$CH_2$-C*H($CF_3$)O- | ditto | ditto | 2 | - | - | 0 | ditto | |
| 722 | $C_6H_{13}$-CH($CF_3$)O- | 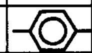 | -COO- | 2 | - | - | 0 | -C*H($CF_3$)-$C_6H_{13}$ | |
| 723 | $C_6H_{13}$-CH($CF_3$)O- | ditto | ditto | 2 | - | - | 0 | ditto | |
| 724 | $C_5H_{11}$-CH($C_2H_5$)O- | ditto | ditto | 2 | - | - | 0 | ditto | |
| 725 | $C_6H_{13}$-CH($C_2H_5$)O- | ditto | ditto | 2 | - | - | 0 | ditto | |
| 726 | $C_2H_5$-CH($CH_3$)-$CH_2$O- | ditto | ditto | 2 | - | - | 0 | ditto | |
| 727 | $C_2H_5$-CH($CH_3$)-($CH_2$)$_3$O- | ditto | ditto | 2 | - | - | 0 | ditto | |
| 728 | $C_2H_5$-OCO-$CH_2$-CH($CF_3$)O- | ditto | ditto | 2 | - | - | 0 | ditto | |
| 729 | $C_6H_{13}$-C*H($CF_3$)O- | 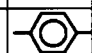 | -COO- | 2 | - | - | 0 | -C*H($CF_3$)-$C_6H_{13}$ | |
| 730 | $C_6H_{13}$-C*H($CH_3$)O- | ditto | ditto | 2 | - | - | 0 | ditto | |
| 731 | $C_5H_{11}$-C*H($C_2H_5$)O- | ditto | ditto | 2 | - | - | 0 | ditto | |
| 732 | $C_6H_{13}$-C*H($C_2H_5$)O- | ditto | ditto | 2 | - | - | 0 | ditto | |
| 733 | $C_2H_5$-C*H($CH_3$)-$CH_2$O- | ditto | ditto | 2 | - | - | 0 | ditto | |
| 734 | $C_2H_5$-C*H($CH_3$)-($CH_2$)$_3$O- | ditto | ditto | 2 | - | - | 0 | ditto | |
| 735 | $C_2H_5$-OCO-$CH_2$-C*H($CF_3$)O- | ditto | ditto | 2 | - | - | 0 | ditto | |

Table 4-8

| Compound Number | R | A' | X | m | B' | Y | n | R* | Exam. No. |
|---|---|---|---|---|---|---|---|---|---|
| 736 | $C_6H_{13}$-CH($CF_3$)O- | 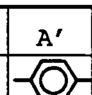 | -COO- | 2 | - | - | 0 | -C*H($CF_3$)-$C_6H_{13}$ | |
| 737 | $C_6H_{13}$-CH($CF_3$)O- | ditto | ditto | 2 | - | - | 0 | ditto | |
| 738 | $C_5H_{11}$-CH($C_2H_5$)O- | ditto | ditto | 2 | - | - | 0 | ditto | |
| 739 | $C_6H_{13}$-CH($C_2H_5$)O- | ditto | ditto | 2 | - | - | 0 | ditto | |
| 740 | $C_2H_5$-CH($CH_3$)-$CH_2$O- | ditto | ditto | 2 | - | - | 0 | ditto | |
| 741 | $C_2H_5$-CH($CH_3$)-($CH_2$)$_3$O- | ditto | ditto | 2 | - | - | 0 | ditto | |
| 742 | $C_2H_5$-OCO-$CH_2$-C*H($CF_3$)O- | ditto | ditto | 2 | - | - | 0 | ditto | |

These carboxylate compounds as mentioned above may be prepared by means of a combination of known synthesis techniques.

For example, the carboxylate compounds of the above formula [III] may be prepared according to the following synthesis route.

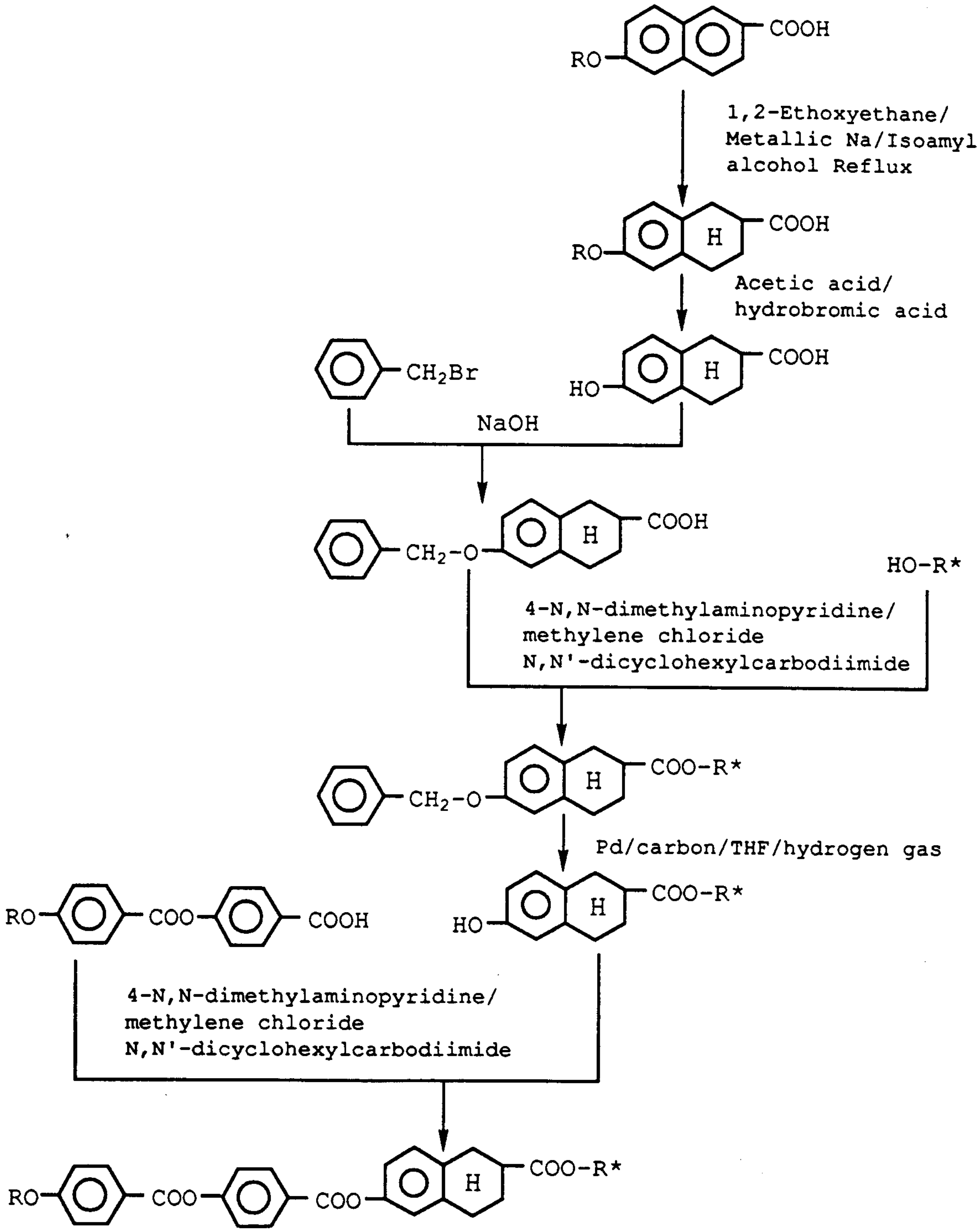


For example, a mixture of 6-n-alkoxynaphthalene-2-carboxylic acid and 1,2-ethoxyethane is refluxed in the presence of metallic sodium while adding dropwise isoamyl alcohol to obtain 1,2,3,4-tetrahydro-6-n-alkoxynaphthalene-2-carboxylic acid.

The thus obtained 1,2,3,4-tetrahydro-6-n-alkoxynaphthalene-2-carboxylic acid is reacted with acetic acid and hydrobromic acid to obtain 1,2,3,4-tetrahydro-6-hydroxynaphthalene-2-carboxylic acid.

The 1,2,3,4-tetrahydro-6-hydronaphthalene-2-carboxylic acid is reacted with benzyl bromide in the presence of potassium hydroxide to produce 1,2,3,4-tetrahydro-6-benzyloxynaphthalene-2-carboxylic acid.

Subsequently, in the presence of 4-N,N-dimethylaminopyridine and a solvent such as methylene chloride, an alcohol having an asymmetric carbon is reacted with the 1,2,3,4-tetrahydro-6-benzyloxynaphthalene-2-carboxylic acid while adding dropwise N,N-dicyclohexylcarbodiimide to the reaction mixture, thereby obtaining 1,2,3,4-tetrahydro-6-benzyloxynaphthalene-2-carboxylate.

The thus obtained 1,2,3,4-tetrahydro-6-benzyloxynaphthalene-2-carboxylate in a solvent such as tetrahydrofuran is reduced with hydrogen gas in the presence of a reducing catalyst such as a palladium/carbon to obtain 1,2,3,4-tetrahydro-6-hydroxynaphthalene-2-carboxylate.

Then, 4-(4'-alkoxybenzoyloxy)benzoic acid prepared separately is reacted with the 1,2,3,4-tetrahydro-6-hydroxynaphthalene-2-carboxylate obtained in the above step in the presence of 4-N,N-dimethylaminopyridine and a solvent such as methylene chloride while adding dropwise N,N'-dicyclohexylcarbodiimide, whereby the above-mentioned carboxylate compound may be obtained.

These processes for the preparation of the carboxylate compounds of the invention are provided only by way of non-limiting illustration.

Among the carboxylate compounds of the invention prepared by the above-mentioned processes, the $^1$H-NMR of 6-[4'-(4''-decyloxybenzoyloxy)benzoyloxy]-1,2,3,4-tetrahydronaphthalene-2-carboxylic acid R-1'''-trifluoromethylheptyl ester [Exemplified compound (404)] represented by the following formula is shown in Fig. 13.

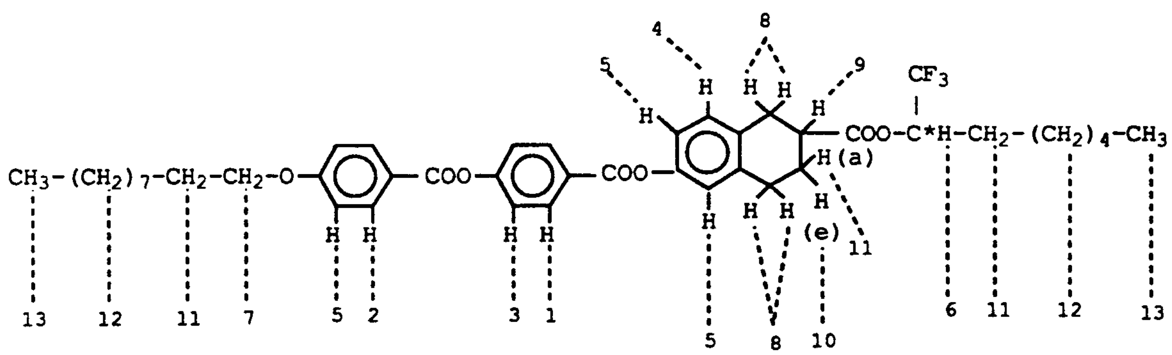

In this formula, (e) represents an equatorical steric conformation, (a) represents an axial steric conformation, and the numbers 1 to 13 show hydrogen atoms corresponding to the peaks in Fig. 13.

Further, the $^1$H-NMR of 6-[4'-(4''-(R-1'''-methylheptyloxy)benzoyloxy)benzoyloxy]-1,2,3,4-tetrahydronaphthalene-2-carboxylic acid R-1'''-trifluoromethyl-heptyl ester [Exemplified compound (545)] represented by the following formula is shown in Fig. 14.

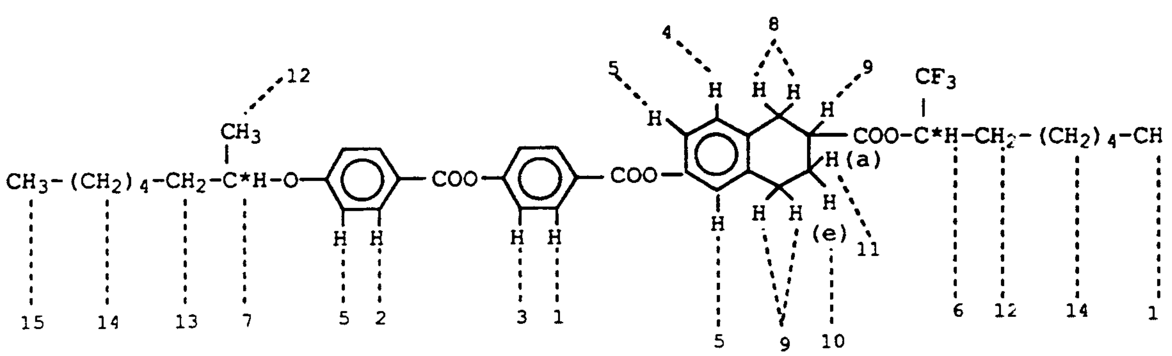

In this formula, the numbers 1 to 15 show hydrogen atoms corresponding to the peaks in Fig. 14.

Among the carboxylate compounds of formula [IV] of the invention, the $^1$H-NMR of 6-[4'-(4''-decyloxybenzoyloxy)benzoyloxy]-5,6,7,8-tetrahydronaphthalene-2-carboxylic acid R-1'''-trifluoromethylheptyl ester [Exemplified compound (575)] represented by the following formula is shown in Fig. 15.

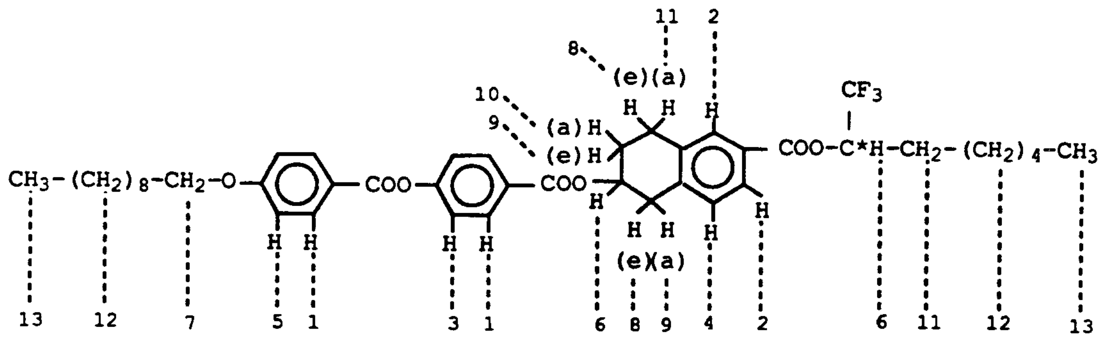

In this formula, the numbers 1 to 13 show hydrogen atoms corresponding to the peaks in Fig. 15.

Further, the $^1$H-NMR of 6-[4'-(4''-(R-1'''-methylheptyloxy)benzoyloxy)benzoyloxy]-5,6,7,8-tetrahydronaphthalene-2-carboxylic acid R-1''''-trifluoromethylheptyl ester [Exemplified compound (716)] represented by the following formula is shown in Fig. 16.

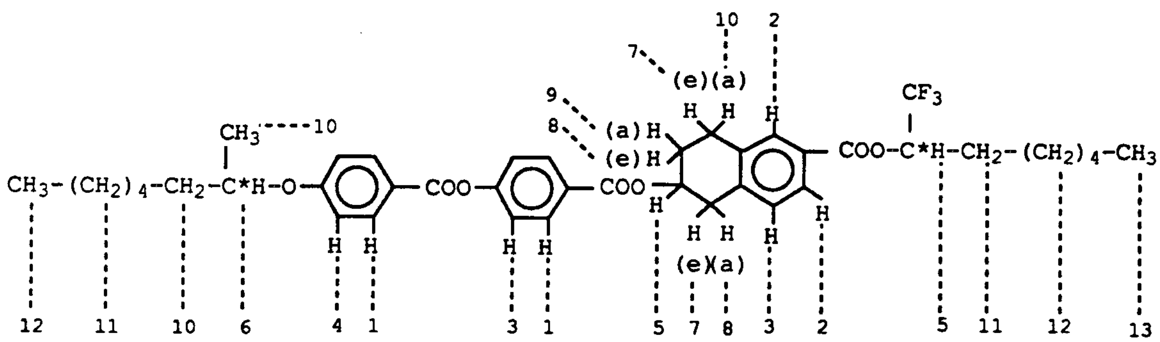

In this formula, the numbers 1 to 13 show hydrogen atoms corresponding to the peaks in Fig. 16.

Figs. 23-34 show the $^1$H-NMR spectra of other compounds according to the present invention.

Figs. 23:

6-(4'-decyloxybenzoyloxy)-1,2,3,4-tetrahydronaphthalene-2-carboxylic acid R-1''-trifluoromethylheptyl ester [Compound (235)] represented by the following formula

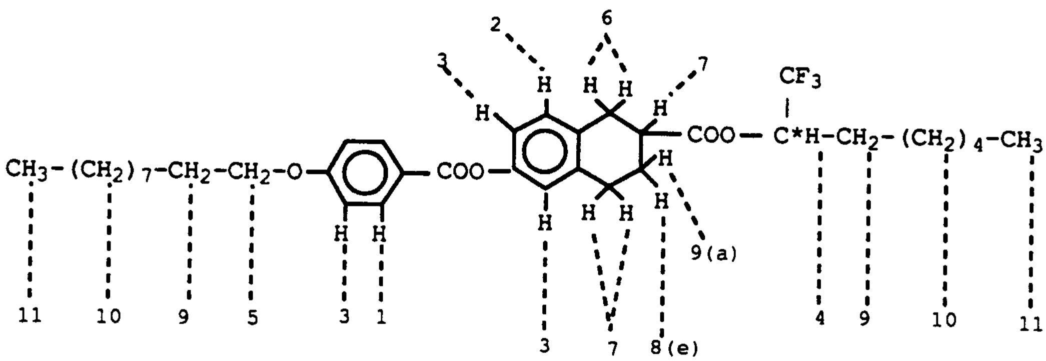

Fig. 24:

Fig. 24:

6-[4'-(4''-undecyloxybiphenyl)carboxy]-1,2,3,4-tetrahydronaphthalene-2-carboxylic acid R-1'''-trifluoromethylheptyl ester [Compound (252)] represented by the following formula

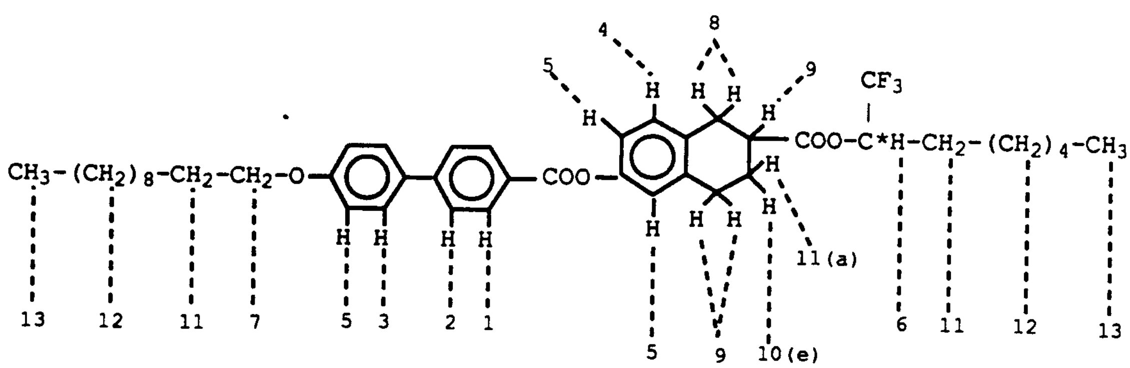

Fig. 25:

Fig. 25:

6-[4'-(4''-dodecyloxybiphenyl)carboxy]-1,2,3,4-tetrahydronaphthalene-2-carboxylic acid R-1'''-trifluoromethylheptyl ester [Compound (253)] represented by the following formula

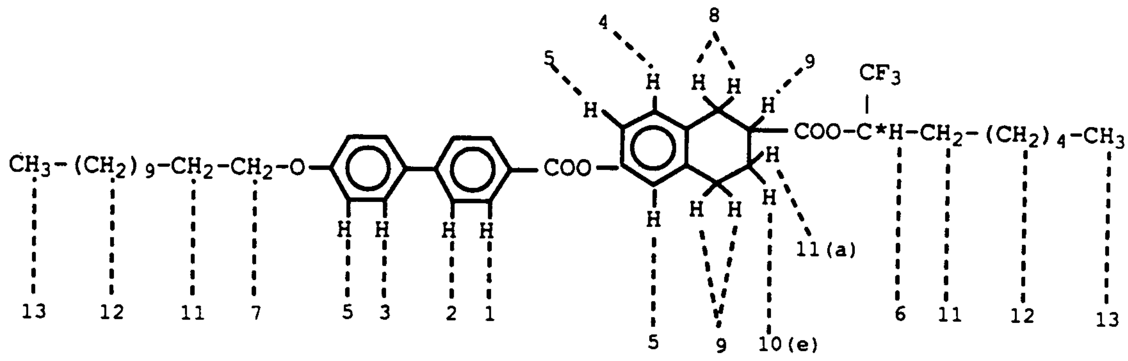

Fig. 26:

Fig. 26:

6-[4'-(4"-octylbiphenyl)carboxy]-1,2,3,4-tetrahydronaphthalene-2-carboxylic acid R-1'''-trifluoromethylheptyl ester represented by the following formula

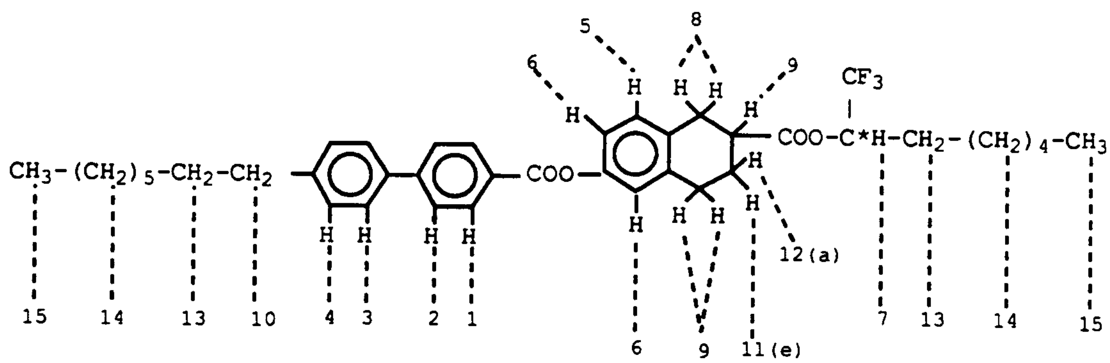

Fig. 27:

Fig. 27:

6-[4'-(4"-decylbiphenyl)carboxy]-1,2,3,4-tetrahydronaphthalene-2-carboxylic acid R-1'''-trifluoromethylheptyl ester represented by the following formula

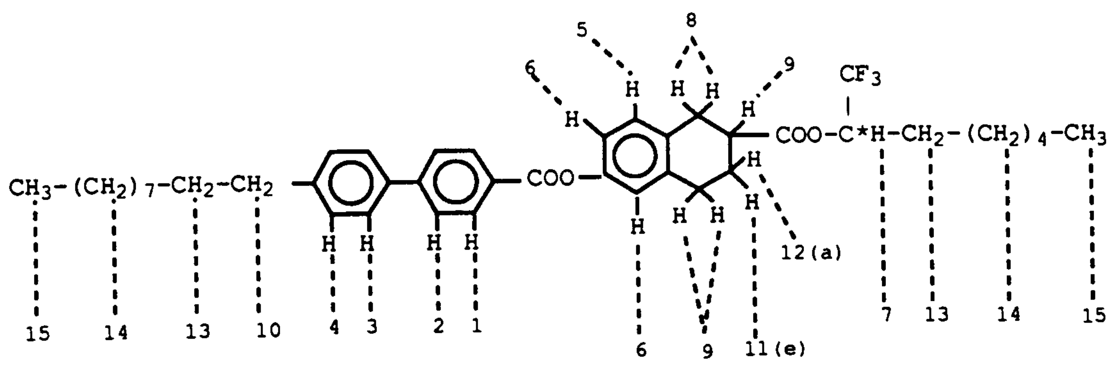

Fig. 28:

Fig. 28:

6-[4'-(4"-dodecylbiphenyl)carboxy]-1,2,3,4-tetrahydronaphthalene-2-carboxylic acid R-1‴-trifluoromethylheptyl ester represented by the following formula

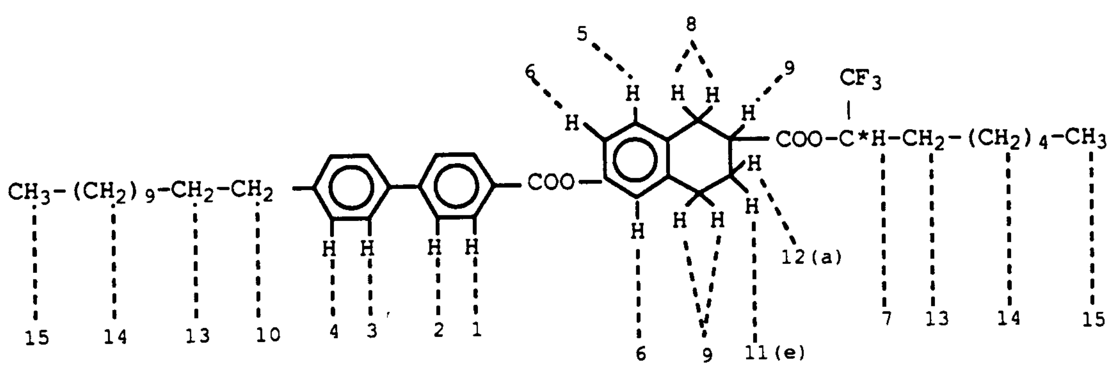

Fig. 29:

Fig. 29:

6-[4'-(4"-octylbiphenyl)carboxy]-1,2,3,4-tetrahydronaphthalene-2-carboxylic acid R-1‴-methylheptyl ester represented by the following formula

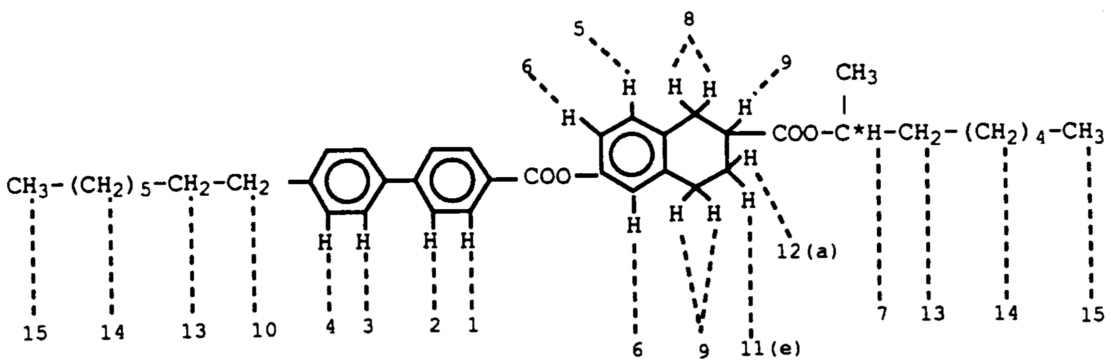

Fig. 30:

Fig. 30:

6-[4'-(4"-decylbiphenyl)carboxy]-1,2,3,4-tetrahydronaphthalene-2-carboxylic acid R-1‴-methylheptyl ester represented by the following formula

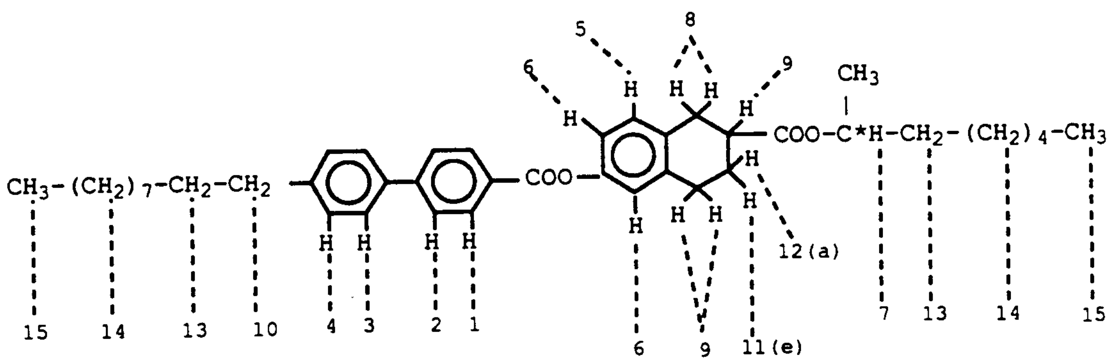

Fig. 31:

Fig. 31:

6-[4'-(4"-(S-1‴-methylheptyloxy)biphenyl)carboxy]-1,2,3,4-tetrahydronaphthalene-2-carboxylic acid R-1''''-methylheptyl ester represented by the following formula

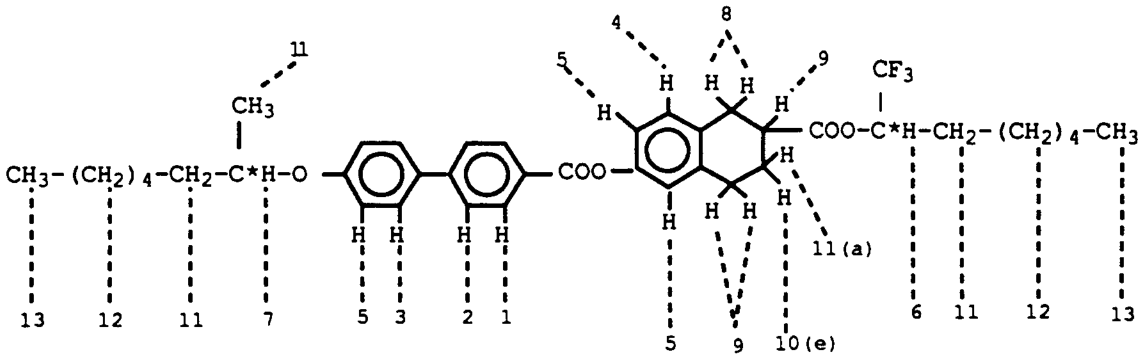

Fig. 32:

Fig. 32:

6-[4'-(4"-octyloxycyclohexyl)carboxy]-1,2,3,4-tetrahydronaphthalene-2-carboxylic acid R-1'''-trifluoromethylheptyl ester [Compound (265)] represented by the following formula

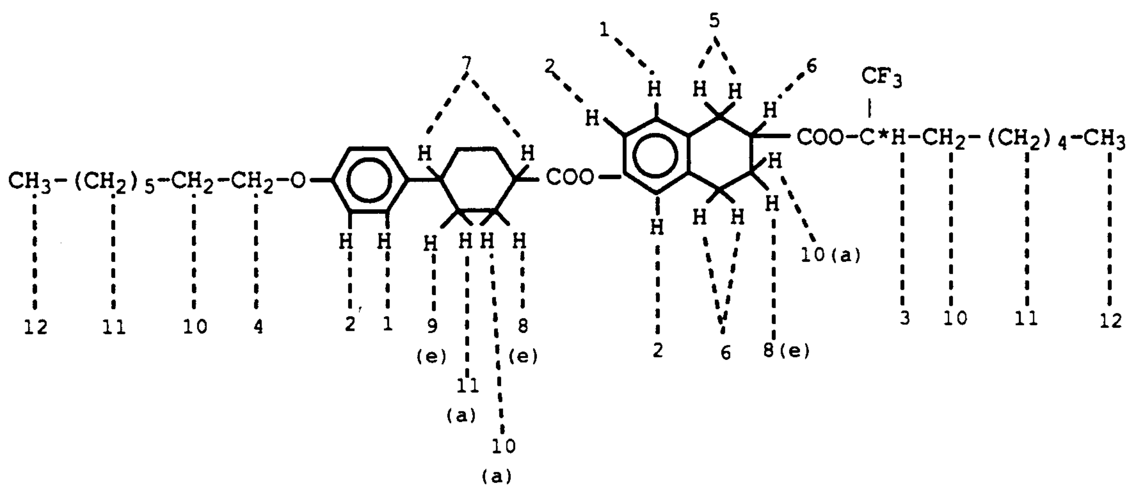

Fig. 33:

Fig. 33:

6-[4'-(4"-undecyloxyphenylcyclohexyl)carboxy]-1,2,3,4-tetrahydronaphthalene-2-carboxylic acid R-1'''-trifluoromethylheptyl ester [Compound (269)] represented by the following formula

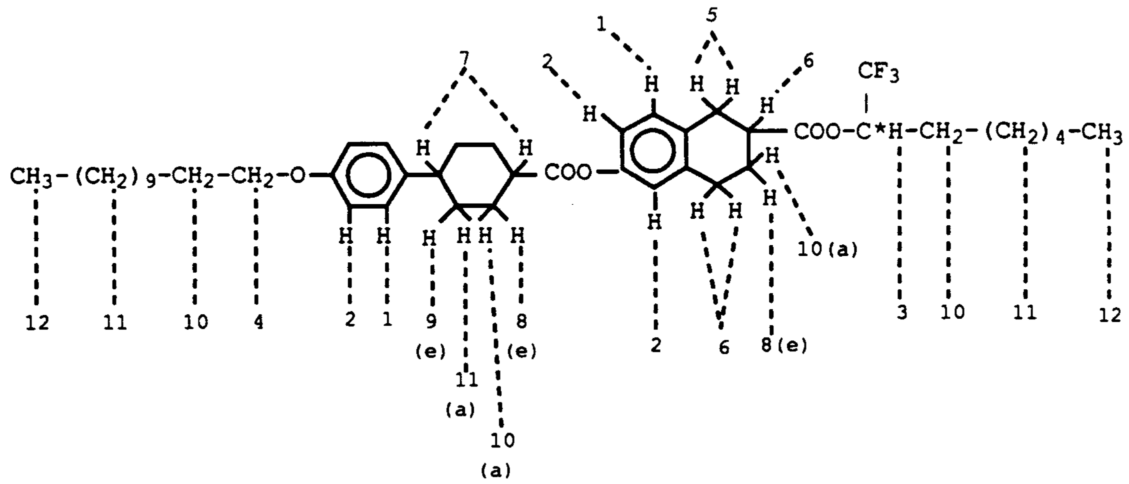

Fig. 34:

Fig. 34:

6-[4'-(4''-decylphenyltranscyclohexyl)carboxy]-1,2,3,4-tetrahydronaphthalene-2-carboxylic acid R-1'''-trifluoromethylheptyl ester represented by the following formula

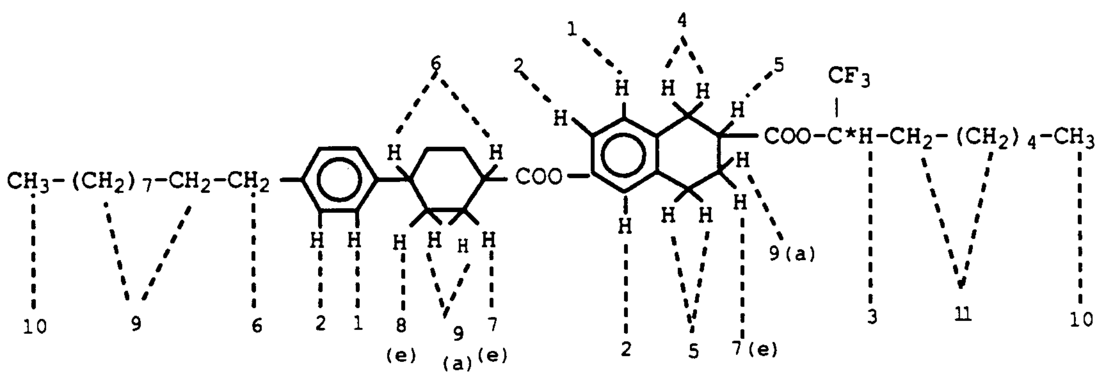

The carboxylate compounds represented by the above-mentioned formula [I], [II], [III] or [IV] may be used, for example, as liquid crystal materials.

Particularly, the carboxylate compounds of the invention can be used as ferroelectric liquid crystal compounds or antiferroelectric liquid crystal compounds

Of the carboxylate compounds of the above formula [I], the following compounds [4], [21], [22] and [130] exhibit particularly excellent liquid crystal properties.

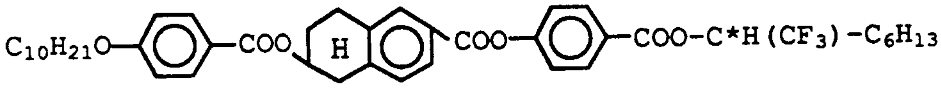

... [4]

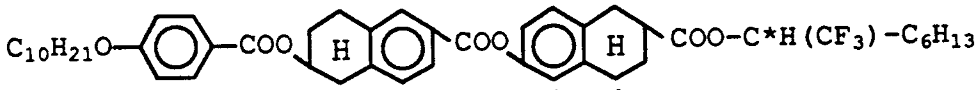

... [21]

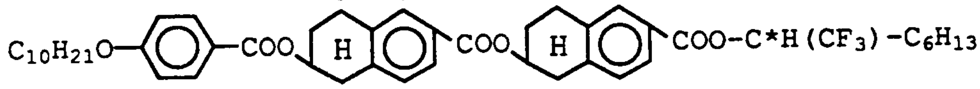

... [22]

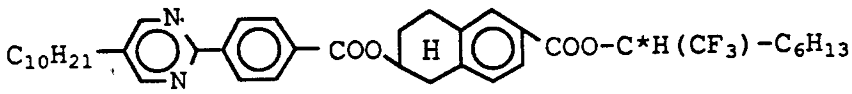

... [130]

The phase transition temperatures of these compounds [4], [21], [22] and [130] are shown in Table 5, wherein Cry represents a crystal phase, SmX represents an unidentified smectic phase, $SmC_A$* represents an antiferroelectric phase, SmA represents a smectic A phase and Iso represents an isotropic liquid.

Table 5

| Compound No. | Cry-SmX or $SmC_A$* or Iso | SmX-$SmC_A$* (4) $SmC_A$*-SmA(21) | $SmC_A$* or SmA-Iso |
|---|---|---|---|
| (4) | 21°C | 49°C | 61°C |
| (21) | 43°C | 47°C | 72°C |
| (22) | 43°C | - | - |
| (130) | 54°C | - | - |

Furthermore, of the carboxylate compounds of the above formula [II], the following compounds [204], [221], [222], [251], [267], [288], [296], [299] and [331] exhibit particularly excellent liquid crystal characteristics.

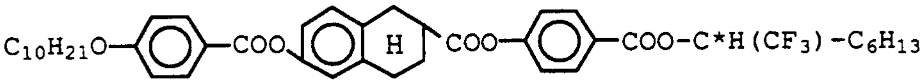 ... [204]

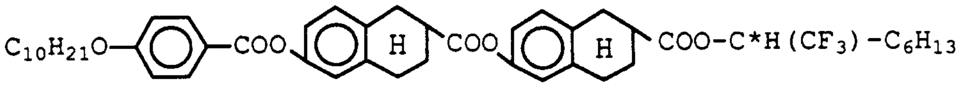 ... [221]

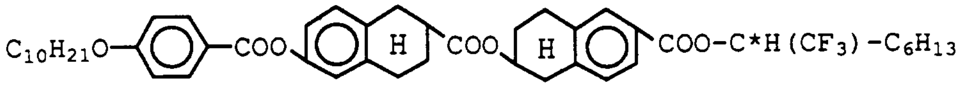 ... [222]

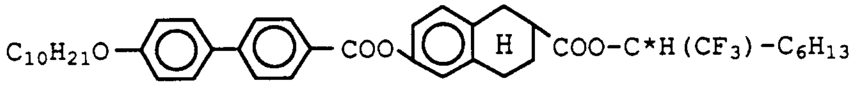 ... [251]

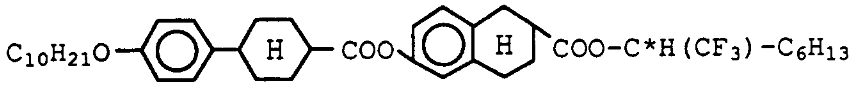 ... [267]

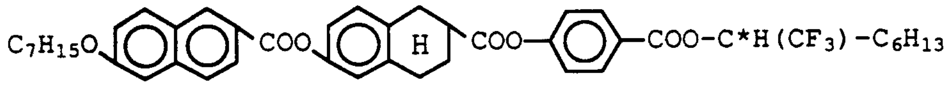 ... [288]

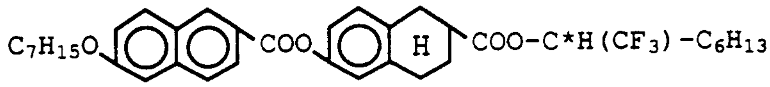 ... [296]

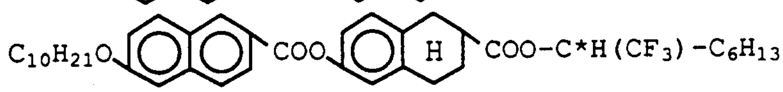 ... [299]

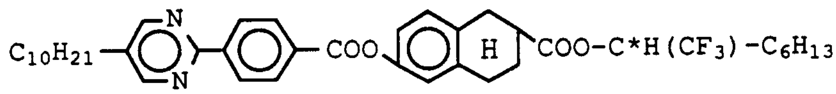 ... [331]

The phase transition temperatures of the compounds [204], [221], [222], [251], [267], [288], [296], [299] and [331] are shown in Table 6

Table 6

| Compound No. | Cry-$SmC_A$* or SmC* or SmA | $SmC_A$*-SmA SmC*-SmA (222) | SmA-Iso |
|---|---|---|---|
| [204] | -23°C | 81°C | 113°C |
| [221] | 46°C | 71°C | 109°C |
| [222] | 30°C | 36°C | 52°C |
| [251] | 21°C | 99°C | 130°C |
| [267] | -5°C | 44°C | 87°C |
| [288] | <-30°C | 42°C | 165°C |
| [296] | 80°C | - | - |
| [299] | 44°C | - | 50°C |
| [331] | 48°C | - | 82°C |

Of the carboxylate compounds represented by the above formula [III] or [IV], the following compounds [404], [545], [575] and [716] exhibit particularly excellent liquid crystal characteristics.

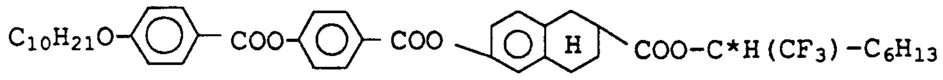 ... [404]

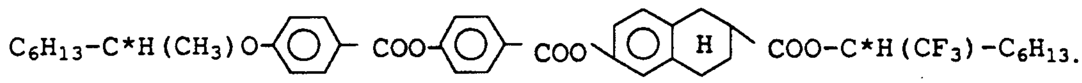 [545]

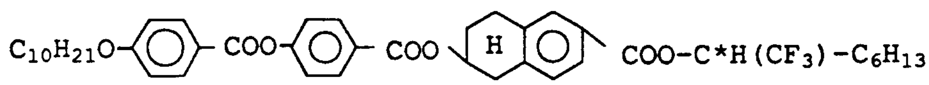 ... [575]

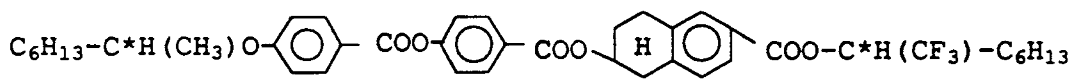 [716]

The phase transition temperatures of the compounds [404], [545], [575] and [716] are shown in Table 7.

Table 7

| Compound No. | Cry-SmC$_A$* or SmA or Iso | SmC$_A$*-SmC* | SmC$_A$* or SmC* or Iso | SmA-Iso |
|---|---|---|---|---|
| (404) | 50°C | 77°C | 85°C | 122°C |
| (545) | 45°C | - | - | - |
| (575) | 23°C | - | 57°C | - |
| (716) | -15°C | - | - | - |

In the liquid crystal materials of the invention, there are many compounds exhibiting a smectic phase over a broad temperature range, as shown in Tables 5 to 7.

There have been known few liquid crystal compounds which, if used alone as a liquid crystal material, exhibit a smectic phase over such a broad temperature range as in the above-mentioned compounds.

In addition to the fact that the liquid crystal materials of the invention exhibit a smectic phase in a broad temperature range, optical switching elements prepared by using said materials have an excellent high speed response.

The liquid crystal materials of the invention may be used either singly or in a mixture with other liquid crystal materials in the form of liquid crystal compositions. For example, the liquid crystal materials of the invention may be used as a principal component of antiferrodielectric liquid crystal compositions or as an assistant of liquid crystal compositions which contain other compounds exhibiting a smectic phase as a principal component. That is, the carboxylate compounds of the invention exhibiting a smectic phase may be used as a principal component of liquid crystal compositions or as an assistant of liquid crystal compositions which contain other liquid crystal materials as a principal component, while the carboxylate compounds which do not exhibit a smectic phase may be used as an assistant of the latter liquid crystal compositions.

Examples of known liquid crystal compounds which may be used along with the compounds of the invention of formulae [I] to [IV] include:

(+)-4'-(2"-methylbutyloxy)phenyl-6-octyloxynaphthalene-2-carboxylate,
4'-decyloxyphenyl-6-((+)-2"-methylbutyloxy)naphthalene-2-carboxylate,

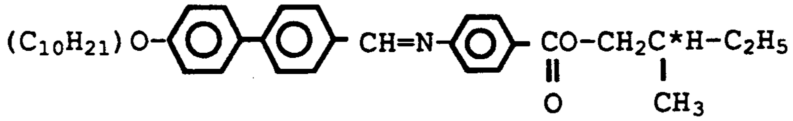,

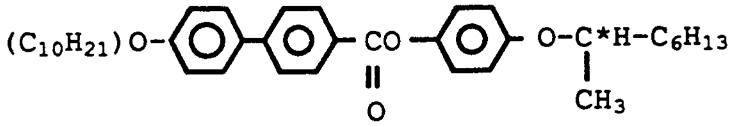 and

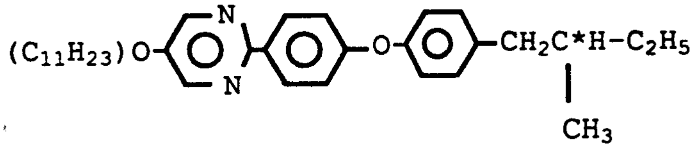

In addition to the above, there may be mentioned compounds having a cyclic system and optical activity, for example:

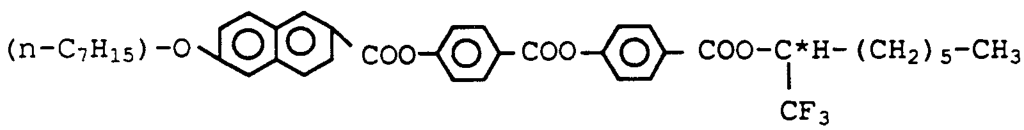

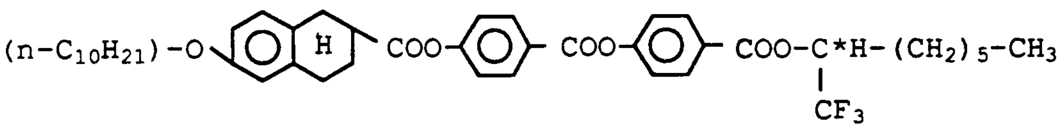

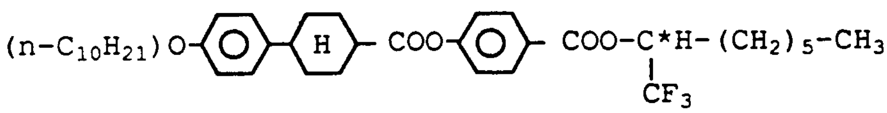

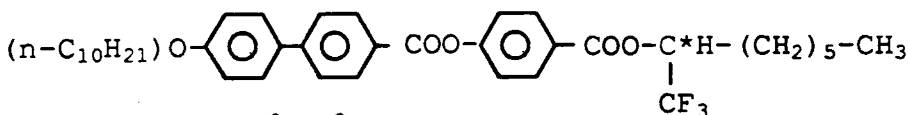

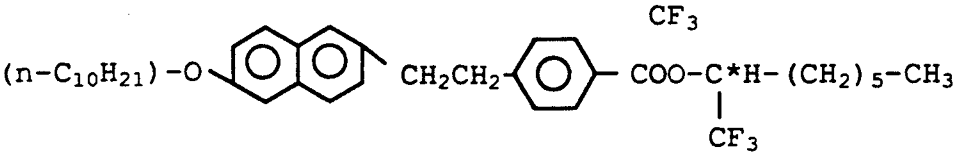

Further, there also may be mentioned Schiff base type liquid crystal compounds such as

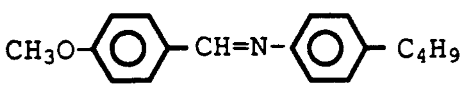

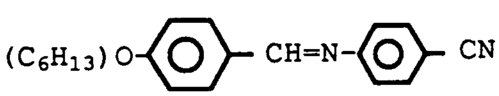

azoxy type liquid crystal compounds such as

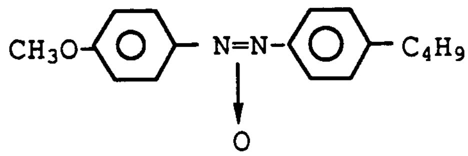

benzoate type liquid crystal compounds such as

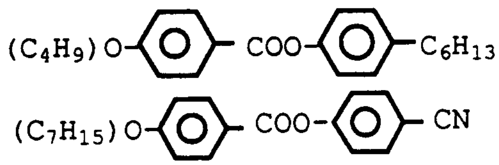

cyclohexylcarboxylate type liquid crystal compounds such as

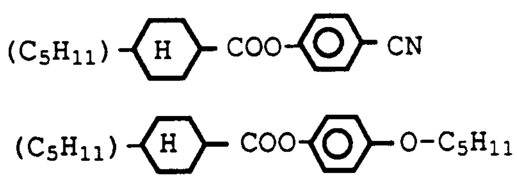

phenyl type liquid crystal compounds such as

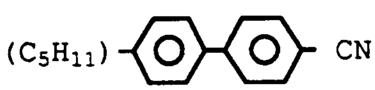

terphenol type liquid crystal compounds such as

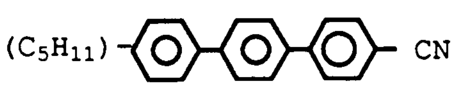

cyclohexyl type liquid crystal compounds such as

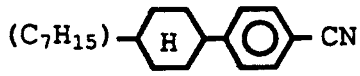

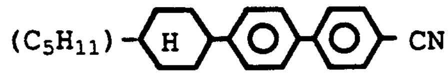

and pyrimidine type liquid crystal compound such as

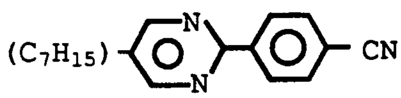

The liquid crystal compositions of the invention contain the carboxylate compounds of formulae [I] to [IV] and other compounds including the compounds exemplified above.

The amount of the carboxylate compounds of formulae [I] to [IV] in the liquid crystal composition may be selected depending on the characteristics of the resulting composition. The composition of the invention usually contains the carboxylate compound of the formulae [I] to [IV] in an amount of 1-99 parts by weight, preferably 5-75 parts by weight, based on 100 parts by weight of total amount of the liquid crystal present in the composition.

In addition to the liquid crystal material mentioned above, the liquid crystal composition may further contain additives conventionally used in liquid crystal compositions, for example electrical conductivity-imparting agents and life-improving agents.

The liquid crystal composition of the invention may be prepared by mixing the above-mentioned carboxylate compound and, if desired, other liquid crystal compounds and additives.

When a voltage is applied to the liquid crystal composition (liquid crystal substance) containing the above-mentioned liquid crystal material, an optical switching phenomenon occurs. Hence a display device exhibiting a good response due to this phenomenon can be produced. In the invention, with regard to the elements utilizing such phenomenon or the method of driving such elements, reference may be made, for example to Japanese Patent L-O-P Publns. Nos. 107216/1981 and 118744/1984.

The liquid crystal substance that may be used in the display device referred to above may include compounds exhibiting any of a smectic C phase, smectic F phase, smectic G phase, smectic H phase, smectic I phase, smectic J phase and smectic K phase. Because the display devices using the liquid crystal substances other than those exhibiting the smectic C phase have in general a low response speed, it has heretofore been considered that it is effective to drive the display device by means of the liquid crystal substance exhibiting the smectic C phase having a high speed response.

However, it have been found that it is possible in the invention to use advantageously the liquid crystal substances exhibiting not only a smectic C phase but also a smectic A phase by a method where the display device is driven by means of the liquid crystal substance exhibiting a smectic A phase as proposed by the present inventors in Japanese Patent L-O-P Publn. No. 3632/1989. That is, by virtue of the utilization of this driving method, the liquid crystal elements of the invention may be driven in a wide phase range and, at the same time, it is possible to speed up the electro-optical response.

The liquid crystal element of the present invention is composed of a cell filled with a liquid crystal substance, and polarizing plates. For example, the liquid crystal element of the invention, as shown in Fig. 17, may be formed from a cell 13 comprising two transparent substrates 11a and 11b arranged so as to form a gap 14 to be filled with a liquid crystal substance 12, and transparent electrodes 15a and 15b formed on the surfaces of the transparent substrates 11a and 11b, said surfaces individually facing the liquid crystal substance 12, the liquid crystal substance 12 charged in the gap 14 of the cell 13, and polarizing plates (not shown) arranged outside at both sides of the cell 13.

Examples of the substrate are glass and transparent polymer plates. When glass substrates are used, in order to prevent deterioration of the liquid crystal substance owing to elution of alkali components of the glass, the glass surfaces may be provided, for example, with an undercoat layer (layer for shielding or preventing unnecessary components) comprising silicon oxide as an essential ingredient. The transparent substrates, for example glass substrates, usually have a thickness of 0.01-1.0 mm.

Flexible transparent substrates may also be used as the transparent substrates. In this case, at least one of the substrates may be a flexible transparent substrate, or both substrates may be flexible transparent substrates. Polymer films are useful as such flexible transparent substrates. When the flexible transparent substrates are used as the transparent substrates in the invention, it is preferable that the thickness t (mm) of said flexible transparent substrates, the modulus of elasticity E ($kgf/m^2$) and the width a (mm) of the gap formed in the cell have the relationship:

$$\frac{a^4}{Et^3} < 0.32$$

The transparent substrates mentioned above are provided on the surfaces of the transparent electrodes. The transparent electrodes may be formed, for example, by coating the transparent substrate surfaces with iridium oxide or tin oxide. The transparent electrodes may be prepared by a known method. The thickness of the transparent electrodes is usually 100 to 2,000 Å.

The transparent substrates having such transparent electrodes may further be provided on the surfaces of the transparent electrodes with an orientation layer or a ferroelectric material layer. The orientation layer may include, for example, an organic film and inorganic film formed by chemical adsorption of an organic silane coupling agent or a carboxylic acid polynuclear complex thereon. Examples of the organic film include films of polymers such as polyethylene, polypropylene, polyester, polyamide, polyvinyl alcohol and polyimide. The organic films may be formed by techniques such as coating, adhesion, vacuum deposition or polymerization (e.g. plasma polymerization) on the substrate.

Examples of the inorganic film may include films of oxides such as silicon oxide, germanium oxide and alumina, films of nitrides such as silicon nitride, and films of other semi-conductors. The inorganic films may be formed by techniques such as vacuum deposition (e.g. rhombic vacuum deposition) and sputtering.

The films mentioned above may be imparted with orientation by imparting anisotropism or form specificity to the film itself during the film formation, or imparting externally orientation to the film after the film formation. Specifically, there may be mentioned a method in which the film is formed by coating a polymer such as a polyimide resin on the transparent electrode, followed by rubbing the film in a definite direction; a method in which a polymer film is subjected to stretching to impart orientation to the stretched film; and a method in which an oxide is subjected to rhombic vacuum depositing to thereby form the oriented oxide film.

Such a film (for example an orientation layer) may be so formed that it may serve simultaneously as a spacer, as mentioned below.

Two transparent substrates as mentioned above are arranged so that the transparent electrodes formed on the substrates face each other and a gap to be filled with a liquid crystal is formed therebetween. The gap usually has a width of 1-10 μm, preferably 1-5 μm. Such gap may be formed, for example, by arranging the two substrates with a spacer between them. A spacer may be, for example, a polyimide polymer obtained, for example, by patterning a photosensitive polyimide precursor. By the use of the spacer, a monodomain is formed by the interfacial effect between the spacer and the liquid crystal.

As shown in Fig. 18 (a), and Fig. 18 (b) showing an A-A cross section of (a), integration of the orientation film with the spacer may be made, for example, by using a concentric spacer 26 which acts as an orientation film. In Figs. 18 (a) and (b), 27 is the transparent substrates, 25 is the transparent electrodes, and 23 is a liquid crystal substance.

As shown in Fig. 19 (a), and Fig. 19 (b) showing an A-A cross section of (a), integration of the orientation film with the spacer may be made, for example, by using a comb-like spacer 36 which acts as an orientation film. In Figs. 19 (a) and (b), 37 is the transparent substrates, 35 is the transparent electrodes, and 33 is a liquid crystal substance.

Instead of the above-mentioned spacers, fibers 46 may also be incorporated into a liquid crystal substance 43 in the manner as shown in Fig. 20, where the fibers 46 hold transparent substrates 47 bearing transparent electrodes 45 so as to form a definite gap between them.

Preferably, the fibers have an average diameter and average length represented by the following formula:

$$3 \leq \frac{q}{d} \leq 100$$

wherein d is an average diameter of the fiber, and q is an average length of the fiber. The fibers are preferably obtained by spinning, particularly alkali glass.

It is also possible to incorporate particulate materials into the liquid crystal substance in place of or in combination with said fibers.

The particulate materials include those of melamine resin, urea resin or benzoguanamine resin having a particle diameter of 1-10 μm.

The two transparent substrates arranged so as to form the gap in the manner mentioned above are combined together by sealing their periphery with a sealer. Examples of the sealer are epoxy resin and silicone resin. The epoxy

resin, for example, used as the sealer may be modified with acrylic materials or silicone rubbers.

The gap of the liquid crystal cell having a structure such as mentioned above is filled with the liquid crystal substance containing the carboxylate compound of formula [I], [II], [III] or [IV].

The liquid crystal substance thus filled in the gap of the liquid crystal cell may be oriented, for example, by utilizing a monoaxial orientation control method such as a temperature gadient method utilizing a spacer edge or a surface treatment method using an orientation film. In the invention, moreover, it is possible to carry out the initial orientation of the liquid crystal substance, for example, by applying a direct bias voltage to the liquid crystal substance while heating said substance.

The liquid crystal cell filled with the liquid crystal substance and subjected to initial orientation in the manner mentioned above is arranged between two polarizing plates. As shown in Fig. 21, two or more cells 58 comprising two transparent substrates 57, two transparent electrodes 55 and the liquid crystal substance 53 may also be disposed between two polarizing plates 56.

In the liquid crystal element of the invention, two polarizing plates may be disposed so that the directions of polarized light passed through the plates cross at an angle of 70-110°, preferably at right angles, i.e., 90°.

Example of such polarizing plates are polarizing films which may be obtained by stretching a resin film such as a polyvinyl alcohol resin film or polyvinyl butyral resin film in the presence of, for example, iodine, thereby absorbing the iodine thereinto, imparting polarizability. The polarizing films may have a multi-layer construction by coating their surface with, for example, another resin.

In the present invention, the above-mentioned liquid crystal cell may be disposed between two polarizing plates disposed in the manner mentioned above so that a cross angle of two polarized directions of light passed through the polarizing plates is within ± 10° based on the cross angle corresponding to the darkest state where the amount of light passed through the plates is the least, preferably the darkest state is attained. The liquid crystal cell may also be disposed between the polarized plates so that a cross angle of two polarized directions of light passed through the polarized plates is within ± 10° based on the brightest state where the amount of light passed through the plates is the most, preferably the brightest state is attained.

The liquid crystal element of the invention may be prepared, as shown in Fig. 21, by filling the gap 14 of the cell 13 with the liquid crystal substance 15 mentioned above, and subjecting said crystal substance 15 to initial orientation.

The liquid crystal substance 15 is usually heated until it reaches a molten state, and the molten substance is injected into the vacuumized gap 14 of the cell 13 through an inlet provided in the cell. After the injection operation, the inlet is sealed.

After sealing the inlet, the cell 13 is heated to a temperature higher than the temperature at which the liquid crystal substance 15 filled in the cell 13 exhibits an isotropic phase, and then the cell is cooled to a temperature at which the liquid crystal substance 15 is in a liquid crystal state.

The cooling is preferably carried out at a rate of lower than 2°C/min, more preferably 0.1-2.0°C/min and particularly 0.1-0.5°C/min. By cooling the cell 13 at such a cooling rate, the state of initial orientation of the liquid crystal substance 15 is improved, and thus it is possible to form a liquid crystal element having a liquid crystal phase consisting of a monodomain with less orientation defects. The initial orientation referred to herein implies the state of arrangement of the liquid crystal substance prior to changing the orientation vector by applying, for example, a voltage.

In comparison with the prior art liquid crystal elements, the liquid crystal elements of the invention prepared in the manner as mentioned above have markedly excellent characteristics such as contrast, and are suitable for use, for example, as surface stabilized ferroelectric liquid crystal elements, helical modulation elements, excess scattering type elements, guest-host type elements and vertical orientation liquid crystal elements.

For instance, the liquid crystal element of the invention may be driven by application of an electric field, usually having a frequency controlled to 1 Hz-100 kHz, preferably 10Hz-10kHz, and a voltage of usually 0.01-60 Vp-p/$\mu m^t$ (voltage per thickness of 1 $\mu$m), preferably 0.05-30 Vp-p/$\mu m^t$.

When the liquid crystal element of the invention is driven by application of an electric field, two kinds of hysteresis curves are drawn, depending on the light volume passing through the liquid crystal element, by changing the width of the wave (driving wave) of the electric field applied. One of the procedures for describing two kinds of the hysteresis curves is a driving method in which a so-called bi-stability type is utilized, and the other is a driving method in which a so-called tri-stability type is utilized.

The liquid crystal element of the invention in which a liquid cell filled with an optically active liquid crystal substance is disposed between two polarizing plates so that the polarizing planes cross at right angles and the darkest state is attained when no electric field is applied, may be driven, for example, by application of an electric field of any waveform having a frequency of 50 Hz-100 kHz, preferably 70 Hz-10 kHz, such as a rectangular wave (or pulse wave), triangular wave, sine wave or a combination thereof. For example, when an electric field of a rectangular wave or a pulse wave or a combination thereof is applied, the driving speed of the liquid crystal element can be increased by employing a width of the electric field of not more than 10 millisec., preferably 0.01-10 millisec. In this range, the liquid crystal element of the invention may be used as a bi-stability type liquid crystal element. On the other hand, by employing a width of the electric field of larger than 10 millisec., preferably 33-1,000 millisec., the liquid crystal element of the invention may be

used a tri-stability type liquid crystal element in the region where not so high driving speed is required.

"Width of the electric field" used herein means, in the electric field of a rectangular wave, for example, the time span for which a designated voltage is maintained.

By using the liquid crystal elements of the invention, there may be manufactured various liquid crystal display devices and electro-optical display devices. Of the liquid crystal elements of the invention, those filled with liquid crystal materials exhibiting a smectic phase may be used for manufacturing memory-type liquid crystal display devices or electro-optical display devices using, for example, a thermal-write or laser-write type liquid display element. Further, in addition to the above-mentioned uses, by the use of liquid crystal materials containing carboxylate compounds having a ferroelectricity, there can be manufactured liquid crystal display devices or electro-optical display devices using, for example, light switching elements of a light shutter or liquid crystal printer, piezoelectric element and pyroelectric element.

That is, the liquid crystal materials of the invention exhibit tri-stability or bi-stability, and hence the liquid crystal elements of the invention have light switching or display function by inverting the electric field so as to accomplish the bistable state.

The liquid crystal substance exhibiting bi-stability used in the invention has a spontaneous polarization, and hence when a voltage is applied once, the liquid crystal substance has a memory effect even after elimination of the electric field. It is not necessary to apply continuously the electric field to the liquid crystal element in order to keep this memory effect. Thus in the display device using the liquid crystal element of the invention, the consumption of electric power can be reduced. Moreover, the display device is very clear because of its stable contrast.

Further, in the switching element in the invention using the liquid crystal material, it is possible to perform a switching operation only by changing the direction of orientation of the molecule. In this case, the first order of the field strength acts on the driving of the switching element, and hence the switching element of the invention can be driven at a low voltage.

By using this switching element, it is possible to attain a high speed response of not more than several 10 $\mu$ seconds, and hence the operating time of the element can be shortened sharply. Accordingly, a display (liquid crystal display device) having a large numbers of scanning lines and a large screen can easily be manufactured by using the liquid crystal element of the invention. Further, in the case of the liquid crystal substance exhibiting tri-stability, its memorizing properties can also be maintained. Moreover, this display can be driven without using an auxiliary means for controlling the driving temperature, because it can be driven at room temperature or lower.

Further, in the liquid crystal substance used in the invention, the molecule inclines inducibly when an electric field is applied thereto even in a smectic A phase which has been generally considered not to exhibit a bi-stability, and hence light switching can be performed in this phase by utilizing such properties of the liquid crystal substance. That is, when ferroelectric liquid crystal compounds are used, a practical response speed cannot be attained, and the smectic A phase thereof is not generally used. However it is possible to drive the display device of the invention by utilizing the driving method and apparatus proposed by the present inventors in EP-A-332,456 (1989). Further, the liquid crystal substance used in the invention exhibits two or more stable states even in the smectic F phase which is higher in order than the smectic C phase, and hence the light switching can be performed in the same manner as above utilizing a plurality of stable states in these phases.

The display device using the liquid crystal element of the invention may be driven by various methods, only a few of which are illustrated as follows.

The first method comprises interposing the liquid crystal element of the invention between two polarizing plates, applying an external voltage to the liquid crystal element to change the orientation vector of the liquid crystal substance filled in the element, and thereby performing the display by double refraction between the polarizing plates and the liquid crystal substance.

The second method is to utilize the dichroism of a a dichromic dye (or pigment) incorporated in a liquid crystal substance. Change of the orientation direction of the liquid crystal compound causes a change in the wavelength of light absorbed by the dye or pigment. The dye or pigment which may be used in this case has a dichromatic property and includes, for example, azo dyes, naphthoquinone dyes, cyanine dyes and anthraquinone dyes.

The display device prepared using the liquid crystal element of the invention may be driven by the electric address display system, light address display system, heat address display system and light beam display system. Any driving means may be employed, such as a static drive, simple matrix drive and composite matrix drive.

Further, when the display device in the invention is driven by application of an electric field, a nonlinear element or an active element may be used as an element for driving each picture element. More particularly, as a nonlinear element of a 2-terminal element, there may be mentioned, for example, an element utilizing the nonlinearities of a varistor, MIM (Metal Insulator Metal) and diode arranged on one transparent substrate, as shown in Fig. 22 (a). In Fig. 22 (a), 43 is a liquid crystal substance, 47 is a transparent substrate, 61 is an ITO (Indium Tin Oxide) layer, 62 is an Al/Mo layer, 63 is a SiO layer, 64 is a Mo layer, 65 is a n+ $\alpha$-Si, 66 is a i$\alpha$-Si and 67 is a protecting layer. Further, as an active element of a 3-terminal element, there may be mentioned, for example, an element in which a TFT (Thin Film Transistor), Si-MOS (Si-Metal Oxide Semi Conductor field-effect Transistor) or SOS (Silicon on Sapphire) is arranged on the picture

element, as shown in Fig. 22 (b). In Fig. 22 (b), 68 is a TFT.

The novel carboxylate compounds of the present invention are useful as liquid crystal materials. A liquid crystal element manufactured using the compound as a liquid crystal material or a liquid crystal composition comprising the same have excellent liquid crystal characteristics.

By combining two or more compounds of the invention or different kinds of liquid crystal substances, it is possible to broaden the temperature range within which the liquid crystal exhibits effective properties without marring the ferroelectric properties and anti-ferroelectric properties of the compounds of the invention.

Accordingly, by the use of such compounds, it is possible to obtain, for example, liquid crystal elements having a high speed response in a broad temperature range, high contrast and stable contrast.

Further, in the liquid crystal display prepared by using such an element, the operating time can be shortened sharply. In such display, the consumption of electric power can be reduced and, at the same time, a high contrast and a stable contrast are obtained. Further, a low voltage drive of the liquid crystal display is also possible.

When the carboxylate compound of the invention is used as an anti-ferroelectric liquid crystal compound, the memory characteristics may be obtained without difficulty, and the orientation characteristics may also be improved.

By using the carboxylate compounds of the invention as liquid crystal materials, there can be obtained various devices having excellent characteristics such as a broad operating temperature range, high (fast) switching speed, very small consumption of electric power, and stable contrast.

The present invention is further illustrated below in the Examples. In the examples, R and S mean the R and S bodies of optically active compounds respectively.

Example 1

Synthesis of 4-[6'-(4"-decyloxybenzoyloxy)-5',6',7',8'-tetrahydro-2'-naphthoyloxy]benzoic acid R-1'''-trifluoromethylheptyl ester [Compound (4)]

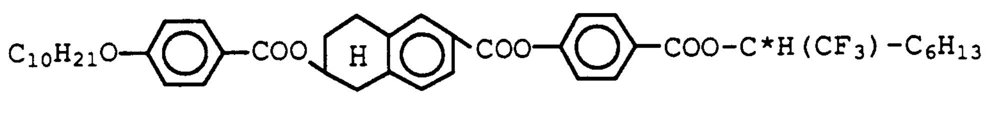

... [4]

First stage

In a 1-liter autoclave, 30g (160 mmol) of 6-hydroxynaphthalene-2-carboxylic acid, 5g of 5% palladium/carbon and 500 ml of tetrahydrofuran were mixed together, and the mixture was heated in a nitrogen atmosphere up to 105°C.

The mixture was allowed to undergo reaction in the autoclave maintained at a hydrogen pressure of 20 kg/cm$^2$ G, and a temperature of 105°C with stirring for 3 hours.

After a three-hour reaction, the reaction system was allowed to cool to room temperature, the hydrogen gas was released, the reaction mixture obtained was filtered using Celite as a filter aid, and the filtrate obtained was concentrated.

The mixture thus obtained was recrystallized from toluene to obtain 16.1 g of a compound as a white crystal.

This compound had a M/e value in FD-mass spectrum of 192.

From this analytical result, this compound was identified as 5,6,7,8-tetrahydro-6-hydroxynaphthalene-2-carboxylic acid.

Second stage

To a mixture of 0.96 g (a mmol) of 5,6,7,8-tetrahydro-6-hydroxynaphthalene-2-carboxylic acid, 1.52 g (5 mmol) 4-hydroxybenzoic acid R-1'-trifluoromethylheptyl ester synthesized separately by conventional means, 0.061 g (0.5 mmol) of 4-N,N-dimethylaminopyridine and 30 ml of methylene chloride was added dropwise 10 ml of a methylene chloride solution containing 1.13 g (5.5 mmol) of N,N'-dicyclohexylcarbodiimide with stirring at room temperature over a period of 5 hours.

Further, the reaction was carried out at room temperature for 14 hours.

The reaction mixture was filtered, and the filtrate was concentrated. The concentrate obtained was separated by column chromatography to obtain 2.18 g (4.56 mmol) of 4-(5',6',7',8'-tetrahydro-6'-hydroxy-2'-naphthoyloxy)benzoic acid R-1"-trifluoromethylheptyl ester as a colorless transparent viscous liquid.

Third stage

To a mixture of 0.48 g (1 mmol) of the 4-(5',6',7',8'-tetrahydro-6'-hydroxy-2'-naphthoyloxy)benzoic acid R-1'-trifluoromethylheptyl ester obtained in the second stage, 0.278 g (1 mmol) of 4-decyloxybenzoic acid synthesized separately by conventional means, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 3 hours.

Further, the reaction was carried out at room temperature for 3 hours.

The reaction mixture was filtered, and the filtrate was concentrated. The concentrate obtained was separated by column chromatography to obtain 0.45 g of a compound as a white solid.

This compound had a M/e value in FD-mass spectrum of 738.

Fig. 1 shows $^1$H-NMR spectrum of this compound.

From these analytical results, this compound was identified as the title compound as desired.

Example 2

Synthesis of 6-[6'-(4''-decyloxybenzoyloxy)-5',6',7',8'-tetrahydro-2'-naphthoyloxy]-1,2,3,4-tetrahydronaphthalene-2-carboxylic acid R-1'''-trifluoromethylheptyl ester [Compound (21)]

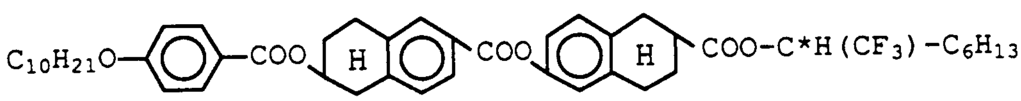

... [21]

First stage

To a mixture of 3.86 g (11.8 mmol) of 6-n-decyloxynaphthalene-2-acid and 130 ml of 1,2-diethoxyethane was added 3.0 g (130 mg atom) of metallic sodium with stirring in a nitrogen atmosphere at 120°C, and the resulting mixture was heated further up to the reflux temperature.

To this mixture was added dropwise 10 g (114 mmol) of isoamyl alcohol over a period of 1 hour, and the resulting mixture was allowed to undergo reaction under reflux for 11 hours. After cooling the reaction system to room temperature, the remaining metallic sodium was solubilized in the form of sodium alcoholate by the addition of ethanol, and the reaction mixture was acidified with 20% hydrochloric acid.

After addition to this reaction mixture of 100 ml of water, the organic phase was separated, followed by water washing.

The organic phase was then concentrated under reduced pressure to obtain 4.25 g of a solid. This solid was recrystallized from toluene to obtain 2.95 g (8.89 mmol) of 1,2,3,4-tetrahydro-6-n-decyloxynaphthalene-2-carboxylic acid.

Second stage

A mixture of 16.6 g (50 mmol) of the 6-decyloxynaphthalene-2-carboxylic acid obtained in the first stage, 250 cc of acetic acid and 86.5 g (0.5 mol) of 47 % hydrobromic acid was heated under reflux at 130°C for 7 hours. After addition to the reaction system of distilled water, the reaction mixture was concentrated under reduced pressure to obtain 10.60 g (50 mmol) of 1,2,3,4-tetrahydro-6-hydroxynaphthalene-2-carboxylic acid.

Third stage

A mixture of 10.60 g (50 mmol) of 1,2,3,4-tetrahydro-6-hydroxynaphthalene-2-carboxylic acid, 12.85 g (75 mmol) of benzyl bromide, 6.6 g (100 mmol) of 85% potassium hydroxide, 0.525 g (3.5 mmol) of sodium iodide, 200 ml of ethanol and 25 ml of distilled water was heated under reflux at 100°C for 12 hours. To this reaction mixture was added 50 ml of 10% potassium hydroxide, followed by heating under reflux at that temperature for 2 hours. After allowing the reaction system to cool up to room temperature, the reaction mixture in cold water was acidified with 36% hydrochloric acid. The resulting deposited product was filtered, and recrystallized from toluene to obtain 13.08 g (46.4 mmol) of 1,2,3,4-tetrahydro-6-benzyloxynaphthalene-2-carboxylic acid.

Fourth stage

To a mixture of 5.64 g (20 mmol) of the 1,2,3,4-tetrahydro-6-benzyloxynaphthalene-2-carboxylic acid obtained in the third stage, 3.68 g (20 mmol) of R-1-trifluoromethylheptanol, 0.244 g (0.2 mmol) of 4-N,N-dimethylaminopyridine and 70 ml of methylene chloride was added dropwise 25 ml of a methylene chloride solution containing 4.53 g (22 mmol) of N,N'-dicylohexylcarbodiimide with stirring at room temperature for a period of 2 hours.

Further, the reaction was carried out at room temperature for 2 hours.

The reaction mixture was then filtered, and the filtrate was concentrated. The concentrate obtained was separated by column chromatography to obtain 8.19 g(18.3 mmol) of 1,2,3,4-tetrahydro-6-benzyloxynaphthalene-2-carboxylic acid R-1'-trifluoromethylheptyl ester.

Fifth stage

Hydrogen gas was blown into a mixture of 8.19 g (18.3 mmol) of the 1,2,3,4-tetrahydro-6-benzyloxynaphthalene-2-carboxylic acid R-1'-trifluoromethylheptyl ester obtained in the fourth stage, 3.6 g of 5% palladium/carbon and 50 ml of tetrahydrofuran with stirring at room temperature and ordinary pressure for 24 hours. The reaction mixture was filtered using Celite as a filter aid, and the resulting filtrate was then concentrated to obtain 6.78 g (18.3 mmol) of 1,2,3,4-tetrahydro-6-hydroxynaphthalene-2-carboxylic acid R-1'-tri-fluoromethylheptyl ester as a brown liquid.

Sixth stage

To a mixture of 1.07 g (3 mmol) of the 1,2,3,4-tetrahydro-6-hydroxynaphthalene-2-carboxylic acid R-1'-trifluoromethylheptyl ester obtained in the fifth stage, 0.576 g (3 mmol) of the 5,6,7,8-tetrahydro-6-hyydroxynaphthalene-2-carboxylic acid obtained in the first stage of Example 1, 0.037 g (0.3 mmol) of 4-N,N-dimethylaminopyridine and 20 ml of methylene chloride was added dropwise 10 ml of a methylene chloride solution containing 0.68 g (3.3 mmol) of N,N'-dicyclohexylcarbodiimide with stirring at room temperature over a period of 2 hours.

Further, the reaction was carried out at room temperature for 18 hours.

The reaction mixture was filtered, and the filtrate was then concentrated. The concentrate obtained was separated by column chromatography to obtain 0.55 g (1.03 mmol) of 5',6',7',8'-tetrahydro-6'-hydroxy-2'-naphthoyloxy]-5,6,7,8-tetrahydronaphthalene-2-carboxylic acid R-1"-trifluoromethylheptyl ester.

Seventh stage

To a mixture of 0.55 g (1.03 mmol) of the 5',6',7',8'-tetrahydro-6'-hydroxy-2'-naphthoyloxy]-5.6.7.8-tetrahyddronaphthalene-2-carboxylic acid R-1"-trifluoromethylheptyl ester obtained in the sixth stage, 0.286 g (1.03 mmol) of 4-decyloxy-benzoic acid synthesized separately by conventional means, 0.013 g (0.103 mmol) of 4-N,N-dimethylaminopyridine and 10 ml of methylene chloride was added dropwise 5 ml of a methylene chloride solution containing 0.255 g (1.2 mmol) of N,N'-dicyclohexylcarbodiimide with stirring at room temperature over a period of 4 hours.

Further, the reaction was carried out for 4 hours.

The reaction mixture was distilled, and the filtrate was then concentrated. The concentrate obtained was separated by column chromatography to obtain 0.46 g of a compound as a white solid.

This compound had a M/e value in FD-mass spectrum of 792.

Fig. 2 shows $^1$H-NMR spectrum of this compound.

From these analytical results, this compound was identified as the title compound as desired.

Example 3

Synthesis of 6-[6'-(4''-decyloxybenzoyloxy)-5',6',7',8'-tetrahydro-2'-naphthoyloxy]-5,6,7,8-tetrahydronaphthalene-2-carboxylic acid R-1'''-trifluoromethylheptyl ester [Compound (22)]

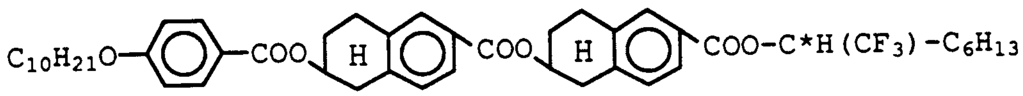

· [22]

First stage

To a mixture of 0.38 g (2 mmol) of 5,6,7,8-tetrahydro-6-hydroxynaphthalene-2-carboxylic acid, 0.22 g (2 mmol) of benzyl alcohol, 0.024 g (0.2 mmol) of 4-N,N-dimethylaminopyridine and 15 ml of methylene chloride was added dropwise 10 ml of a methylene chloride solution containing 0.45 g (2.2 mmol) of N,N'-dicyclohexylcarbodiimide with stirring at room temperature over a period of 1.5 hours.

Further, the reaction was carried out for 14 hours.

The reaction mixture was filtered, and the filtrate was concentrated. The concentrate obtained was separated by column chromatography to obtain 0.40 g (1.4 mmol) of 5,6,7,8-tetrahydro-6-hydroxynaphthalene-2-carboxylic acid benzyl ester.

Second stage

To a mixture of 0.04 g (1.42 mmol) of the 5,6,7,8-tetrahydro-6-hydroxynaphthalene-2-carboxylic acid benzyl ester obtained in the first stage, 0.395 g (1.42 mmol) of 4-decyloxy-benzoic acid synthesized separately by conventional means, 0.017 g (0,14 mmol) of 4-N,N-dimethylaminopyridine and 10 ml of methylene chloride was added dropwise 5 ml of a methylene chloride solution containing 0.35 g (1.42 mmol) of N,N'-dicyclohexylcarbodiimide with stirring at room temperature over a period of 4 hours.

Further, the reaction was carried out for 16 hours.

The reaction mixture was filtered, and the filtrate was concentrated. The concentrate obtained was separated by column chromatography to obtain 0.53 g (1.42 mmol) of 6-(4'-decyloxybenzoyloxy)-5,6,7,8-tetrahydronaphthalene-2-carboxylic acid benzyl ester.

Third stage

Hydrogen gas was blown into a mixture of 0.53 g (1.0 mmol) of 6-(4'-decyloxybenzoyloxy)-5,6,7,8-tetrahydronaphthalene-2-carboxylic acid benzyl ester, 0.11 g of 5% palladium/carbon and 10 ml of tetrahydrofuran with stirring at room temperature and ordinary pressure for 15 hours. The reaction mixture was filtered using Celite as a filter aid, and the filtrate was concentrated to obtain 0.42 g (0.93 mmol) of 6-(4'-decyloxybenzoyloxy)-5,6,7,8-tetrahydronaphthalene-2-carboxylic acid.

Fourth stage

To a mixture of 2.88 g (15 mmol) of the 5,6,7,8-tetrahydro-6-hydronaphthalene-2-carboxylic acid obtained in the first stage of Example 1, 2.76 g (15 mmol) of 1-trifluoromethylheptyl alcohol, 0.183 g (1.5 mmol) of 4-N,N-dimethylaminopyridine and 50 ml of methylene chloride was added dropwise 20 ml of a methylene chloride solution containing 3.40 g (16.5 mmol) of N,N'-dicyclohexylcarbodiimide with stirring at room temperature for a period of 24 hours.

Further, 2 ml of a methylene chloride solution containing 0.41 g (2.0 mmol) of N,N'-dicyclohexylcarbodiimide was added to the mixture, and the resulting mixture was allowed to undergo reaction at room temperature for 72 hours.

The reaction mixture was filtered, and the filtrate was then concentrated. The concentrate obtained was separated by olumn chromatography to obtain 3.11 g (8.69 mmol) of 5,6,7,8-tetrahydro-6-hydroxynaphthalene-2-carboxylic acid 1'-trifluoromethylheptyl ester.

Fifth stage

To a mixture of 0.42 g (0. 93 mmol) of the 6-(4'-decyloxybenzoyloxy)-5,6,7,8-tetrahydronaphthalene-2-carboxylic

acid obtained in the third stage, 0.44 g (0.93 mmol) of the 5,6,7,8-tetrahydro-6-hydroxynaphthalene-2-carboxylic acid 1'-trifluoromethylheptyl ester obtained in the fourth stage, 0.011 g (0.092 mmol) of 4-N,N-dimethylamino pyridine and 10 ml of methylene chloride was added dropwise 5 ml of a methylene chloride solution containing 0.23 g (1.1 mmol) of N,N'-dicyclohexylcarbodiimide with stirring at room temperature over a period of 3 hours.

Further, the reaction was carried out at room temperature for 120 hours.

The reaction mixture was filtered, and the filtrate was concentrated. The concentrate obtained was separated by column chromatography to obtain 0.43 g of a compound as a white solid.

This compound had a M/e value in FD-mass spectrum of 792.

Fig. 3 shows $^{1}$H-NMR spectrum of this compound.

From these analytical results, this compound was identified as the title compound as desired.

Example 4

Synthesis of 6-[4''-(5'-decyl-2'-pyrimidinyl)benzoyloxy]-5,6,7,8-tetrahydronaphthalene-2-carboxylic acid R-1'''-trifluoromethylheptyl ester [Compound (130)]

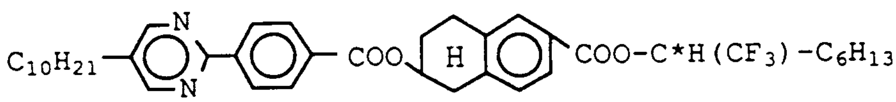

... [130]

First stage

To a mixture of 0.44 g (0,93 mmol) of the 5,6,7,8-tetrahydro-6-hydroxynaphthalene-2-carboxylic acid 1'-trifluoromethylheptyl ester obtained in the fourth stage of Example 3, 0.264 g (0.8 mmol) of 4-(5'-decyl-2'-pyrimidinyl)benzoic acid (produced and sold by Kojima Kagaku K.K.), 0.01 g (0.08 mmol) of 4-N,N-dimethylaminopyridine and 20 ml of methylene chloride was added dropwise 5 ml of a methylene chloride solution containing 0.18 g (0.88 mmol) of N,N'-dicyclohexylcarbodiimide with stirring at room temperature over a period of 1.5 hours.

Further, the reaction was carried out for 4 hours.

The reaction mixture was filtered and the filtrate was then concentrated. The concentrate obtained was separated by column chromatography to obtain 0.39 g of a white solid.

This compound had a M/e value in FD-mass spectrum of 680.

Fig. 4 shows $^{1}$H-NMR spectrum of this compound.

From these analytical results, this compound was identified as the title compound as desired.

Example 5

The compound (4) of the following formula [4] obtained in Example 1 was mixed with compound (A) represented by the following formula [A] in the weight ratio of 75:25 to obtain a liquid crystal composition of the invention:

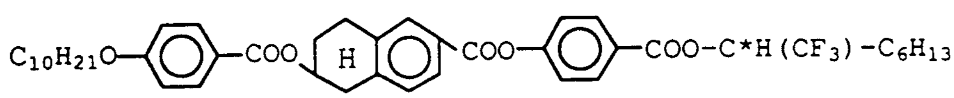

... [4]

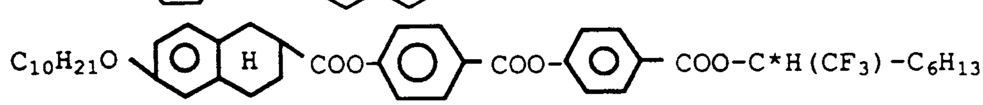

... [A]

Phase transition temperatures of this composition measured are shown in Table 8 together with those of the compound (4) and the compound (A).

Table 8

| Compound Number | Cry-$SmC_A$* or SmX | SmX-$SmAC_A$* or $SmC_A$*-SmA | SmA-Iso |
|---|---|---|---|
| (4) | 21°C | 49°C | 61°C |
| (A) | 44°C | 78°C | 94°C |
| (4)75%+(A)25% | 45°C | 68°C | 74°C |

Example 6

The compound (21) of the following formula [21] obtained in Example 2 was mixed with compound (A) represented by the following formula [A] in a weight ratio of 50:50 to obtain a liquid crystal composition of the invention.

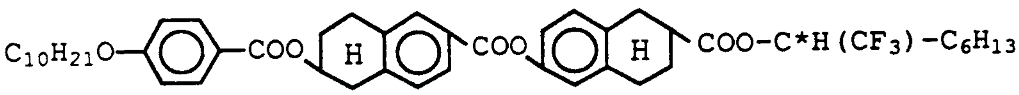
··· [21]

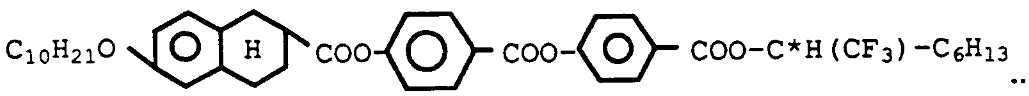
.. [A]

The phase transition temperatures of this composition are shown in Table 9 together with those of compound (21) and compound (A).

Table 9

| Compound Number | Cry-$SmC_A$* | $SmC_A$*-SmA | SmA-Iso |
|---|---|---|---|
| (21) | 43°C | 47°C | 72°C |
| (A) | 44°C | 78°C | 94°C |
| (21)50%+(A)50% | <-30°C | 70°C | 98°C |

Example 7

The compound (22) of the following formula [22] obtained in Example 3 was mixed with compound (A) represented by the following formula [A] in a weight ratio of 50:50 to obtain a liquid crystal composition of

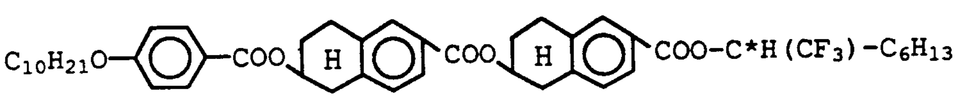
··· [22]

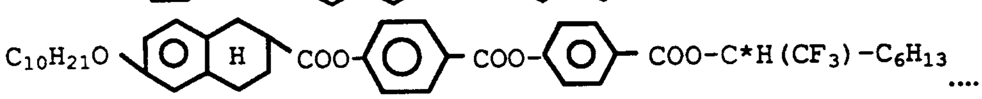
.... [A]

The phase transition temperatures of this composition measured are shown in Table 10 together with those of the compound (22) and the compound (A).

Table 10

| Compound Number | Cry-$SmC_A$* or Iso | $SmC_A$*-SmA or Iso | SmA-Iso |
|---|---|---|---|
| (22) | 43°C | - | - |
| (A) | 44°C | 78°C | 94°C |
| (22)50%+(A)50% | <-30°C | 68°C | - |

Example 8

Synthesis of 4-[6'-(4"-decyloxybenzoyloxy)-1',2',3',4'-tetrahydro-2'-naphthoyloxy]benzoic acid R-1'''- trifluoromethylheptyl ester [Compound (204)]

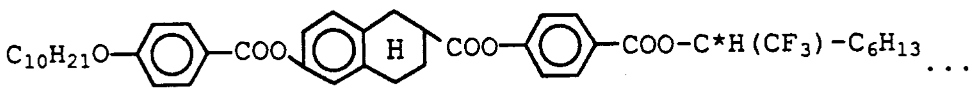

[204]

First stage

To a mixture of 3.86g (11.8 mmol) of 6-n-decyloxynaphthalene-2-carboxylic acid and 130 ml of 1,2-diethoxyethane was added 3.0 g (130 mg atom) of metallic sodium with stirring in a nitrogen atmosphere at 120 °C, and the resulting mixture was further heated up to the reflux temperature.

To this mixture was added dropwise 10 g (114 mmol) of isoamyl alcohol over a period of 1 hour, and the resulting mixture was allowed to undergo reaction under reflux for 11 hours. After cooling the reaction system to room temperature, the remaining mettalic sodium was converted to alcoholate by the addition of ethanol, and the reaction mixture was acidified with 20 % hydrochloric acid.

To the reaction mixture was added 100 ml of water, and then the organic phase was separated and washed with water.

The organic phase was concentrated under reduced pressure to obtain 4.25 g of a solid, which was recrystallized from toluene to obtain 2.95 g (8.89 mmol) of 1,2,3,4-tetrahydro-6-n-decyloxynaphthalene-2-carboxylic acid.

Second stage

16.6 g (50 mmol) of the 6-decyloxynaphthalene-2-carboxylic acid obtained in the first stage was heated under reflux at 130°C for 7 hours in the presence of 250 cc of acetic acid and 86.5 g (0.5 mol) of 47 % hydrobromic acid. To the reaction mixture was added distilled water, and then the resulting mixture was concentrated under reduced pressure to obtain 10.60 g (50 mmol) of 1,2,3,4-tetrahydro-6-hydroxynaphthalene-2-carboxylic acid.

Third stage

A mixture of 10.60 g (50 mmol) of the 1,2,3,4-tetrahydro-6-hydroxynaphthalene-2-carboxylic acid obtained in the second stage, 12.85 g (75 mmol) of benzyl bromide, 6.6 g (100 mmol) of 85 % potassium hydroxide, 0.525 g (3.5 mmol) of sodium iodide , 200 ml of ethanol and 25 ml of distilled water was heated under reflux at 100°C for 12 hours. After 50 ml of 10 % potassium hydroxide was added, the mixture was further heated under reflux for 2 hours. After allowing the reaction system to cool up to room temperature, the reaction mixture in cold water was acidified with 36 % hydrochloric acid. The deposited product was separated by filtration and recrystallized from toluene to obtain 13.08 g (46.4 mmol) of 1,2,3,4-tetrahydro-6-benzyloxynaphthalene-2-carboxylic acid.

Fourth stage

To a mixture of 0.846 g (3 mmol) of the 1,2,3,4-tetrahydro-6-benzyloxynaphthalene-2-carboxylic acid obtained in

the third stage, 0.912 g (3 mmol) of 4-hydroxybenzoic acid R-1'-trifluoromethylheptyl ester synthesized separately by conventional means, 0.037 g (0.3 mmol) of 4-N,N-dimetylaminopyridine and 15 ml of methylene chloride was added dropwise 7 ml of a methylene chloride solution containing 0.68 g (3.3 mmol) of N,N'-dicyclohexylcarbodiimide with stirring at room temperature over a period of 1 hour.

Further, the reaction was carried out at room temperature for 3 hours.

The reaction mixture was filtered, and the filtrate was concentrated. The concentrate obtained was separated by column chromatography to obtain 1.56 g (2.75 mmol) of 4-(1',2',3',4'-tetrahydro-6'-benzyloxy-2'-naphhoyloxy)benzoic acid R-1"-trifluoromethylheptyl ester as a white solid.

Fifth stage

Hydrogen gas was blown into a mixture of 1.56 g (2.75 mmol) of the 4-(1',2',3',4'-tetrahydro-6'-benzyloxy-2'-naphthoyloxy)benzoic acid R-1"-trifluoromethylheptyl ester obtained in the fourth stage, 0.16 g of 5 % palladium/carbon and 20 ml of tetrahydrofuran with stirring at room temperature and ordinary pressure for 20 hours. The reaction mixture was filtered using Celite as a filter aid, and the filtrate was concentrated to obtain 1.36 g (2.75 mmol) of 4-(1',2',3',4'-tetrahydro-6'-hydroxy-2'-naphthoyloxy)benzoic acid R-1"-trifluoromethylheptyl ester.

Sixth state

To a mixture of 0.478 g (1 mmol) of the 4-(1',2',3',4'-tetrahydro-6'-hydroxy-2'-naphthoyloxy)benzoic acid R-1"-trifluoromethylheptyl ester obtained in the fifth stage, 0.278 g (1 mmol) of 4-decyloxybenzoic acid synthesized separately by conventional means, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 1 hour.

Further, the reaction was carried out at room temperature for 5 hours.

The reaction mixture was filtered, and the filtrate was concentrated. The concentrated obtained was separated by column chromatography to obtain 0.43 g of a compound as a colorless semisolid.

This compound had a M/e value in FD-mass spectrum of 738.

Fig. 5 shows 1H-NMR spectrum of this compound.

From these analytical results, this compound was identified as the title compound as desired.

Example 9

Synthesis of 6-[6'-(4"-decyloxybenzoyloxy)-1',2',3',4'-tetrahydro-2'-naphthoyloxy]-1,2,3,4-tetrahydronaphthalene-2-carboxylic acid R-1‴-trifluoromethylheptyl ester [Compound (221)]

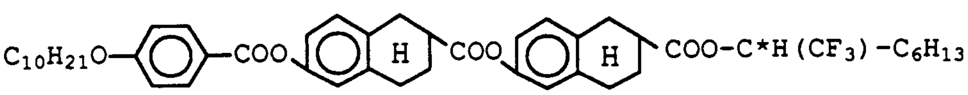

... [221]

First stage

To a mixture of 5.64 g (20 mmol) of the 1,2,3,4-tetrahydro-6-benzyloxynaphthalene-2-carboxylic acid obtained in the third stage of Example 8, 3.68 g (20 mmol) of R-1-trifluoromethylheptanol, 0.244 g (0.2 mmol) of 4-N,N-dimethylaminopyridine and 70 ml of methylene chloride was added dropwise 25 ml of a methylene chloride solution containing 4.53 g (22 mmol) of N,N'-dicyclohexylcarbodiimide with stirring at room temperature over a period of 2 hours.

Further, the reaction was carried out at room temperature for 2 hours.

The reaction mixture was filtered, and the filtrate was concentrated. The concentrated obtained was separated by column chromatography to obtain 8.19 g (18.3 mmol) of 1,2,3,4-tetrahydro-6-benzyloxynaphthalene-2-carboxylic acid R-1'-trifluoromethylheptyl ester as a colorless liquid.

Second stage

Hydrogen gas was blown into a mixture of 8.19 g (18.3 mmol) of the 1,2,3,4-tetrahydro-6-benzyloxynaphthalene-2-carboxylic acid R-1'-trifluoromethylheptyl ester obtained in the first stage, 3.6 g of 5 % palladium/carbon and 50 ml of

tetrahydrofuran with stirring at room temperature and or dinary pressure for 24 hours. The reaction mixture was filtered using Celite as a filter aid, and the filtrate was concentrated to obtain 6.78 g (18.3 mmol) of 1,2,3,4-tetrahydro-6-hydroxynaphthalene-2-carboxylic acid R-1'-trifluoromethylheptyl ester as a brown liquid.

Third stage

To a mixture of 0.43 g (1.2 mmol) of the 1,2,3,4-tetrahydro-6-hydroxynaphthalene-2-carboxylic acid R-1'-trifluoromethylheptyl ester obtained in the second stage, 0.34 g (1.2 mmol) of the 1,2,3,4-tetrahydro-6-benzyloxynap hthalene-2-carboxylic acid obtained in the third stage of Example 8, 0.015 g (0.12 mmol) of 4-N,N-dimethylaminopyridine and 10 ml of methylene chloride was added dropwise 5 ml of a methylene chloride solution containing 0.30 g (1.4 mmol) of N,N'-dicyclohexylcarbodiimide with stirring at room temperature over a period of 2 hours.

Further, the reaction was carried out at room temperature for 6 hours.

The reaction mixture was filtered, and the filtrate was concentrated. The concentrate obtained was separated by column chromatography to obtain 0.70 g (0.125 mmol) of 6-(1',2',3',4'-tetrahydro-6'-benzyloxy-2'-naphthoyloxy)-1,2,3,4-tetrahydroxynaphthalene-2-carboxylic acid R-1"-trifluoromethylheptyl ester as a white solid.

Fourth stage

Hydrogen gas was blown into a mixture of 0.70 g (1.125 mmol) of the 6-(1',2',3',4'-tetrahydro-6'-benzyloxy-2'-naphthoyloxy)-1,2,3,4-tetrahydroxynaphthalene-2-carboxylic acid R-1"-trifluoromethylheptyl ester obtained in the third stage, 0.14 g of 5 % palladium/carbon and 10 ml of tetrahyd rofuran with stirring at room temperature and ordinary pressure for 16 hours. The reaction mixture was filtered using Celite as a filter aid, and the filtrate obtained was concentrated to obtain 0.64 g (1.125 mmol) of 6-(1',2',3',4'-tetrahydro-6'-benzyloxy-2'-naphthoyloxy)-1,2,3,4-tetrahydroxynaphthalene-2-carboxylic acid R-1"-trifluoromethylheptyl ester as a white solid.

Fifth stage

To a mixture of 0.64 g (1.125 mmol) of 6-(1',2',3',4'-tetrahydro-6'-benzyloxy-2'-naphthoyloxy)-1,2,3,4-tetrahydroxynaphthalene-2-carboxylic acid R-1"-trifluoromethylheptyl ester obtained in the fourth stage, 0.313 g (1.125 mmol) of 4-decyloxybenzoic acid synthesized separately by conventional means, 0.014 g (0.113 mmol) of 4-N,N-dimethylaminopyridine and 10 ml of methylene chloride was added dropwise 5 ml of a methylene chloride solution containing 0.28 g (1.5 mmol) of N,N'-dicyclohexylcarbodiimide with stirring over a period of 2 hours.

Further, the reaction was carried out at room temperature for 5.5 hours.

The reaction mixture was filtered, and the filtrate was concentrated. The concentrate obtained was separated by column chromatography to obtain 0.76 g of a compound as a colorless semisolid.

This compound had a M/e value in FD-mass spectrum of 792.

Fig. 6 shows 1H-NMR spectrum of this compound.

From these analytical results, this compound was identified as the title compound as desired.

Example 10

Synthesis of 6-[6'-(4"-decyloxybenzoyloxy)-1',2',3',4'-tetrahydro-2'-naphthoyloxy]-5,6,7,8-tetrahydronaphthalene-2-carboxylic acid R-1'''-trifluoromethylheptyl ester [Compound (222)]

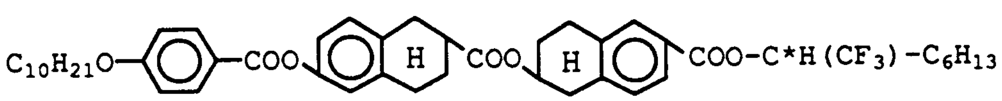

... [222]

First stage

In a 1-liter autoclave, 30 g (160 mmol) of 6-hydroxynaphthalene-2-carboxylic acid, 5 g of 5 % palladium/carbon and 500 ml of tetrahydrofuran were mixed together, and the mixture was heated in a nitrogen atmosphere up to 105 °C.

The mixture was allowed to undergo reaction in the autoclave maintained at a hydrogen pressure of 20 kg/cm2 G and a temperature of 105°C with stirring for 3 hours.

After a three-hour reaction, the reaction system was allowed to cool to room temperature, the hydrogen gas was

released, the reaction mixture obtained was filtered using Celite as a filter aid, and the filtrate obtained was concentrated.

The mixture thus obtained was recrystallized from toluene to obtain 16.1 g of a compound of a white crystal.

This compound had a M/e value in FD-mass spectrum of 192.

From this analytical result, this compound was identified as 5,6,7,8-tetrahydro-6-hydroxynaphthalene-2-carboxylic acid.

Second stage

To a mixture of 2.88 g (15 mmol) of the 5,6,7,8-tetrahydro-6-hydroxynaphthalene-2-carboxylic acid obtained in the first stage, 2.76 g (15 mmol) R-1'-trifluoromethylheptanol, 0.183 g (1.5 mmol) of 4-N,N-dimethylaminopyridine and 50 ml of methylene chloride was added dropwise 20 ml of a methylene chloride solution containing 3.40 g (16.5 mmol) of N,N'-dicyclohexylcarbodiimide with stirring at room temperature over a period of 5 hours.

After the resulting mixture was further allowed to undergo reaction at room temperature for 24 hours, 2 ml of a methylene chloride solution containing 0.41 g (2 mmol) of N,N'-dicyclohexylcarbodiimide was added thereto, and allowed to undergo reaction for additional 3 days.

The reaction mixture was filtered, and the filtrate was concentrated. The concentrate obtained was separated by column chromatography to obtain 3.11 g (8.69 mmol) of 5,6,7,8-tetrahydro-6-hydroxynaphthalene-2-carboxylic acid R-1'-trifluoromethylheptyl ester as a colorless viscous liquid.

Third stage

To a mixture of 0.716 g (2 mmol) of the 5,6,7,8-tetrahydro-6-hydroxynaphthalene-2-carboxylic acid R-1'-trifluoromethylheptyl ester obtained in the second stage, 0.564 g (2 mmol) of the 1,2,3,4-tetrahydro-6-benzyloxynaphthalene-2-carboxylic acid obtained in the third stage of Example 8, 0.0244 g (0.2 mmol) of 4-N,N-dimethylaminopyridine and 15 ml of methylene chloride was added dropwise 10 ml of a methylene chloride solution containing 0.45 g (2.2 mmol) of N,N'-dicyclohexylcarbodiimide with stirring at room temperature over a period of 4 hours.

Further, the reaction was carried out at room temperature for 23 hours.

The reaction mixture was filtered, and the filtrate was concentrated. The concentrate obtained was separated by column chromatography to obtain 1.15 g (1.85 mmol) of 6-(1',2',3',4'-tetrahydro-6'-benzyloxy-2'-naphthoyloxy)-5,6,7,8-tetrahydroxynaphthalene-2-carboxylic acid R-1"-trifluoromethylheptyl ester as a white solid.

Fourth stage

Hydrogen gas was blown into a mixture of 1.15 g (1.85 mmol) of the 6-(1',2',3',4'-tetrahydro-6'-benzyloxy-2'-naphthoyloxy)-5,6,7,8-tetrahydroxynaphthalene-2-carboxylic acid R-1"-trifluoromethylheptyl ester obtained in the third stage, 0.23 g of 5 % palladium/carbon and 10 ml of tetrahydrofuran with stirring at room temperature and ordinary pressure for 16 hours. The reaction mixture was filtered using Celite as a filter aid, and th e resulting filtrate was then concentrated to obtain 0.98 g (1.84 mmol) of 6-(1',2',3',4'-tetrahydro-6'-hydroxy-2'-naphthoyloxy)-5,6,7,8-tetrahydroxynaphthalene-2-carboxylic acid R-1"-trifluoromethylheptyl ester as a white solid.

Fifth stage

To a mixture of 0.48 g (0.9 mmol) of the 6-(1',2',3',4'-tetrahydro-6'-hydroxy-2'-naphthoyloxy)-5,6,7,8-tetrahydroxynaphthalene-2-carboxylic acid R-1"-trifluoromethylheptyl ester obtained in the fourth stage, 0.25 g (0.9 mmol) of 4-decyloxybenzoic acid synthesized separately by conventional means, 0.011 g (0.09 mmol) of 4-N,N-dimethylaminopyridine and 10 ml of methylene chloride was added dropwise 5 ml of a methylene chloride solution containing 0.22 g (1.1 mmol) of N,N'-dicyclohexylcarbodiimide with stirring at room temperature over a period of 1 hour.

Further, the reaction was carried out at room temperature for 65 hours.

The reaction mixture was filtered, and the filtrate was concentrated. The concentrate obtained was separated by column chromatography to obtain 0.51 g of a compound as a colorless semisolid.

This compound had a M/e value in FD-mass spectrum of 792.

Fig. 7 shows 1H-NMR spectrum of this compound.

From these analytical results, this compound was identified as the title compound as desired.

Example 11

Synthesis of 6-[4'-(4"-decyloxybiphenyl)carboxy]-1,2,3,4-tetrahydronaphthalene-2-carboxylic acid R-1‴-trifluoromethylheptyl ester [Compound (251)]

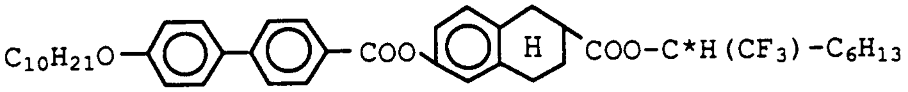

... [251]

First stage

A mixture of 21.4g (0.1 mol) of 4'-hydroxybiphenyl-4-carboxylic acid, 33.15 g (0.15 mol) of n-decyl bromide, 13.20 g (0.2 mol) of 85 % potassium hydroxide, 1.05 g (7 mmol) of sodium iodide , 500 ml of ethanol and 100 ml of distilled water was heated under reflux at 100°C for 12 hours. After 40 ml of 25 % potassium hydroxide was added, the mixture was further heated under reflux for 2 hours. After allowing the reaction system to cool up to room temperature, the reaction mixture in cold water was acidified with 36 % hydrochloric acid. The deposited product was separated by filtration and dissolved in acetone with heating, followed by filtration. The resulting filtrate was concentrated to obtain 1.97 g (6 mmol) of 4'-decyloxybiphenyl-4-carboxylic acid.

Second stage

To a mixture of 0.35 g (1 mmol) of the 4'-decyloxybiphenyl-4-carboxylic acid obtained in the first stage, 0.36 g (1 mmol) of the 1,2,3,4-tetrahydro-6-hydroxynaphthalene-2-carboxylic acid R-1'-trifluoromethylheptyl ester obtained in the second stage of Example 9, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2 hours.

Further, the reaction was carried out at room temperature for 48 hours.

The reaction mixture was filtered, and the filtrate was concentrated. The concentrate obtained was separated by column chromatography to obtain 0.51 g of a compound as a colorless semisolid.

This compound had a M/e value in FD-mass spectrum of 694.

Fig. 8 shows 1H-NMR spectrum of this compound.

From these analytical results, this compound was identified as the title compound as desired.

Example 12

Synthesis of 6-[4'-(4"-decyloxyphenylcyclohexane)carboxy]-1,2,3,4-tetrahydronaphthalene-2-carboxylic acid R-1‴-trifluoromethylheptyl ester [Compound (267)]

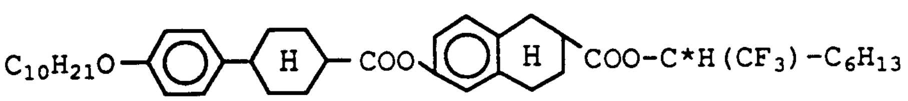

... [267]

First stage

A mixture of 44g (0.2 mol) of 4'-hydroxyphenylcyclohexane-4-carboxylic acid, 66.4 g (0.3 mol) of n-decyl bromide, 26.4 g (0.4 mol) of 85 % potassium hydroxide, 3.0 g (20 mmol) of sodium iodide, 360 g of ethanol and 180 g of distilled water was heated under reflux at 100°C for 9 hours. After 100 ml of 10 % potassium hydroxide was added, the mixture was further heated under reflux for 20 hours. After allowing the reaction system to cool up to room temperature, the reaction system in cold water was acidified with 36 % hydrochloric acid. The deposited product was separated by filtration and washed with hexane to obtain 67.4 g (0.187 mmol) of 4'-decyloxyphenylcyclohexane-4-carboxylic acid.

Second stage

To a mixture of 0.36 g (1 mmol) of the 4'-decyloxyphenylcyclohexane-4-carboxylic acid obtained in the first stage, 0.36 g (1 mmol) of the 1,2,3,4-tetrahydro-6-hydroxynaphthalene-2-carboxylic acid R-1'-trifluoromethylheptyl ester obtained in the second stage of Example 9, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2 hours.

Further, the reaction was carried out at room temperature for 19 hours.

The reaction mixture was filtered, and the filtrate was concentrated. The concentrate obtained was separated by column chromatography to obtain 0.58 g of a compound as a colorless semisolid.

This compound had a M/e value in FD-mass spectrum of 700.

Fig. 9 shows 1H-NMR spectrum of this compound.

From these analytical results, this compound was identified as the title compound as desired.

Example 13

Synthesis of 4-[6'-(6"-heptyloxy-2"-naphthoyloxy)-1',2',3',4'-tetrahydro-2'-naphthoyloxy] benzoic acid R-1'''-trifluoromethylheptyl ester [Compound (288)]

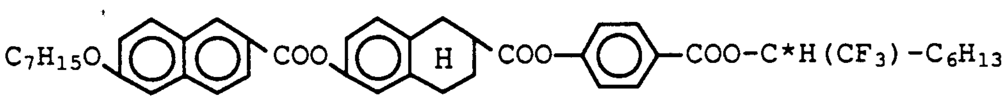

[288]

First stage

To a mixture of 0.24g (0.84 mmol) of 6-heptyloxynaphthalene-2-carboxylic acid synthesized by conventional means, 0.56 g (0.84 mmol) of the 4-(1',2',3',4'-tetrahydro-6'-hydroxy-2'-naphthoyloxy)benzoic acid R-1"-trifluoromethylheptyl ester obtained in the fifth stage of Example 8, 0.012 g (0.1 mmol) of 4-N,N-dimethylaminopyridine and 20 ml of methylene chloride was added dropwise 2 ml of a methylene chloride solution containing 0.20 g (1 mmol) of N,N'-dicyclohexylcarbodiimide with stirring at room temperature over a period of 1 hour.

Further, the reaction was carried out at room temperature for 14 hours.

The reaction mixture was filtered, and the filtrate was concentrated. The concentrate obtained was separated by column chromatography to obtain 0.05 g of a compound as a colorless semisolid.

This compound had a M/e value in FD-mass spectrum of 746.

From the analytical result, this compound was identified as the title compound as desired.

Example 14

Synthesis of 6-(6'-heptyloxy-2'-naphthoyloxy)-1,2,3,4-tetrahydronaphthalene-2-carboxylic acid R-1"-trifluoromethylheptyl ester [Compound (296)]

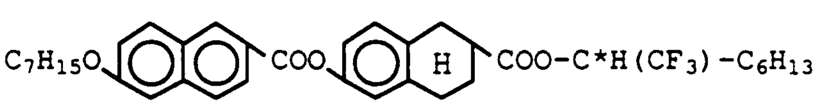

... [296]

First stage

To a mixture of 0.24g (0.84 mmol) of 6-heptyloxynaphthalene-2-carboxylic acid synthesized by conventional means, 0.69 g (0.84 mmol) of the 1,2,3,4-tetrahydro-6-hydroxynaphthalene-2-carboxylic acid R-1'-trifluoromethylheptyl ester obtained in the second stage of Example 9, 0.012 g (0.1 mmol) of 4-N,N-dimethylaminopyridine and 20 ml of methylene chloride was added dropwise 2 ml of a methylene chloride solution containing 0.17 g (0.84 mmol) of N,N'-dicyclohexylcarbodiimide with stirring at room temperature over a period of 1 hour.

Further, the reaction was carried out at room temperature for 14 hours.

The reaction mixture was filtered, and the filtrate was concentrated. The concentrate obtained was separated by column chromatography to obtain 0.27 g of a compound as a colorless semisolid

This compound had a M/e value in FD-mass spectrum of 626.

Fig. 10 shows 1H-NMR spectrum of this compound.

From these analytical results, this compound was identified as the title compound as desired

Example 15

Synthesis of 6-(6'-decyloxy-2'-naphthoyloxy)-1,2,3,4-tetrahydronaphthalene-2-carboxylic acid R-1''-trifluoromethylheptyl ester [Compound (299)]

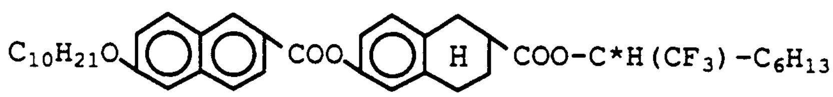

··· [299]

First stage

The reaction was conducted in the same manner as in Example 14 except that 6-decyloxynaphthalene-2-carboxylic acid was used instead of 6-heptyloxynaphthalene-2-carboxylic acid, to thereby obtain 0.64 g of a compound as a colorless semisolid

This compound had a M/e value in FD-mass spectrum of 668.

Fig. 11 shows $^1$H-NMR spectrum of this compound.

From these analytical results, this compound was identified as the title compound as desired.

Example 16

Synthesis of 6-[4'-(5''-decyl-2''-pyrimidinyl)benzoyloxy]-1,2,3,4-tetrahydronaphthalene-2-carboxylic acid R-1'''-trifluoromethyl heptyl ester [Compound (331)]

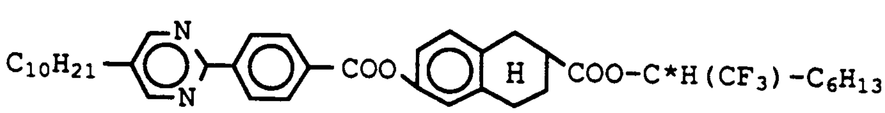

···[331]

First stage

To a mixture of 0.358 g (1 mmol) of the 1,2,3,4-tetrahydro-6-hydroxynaphthalene-2-carboxylic acid R-1'-trifluoromethylheptyl ester obtained in the second stage of Example 9, 0.33 g (1 mmol) of 4-(5'-decyl-2'-pyrimidinyl)benzoic acid (manufactured by Kojima Kagaku Kabusiki Kaisha), 0.01 g (0.1 mmol) of 4-N,N-dimethylaminopyridine and 10 ml of methylene chloride was added dropwise 3 ml of a methylene chloride solution containing 0.23 g (1.1 mmol) of N,N'-dicyclohexylcarbodiimide with stirring at room temperature over a period of 1.5 hours.

Further, the reaction was carried out at room temperature for 5 hours.

The reaction mixture was filtered, and the filtrate was concentrated. The concentrate obtained was separated by column chromatography to obtain 0.74 g of a compound as a colorless semisolid.

This compound had a M/e value in FD-mass spectrum of 680.

Fig. 12 shows 1H-NMR spectrum of this compound.

From these analytical results, this compound was identified as the title compound as desired.

Example 17

Synthesis of [6-(4'-decyloxybenzoyloxy)-1,2,3,4-tetrahydro-naphthalene-2-carboxylic acid] R-1''-trifluoromethylheptyl ester [Compound (235)]

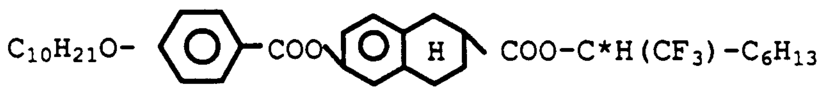

..[235]

First stage

To a mixture of 0.28 g (1.0 mmol) of the 4-decyloxybenzoic acid synthesized separately by conventional means, 0.36 g (1.0 mmol) of 1,2,3,4-tetrahydro-6-hydroxynaphthalene-2-carboxylic acid R-1'-trifluoromethylheptyl ester obtained in the second stage of Example 9, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2 hours.

Further, the reaction was carried out at room temperature for 48 hours.

The reaction mixture was filtered, and the filtrate was concentrated. The concentrate obtained was separated by column chromatography to obtain 0.50 g of a compound as a white solid.

This compound had a M/e value in FD-mass spectrum of 618.

From the analytical result, this compound was identified as the title compound as desired.

Example 18

The compound (204) represented by the following formula [204] obtained in Example 8 was mixed with compound (A) represented by the following formula [A] in a weight ratio of 50:50 to obtain a liquid crystal composition of the present invention.

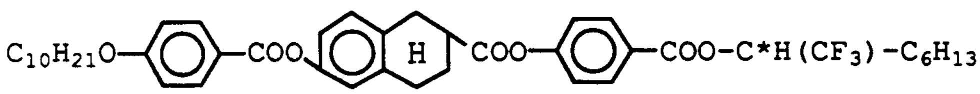

...[204]

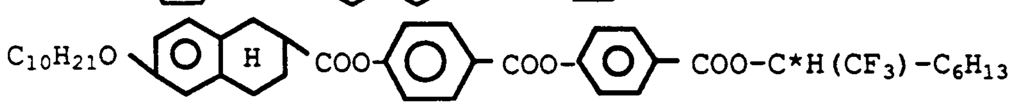

.. [A]

The phase transition temperatures of this composition measured are shown in Table 11.

Table 11

| Compound Number | Cry-$SmC_A$* | $SmC_A$*-SmA | SmA-Iso |
|---|---|---|---|
| (204) | -23°C | 81°C | 113°C |
| (A) | 44°C | 78°C | 94°C |
| (204)50%+(A)50% | <-30°C | 82°C | 110°C |

Example 19

The compound (251) represented by the following formula [251] obtained in Example 11 was mixed with compound (B) represented by the following formula [B] in a weight ratio of 80:20 to obtain a liquid crystal composition of the present invention.

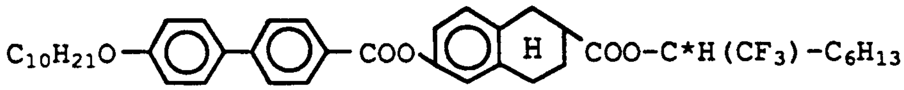
... [251]

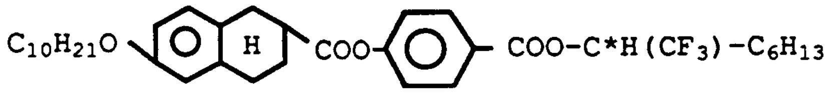
... [B]

The phase transition temperatures of this composition measured are shown in Table 12.

Table 12

| Compound Number | Cry-SmC$_A$* or SmA | SmC$_A$*-SmA | SmA-Iso |
|---|---|---|---|
| (251) | 21°C | 99°C | 130°C |
| (B) | <-30°C | - | -14°C |
| (251)80%+(B)20% | <-30°C | 67°C | 106°C |

Example 20

The compound (267) represented by the following formula [267] obtained in Example 12 was mixed with the compound (251) represented by the following formula [251] obtained in Example 11 in a weight ratio of 50:50 to obtain a liquid crystal composition of the present invention.

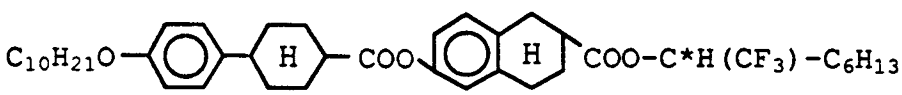
·· [267]

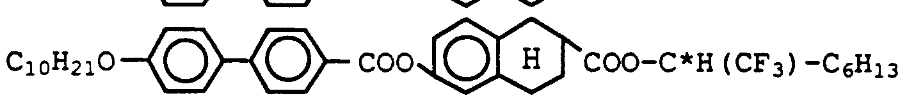
·· [251]

The phase transition temperatures of this composition measured are shown in Table 13.

Table 13

| Compound Number | Cry-SmC$_A$* | SmC$_A$*-SmC | SmC$_A$*-SmA or SmC* | SmA-Iso |
|---|---|---|---|---|
| (267) | -5°C | - | 44°C | 87°C |
| (251) | 21°C | - | 99°C | 130°C |
| (267)50%+(251)50% | 18°C | 70°C | 73°C | 110°C |

Example 21

The compound (296) represented by the following formula [296] obtained in Example 14 was mixed with compound (A) represented by the following formula [A] in a weight ratio of 50:50 to obtain a liquid crystal composition of the present invention.

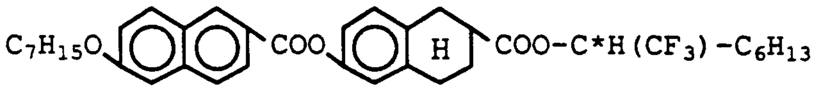
... [296]

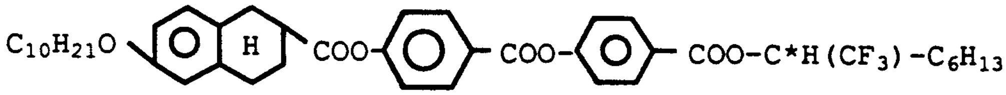
[A]

The phase transition temperatures of this composition measured are shown in Table 14.

Table 14

| Compound Number | Cry-SmC$_A$* or SmA or Iso | SmC$_A$*-SmA | SmA-Iso |
|---|---|---|---|
| (296) | 80°C | - | - |
| (A) | 44°C | 78°C | 94°C |
| (296)50%+(A)50% | 16°C | - | 81°C |

Example 22

The compound (331) represented by the following formula [331] obtained in Example 16 was mixed with the compound (251) represented by the following formula [251] obtained in Example 11 in a weight ratio of 10:90 to obtain a liquid crystal composition of the present invention.

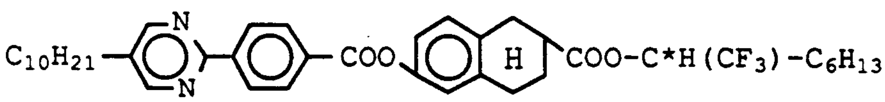
... [331]

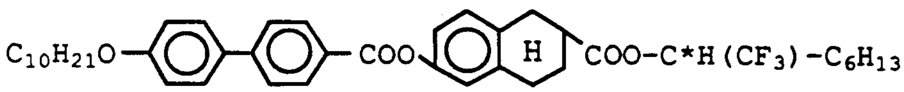
... [251]

The phase transition temperatures of this composition measured are shown in Table 15.

Table 15

| Compound Number | Cry-SmC$_A$* | SmC$_A$*-SmC | SmC$_A$* or SmC*-SmA | SmA-Iso |
|---|---|---|---|---|
| (331) | 48°C | - | - | 82°C |
| (251) | 21°C | - | 99°C | 130°C |
| (331)10%+(251)90% | 36°C | 85°C | 90°C | 124°C |

Example 23

Synthesis of 6-[4'-(4"-decyloxybenzoyloxy)benzoyloxy]-1,2,3,4-tetrahydronaphthalene-2-carboxylic acid R-1'"-trifluoromethylheptyl ester [Compound (404)]

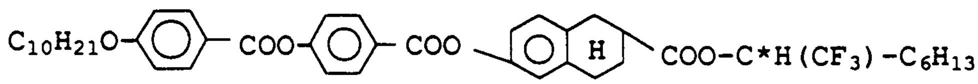

... [404]

First stage

To a mixture of 3.86g (11.8 mmol) of 6-n-decyloxynaphthalene-2-carboxylic acid and 130 ml of 1,2-diethoxyethane was added 3.0 g (130 mg atom) of metallic sodium with stirring in a nitrogen atmosphere at 120 °C, and the resulting mixture was further heated up to the reflux temperature.

To this mixture was added dropwise 10 g (114 mmol) of isoamyl alcohol over a period of 1 hour, and the resulting mixture was allowed to undergo reaction under reflux for 11 hours. After cooling the reaction system to room temperature, the remaining metallic sodium was converted to alcholate by the addition of ethanol, and the reaction mixture was acidified with 20 % hydrochloric acid.

To the reaction mixture was added 100 ml of water, and then the organic phase was separated and washed with water.

The organic phase was concentrated under reduced pressure to obtain 4.25 g of a solid, which was recrystallized from toluene to obtain 2.95 g (8.89 mmol) of 1,2,3,4-tetrahydro-6-n-decyloxynaphthalene-2-carboxylic acid.

Second stage

A mixture of 16.6 g (50 mmol) of the 1,2,3,4-tetrahydro-6-decyloxynaphthalene-2-carboxylic acid obtained in the first stage, 250 cc of acetic acid and 86.5 g (0.5 mol) of 47 % hydrobromic acid was heated under reflux at 130°C for 7 hours. To the reaction mixture was added distilled water, and then the resulting mixture was concentrated under reduced pressure to obtain 10.60 g (50 mmol) of 1,2,3,4-tetrahydro-6-hydroxynaphthalene-2-carboxylic acid.

Third stage

A mixture of 10.60 g (50 mmol) of the 1,2,3,4-tetrahydro-6-hydroxynaphthalene-2-carboxylic acid obtained in the second stage, 12.85 g (75 mmol) of benzyl bromide, 6.6 g (100 mmol) of 85 % potassium hydroxide, 0.525 g (3.5 mmol) of sodium iodide , 200 ml of ethanol and 25 ml of distilled water was heated under reflux at 100°C for 12 hours. After 50 ml of 10 % potassium hydroxide was added, the mixture was further heated under reflux for 2 hours. After allowing the reaction system to cool up to room temperature, the reaction mixture in cold water was acidified with 36 % hydrochloric acid. The deposited product was separated by filtration and recrystallized from toluene to obtain 13.08 g (46.4 mmol) of 1,2,3,4-tetrahydro-6-benzyloxynaphthalene-2-carboxylic acid.

Fourth stage

To a mixture of 5.64 g (20 mmol) of the 1,2,3,4-tetrahydro-6-benzyloxynaphthalene-2-carboxylic acid obtained in the third stage, 3.68 g (20 mmol) of R-1-trifluoromethylheptanol, 0.244 g (0.2 mmol) of 4-N,N-dimetylaminopyridine and 70 ml of methylene chloride was added dropwise 25 ml of a methylene chloride solution containing 4.53 g (22 mmol) of N,N'-dicyclohexylcarbodiimide with stirring at room temperature over a period of 2 hours.

Further, the reaction was carried out at room temperature for 2 hours.

The reaction mixture was filtered, and the filtrate was concentrated. The concentrate obtained was separated by column chromatography to obtain 8.19 g (18.3 mmol) of 1,2,3,4-tetrahydro-6-benzyloxynaphthalene-2-carboxylic acid R-1'-trifluoromethylheptyl ester as a colorless liquid.

Fifth stage

Hydrogen gas was blown into a mixture of 8.19 g (18.3 mmol) of the 1,2,3,4-tetrahydro-6-benzyloxynaphthalene-2-carboxylic acid R-1'-trifluoromethylheptyl ester obtained in the fourth stage, 3.6 g of 5 % palladium/carbon as a catalyst and 50 ml of tetrahydrofuran with stirring at room temperature and ordinary pressure for 24 hours. The reaction mixture

was filtered using Celite as a filter aid, and the filtrate obtained was concentrated to obtain 6.78 g (18.3 mmol) of 1,2,3,4-tetrahydro-6-hydroxynaphthalene-2-carboxylic acid R-1'-trifluoromethylheptyl ester as a brown liquid.

Sixth stage

To a mixture of 0.29 g (0.8 mmol) of the 1,2,3,4-tetrahydro-6-hydroxynaphthalene-2-carboxylic acid R-1'-trifluoromethylheptyl ester obtained in the fifth stage, 0.32 g (0.8 mmol) of 4-(4'-decyloxybenzoyloxy)benzoic acid synthesized separately by conventional means, 0.01 g (0.08 mmol) of 4-N,N-dimethylaminopyridine and 10 ml of methylene chloride was added dropwise 5 ml of a methylene chloride solution containing 0.20 g (0.96 mmol) of N,N'-dicyclohexylcarbodiimide with stirring at room temperature over a period of 40 minutes.

Further, the reaction was carried out at room temperature for 3.5 hours.

The reaction mixture was filtered, and the filtrate was concentrated. The concentrate obtained was separated by column chromatography to obtain 0.38 g of a compound as a white solid.

This compound had a M/e value in FD-mass spectrum of 738.

Fig. 13 shows $^1$H-NMR spectrum of this compound.

From these analytical results, this compound was identified as the title compound as desired.

Example 24

Synthesis of 6-[4'-(4''-R-1'''-methylheptyloxy)benzoyloxy)benzoyloxy]-1,2,3,4-tetrahydronaphthalene-2-carboxylic acid R-1''''-trifluoromethylheptyl ester [Compound (545)]

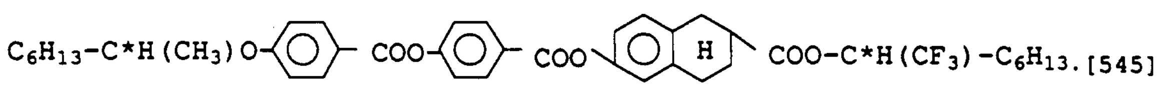 [545]

First stage

To a mixture of 0.50 g (2 mmol) of 4-(R-1'-methylheptyloxy)benzoic acid (manufactured by Arakawa Kagaku Kogyo K.K.), 0.46 g (2 mmol) of 4-hydroxybenzoic acid benzyl ester synthesized separately by conventional means, 0.02 g (0.2 mmol) of 4-N,N-dimethylaminopyridine and 20 ml of methylene chloride was added dropwise 8 ml of a methylene chloride solution containing 0.45 g (2.2 mmol) of N,N'-dicyclohexylcarbodiimide with stirring at room temperature over a period of 1 hour.

Further, the reaction was carried out at room temperature for 3 hours.

The reaction mixture was filtered, and the filtrate was concentrated. The concentrate obtained was separated by column chromatography to obtain 0.8 g (1.74 mmol) of 4-[4'-(R-1''-methylheptyloxy)benzoyloxy)benzoic acid] benzyl ester as a white solid.

Second stage

Hydrogen gas was blown into a mixture of 0.8 g (1.74 mmol) of the 4-[4'-(R-1''-methylheptyloxy)benzoyloxy)benzoic acid] benzyl ester obtained in the first stage, 0.16 g of 5 % palladium/carbon as a catalyst and 10 ml of tetrahydrofuran with stirring at room temperature and ordinary pressure for 16 hours. The reaction mixture was filtered using Celite as a filter aid, and the filtrate obtained was concentrated to obtain 0.66 g (1.74 mmol) of 4-[4'-(R-1''-methylheptyloxy)benzoyloxy] benzoic acid as a white solid.

Third stage

To a mixture of 0.30 g (0.8 mmol) of the 4-[4'-(R-1''-methylheptyloxy)benzoyloxy] benzoic acid obtained in the second stage, 0.29 g (0.8 mmol) of the 1,2,3,4-tetrahydro-6-hydroxynaphthalene-2-carboxylic acid R-1'-trifluoromethylheptyl ester obtained in the fifth stage of Example 23, 0.01 g (0.08 mmol) of 4-N,N-dimethylaminopyridine and 10 ml of methylene chloride was added dropwise 5 ml of a methylene chloride solution containing 0.18 g (0.88 mmol) of N.N'-dicyclohexylcarbodiimide with stirring at room temperature over a period of 1 hour.

Further, the reaction was carried out at room temperature for 3 hours.

The reaction mixture was filtered, and the filtrate was concentrated. The concentrate obtained was separated by column chromatography to obtain 0.48 g of a compound as a white solid.

This compound had a M/e value in FD-mass spectrum of 710.

Fig. 14 shows $^1$H-NMR spectrum of this compound.

From these analytical results, this compound was identified as the title compound as desired.

Example 25

Synthesis of 6-[4'-(4"-decyloxybenzoyloxy)benzoyloxy]-5,6,7,8-tetrahydronaphthalene-2-carboxylic acid R-1'''-trifluoromethylheptyl ester [Compound (575)]

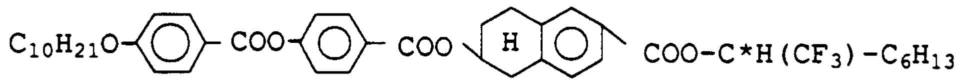

...[575]

First stage

In a 1-liter autoclave, 30 g (160 mmol) of 6-hydroxynaphthalene-2-carboxylic acid, 5 g of 5 % palladium/carbon as a catalyst and 500 ml of tetrahydrofuran were mixed together, and the mixture was heated in a nitrogen atmosphere up to 105 °C.

The mixture was allowed to undergo reaction in the autoclave maintained at a hydrogen pressure of 20 kg/cm2 G and a temperature of 105°C with stirring for 3 hours.

After a three-hour reaction, the reaction system was allowed to cool to room temperature, the hydrogen gas was released, the reaction mixture obtained was filtered using Celite as a filter aid, and the filtrate obtained was concentrated.

The mixture obtained was recrystallized from toluene to obtain 16.1 g of a compound as a white crystal.

This compound had a M/e value in FD-mass spectrum of 192.

From this analytical result, this compound was identified as 5,6,7,8-tetrahydro-6-hydroxynaphthalene-2-carboxylic acid.

Second stage

To a mixture of 2.88 g (15 mmol) of the 5,6,7,8-tetrahydro-6-hydroxynaphthalene-2-carboxylic acid obtained in the first stage, 2.76 g (15 mmol) of R-1-trifluoromethylheptanol, 0.183 g (1.5 mmol) of 4-N,N-dimethylaminopyridine and 50 ml of methylene chloride was added dropwise 20 ml of a methylene chloride solution containing 3.40 g (16.5 mmol) of N,N'-dicyclohexylcarbodiimide with stirring at room temperature over a period of 24 hours.

Further, 2 ml of a methylene chloride solution containing 0.41 g (2.0 mmol) of N,N'-dicyclohexylcarbodiimide was added thereto, and allowed to undergo reaction at room temperature for 72 hours.

The reaction mixture was filtered, and the filtrate was concentrated. The concentrate obtained was separated by column chromatography to obtain 3.11 g (8.69 mmol) of 5,6,7,8-tetrahydro-6-hydroxynaphthalene-2-carboxylic acid R-1'-trifluoromethylheptyl ester as a white solid.

Third stage

To a mixture of 0.29 g (0.8 mmol) of the 5,6,7,8-tetrahydro-6-hydroxynaphthalene-2-carboxylic acid R-1'-trifluoromethylheptyl ester obtained in the second stage, 0.32 g (0.8 mmol) of the 4-(4'-decyloxybenzoyloxy)benzoic acid synthesized separately by conventional means, 0.01 g (0.08 mmol) of 4-N,N-dimethylaminopyridine and 10 ml of methylene chloride was added dropwise 5 ml of a methylene chloride solution containing 0.20 g (0.96 mmol) of N,N'-dicyclohexylcarbodiimide with stirring at room temperature over a period of 1 hour.

Further, the reaction was carried out at room temperature for 4 hours.

The reaction mixture was filtered, and the filtrate was concentrated. The concentrate obtained was separated by column chromatography to obtain 0.23 g of a compound as a viscous semisolid.

This compound had a M/e value in FD-mass spectrum of 738.

Fig. 15 shows 1H-NMR spectrum of this compound.

From these analytical results, this compound was identified as the title compound as desired.

Example 26

Synthesis of 6-[4'-(4"-R-1'''-methylheptyloxy)benzoyloxy)benzoyloxy]-5,6,7,8-tetrahydronaphthalene-2-carboxylic acid R-1''''-trifluoromethylheptyl ester [Compound (716)]

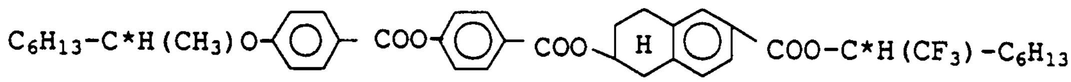 [716]

First stage

To a mixture of 0.30 g (0.8 mmol) of 4-[4'-(R-1"-methylheptyloxy)benzoyloxy]benzoic acid obtained in the second stage of Example 24, 0.29 g (0.8 mmol) of the 5,6,7,8-tetrahydro-6-hydroxynaphthalene-2-carboxylic acid R-1'-trifluoromethylheptyl ester obtained in the second stage of Example 25, 0.01 g (0.08 mmol) of 4-N,N-dimethylaminopyridine and 10 ml of methylene chloride was added dropwise 5 ml of a methylene chloride solution containing 0.18 g (0.88 mmol) of N,N'-dicyclohexylcarbodiimide with stirring at room temperature over a period of 1 hour.

Further, the reaction was carried out at room temperature for 14 hours.

The reaction mixture was filtered, and the filtrate was concentrated. The concentrate obtained was separated by column chromatography to obtain 0.34 g of a compound an a white solid.

This compound had a M/e value in FD-mass spectrum of 710.

Fig. 16 shows 1H-NMR spectrum of this compound.

From these analytical results, this compound was identified as the title compound as desired.

Example 27

The compound (404) represented by the following formula [404] obtained in Example 23 was mixed with compound (A) represented by the following formula [A] in a weight ratio of 50:50 to obtain a liquid crystal composition of the present invention.

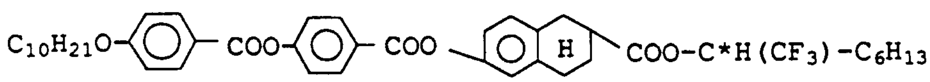 ...[404]

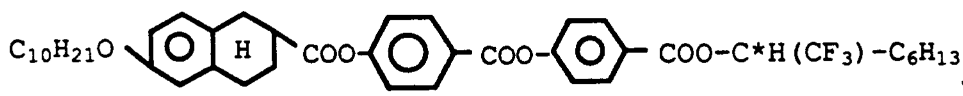 ....[A]

The phase transition temperatures of this composition were measured.

The results are shown in Table 16.

The phase transition temperature of the compound (404) and the compound (A) are also shown in Table 16.

Table 16

| Compound Number | Cry-$SmC_A$* | $SmC_A$*-SmC* | $SmC_A$* or SmC*-SmA | SmA-Iso |
|---|---|---|---|---|
| (404) | 50°C | 77°C | 85°C | 122°C |
| (A) | 44°C | - | 78°C | 94°C |
| (404)50%+(A)50% | <-30°C | - | 85°C | 120°C |

Example 28

The compound (545) represented by the following formula [545] obtained in Example 24 was mixed with compound (A) represented by the following formula [A] in a weight ratio of 50:50 to obtain a liquid crystal composition of the present invention.

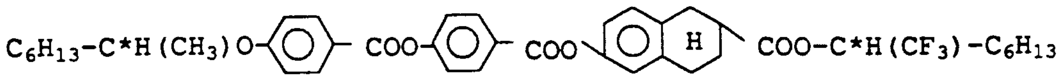 [545]

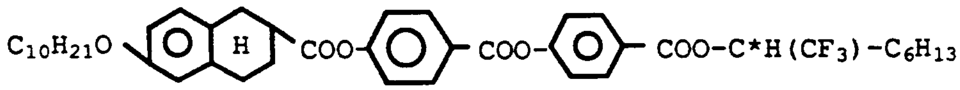 .... [A]

The phase transition temperatures of this composition were measured.
The results are shown in Table 17.
The phase transition temperature of the compound (545) and the compound (A) are also shown in Table 17.

Table 17

| Compound Number | Cry-$SmC_A$* or SmA or Iso | $SmC_A$*-SmA | SmA-Iso |
|---|---|---|---|
| (545) | 45°C | - | - |
| (A) | 44°C | 78°C | 94°C |
| (545)50%+(A)50% | <-30°C | - | 88°C |

Example 29

The compound (575) represented by the following formula [575] obtained in Example 25 was mixed with compound (A) represented by the following formula [A] in a weight ratio of 25:75 to obtain a liquid crystal composition of the present invention.

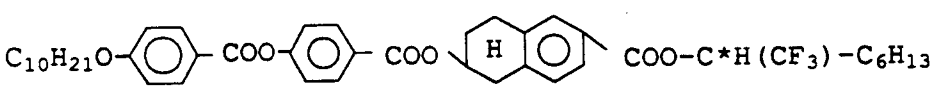 ... [575]

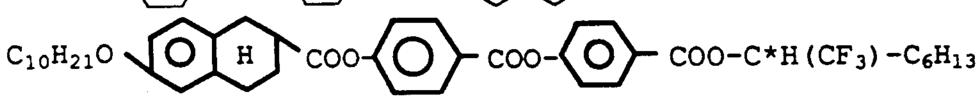 .... [A]

The phase transition temperatures of this composition were measured.
The results are shown in Table 18.
The phase transition temperature of the compound (575) and the compound (A) are also shown in Table 18.

Table 18

| Compound Number | Cry-$SmC_A$* | $SmC_A$*-SmA or Iso | SmA-Iso |
|---|---|---|---|
| (575) | 23°C | 57°C | - |
| (A) | 44°C | 78°C | 94°C |
| (575)75%+(A)25% | <-30°C | 66°C | 72°C |

Example 30

Synthesis of 6-(4'-decyloxybenzoyloxy)-1,2,3,4-tetrahydronaphthalene-2-carboxylic acid R-1"-trifluoromethylheptyl ester [Compound (235)]

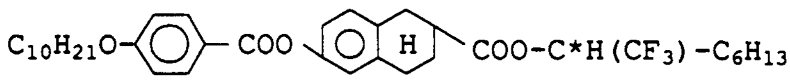 ... [235]

First stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.28g (1 mmol) of 4-decyloxybenzoic acid synthesized separately by conventional means, 0.012 g (0.1 mmol) of 4-N,N-dimethylaminopyridine and 10 ml of methylene chloride was added dropwise 5ml of a methylene chloride solution containing 0.25g (1.2 mmol) of N,N'-dicyclohexylcarbodiimide with stirring at room temperature over a period of 2 hours.

Further, the reaction was carried out at room temperature for 48 hours.

The reaction mixture was filtered, and the filtrate was concentrated. The concentrate was separated by column chromatography to obtain 0.50 g of a white solid.

This compound had a M/e value in FD-mass spectrum of 618.

Fig. 23 shows 1H-NMR spectrum of this compound.

From these analytical results, this compound was identified as the title compound as desired.

Example 31

Synthesis of 6-[4'-(4"-undecyloxybiphenyl)carboxy]-1,2,3,4-tetrahydronaphthalene-2-carboxylic acid R-1‴-trifluoromethylheptyl ester [Compound (252)]

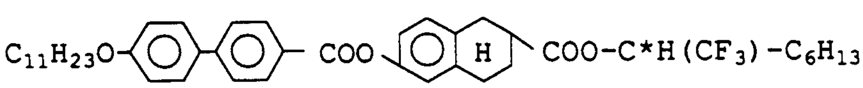 ... [252]

First stage

To a mixture of 0.76 g (2 mmol) of 1,2,3,4-tetrahydro-6-hydroxynaphthalene-2-carboxylic acid R-1'-trifluoromethylheptyl ester obtained in the fifth stage of Example 23, 0.773 g (2.1 mmol) of 4'-undecyloxybiphenyl-4-carboxylic acid prepared as in the first stage of Example 11, 0.024 g (0.2 mmol) of 4-N,N-dimethylaminopyridine and 20 ml of methylene chloride was added dropwise 10 ml of a methylene chloride solution containing 0.495 g (2.4 mmol) of N,N'-dicyclohexylcarbodiimide with stirring at room temperature over a period of 2 hours.

Further, the reaction was carried out at room temperature for 12 hours.

The reaction mixture was filtered, and the filtrate was concentrated. The concentrate was separated by column chromatography to obtain 1.06 g of a white solid.

This compound had a M/e value in FD-mass spectrum of 708.

Fig. 24 shows 1H-NMR spectrum of this compound.

From these analytical results, this compound was identified as the title compound as desired.

Example 32

Synthesis of 6-[4'-(4"-dodecyloxybiphenyl)carboxy]-1,2,3,4-tetrahydronaphthalene-2-carboxylic acid R-1'''-trifluoromethylheptyl ester [Compound (253)]

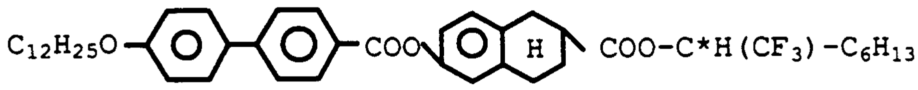
... [253]

First stage

To a mixture of 0.716 g (2 mmol) of 1,2,3,4-tetrahydro-6-hydroxynaphthalene-2-carboxylic acid R-1'-trifluoromethylheptyl ester obtained in the fifth stage of Example 23, 0.802 g (2.1 mmol) of 4'-dodecyloxybiphenyl-4-carboxylic acid prepared as in the first stage of Example 11, 0.024 g (0.2 mmol) of 4-N,N-dimethylaminopyridine and 20 ml of methylene chloride was added dropwise 10 ml of a methylene chloride solution containing 0.495 g (2.4 mmol) of N,N'-dicyclohexylcarbodiimide with stirring at room temperature over a period of 2 hours.

Further, the reaction was carried out at room temperature for 12 hours.

The reaction mixture was filtered, and the filtrate was concentrated. The concentrate was separated by column chromatography to obtain 1.08 g of a white solid.

This compound had a M/e value in FD-mass spectrum of 722.

Fig. 25 shows 1H-NMR spectrum of this compound.

From these analytical results, this compound was identified as the title compound as desired.

Example 33

Synthesis of 6-[4'-(4"-octylbiphenyl)carboxy]-1,2,3,4-tetrahydronaphthalene-2-carboxylic acid R-1'''-trifluoromethylheptyl ester

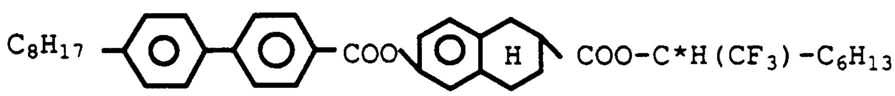

First stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.31 g (1 mmol) of 4'-octylbiphenyl-4-carboxylic acid (FK-1124-8 from Teikoku Kagaku Sangyo K.K.), 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2.5 hours.

Further, the reaction was carried out at room temperature for 36 hours.

The reaction mixture was filtered, and the filtrate was concentrated. The concentrate was separated by column chromatography to obtain 0.55 g of a colorless semisolid.

This compound had a M/e value in FD-mass spectrum of 650.

Fig. 26 shows 1H-NMR spectrum of this compound.

From these analytical results, this compound was identified as the title compound as desired.

Example 34

Synthesis of 6-[4'-(4''-decylbiphenyl)carboxy]-1,2,3,4-tetrahydronaphthalene-2-carboxylic acid R-1'''-trifluoromethylheptyl ester

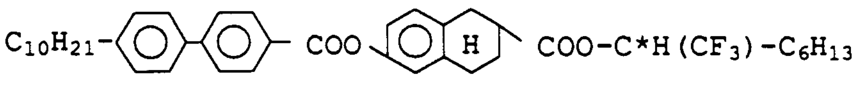

First stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.34 g (1 mmol) of 4'-decylbiphenyl-4-carboxylic acid (FK-1124-10 from Teikoku Kagaku Sangyo K.K.), 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2.5 hours.

Further, the reaction was carried out at room temperature for 20 hours.

The reaction mixture was filtered, and the filtrate was concentrated. The concentrate was separated by column chromatography to obtain 0.50 g of a colorless semisolid.

This compound had a M/e value in FD-mass spectrum of 678.

Fig. 27 shows 1H-NMR spectrum of this compound.

From these analytical results, this compound was identified as the title compound as desired.

Example 35

Synthesis of 6-[4'-(4''-dodecylbiphenyl)carboxy]-1,2,3,4-tetrahydronaphthalene-2-carboxylic acid R-1'''-trifluoromethylheptyl ester

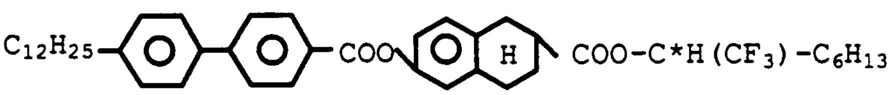

First stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.37 g (1 mmol) of 4'-dodecylbiphenyl-4-carboxylic acid (FK-1124-12 from Teikoku Kagaku Sangyo K.K.), 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 2.5 hours.

Further, the reaction was carried out at room temperature for 20 hours.

The reaction mixture was filtered, and the filtrate was concentrated. The concentrate was separated by column chromatography to obtain 0.54 g of a colorless semisolid.

This compound had a M/e value in FD-mass spectrum of 706.

Fig. 28 shows 1H-NMR spectrum of this compound.

From these analytical results, this compound was identified as the title compound as desired.

Example 36

Synthesis of 6-[4'-(4"-octylbiphenyl)carboxy]-1,2,3,4-tetrahydronaphthalene-2-carboxylic acid R-1'''-methylheptyl ester

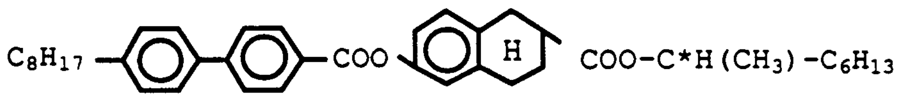

First stage

To a mixture of 0.31 g (1 mmol) of 1,2,3,4-tetrahydro-6-hydroxynaphthalene-2-carboxylic acid R-1'-methylheptyl ester obtained as in the fourth stage of Example 23 where R-1-methyl heputanol was used in stead of R-1-trifluoromethylheptanol, 0.31 g (1 mmol) of 4'-octylbiphenyl-4-carboxylic acid (FK-1124-8 from Teikoku Kagaku Sangyo K.K.), 0.02 g (0.16 mmol) of 4-N,N-dimethylaminopyridine and 15 ml of methylene chloride was added dropwise 3 ml of a methylene chloride solution containing 0.27 g (1.3 mmol) of N,N'-dicyclohexylcarbodiimide with stirring at room temperature over a period of 3 hours.

Further, the reaction was carried out at room temperature for 20 hours.

The reaction mixture was filtered, and the filtrate was concentrated. The concentrate was separated by column chromatography to obtain 0.45 g of a colorless semisolid.

This compound had a M/e value in FD-mass spectrum of 596.

Fig. 29 shows 1H-NMR spectrum of this compound.

From these analytical results, this compound was identified as the title compound as desired.

Example 37

Synthesis of 6-[4'-(4"-decylbiphenyl)carboxy]-1,2,3,4-tetrahydronaphthalene-2-carboxylic acid R-1'''-methylheptyl ester

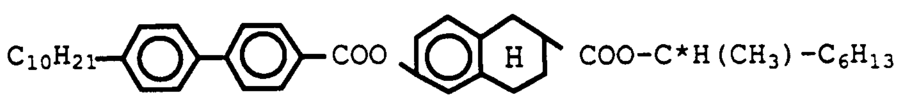

First stage

To a mixture of 0.37 g (1.1 mmol) of 1,2,3,4-tetrahydro-6-hydroxynaphthalene-2-carboxylic acid R-1'-methylheptyl ester obtained as in the fourth stage of Example 23 where R-1-methyl heputanol was used in stead of R-1-trifluoromethylheptanol, 0.33 g (1.1 mmol) of 4'-decylbiphenyl-4-carboxylic acid (FK-1124-10 from Teikoku Kagaku Sangyo K.K.), 0.013 g (0.11 mmol) of 4-N,N-dimethylaminopyridine and 10 ml of methylene chloride was added dropwise 5 ml of a methylene chloride solution containing 0.27 g (1.3 mmol) of N,N'-dicyclohexylcarbodiimide with stirring at room temperature over a period of 2.5 hours.

Further, the reaction was carried out at room temperature for 60 hours.

The reaction mixture was filtered, and the filtrate was concentrated. The concentrate was separated by column chromatography to obtain 0.60 g of a colorless semisolid.

This compound had a M/e value in FD-mass spectrum of 624.

Fig. 30 shows 1H-NMR spectrum of this compound.

From these analytical results, this compound was identified as the title compound as desired.

Example 38

Synthesis of 6-[4'-(4''-(S-1'''-methylheptyloxy)biphenyl)carboxy]-1,2,3,4-tetrahydronaphthalene-2-carboxylic acid R-1''''-trifluoromethylheptyl ester

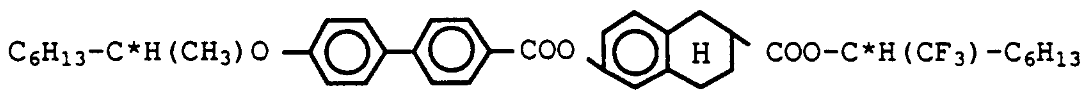

First stage

To a mixture of 0.358 g (1 mmol) of 1,2,3,4-tetrahydro-6-hydroxynaphthalene-2-carboxylic acid R-1'-trifluoromethylheptyl ester obtained in the fifth stage of Example 23, 0.326 g (1 mmol) of 4'-(S-1''-methylheptyloxy)biphenyl-4-carboxylic acid (KB-502-8(S) from Arakawa Kagaku kogyo K.K.), 0.012 g (0.12 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 3 hours.

Further, the reaction was carried out at room temperature for 47 hours.

The reaction mixture was filtered, and the filtrate was concentrated. The concentrate was separated by column chromatography to obtain 0.54 g of a colorless semisolid.

This compound had a M/e value in FD-mass spectrum of 666.

Fig. 31 shows 1H-NMR spectrum of this compound.

From these analytical results, this compound was identified as the title compound as desired.

Example 39

Synthesis of 6-[4'-(4''-octyloxycyclohexyl)carboxy]-1,2,3,4-tetrahydronaphthalene-2-carboxylic acid R-1'''-trifluoromethylheptyl ester [Compound (265)]

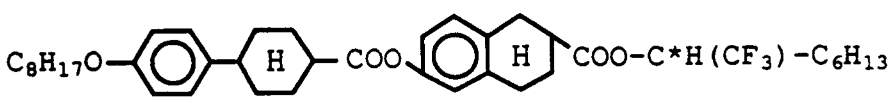

... [265]

First stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.33 g (1 mmol) of 4'-octyloxycyclohexane-4-carboxylic acid obtained as in the first stage of Example 12 where n-octyl bromide was used instead of n-decyl bromide, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 4 hours.

Further, the reaction was carried out at room temperature for 20 hours.

The reaction mixture was filtered, and the filtrate was concentrated. The concentrate was separated by column chromatography to obtain 0.35 g of a colorless semisolid.

This compound had a M/e value in FD-mass spectrum of 672.

Fig. 32 shows 1H-NMR spectrum of this compound.

From these analytical results, this compound was identified as the title compound as desired.

Example 40

Synthesis of 6-[4'-(4"-undecyloxyphenylcyclohexyl)carboxy]-1,2,3,4-tetrahydronaphthalene-2-carboxylic acid R-1'''-trifluoromethylheptyl ester [Compound (269)]

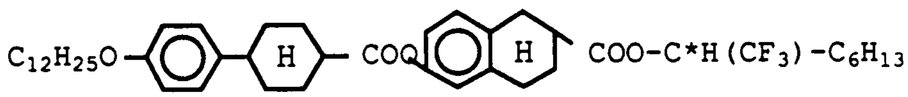
... [269]

First stage

To a mixture of 0.54 g (1.5 mmol) of 1,2,3,4-tetrahydro-6-hydroxynaphthalene-2-carboxylic acid R-1'-trifluoromethylheptyl ester obtained in the fifth stage of Example 23, 0.61 g (1.7 mmol) of 4'-undecyloxyphenylcyclohexane-4-carboxylic acid obtained as in the first stage of Example 12 where n-undecyl bromide was used instead of n-decyl bromide, 0.018 g (0.15 mmol) of 4-N,N-dimethylaminopyridine and 20 ml of methylene chloride was added dropwise 10 ml of a methylene chloride solution containing 0.37 g (1.8 mmol) of N,N'-dicyclohexylcarbodiimide with stirring at room temperature over a period of 4 hours.

Further, the reaction was carried out at room temperature for 20 hours.

The reaction mixture was filtered, and the filtrate was concentrated. The concentrate was separated by column chromatograptiy to obtain 0.53 g of a colorless semisolid.

This compound had a M/e value in FD-mass spectrum of 700.

Fig. 33 shows 1H-NMR spectrum of this compound.

From these analytical results, this compound was identified as the title compound as desired.

Example 41

Synthesis of 6-[4'-(4"-decylphenyltranscyclohexyl)carboxy]-1,2,3,4-tetrahydronaphthalene-2-carboxylic acid R-1'''-trifluoromethylheptyl ester

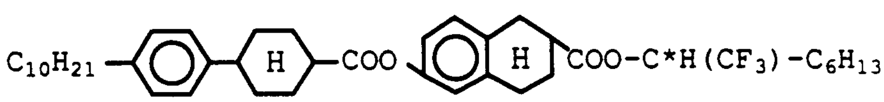

First stage

A mixture of 5.07 g (15 mmol) of 4'-decylbiphenyl-4-carboxylic acid (FK-1124-10 from Teikoku Kagaku Sangyo K.K.), 4.49 g (0.195 mol) of metallic sodium and 200 ml of 1,2-diethoxyethane was heated at 150°C for 4 hours while stirring. To the mixture was added dropwise 20 g of isoamyl alcohol over a period of 8 hours under reflux at 150°C and while stirring, followed by stirring for further 2 hours under reflux.

After cooling the reaction mixture, 80 ml of ethanol and 100ml of distilled water were added to convert the remaining metallic sodium into alcholate. The aqueous phase separated was neutralized by the addition of hydrochloric acid to form a precipitate, which was then purified by column chromatography to separate into cis and trans isomers. Yield was 2.24 g of 4'-decylphenyltranscyclohexane-4-carboxylic acid as desired.

Second stage

To a mixture of 0.36 g (1 mmol) of 1,2,3,4-tetrahydro-6-hydroxynaphthalene-2-carboxylic acid R-1'-trifluoromethylheptyl ester obtained in the fifth stage of Example 23, 0.39 g (1.1 mmol) of 4'-decylphenyltranscyclohexane-4-carboxylic acid obtained in the first stage, 0.012 g (0.1 mmol) of 4-N,N-dimethylaminopyridine and 10 ml of methylene chloride was added dropwise 5 ml of a methylene chloride solution containing 0.25 g (1.2 mmol) of N,N'-dicyclohexylcarbodiimide with stirring at room temperature over a period of 4 hours.

Further, the reaction was carried out at room temperature for 19 hours.

The reaction mixture was filtered, and the filtrate was concentrated. The concentrate was separated by column chromatography to obtain 0.59 g of a colorless semisolid.

This compound had a M/e value in FD-mass spectrum of 684.

Fig. 34 shows 1H-NMR spectrum of this compound.

From these analytical results, this compound was identified as the title compound as desired.

Example 42

Synthesis of 6-[4'-(4"-decylbenzoyloxy)-1',2',3',4'-tetrahydro-2'-naththoyloxy]benzoic acid R-1'''-trifluoromethylheptyl ester

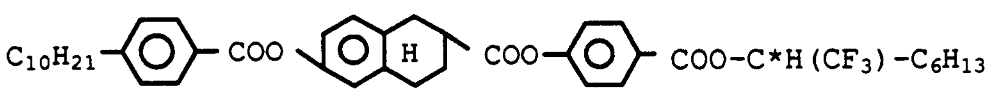

First stage

To a mixture of 0.69 g (1.46 mmol) of 4-(1',2',3',4'-tetrahydro-6'-hydroxy-2'-naphthoyloxy)benzoic acid R-1"-trifluoromethylheptyl ester obtaind in the fifth stage of Example 8, 0.39 g (1.5 mmol) of 4-decylbenzoic acid synthesized separately by conventional means, 0.018 g (0.15 mmol) of 4-N,N-dimethylaminopyridine and 30 ml of methylene chloride was added dropwise 10 ml of a methylene chloride solution containing 0.37 g (1.8 mmol) of N,N'-dicyclohexylcarbodiimide with stirring at room temperature over a period of 4 hours.

Further, the reaction was carried out at room temperature for 20 hours.

The reaction mixture was filtered, and the filtrate was concentrated. The concentrate was separated by column chromatography to obtain 0.39 g of a colorless semisolid.

This compound had a M/e value in FD-mass spectrum of 718.

Fig. 33 shows 1H-NMR spectrum of this compound.

From the analytical result, this compound was identified as the title compound as desired.

**Claims**

1. A carboxylic acid ester compound represented by the following formula (I) or (II)

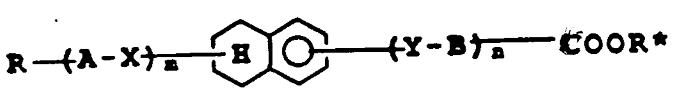

... (I)

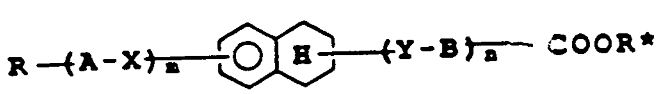

... (II)

wherein

R is a group selected from an alkyl of 3-20 carbon atoms, an alkoxy of 3-20 carbon atoms and a halogenated alkyl of 3-20 carbon atoms,

X and Y are independently a group selected from -COO-, -OCO-, $-CH_2CH_2-$, $-CH_2O-$, $-OCH_2-$, -S-S-, $-CO-CH_2-$, and $-CH_2-CO-$, or a single bond,

A is a group selected from

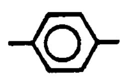 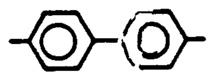 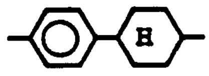 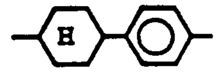

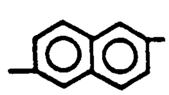 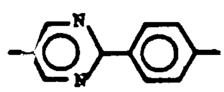 and 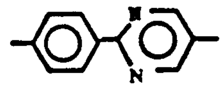

B is a group selected from

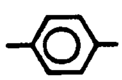 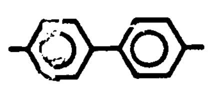 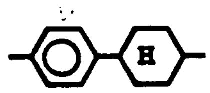 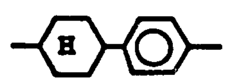 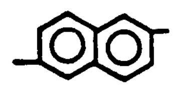

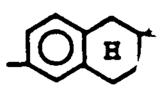 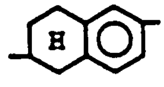 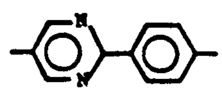 and 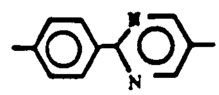

R* is an optically active group of 4-20 carbon atoms containing at least one asymmetric carbon atom where hydrogen atoms attached to the carbon atoms constituting said optically active group may be substituted with a halogen atom, m is 1 or 2, and n is an integer of 0-2.

**2.** A compound according to claim 1 where R * is a group represented by the following formula (V)

$$-Q^1-C^*H(Q^2)-Q^3 \qquad (V)$$

wherein

$Q^1$ is $-(CH_2)_q-$ in which q is an integer of 0-6, and
$Q^2$ and $Q^3$ are different from each other, and are each independently an alkyl of 1-10 carbon atoms, a fluoroalkyl of 1-10 carbon atoms or a halogen atom, and
at least one of the groups $CH_2$ or $CF_2$ in the $Q^1$, $Q^2$ and $Q^3$ may be replaced by at least one group selected from -O-, -S-, -CO-, -CHX- in which X is a halogen atom, -CHCN-, -O-CO-, -O-COO-, -CO-O- and -CH=CH- in such a manner that two hetero atoms do not bond directly.

**3.** A compound according to claim 2 wherein R* is $-C^*H(CF_3)-C_6H_{13}$, $-C^*H(CH_3)-C_6H_{13}$, $-C^*H(CH_3)-C_5H_{11}$, $-C^*H(C_2H_5)-C_5H_{11}$, $-C^*H(C_2H_5)-C_6H_{13}$, $-CH_2-C^*H(CH_3)-C_2H_5$, $-(CH_2)_3-C^*H(CH_3)-C_2H_5$ or $-C^*H(CF_3)-CH_2-COO-C_2H_5$.

**4.** A compound according to claim 1, 2 or 3 wherein X and Y are independently a group -COO-, -OCO- or a single bond.

**5.** A compound according to any one of the preceding claims wherein R is an alkoxy.

**6.** A compound according to any one of the preceding claims wherein R has an asymmetric carbon atom.

**7.** A compound according to any one of the preceding claims wherein A is a group selected from

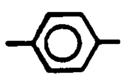 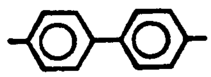 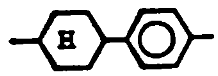

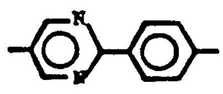 and 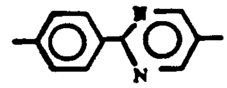

8. A carboxylic acid ester compound represented by the following formula (III) or (IV)

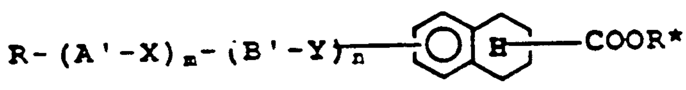 ... (III)

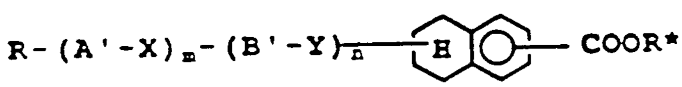 ... (IV)

wherein

R is a group selected from an alkyl of 3-20 carbon atoms, an alkoxy of 3-20 carbon atoms, a halogenated alkyl of 3-20 carbon atoms and a hydrocarbon group having -OCO- group wherein at least one of the hydrogen atoms constituting said hydrocarbon group may be substituted with halogen atoms, and R may have at least one asymmetric carbon atom,
X and Y are independently a group selected from -COO-, -OCO-,$-CH_2CH_2-$,$-CH_2O-$, $-OCH_2-$, -S-S-, $-CO-CH_2-$ and $-CH_2-CO-$, or a single bond,
A' and B' are independently a group selected from

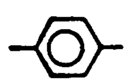 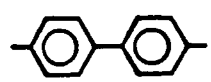 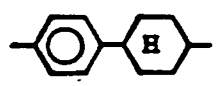 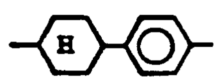

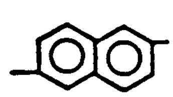 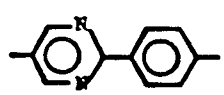 and 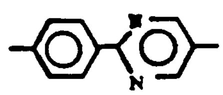

R* is an optically active group of 4-20 carbon atoms containing at least one asymmetric carbon atom where hydrogen atoms attached to the carbon atoms constituting said optically active group may be substituted with a halogen atom,
m is 1 or 2, n is an integer of 0-2 and $m+n \geq 2$.

9. A compound according to claim 8 wherein R* is a group represented by the following formula (V)

$$-Q^1-C^*H(Q^2)-Q^3 \qquad (V)$$

wherein

$Q^1$ is $-(CH_2)_q-$ in which q is an integer of 0-6, and
$Q^2$ and $Q^3$ are different from each other, and are each independently an alkyl of 1-10 carbon atoms, a fluoroalkyl of 1-10 carbon atoms or a halogen atom, and
at least one of the groups $CH_2$ or $CF_2$ in $Q^1$, $Q^2$ and $Q^3$ may be replaced by at least one group selected from -O-, -S-, -CO-, -CHX- in which X is a halogen atom, -CHCN-, -O-CO-, O-COO-, -CO-O- and -CH=CH- in such a manner that the two hereto atoms do not bond directly.

**10.** A compound according to claim 8 or 9 wherein R* is a group selected from $-C^*H(CF_3)-C_6H_{13}$, $-C^*H(CH_3)-C_6H_{13}$, $-C^*H(CH_3)-C_5H_{11}$, $-C^*H(C_2H_5)-C_5H_{11}$, $-C^*H(C_2H_5)-C_6H_{13}$, $-CH_2-C^*H(CH_3)-C_2H_5$, $-(CH_2)_3-C^*H(CH_3)C_2H_5$ and $-C^*H(CF_3)-CH_2-COO-C_2H_5$.

**11.** A compound according to any one of claims 8 to 10 wherein R is an alkoxy optionally having an asymmetric carbon atom.

**12.** A compound according to any one of claim 8 to 11 wherein R is a group having an asymmetric carbon atom.

**13.** A compound according to any one of claims 8 to 12 wherein X and Y are independently a group -COO-, -OCO- or a single bond.

**14.** A compound according to any one of claims 8 to 13 wherein A is selected from

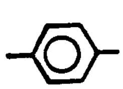 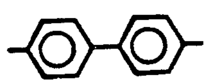 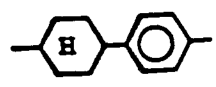

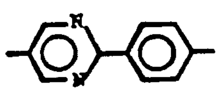 and 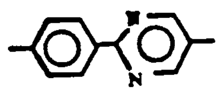

**15.** A liquid crystal material comprising a compound as claimed in any one of the preceding claims.

**16.** A liquid crystal composition comprising a compound as claimed in any one of claims 1 to 14.

**17.** A liquid crystal element comprising a cell composes of two substrates opposite to each other, a gap formed by said substrates and a liquid crystal substance as claimed in any one of claims 1 to 14 filled in said gap.

**Patentansprüche**

**1.** Carbonsäureesterverbindung, angegeben durch die folgende Formel (I) oder (II)

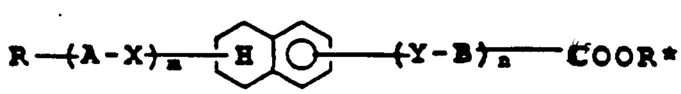 ... (I)

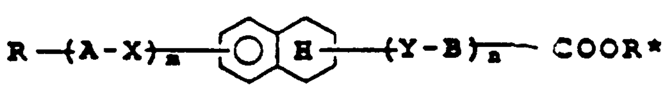 ... (II)

wobei

R eine Gruppe ist, ausgewählt aus einer Alkylgruppe mit 3-20 Kohlenstoffatomen, einer Alkoxygruppe mit 3-20 Kohlenstoffatomen und einer halogenierten Alkylgruppe mit 3-20 Kohlenstoffatomen,

X und Y unabhängig eine Gruppe sind, ausgewählt aus -COO-, -OCO-, $-CH_2CH_2-$, $-CH_2O-$, $-OCH_2-$, -S-S-, $-CO-CH_2-$ und $-CH_2-CO-$, oder eine Einfachbindung,

A eine Gruppe ist, ausgewählt aus

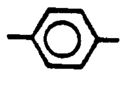 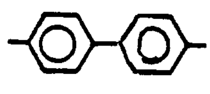 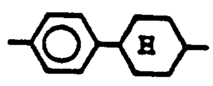 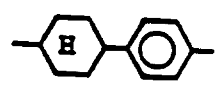

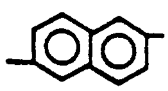 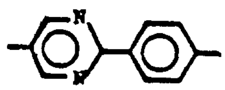 und 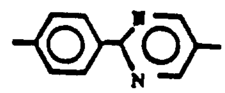

B eine Gruppe ist, ausgewählt aus

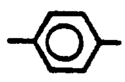 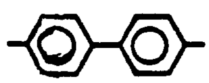 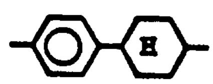 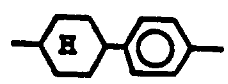 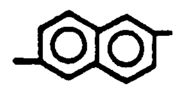

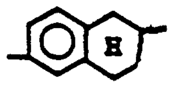 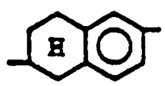 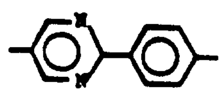 und 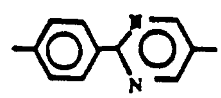

R* eine optisch aktive Gruppe mit 4-20 Kohlenstoffatomen ist, enthaltend mindestens ein asymmetrisches Kohlenstoffatom, wobei Wasserstoffatome, die an die die optische aktive Gruppe bildenden Kohlenstoffatome gebunden sind, durch ein Halogenatom ersetzt sein können, m 1 oder 2 ist und n eine ganze Zahl von 0-2 ist.

**2.** Verbindung nach Anspruch 1, wobei R* eine Gruppe ist, angegeben durch die folgende Formel (V)

$$-Q^1\text{-}C^*H(Q^2)\text{-}Q^3 \qquad (V)$$

wobei

$Q^1$ $-(CH_2)_q-$ ist, wobei q eine ganze Zahl von 0-6 ist, und

$Q^2$ und $Q^3$ voneinander verschieden sind und jeweils unabhängig eine Alkylgruppe mit 1-10 Kohlenstoffatomen, eine Fluoralkylgruppe mit 1-10 Kohlenstoffatomen oder ein Halogenatom bedeuten, und

mindestens eine der Gruppen $CH_2$ oder $CF_2$ in $Q^1$, $Q^2$ und $Q^3$ ersetzt sein kann durch mindestens eine Gruppe, ausgewählt aus -O-, -S-, -CO-, -CHX-, wobei X ein Halogenatom ist, -CHCN-, -O-CO-, -O-COO-, -CO-O- und -CH=CH-, so daß zwei Heteroatome nicht direkt aneinander gebunden sind.

**3.** Verbindung nach Anspruch 2, wobei R* $-C^*H(CF_3)-C_6H_{13}$, $-C^*H(CH_3)-C_6H_{13}$, $-C^*H(CH_3)-C_5H_{11}$, $-C^*H(C_2H_5)-C_5H_{11}$, $-C^*H(C_2H_5)-C_6H_{13}$, $-CH_2-C^*H(CH_3)-C_2H_5$, $-(CH_2)_3-C^*H(CH_3)-C_2H_5$ oder $-C^*H(CF_3)-CH_2-COO-C_2H_5$ ist.

**4.** Verbindung nach Anspruch 1, 2 oder 3, wobei X und Y unabhängig eine Gruppe -COO-, -OCO- oder eine Einfachbindung sind.

**5.** Verbindung nach einem der vorangehenden Ansprüche, wobei R eine Alkoxygruppe ist.

**6.** Verbindung nach einem der vorangehenden Ansprüche, wobei R ein asymmetrisches Kohlenstoffatom enthält.

**7.** Verbindung nach einem der vorangehenden Ansprüche, wobei A eine Gruppe ist, ausgewählt aus

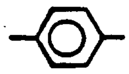 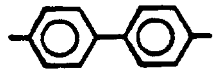 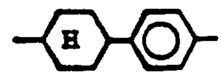

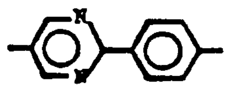 und 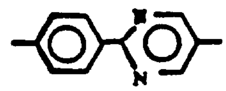

8. Carbonsäureesterverbindung, angegeben durch die folgende Formel (III) oder (IV)

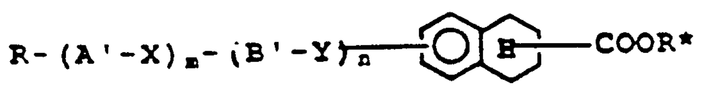 ... (III)

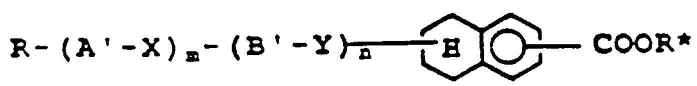 ... (IV)

wobei

R eine Gruppe ist, ausgewählt aus einer Alkylgruppe mit 3-20 Kohlenstoffatomen, einer Alkoxygruppe mit 3-20 Kohlenstoffatomen, einer halogenierten Alkylgruppe mit 3-20 Kohlenstoffatomen und einer Kohlenwasserstoffgruppe mit einer -OCO- Gruppe, wobei mindestens eines der Wasserstoffatome, die die Kohlenwasserstoffgruppe bilden, durch Halogenatome ersetzt sein kann, und R mindestens ein asymmetrisches Kohlenstoffatom enthalten kann,

X und Y unabhängig eine Gruppe sind, ausgewählt aus -COO-, -OCO-, $-CH_2CH_2-$, $-CH_2O-$, $-OCH_2-$, -S-S-, $-CO-CH_2-$ und $-CH_2-CO-$, oder eine Einfachbindung,

A' und B' unabhängig eine Gruppe sind, ausgewählt aus

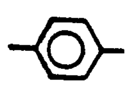 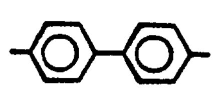 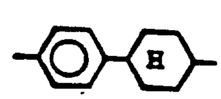 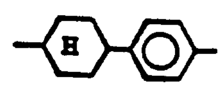

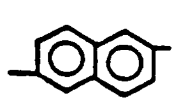 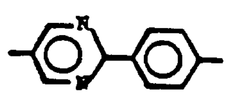 und 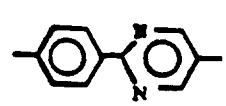

R* eine optisch aktive Gruppe mit 4-20 Kohlenstoffatomen ist, enthaltend mindestens ein asymmetrisches Kohlenstoffatom, wobei Wasserstoffatome, die an die die optische aktive Gruppe bildenden Kohlenstoffatome gebunden sind, durch ein Halogenatom ersetzt sein können, m 1 oder 2 ist, n eine ganze Zahl von 0-2 ist und $m+n \geq 2$ ist.

9. Verbindung nach Anspruch 8, wobei R* eine Gruppe ist, angegeben durch die folgende Formel (V)

$$-Q^1-C^*H(Q^2)-Q^3 \quad (V)$$

wobei

$Q^1$ $-(CH_2)_q-$ ist, wobei q eine ganze Zahl von 0-6 ist, und

$Q^2$ und $Q^3$ voneinander verschieden sind und jeweils unabhängig eine Alkylgruppe mit 1-10 Kohlenstoffatomen, eine Fluoralkylgruppe mit 1-10 Kohlenstoffatomen oder ein Halogenatom bedeuten, und

mindestens eine der Gruppen $CH_2$ oder $CF_2$ in $Q^1$, $Q^2$ und $Q^3$ ersetzt sein kann durch mindestens eine Gruppe, ausgewählt aus -O-, -S-, -CO-, -CHX-, wobei X ein Halogenatom ist, -CHCN-, -O-CO-, -O-COO-, -CO-O- und -CH=CH-, so daß zwei Heteroatome nicht direkt aneinander gebunden sind.

**10.** Verbindung nach Anspruch 8 oder 9, wobei R* eine Gruppe ist, ausgewählt aus $-C^*H(CF_3)-C_6H_{13}$, $-C^*H(CH_3)-C_6H_{13}$, $-C^*H(CH_3)-C_5H_{11}$, $-C^*H(C_2H_5)-C_5H_{11}$, $-C^*H(C_2H_5)-C_6H_{13}$, $-CH_2-C^*H(CH_3)-C_2H_5$, $-(CH_2)_3-C^*H(CH_3)C_2H_5$ und $-C^*H(CF_3)-CH_2-COO-C_2H_5$.

**11.** Verbindung nach einem der Ansprüche 8 bis 10, wobei R eine Alkoxygruppe ist, die gegebenenfalls ein asymmetrisches Kohlenstoffatom enthält.

**12.** Verbindung nach einem der Ansprüche 8 bis 11, wobei R eine Gruppe mit einem asymmetrischen Kohlenstoffatom ist.

**13.** Verbindung nach einem der Ansprüche 8 bis 12, wobei X und Y unabhängig eine Gruppe -COO-, -OCO- oder eine Einfachbindung sind.

**14.** Verbindung nach einem der Ansprüche 8 bis 13, wobei A ausgewählt ist aus

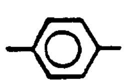 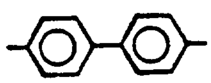 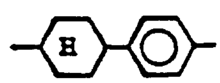

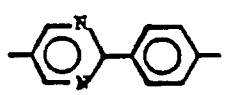 und 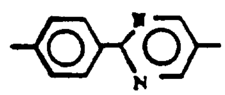

**15.** Flüssigkristallmaterial, umfassend eine Verbindung nach einem der vorangehenden Ansprüche.

**16.** Flüssigkristallmasse, umfassend eine Verbindung nach einem der Ansprüche 1 bis 14.

**17.** Flüssigkristallelement, umfassend eine Zelle, gebildet aus zwei Substraten, die einander gegenüberliegen, einem Zwischenraum, der durch die Substrate gebildet wird, und einer Flüssigkristallsubstanz nach einem der Ansprüche 1 bis 14, die in den Zwischenraum eingefüllt ist.

## Revendications

**1.** Ester d'acide carboxylique représenté par la formule (I) ou (II) suivante :

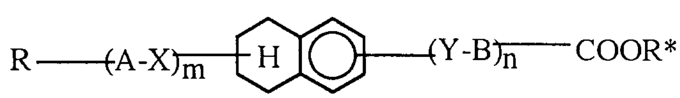 ... (I)

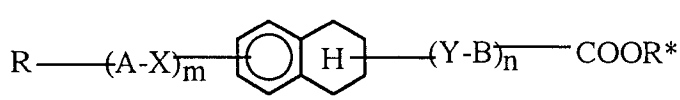 ... (II)

formules dans lesquelles

R représente un groupe alkyle ayant 3-20 atomes de carbone, un groupe alcoxy ayant 3-20 atomes de carbone ou un groupe alkyle halogéné ayant 3-20 atomes de carbone,

X et Y représentent chacun indépendamment un groupe choisi parmi -COO-, -OCO-, $-CH_2CH_2-$, $-CH_2O-$, $-OCH_2-$, -S-S-, $-CO-CH_2-$, et $-CH_2-CO-$, ou une liaison simple,

A représente un groupe choisi parmi

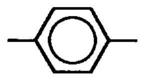 , 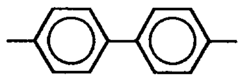 , 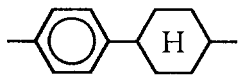 , 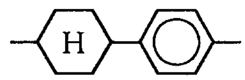 ,

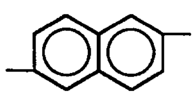 , 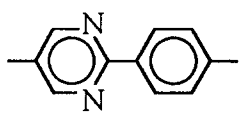 et 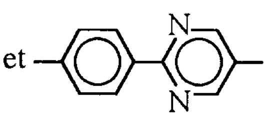 ,

B représente un groupe choisi parmi

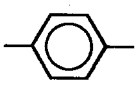 , 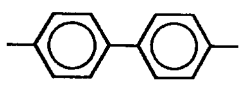 , 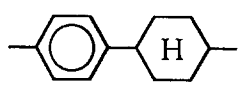 , 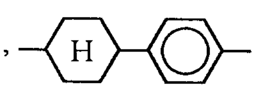 , 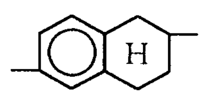 ,

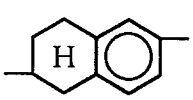 , 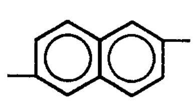 , 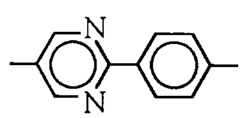 et 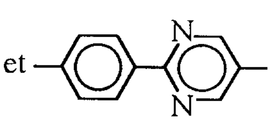 ,

R* représente un groupe optiquement actif, ayant 4-20 atomes de carbone et contenant au moins un atome de carbone asymétrique, des atomes d'hydrogène liés aux atomes de carbone constituant ledit groupe optiquement actif pouvant être remplacés par un atome d'halogène, m est égal à 1 ou 2, et n représente un nombre entier ayant une valeur de 0 à 2.

2. Composé selon la revendication 1, dans lequel R* est un groupe représenté par la formule (V) suivante :

$$-Q^1-C^*H(Q^2)-Q^3 \qquad (V)$$

dans laquelle

$Q^1$ représente un groupe $-(CH_2)_q-$ dans lequel q est un nombre entier ayant une valeur de 0-6,
$Q^2$ et $Q^3$ sont différents l'un de l'autre et représentent chacun indépendamment un groupe alkyle ayant 1-10 atomes de carbone, un groupe fluoroalkyle ayant 1-10 atomes de carbone ou un atome d'halogène, et
au moins un des groupes $CH_2$ ou $CF_2$ de $Q^1$, $Q^2$ et $Q^3$ peut être remplacé par au moins un groupe choisi parmi -O-, -S-, -CO-, -CHX- dans lequel X représente un atome d'halogène, -CHCN-, -O-CO-, -O-COO-, -CO-O- et -CH=CH-, de telle manière que deux hétéroatomes ne sont pas reliés directement.

3. Composé selon la revendication 2, dans lequel R* représente :
$-C^*H(CF_3)-C_6H_{13}$, $-C^*H(CH_3)-C_6H_{13}$, $-C^*H(CH_3)-C_5H_{11}$,

$-C^*H(C_2H_5)-C_5H_{11}$, $-C^*H(C_2H_5)-C_6H_{13}$, $-CH_2-C^*H(CH_3)-C_2H_5$, $-(CH_2)_3-C^*H(CH_3)-C_2H_5$, ou $-C^*H(CF_3)-CH_2-COO-C_2H_5$.

4. Composé selon l'une quelconque des revendications 1, 2 et 3, dans lequel X et Y représentent chacun indépendamment un groupe -COO- ou -OCO- ou une liaison simple.

5. Composé selon l'une quelconque des revendications, précédentes dans lequel R représente un groupe alcoxy.

6. Composé selon l'une quelconque des revendications précédentes, dans lequel R possède un atome de carbone asymétrique.

7. Composé selon l'une quelconque des revendications précédentes, dans lequel A représente un groupe choisi parmi

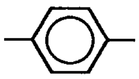,
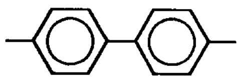,
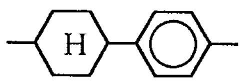,

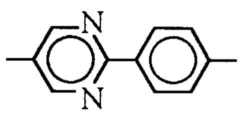 et 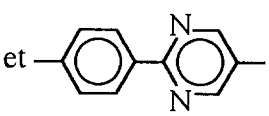

8. Ester d'acide carboxylique représenté par la formule (III) ou (IV) suivante :

R - (A'- X)$_m$ - (B' - Y)$_n$ 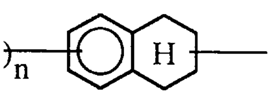 COOR* ... (III)

R - (A'- X)$_m$ - (B' - Y)$_n$ 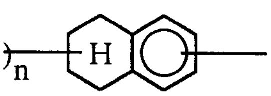 COOR* ... (IV)

formules dans lesquelles

R représente un groupe alkyle ayant 3-20 atomes de carbone, un groupe alcoxy ayant 3-20 atomes de carbone, un groupe alkyle halogéné ayant 3-20 atomes de carbone ou un groupe hydrocarboné possédant un groupe -OCO-, au moins l'un des atomes d'hydrogène constituant ledit groupe hydrocarboné pouvant être remplacé par un atome d'halogène, et R peut posséder au moins un atome de carbone asymétrique,
X et Y représentent chacun indépendamment un groupe choisi parmi -COO-, -OCO-, $-CH_2CH_2-$, $-CH_2O-$, $-OCH_2-$, -S-S-, $-CO-CH_2-$, et $-CH_2-CO-$, ou une liaison simple,
A' et B' représentent chacun indépendamment un groupe choisi parmi :

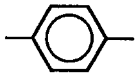 , 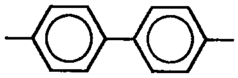 , 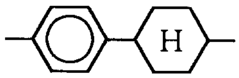 , 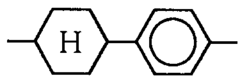 ,

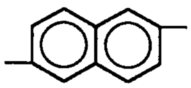 , 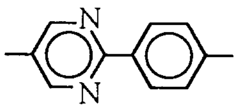 et 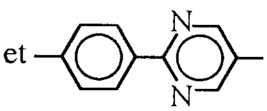 ,

R* représente un groupe optiquement actif, ayant 4-20 atomes de carbone et contenant au moins un atome de carbone asymétrique, des atomes d'hydrogène liés aux atomes de carbone constituant ledit groupe optiquement actif pouvant être remplacés par un atome d'halogène,

m est égal à 1 ou 2, n représente un nombre entier ayant une valeur de 0-2 et la somme $m+n \geq 2$.

**9.** Composé selon la revendication 8, dans lequel R* est un groupe représenté par la formule (V) suivante :

$$-Q^1\text{-}C^*H(Q^2)\text{-}Q^3 \qquad (V)$$

dans laquelle

$Q^1$ représente un groupe $-(CH_2)_q-$ dans lequel q est un nombre entier ayant une valeur de 0-6,
$Q^2$ et $Q^3$ sont différents l'un de l'autre et représentent chacun indépendamment un groupe alkyle ayant 1-10 atomes de carbone, un groupe fluoroalkyle ayant 1-10 atomes de carbone ou un atome d'halogène, et
au moins l'un des groupes $CH_2$ ou $CF_2$ de $Q^1$, $Q^2$ et $Q^3$ peut être remplacé par au moins un groupe choisi parmi -O-, -S-, -CO-, -CHX- dans lequel X représente un atome d'halogène, -CHCN-, -O-CO-, -O-COO-, -CO-O- et -CH=CH-, de telle manière que deux hétéroatomes ne sont pas reliés directement.

**10.** Composé selon la revendication 8 ou 9, dans lequel R* représente un groupe choisi parmi :
$-C^*H(CF_3)-C_6H_{13}$, $-C^*H(CH_3)-C_6H_{13}$, $-C^*H(CH_3)-C_5H_{11}$,
$-C^*H(C_2H_5)-C_5H_{11}$, $-C^*H(C_2H_5)-C_6H_{13}$, $-CH_2-C^*H(CH_3)-C_2H_5$,
$-(CH_2)_3-C^*H(CH_3)-C_2H_5$, et $-C^*H(CF_3)-CH_2-COO-C_2H_5$.

**11.** Composé selon l'une quelconque des revendications 8 à 10, dans lequel R représente un groupe alcoxy possédant éventuellement un atome de carbone asymétrique.

**12.** Composé selon l'une quelconque des revendications 8 à 11, dans lequel R est un groupe possédant un atome de carbone asymétrique.

**13.** Composé selon l'une quelconque des revendications 8 à 12, dans lequel X et Y représentent indépendamment un groupe -COO- ou -OCO-, ou une liaison simple.

**14.** Composé selon l'une quelconque des revendications 8 à 13, dans lequel A représente un groupe choisi parmi :

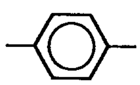 , 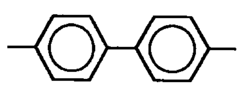 , 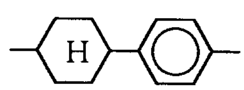 ,

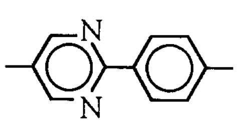 et 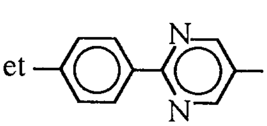

**15.** Produit cristal liquide comprenant un composé tel que revendiqué dans l'une quelconque des revendications précédentes.

16. Composition cristal liquide comprenant un composé tel que revendiqué dans l'une quelconque des revendications 1 à 14.

17. Elément à cristal liquide, comprenant une cellule constituée de deux supports se faisant face, d'un intervalle formé par lesdits supports et d'une substance cristal liquide, telle que revendiquée dans l'une quelconque des revendications 1 à 14, remplissant ledit intervalle.

Fig. 1

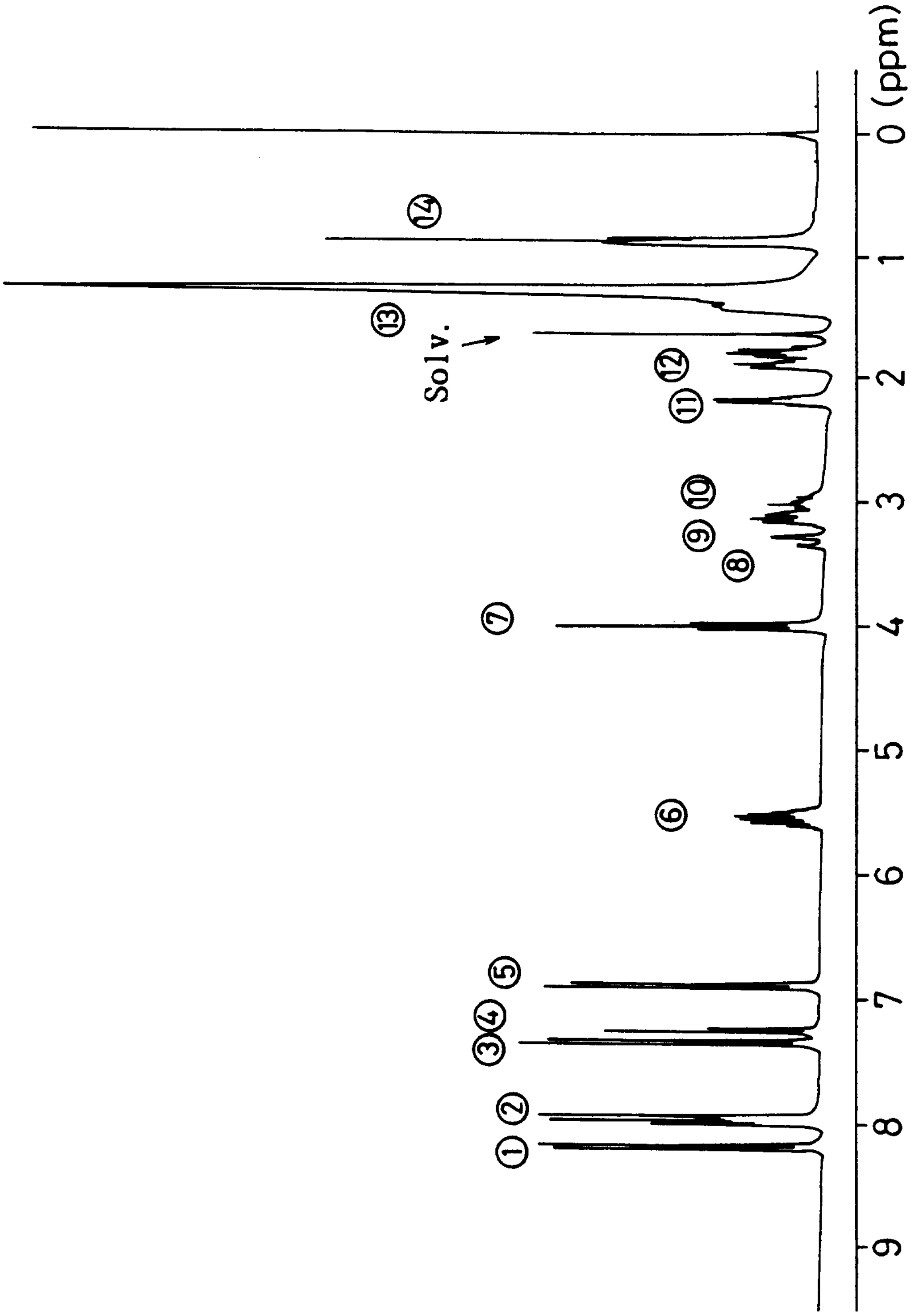

Fig. 2

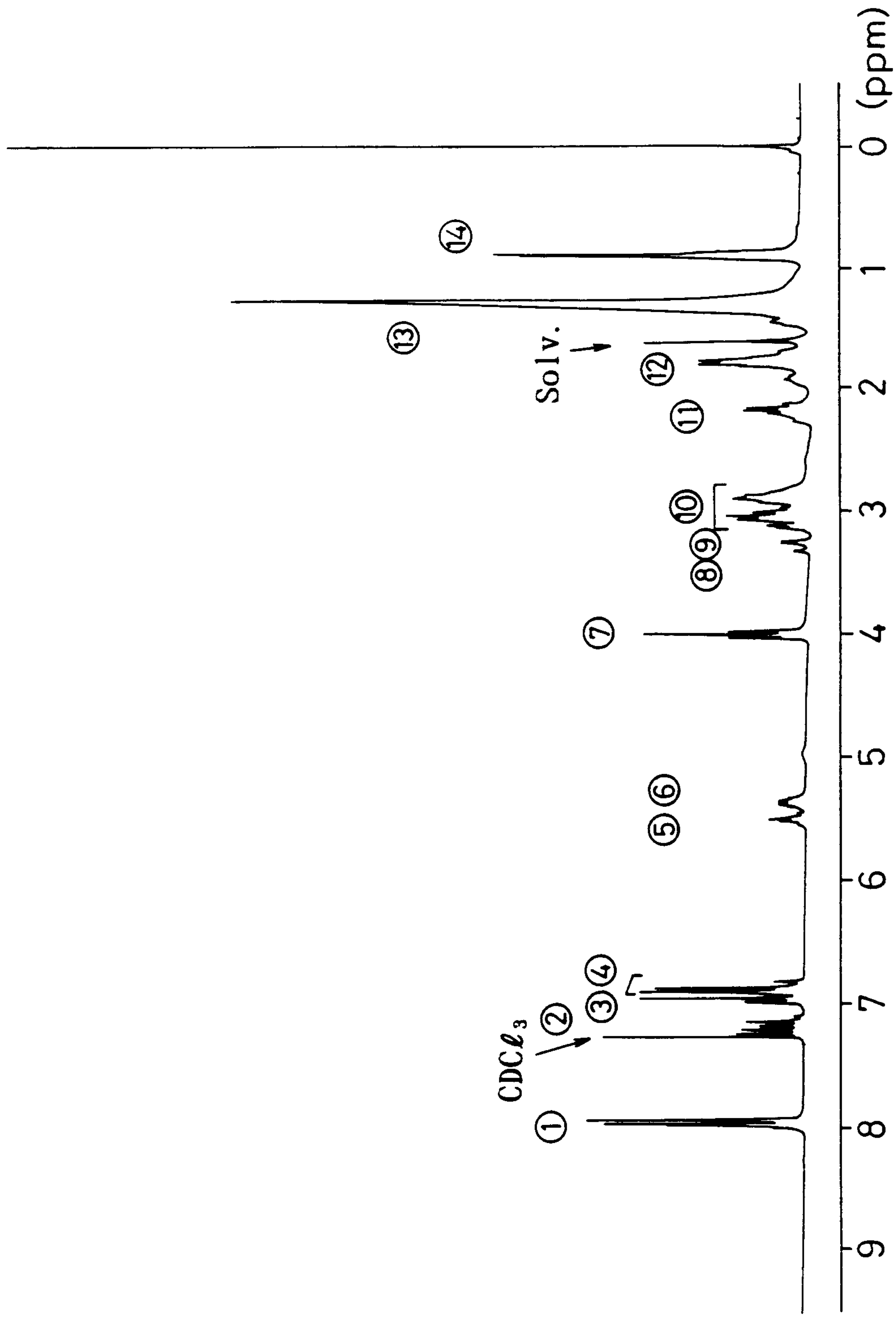

*Fig. 3*

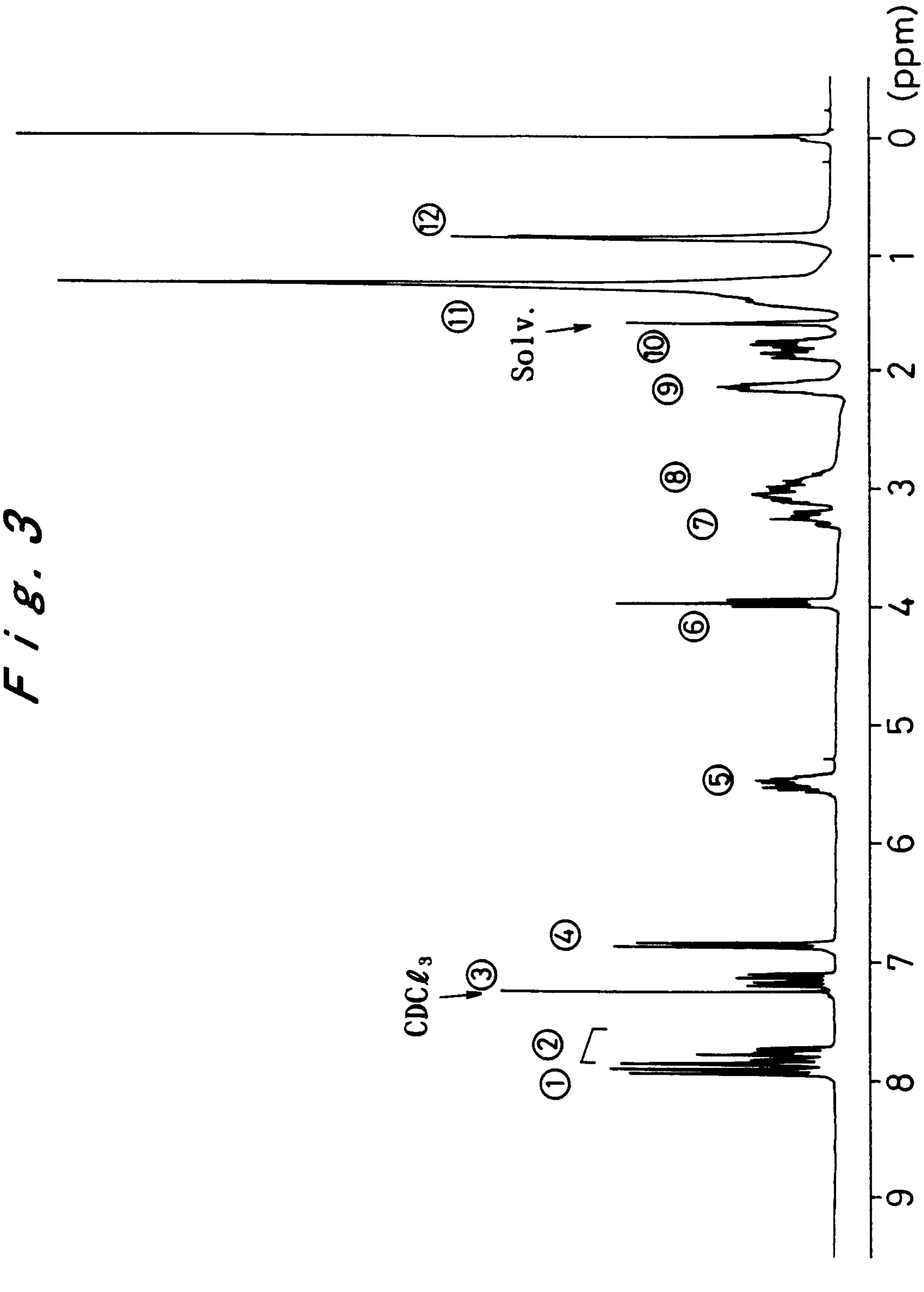

*Fig. 4*

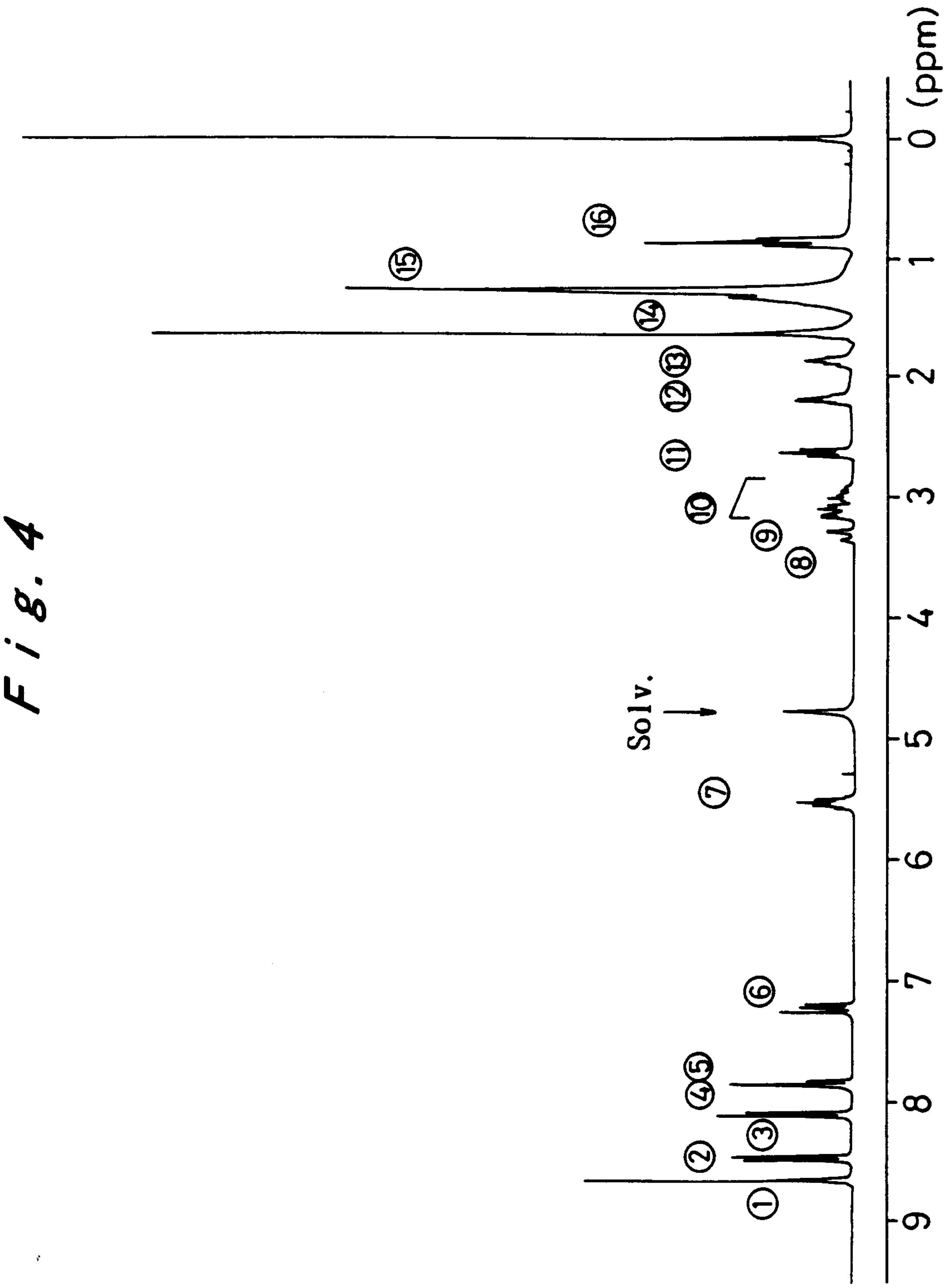

Fig. 5

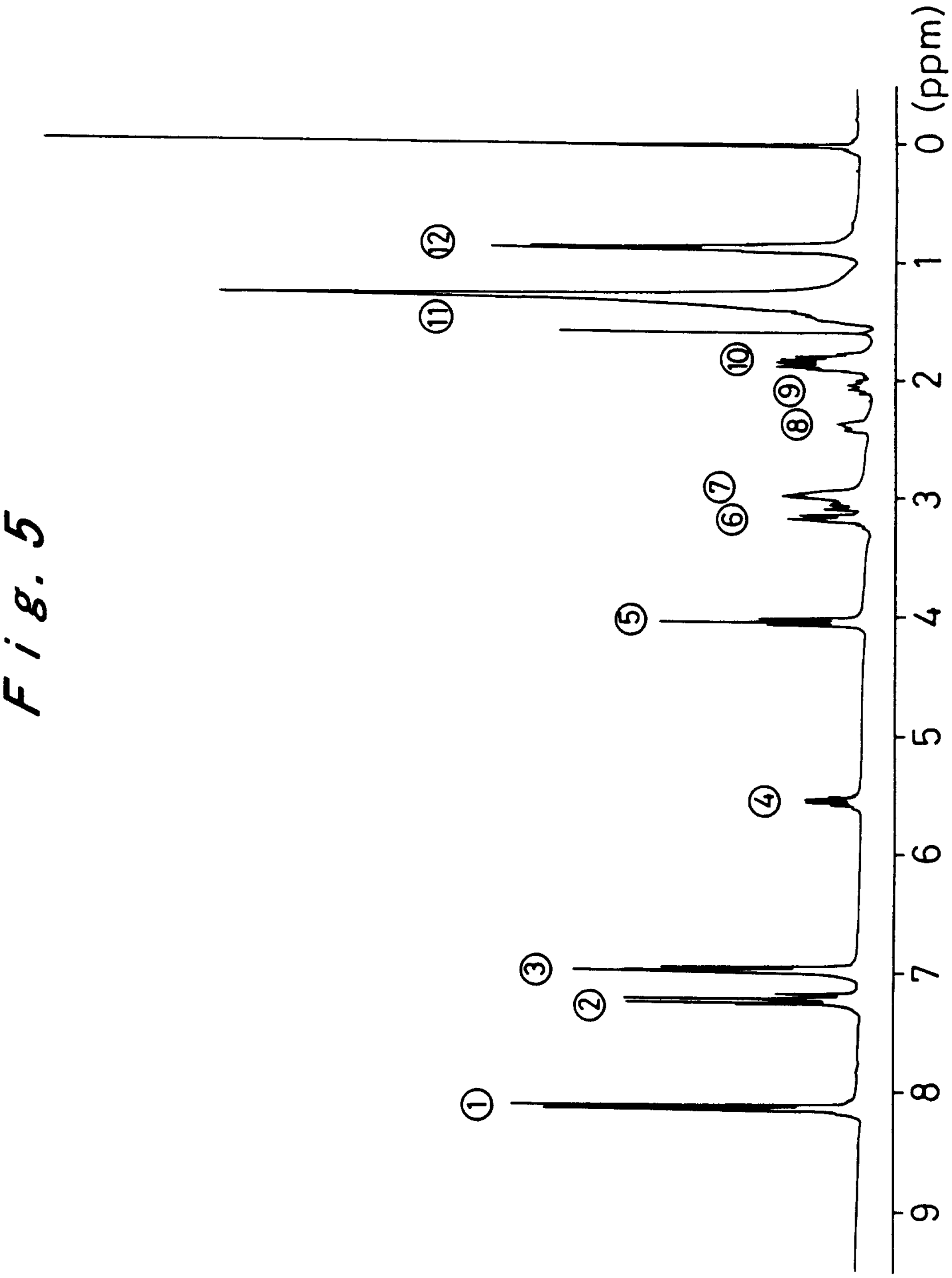

Fig. 6

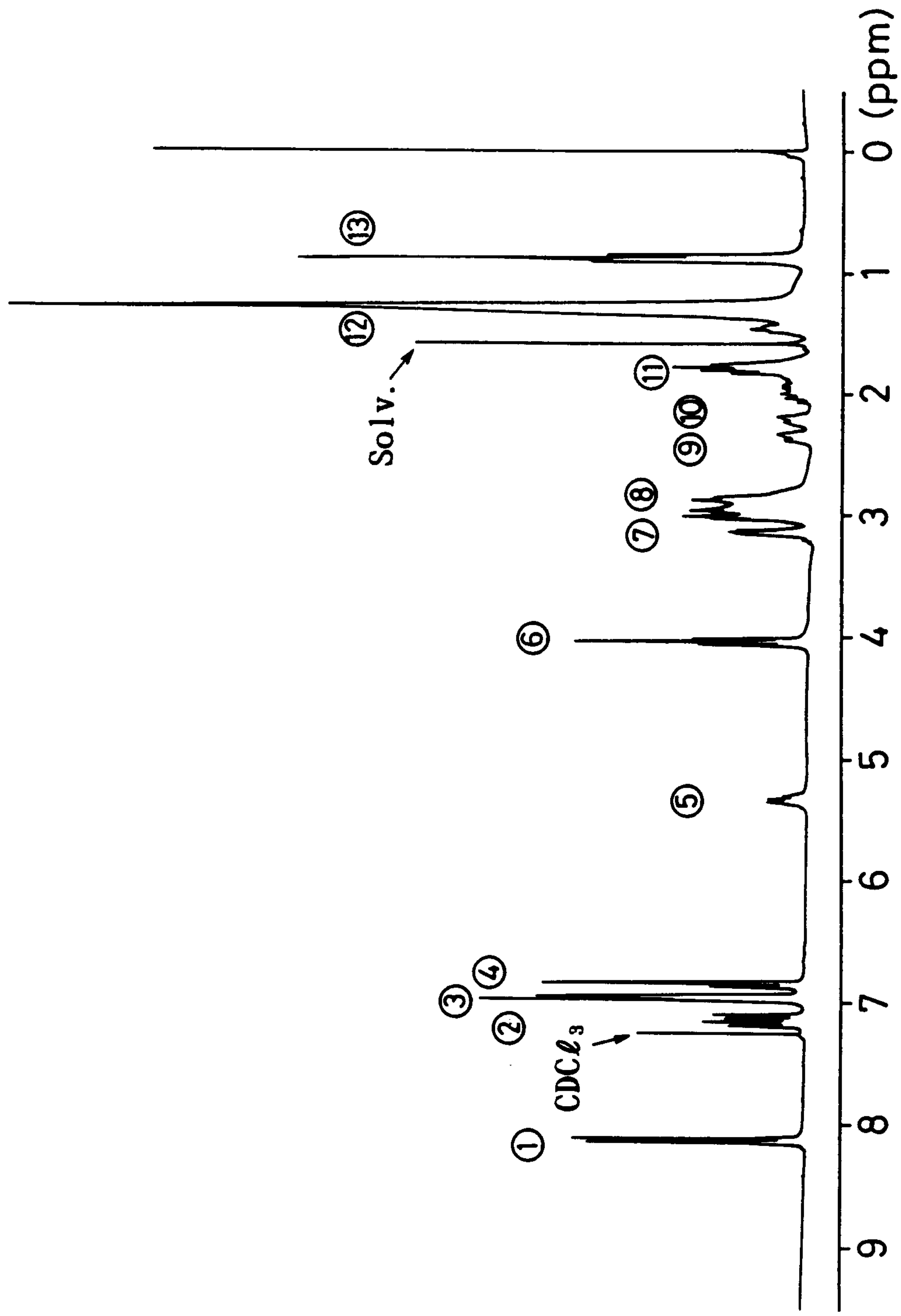

Fig. 7

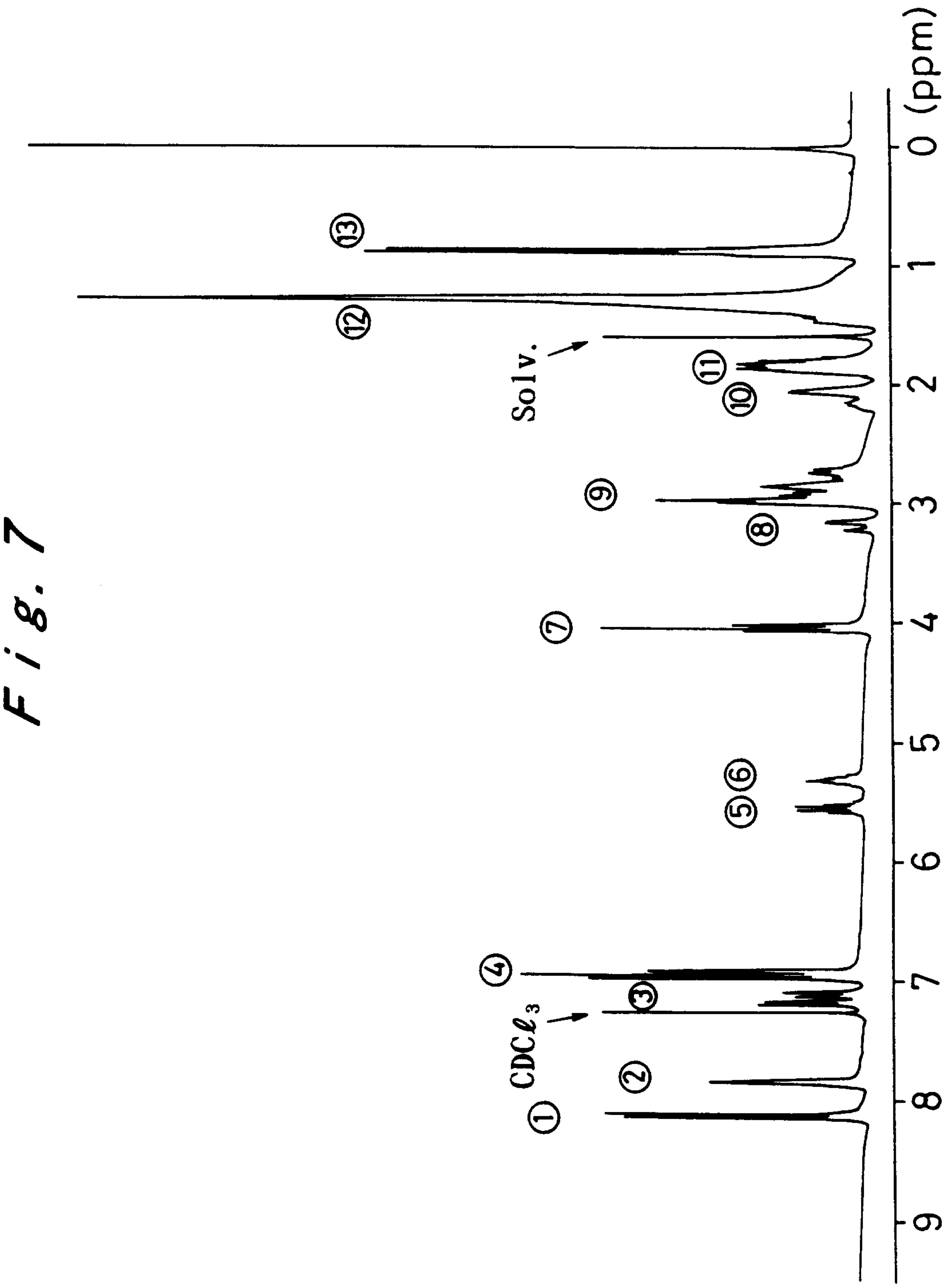

Fig. 8

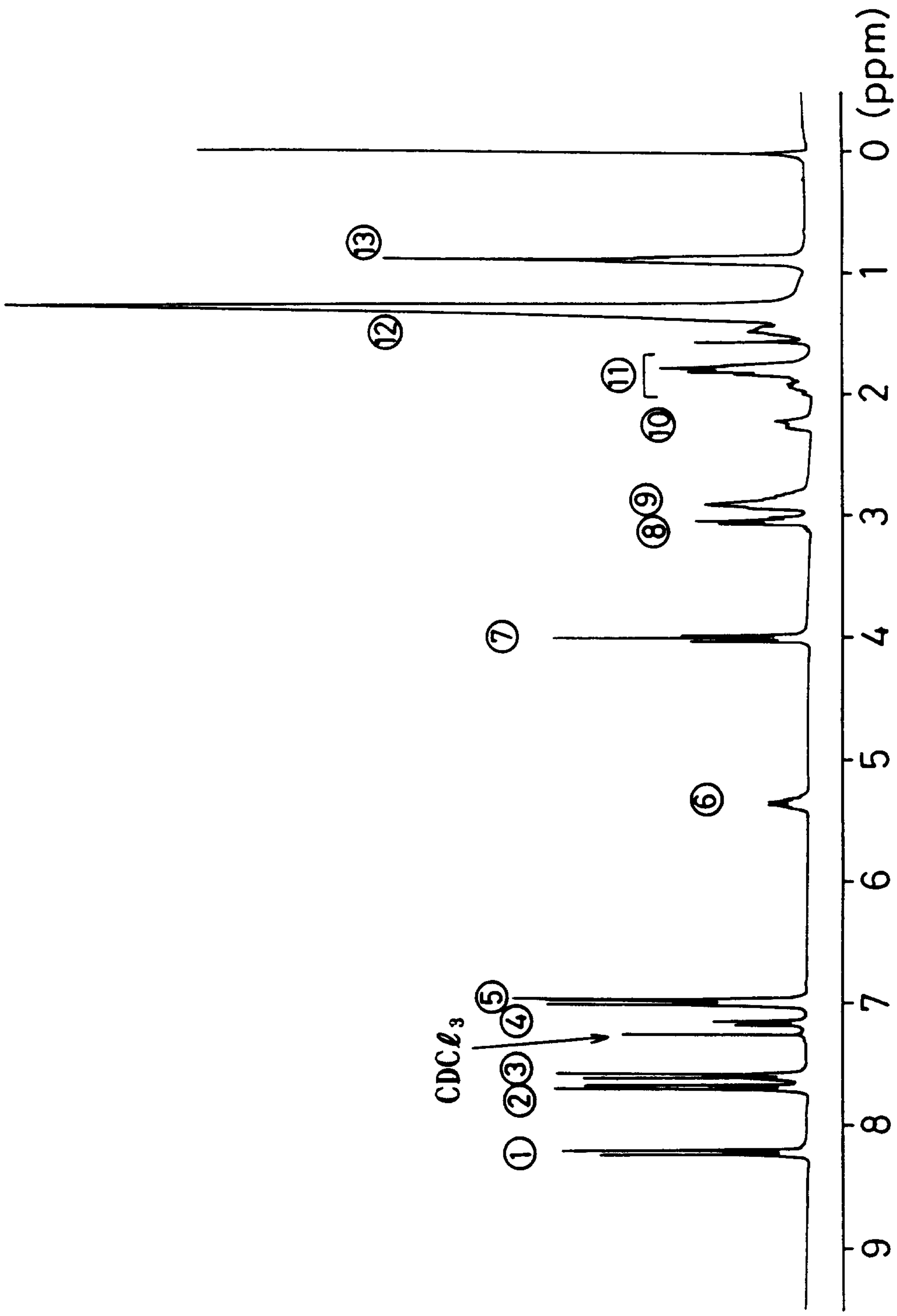

Fig. 9

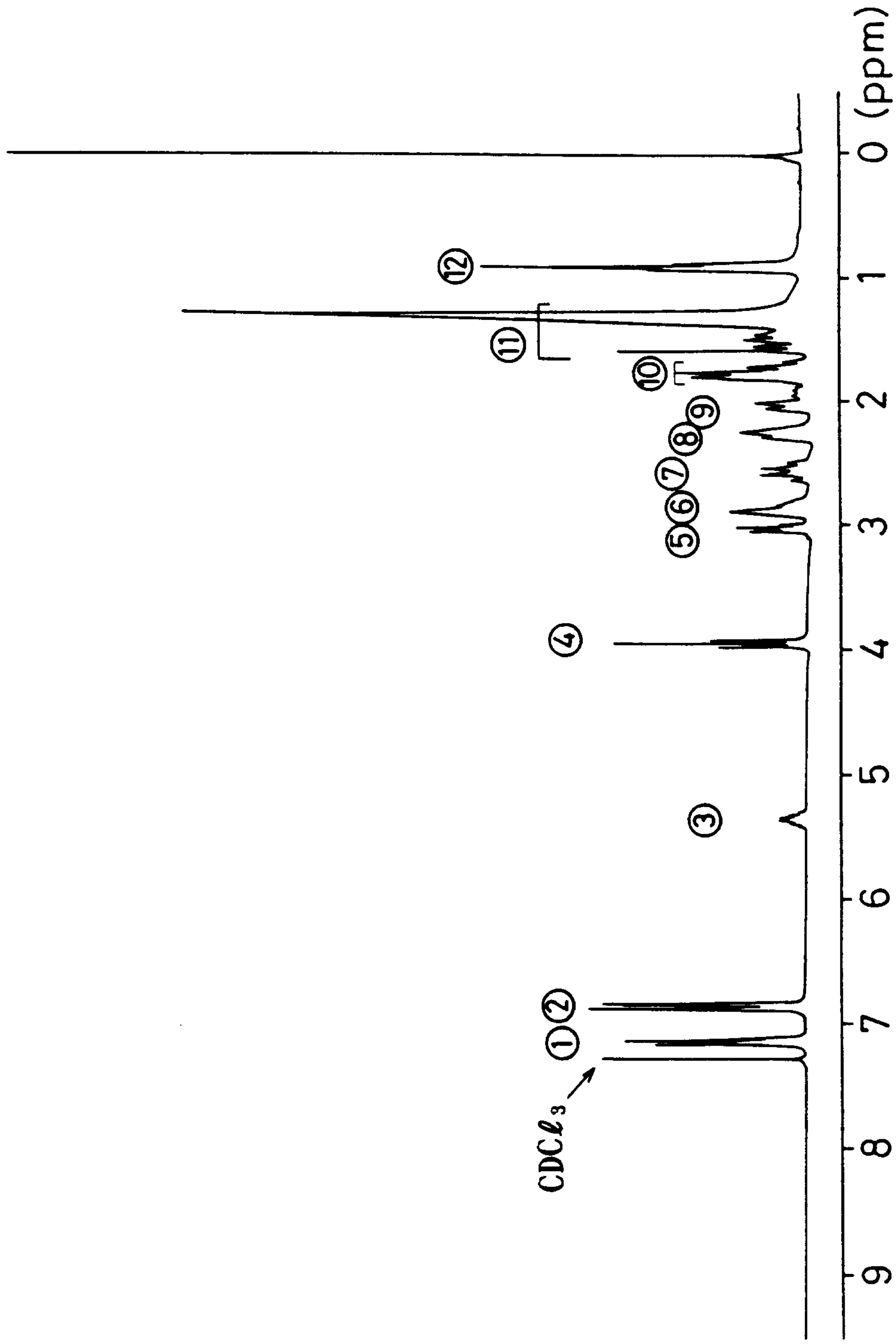

Fig. 10

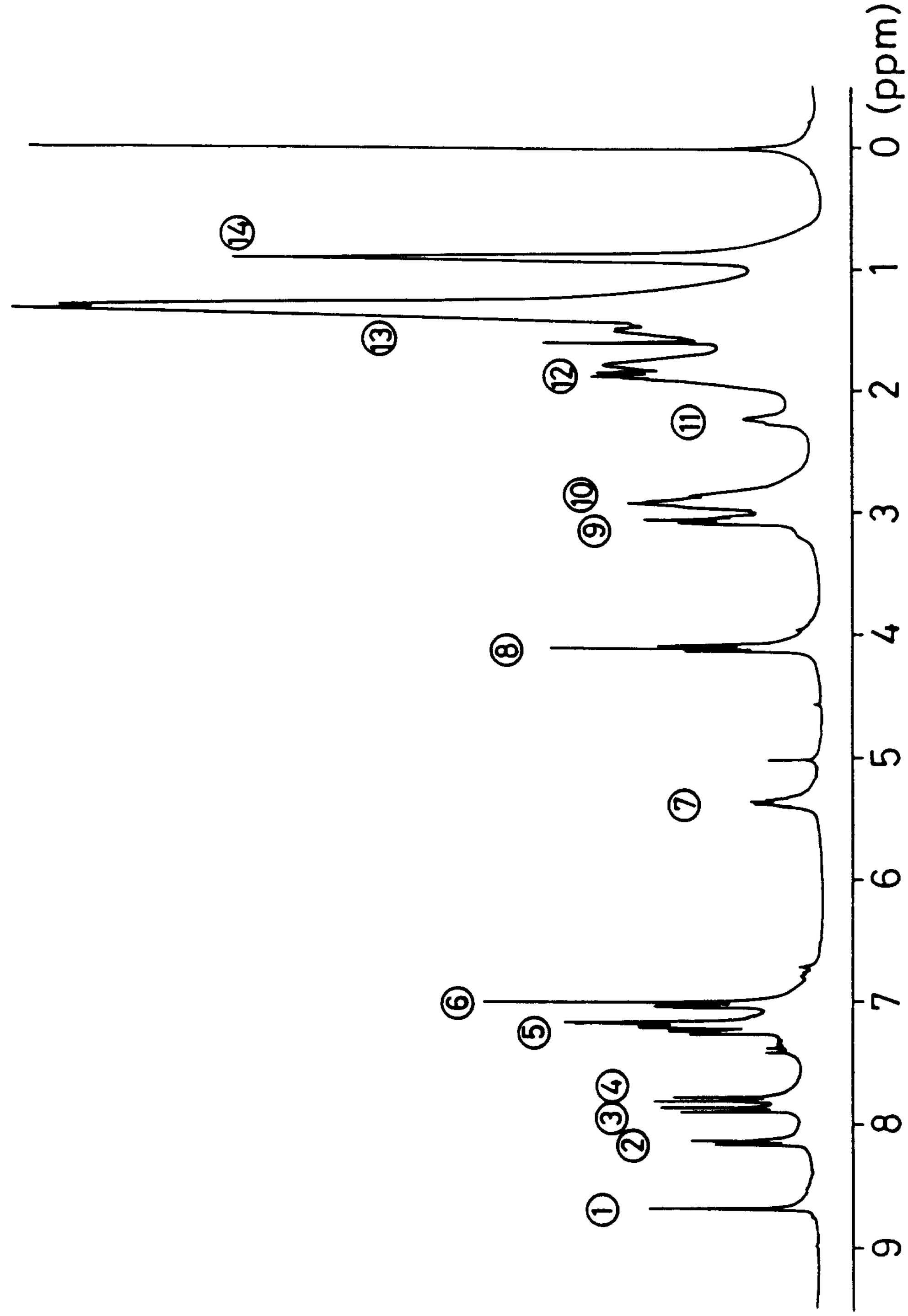

Fig. 11

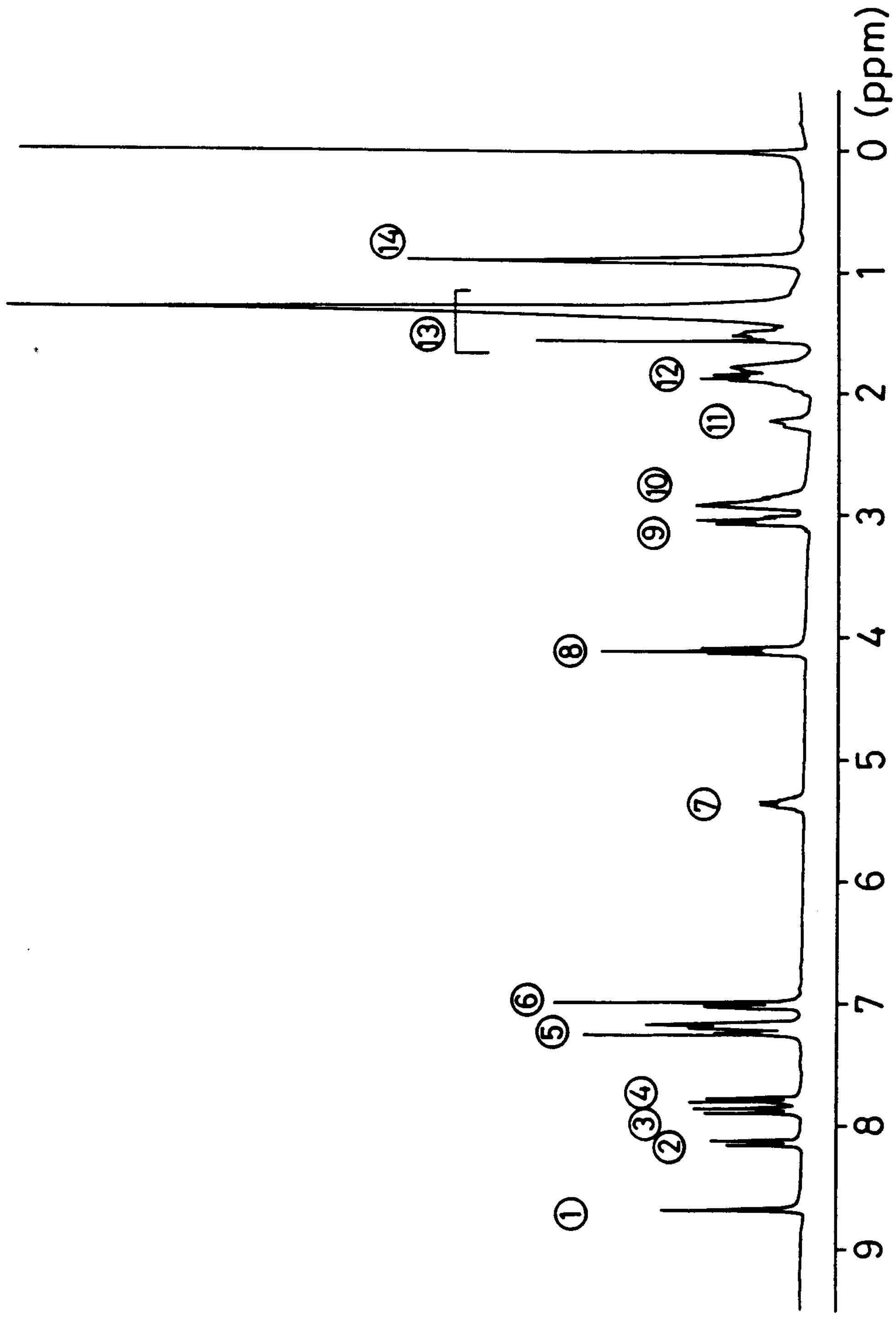

*Fig. 12*

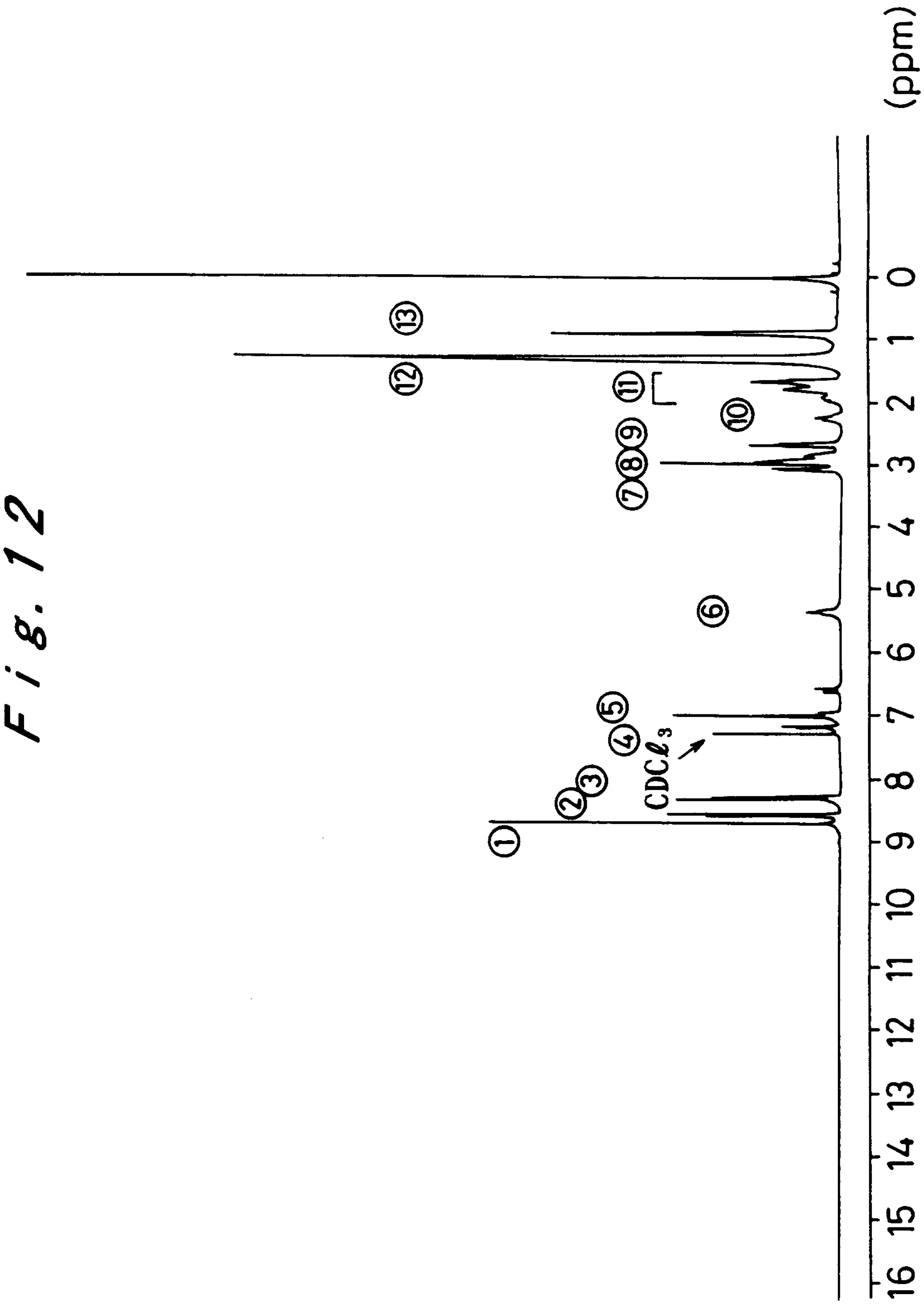

*Fig. 13*

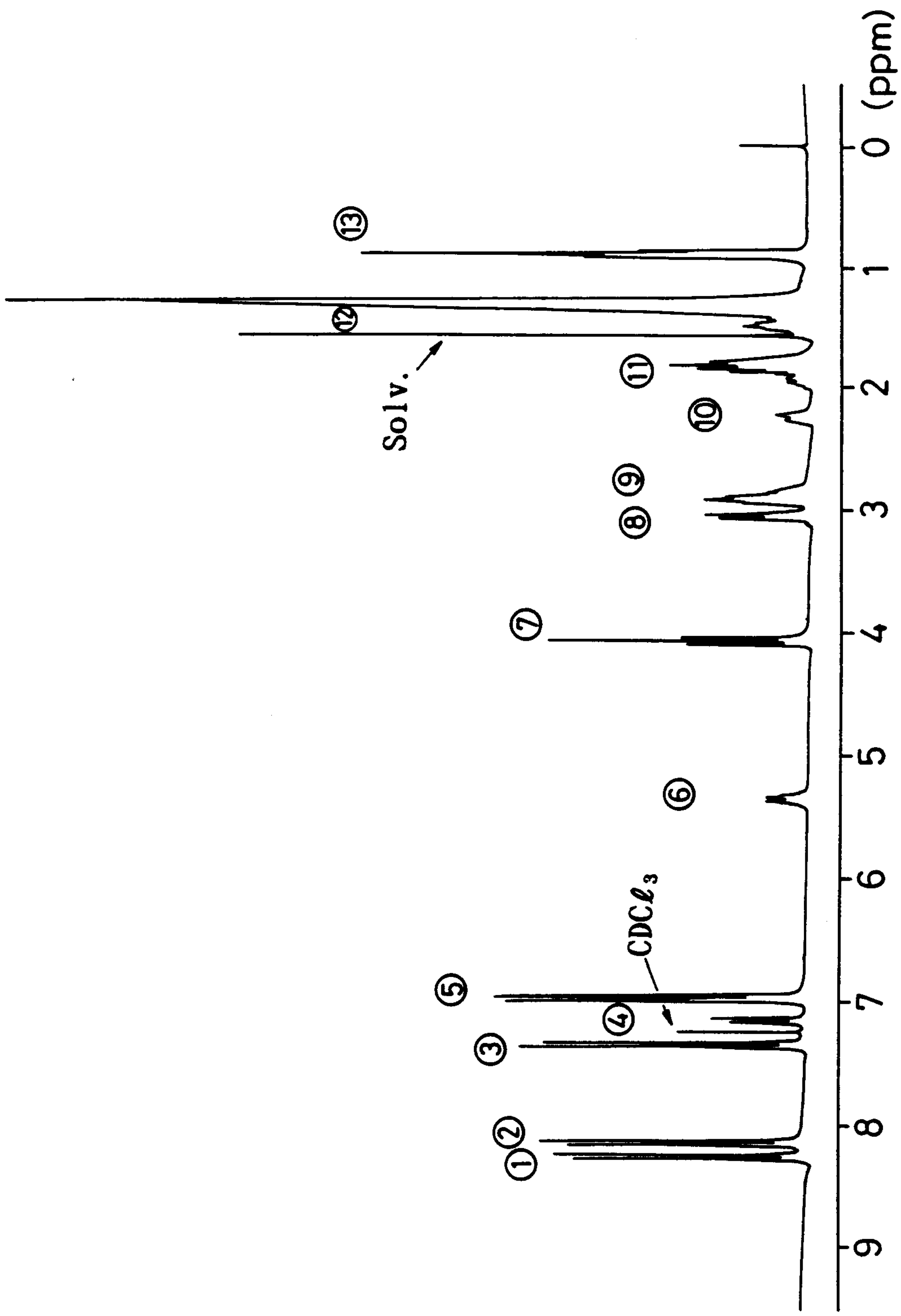

Fig. 14

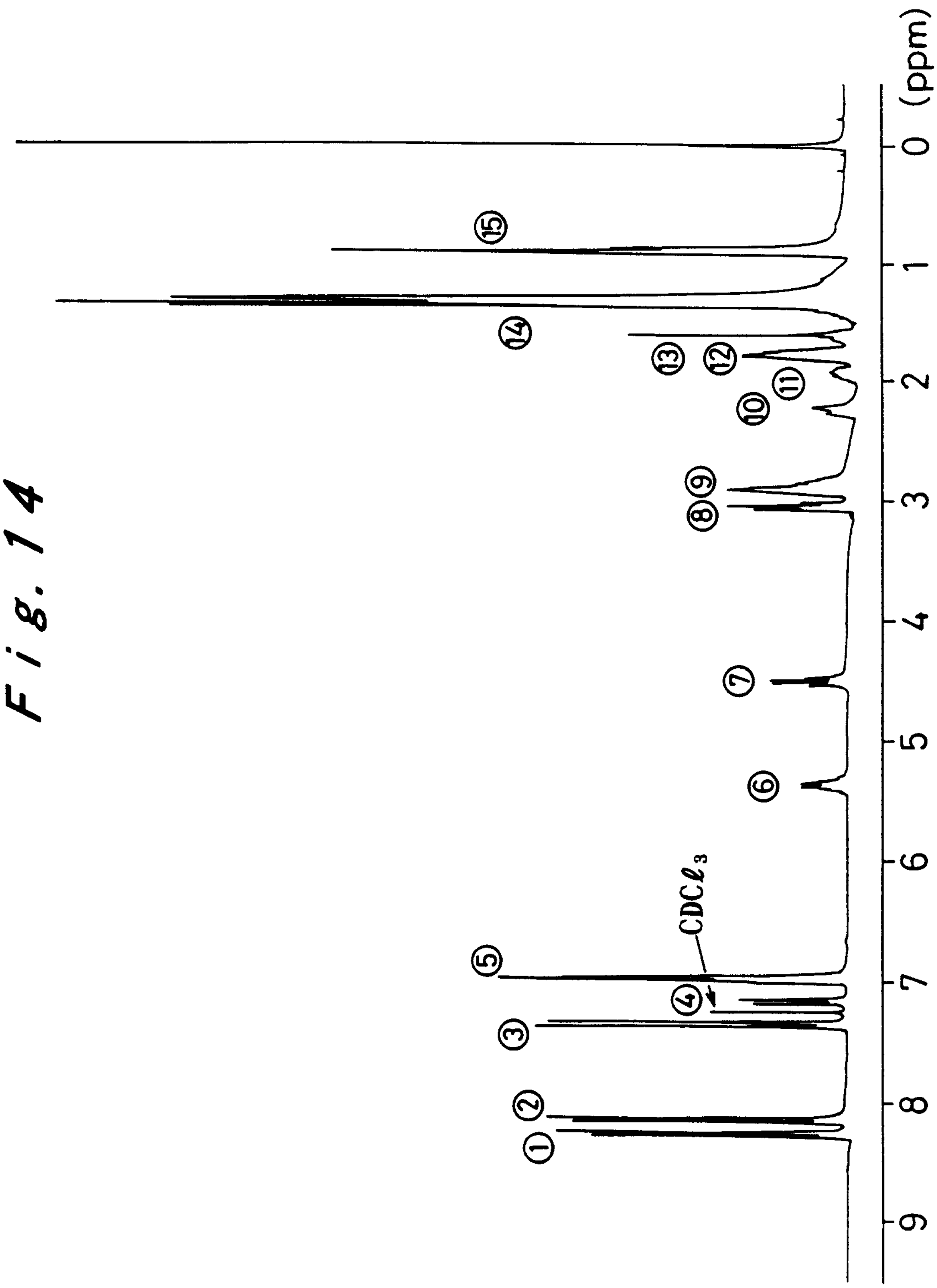

Fig. 15

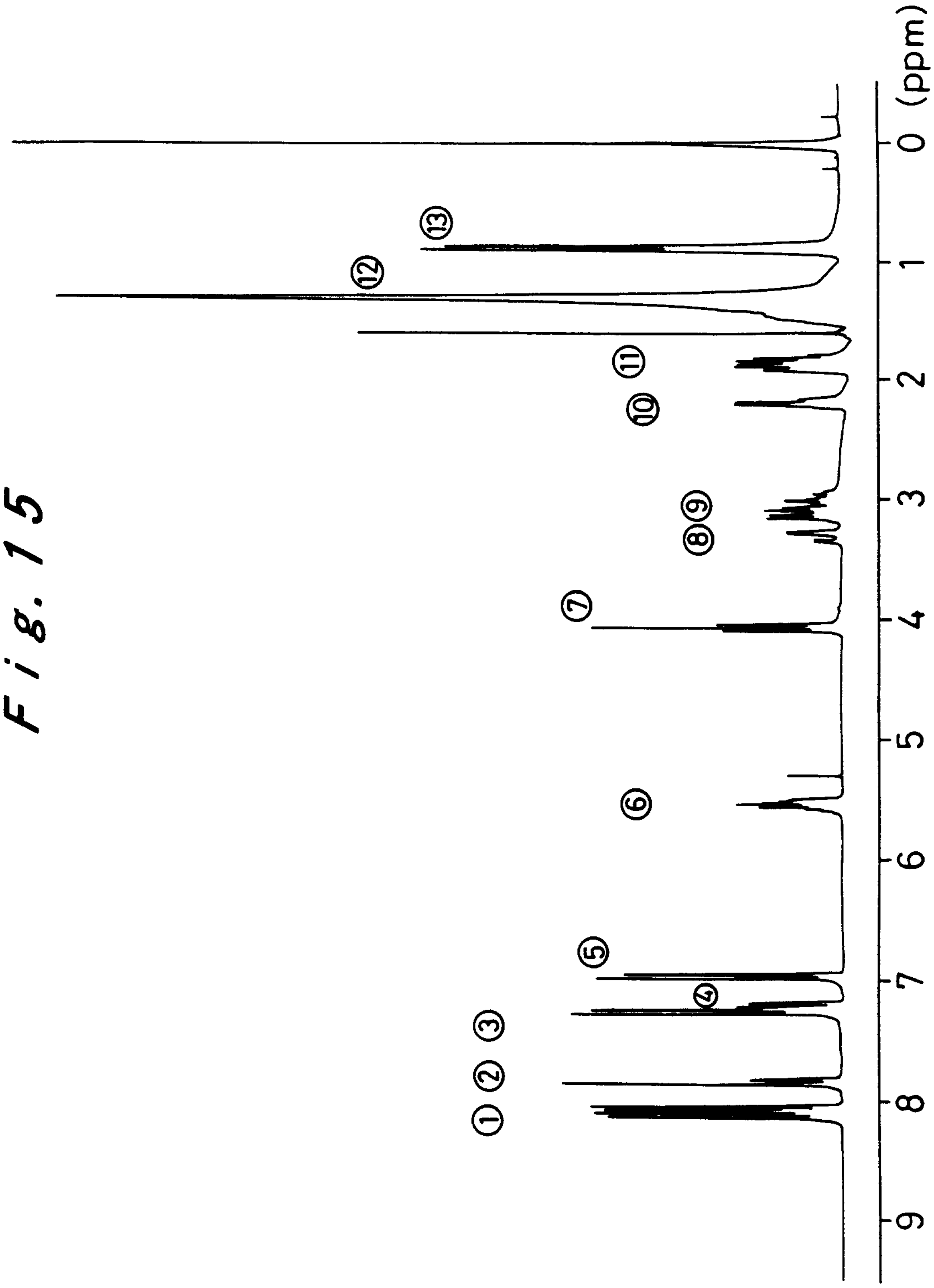

Fig. 16

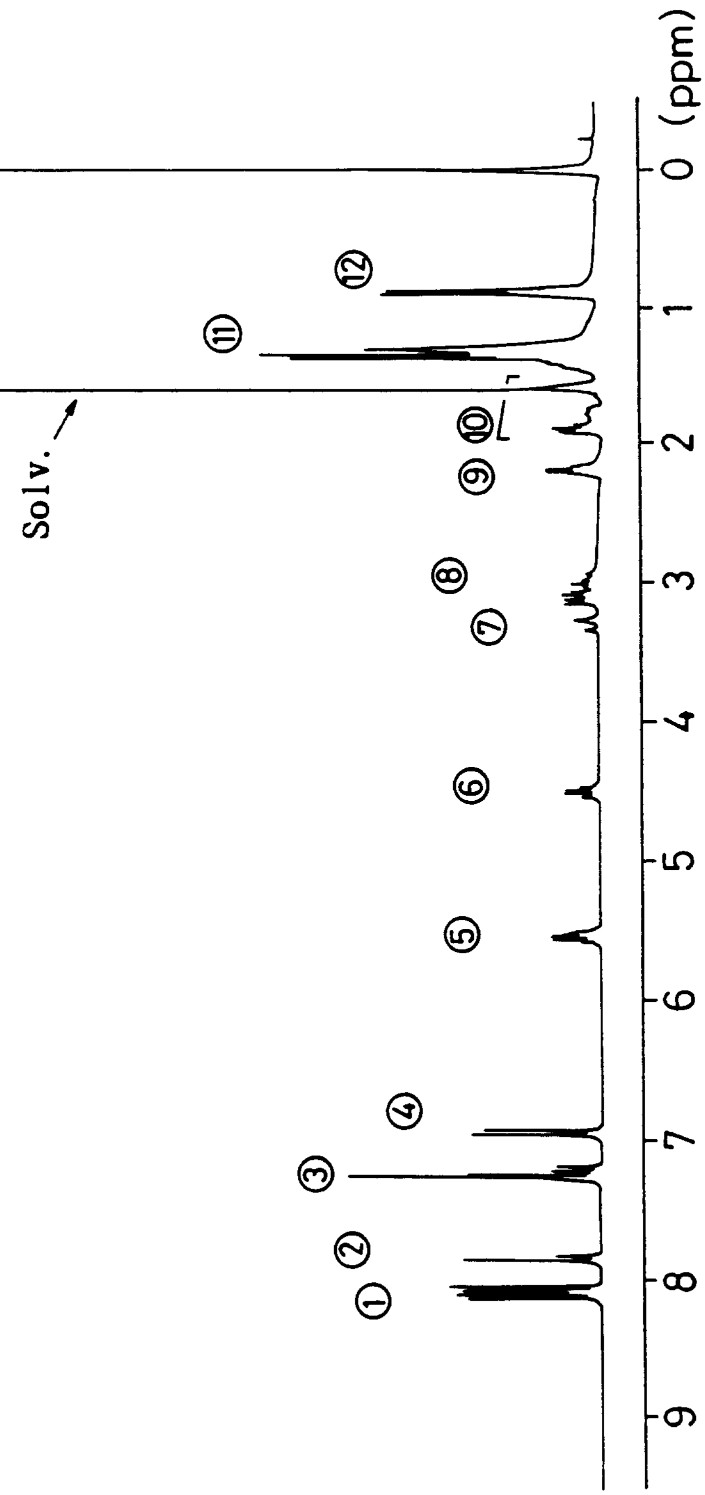

*Fig. 17*

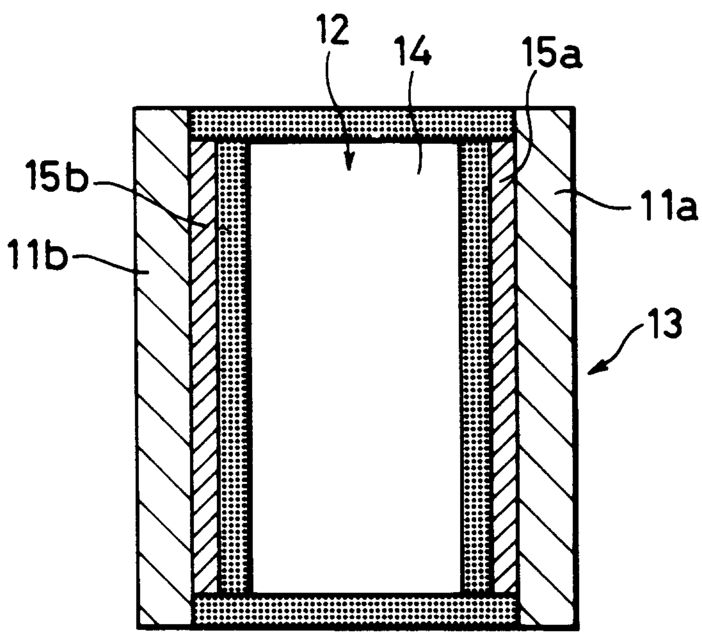

Fig. 18 (a)

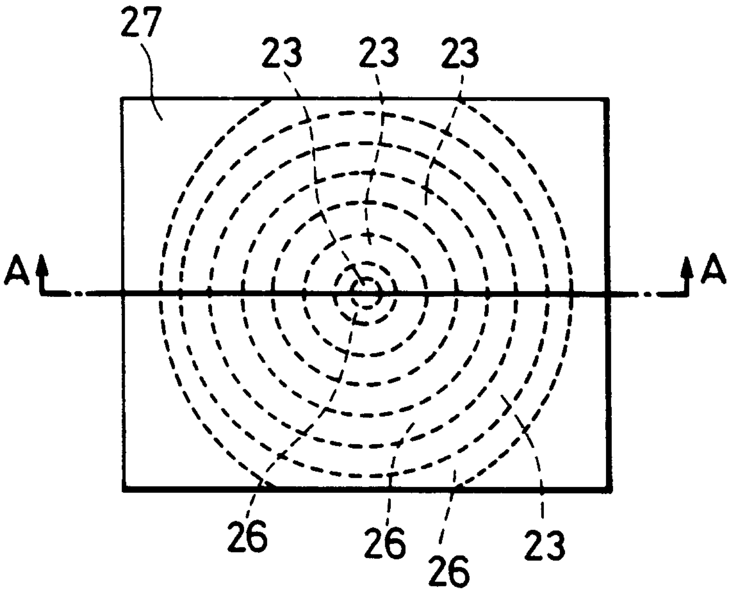


Fig. 18 (b)

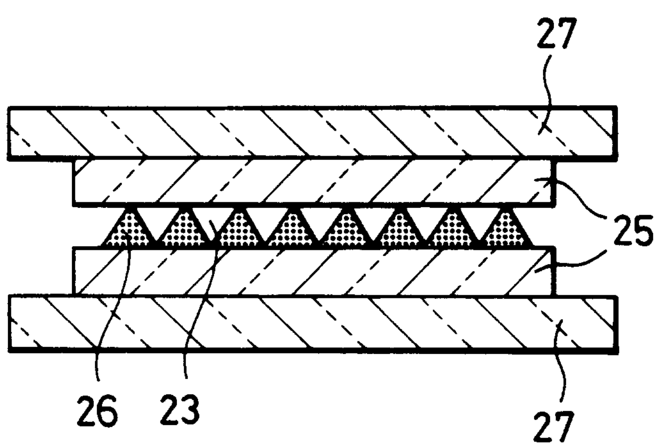

Fig. 19 (a)

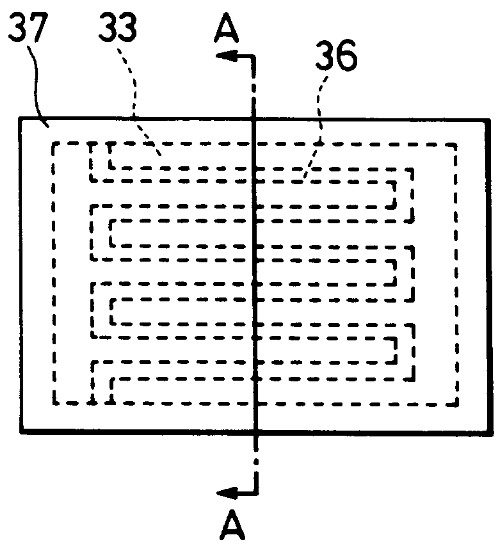


Fig. 19 (b)

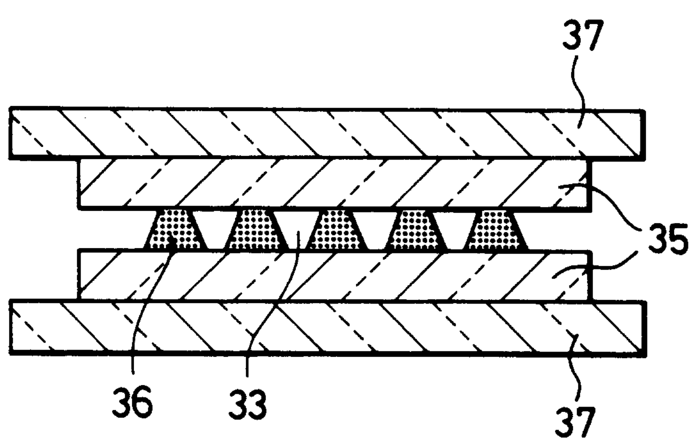

Fig. 20

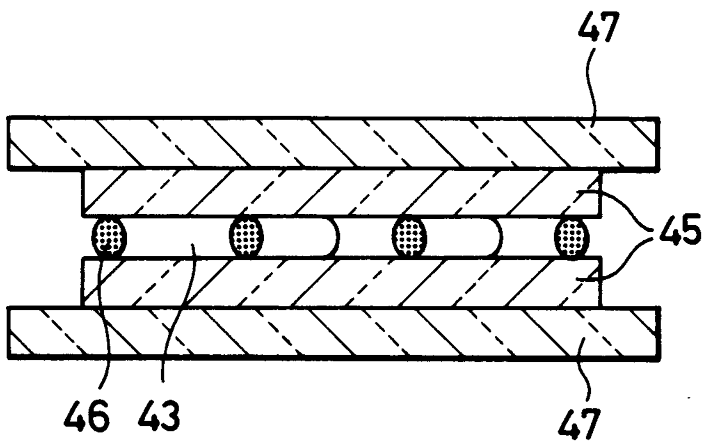


Fig. 21

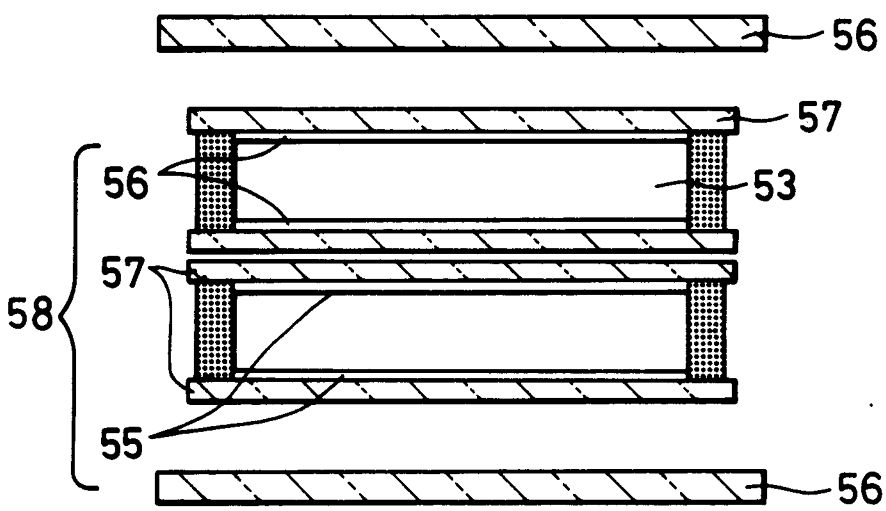

Fig. 22 (a)

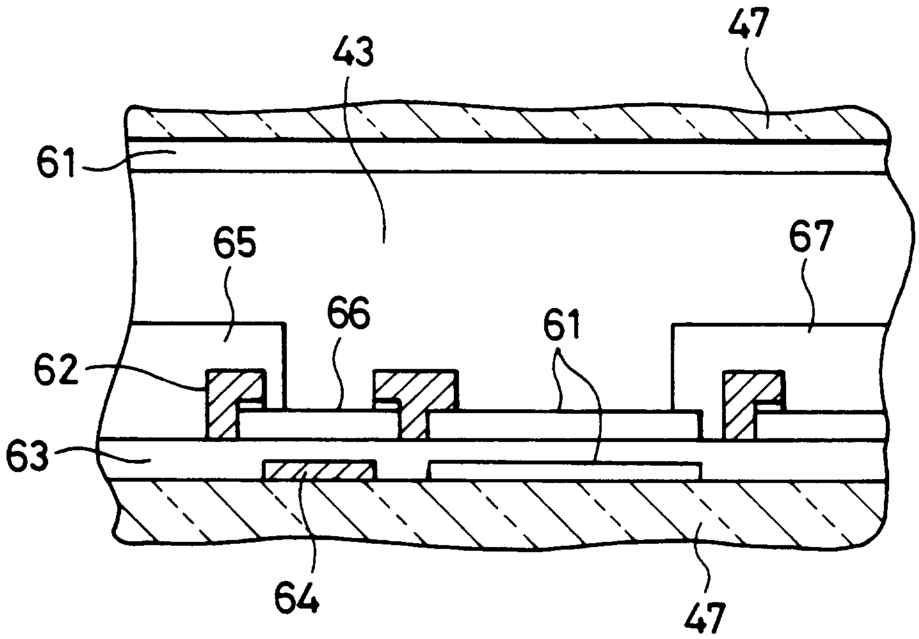


Fig. 22 (b)

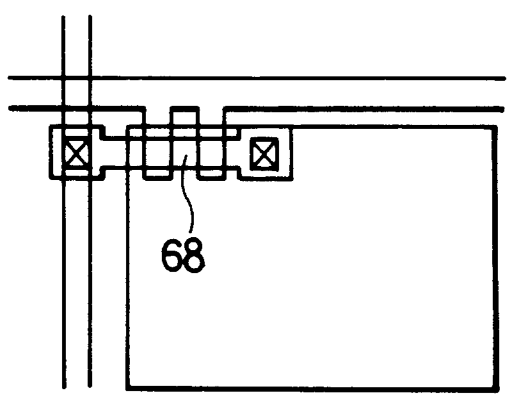

Fig. 23

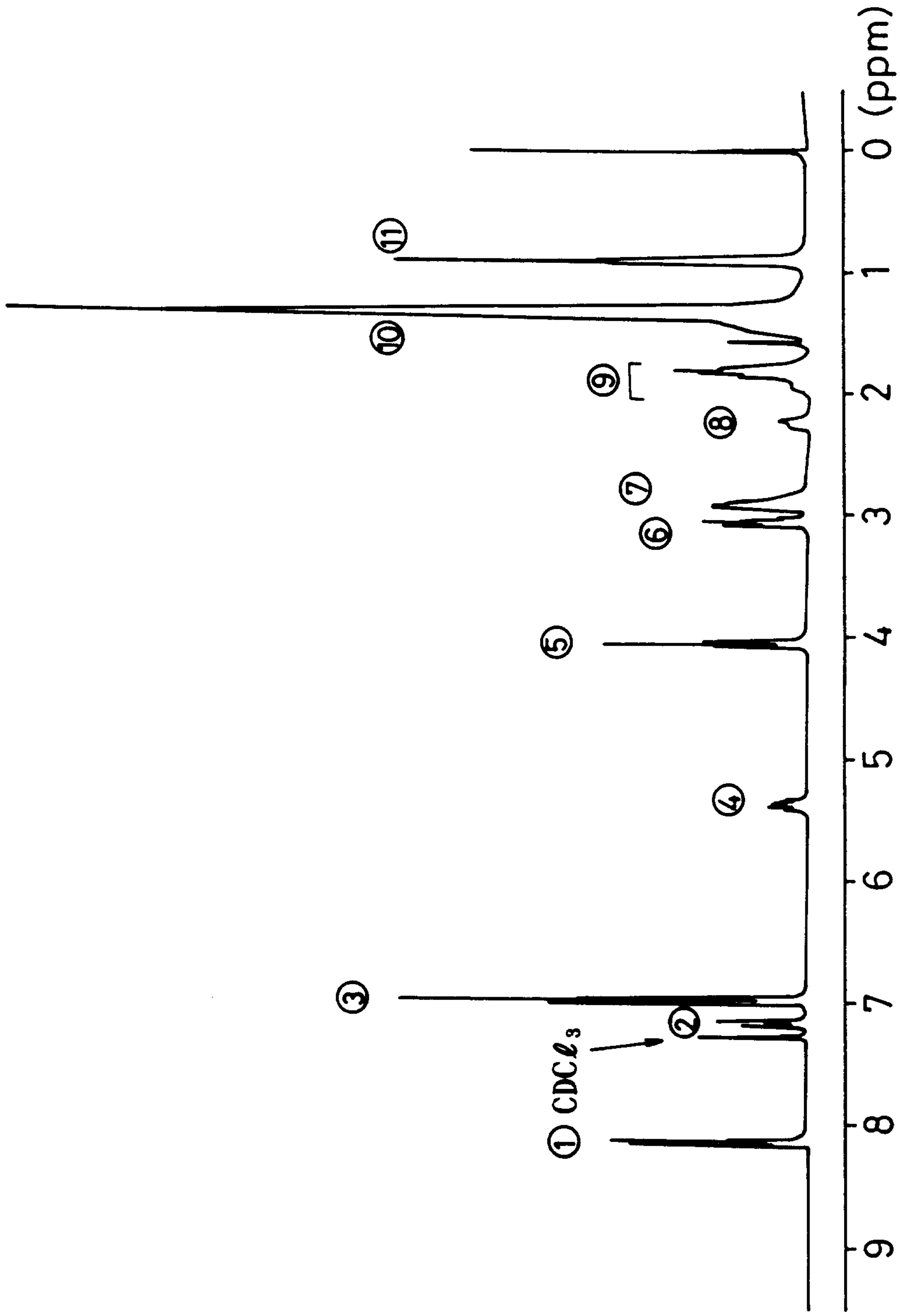

Fig. 24

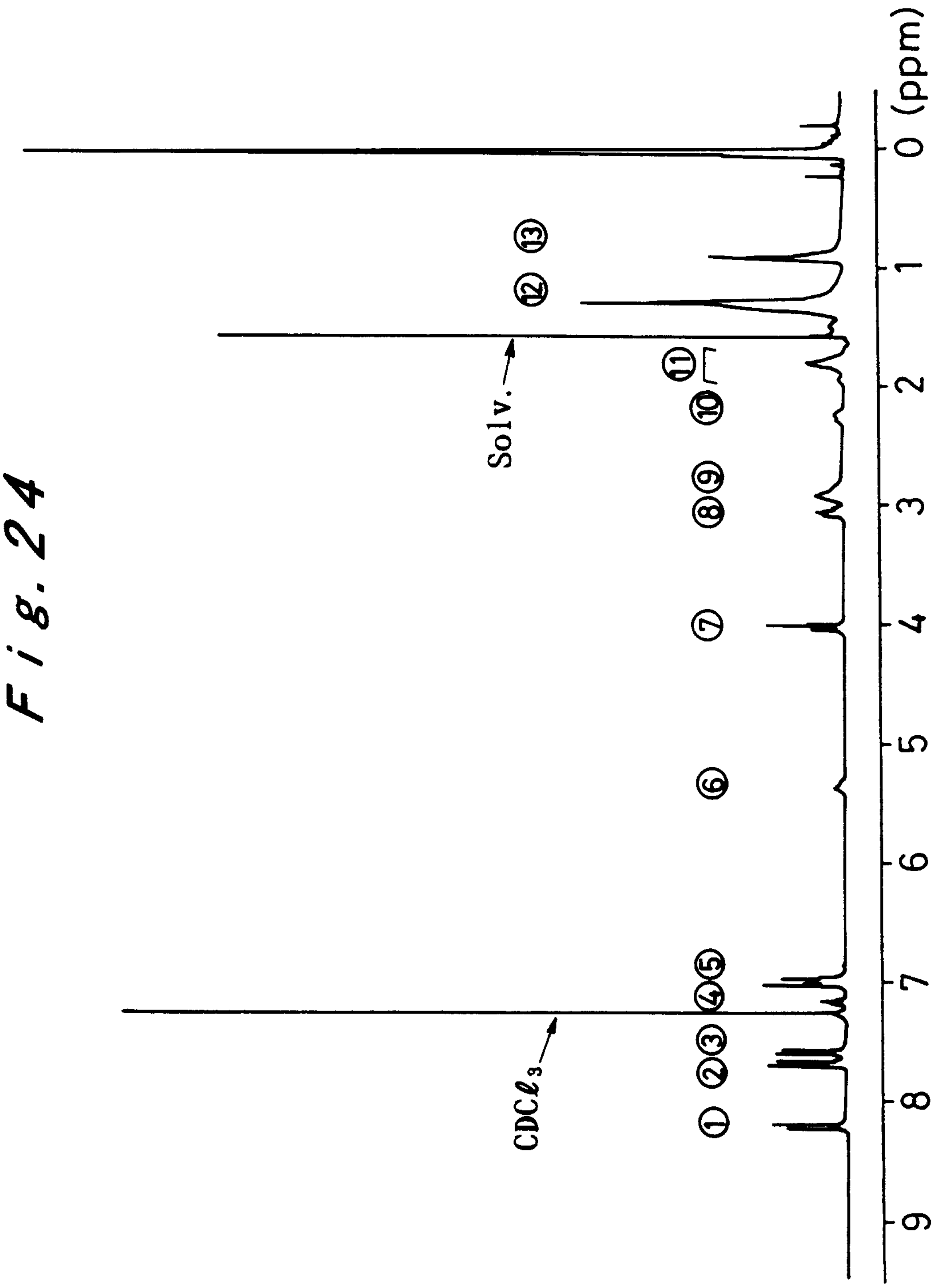

Fig. 25

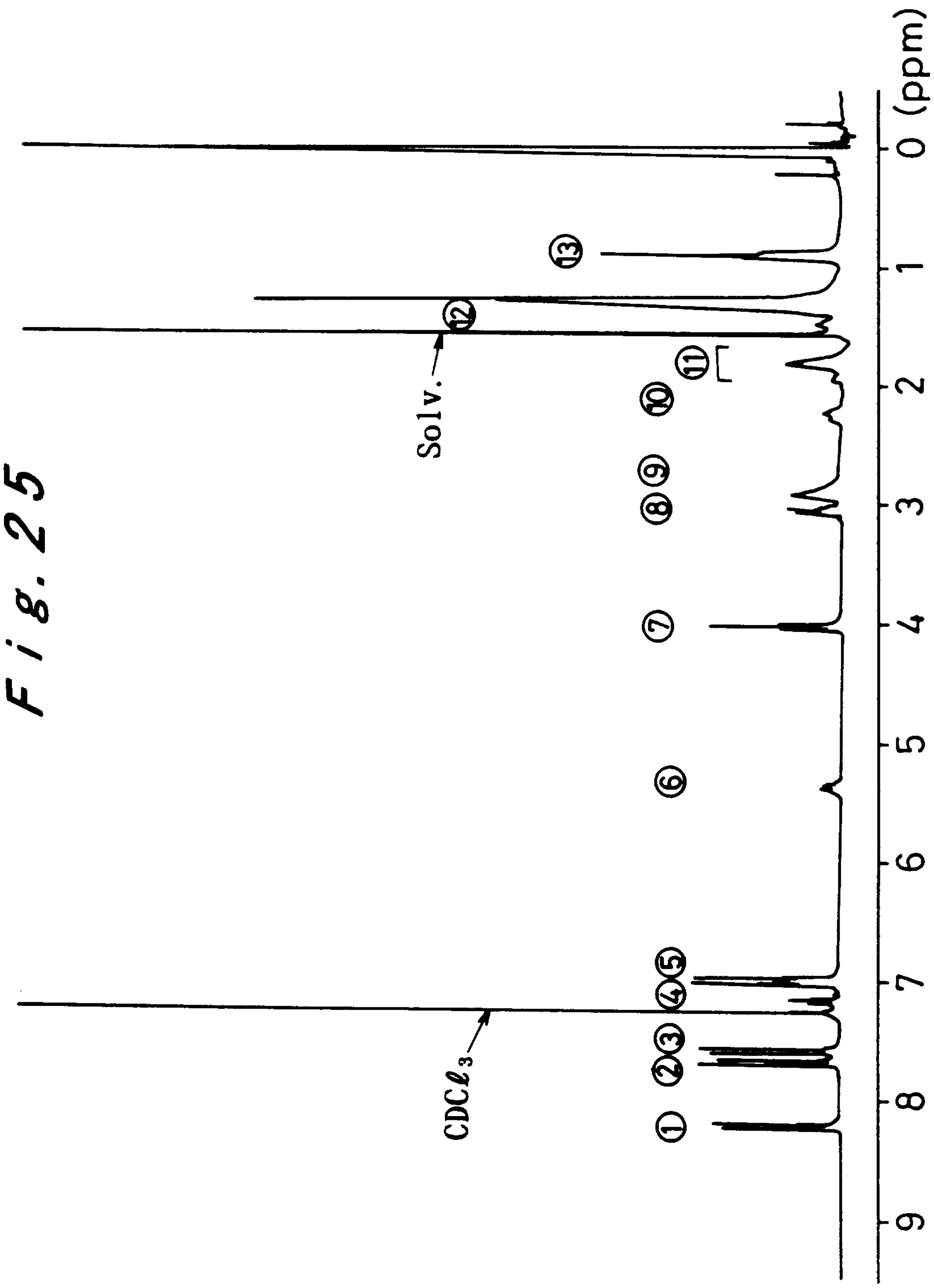

Fig. 26

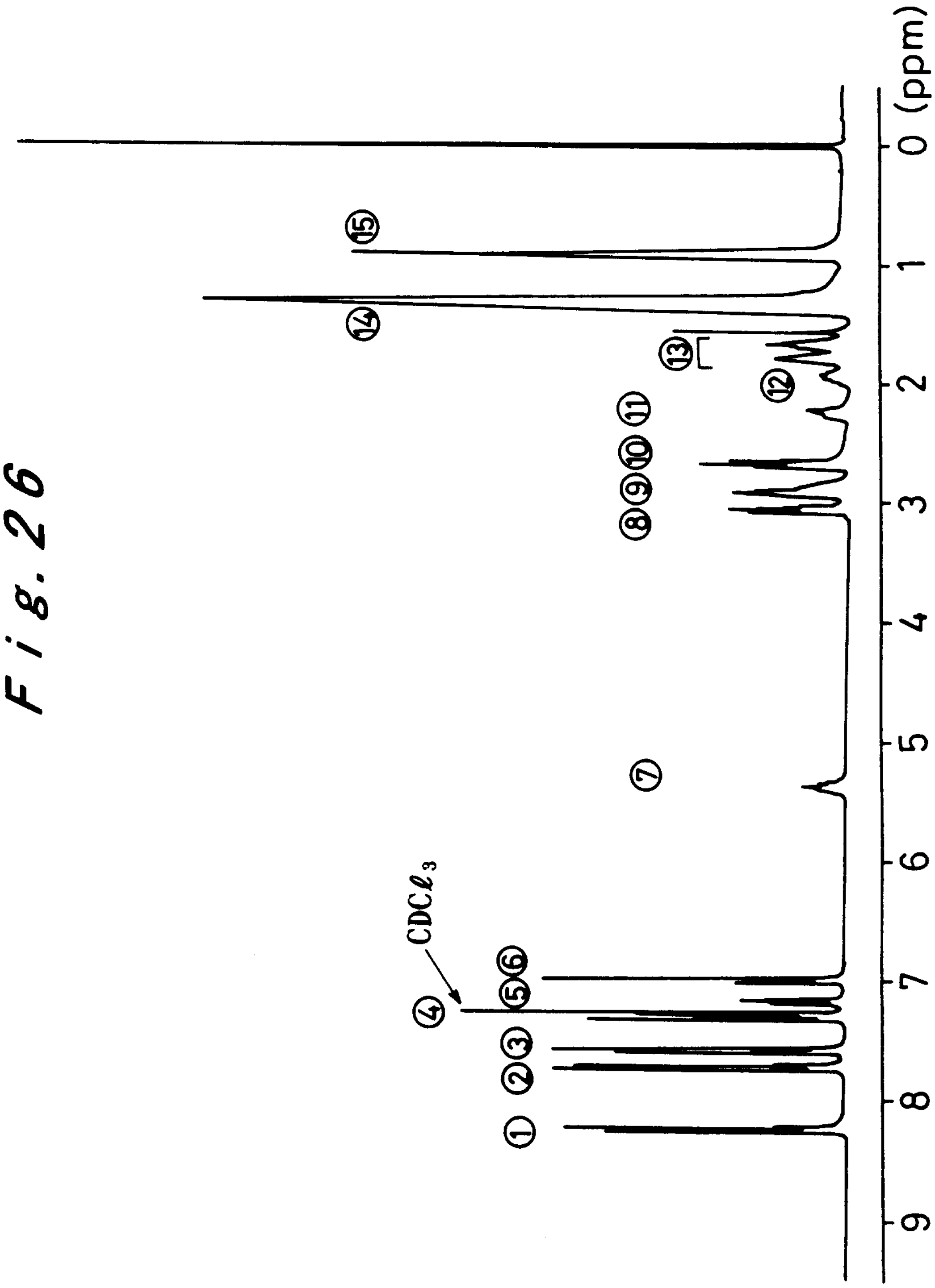

*Fig. 27*

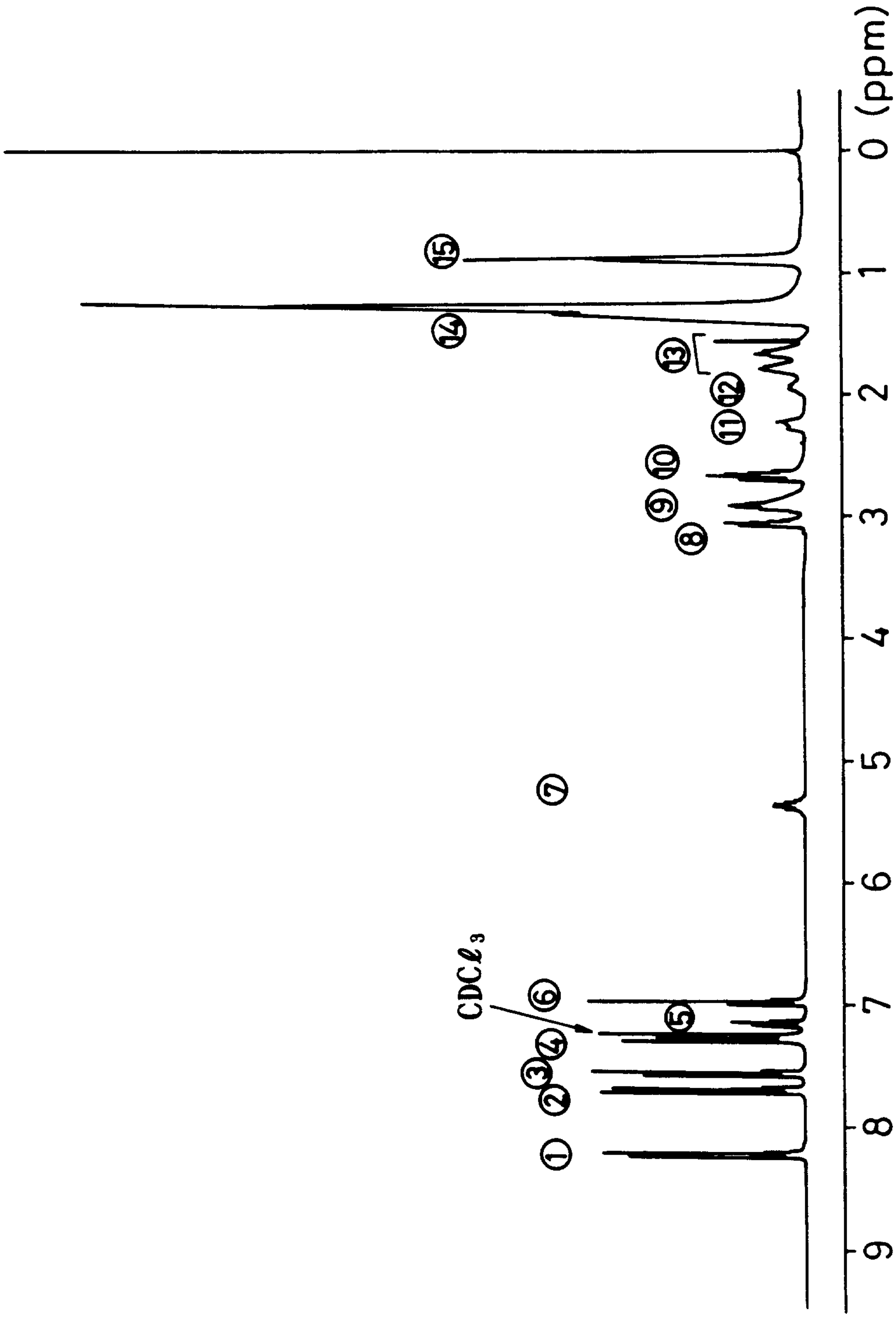

Fig. 28

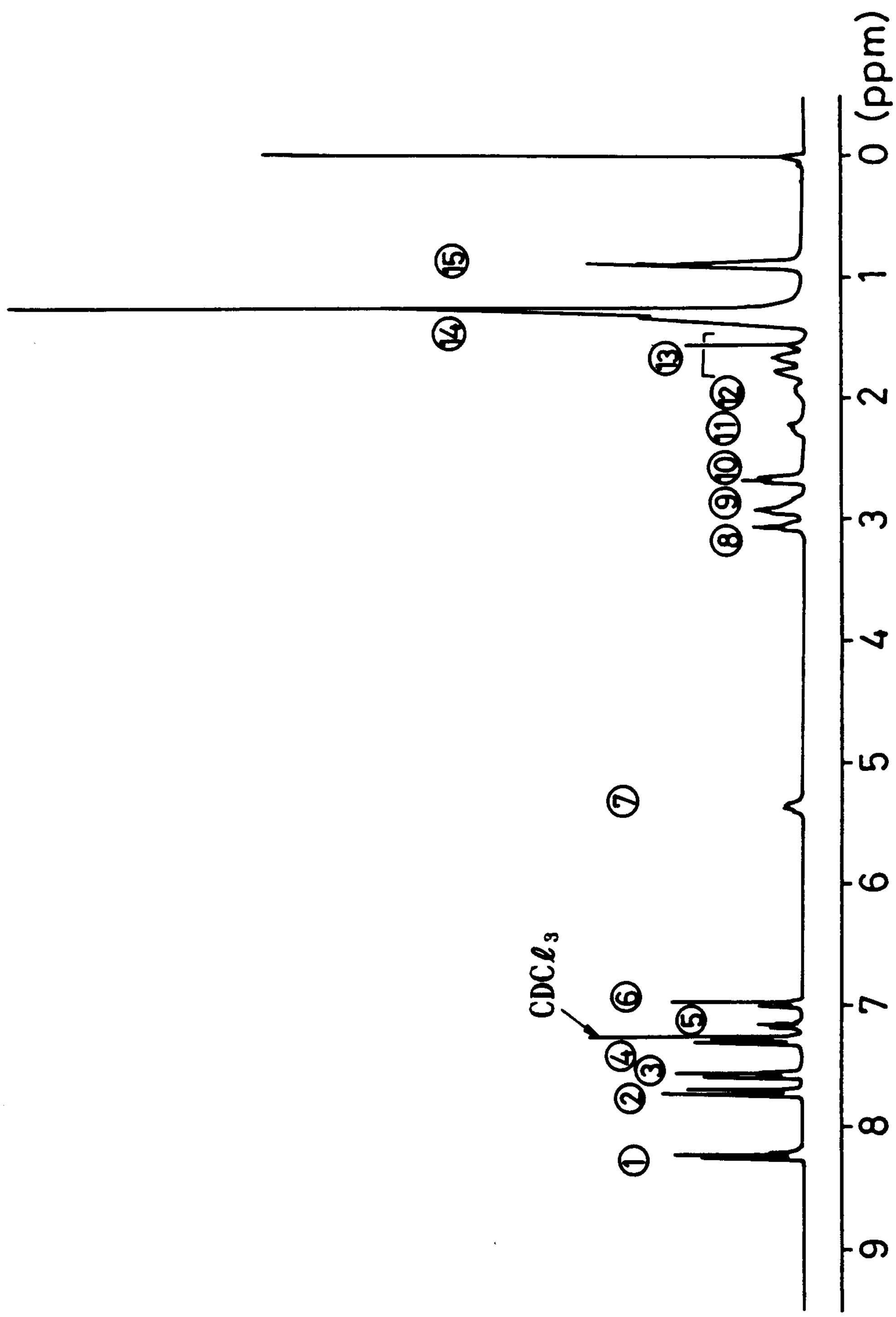

*Fig. 29*

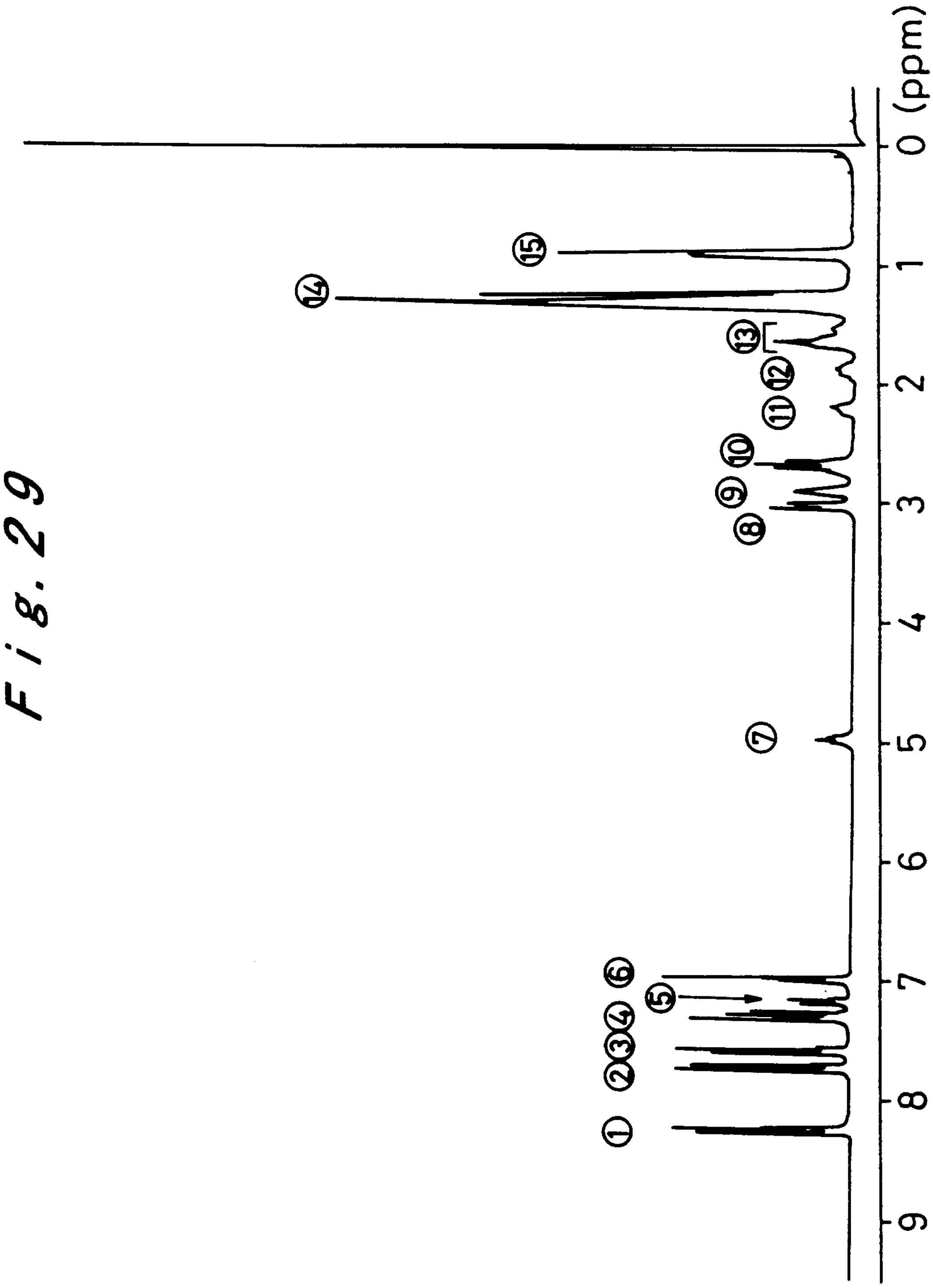

Fig. 30

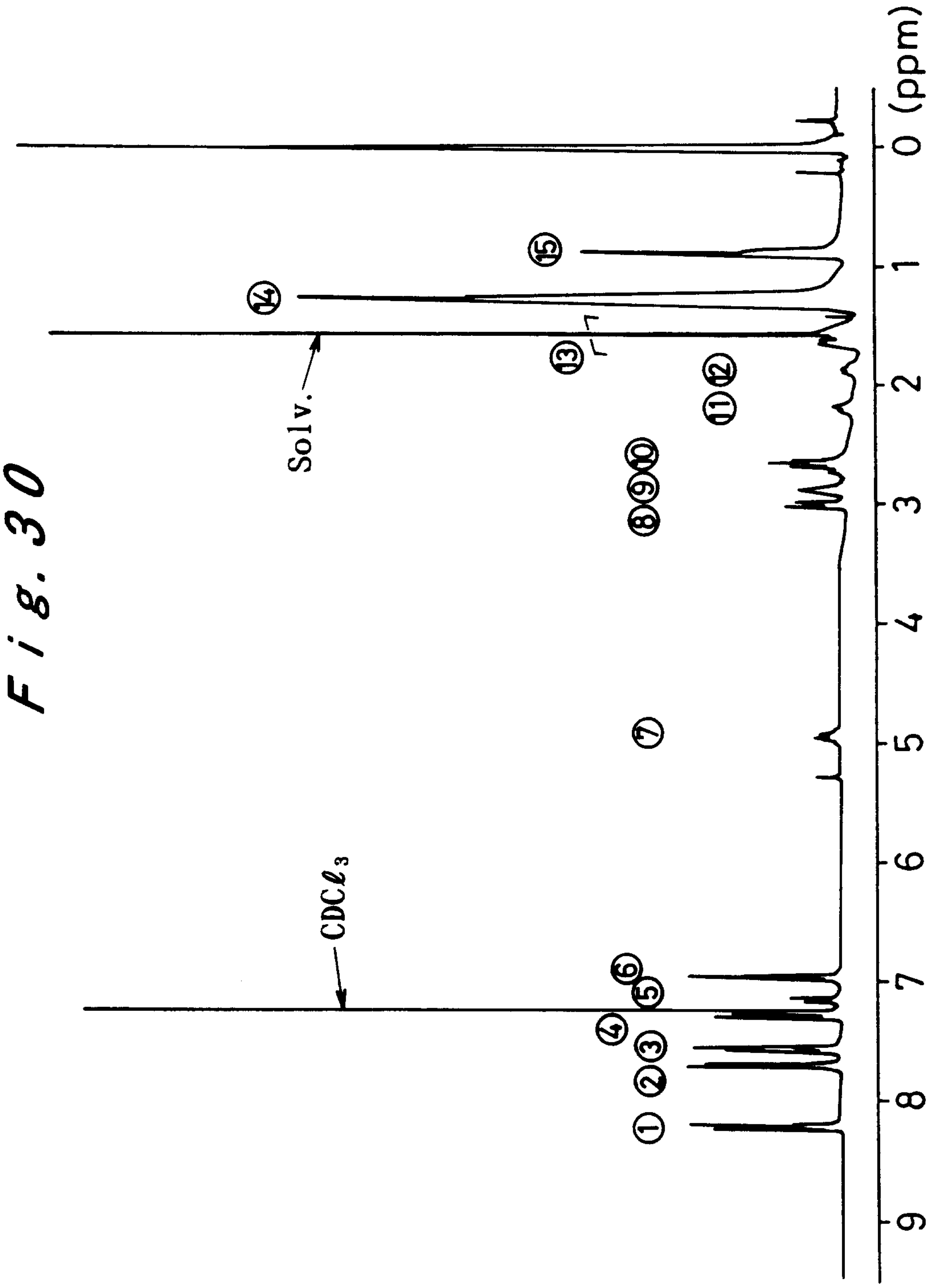

Fig. 31

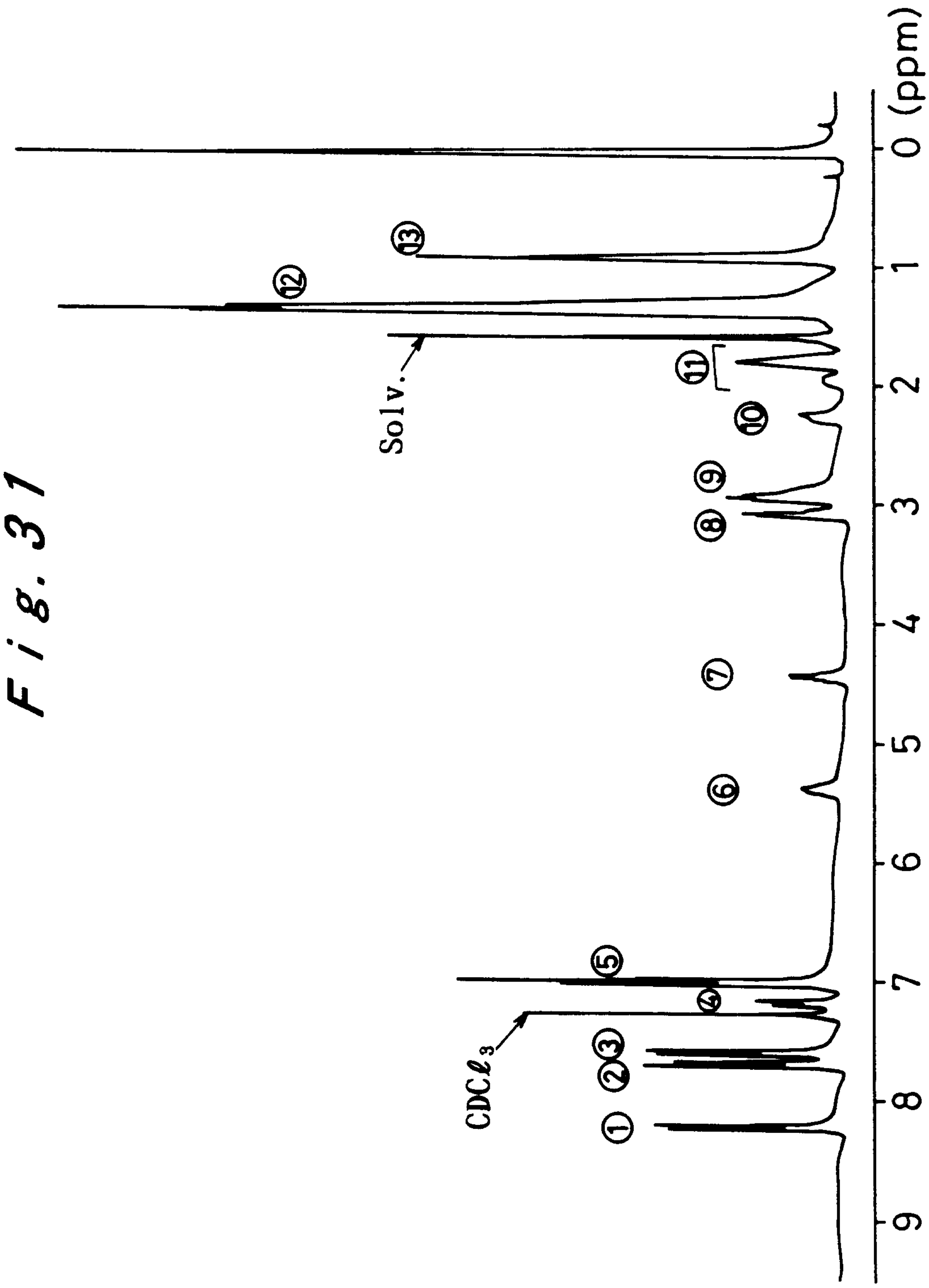

Fig. 32

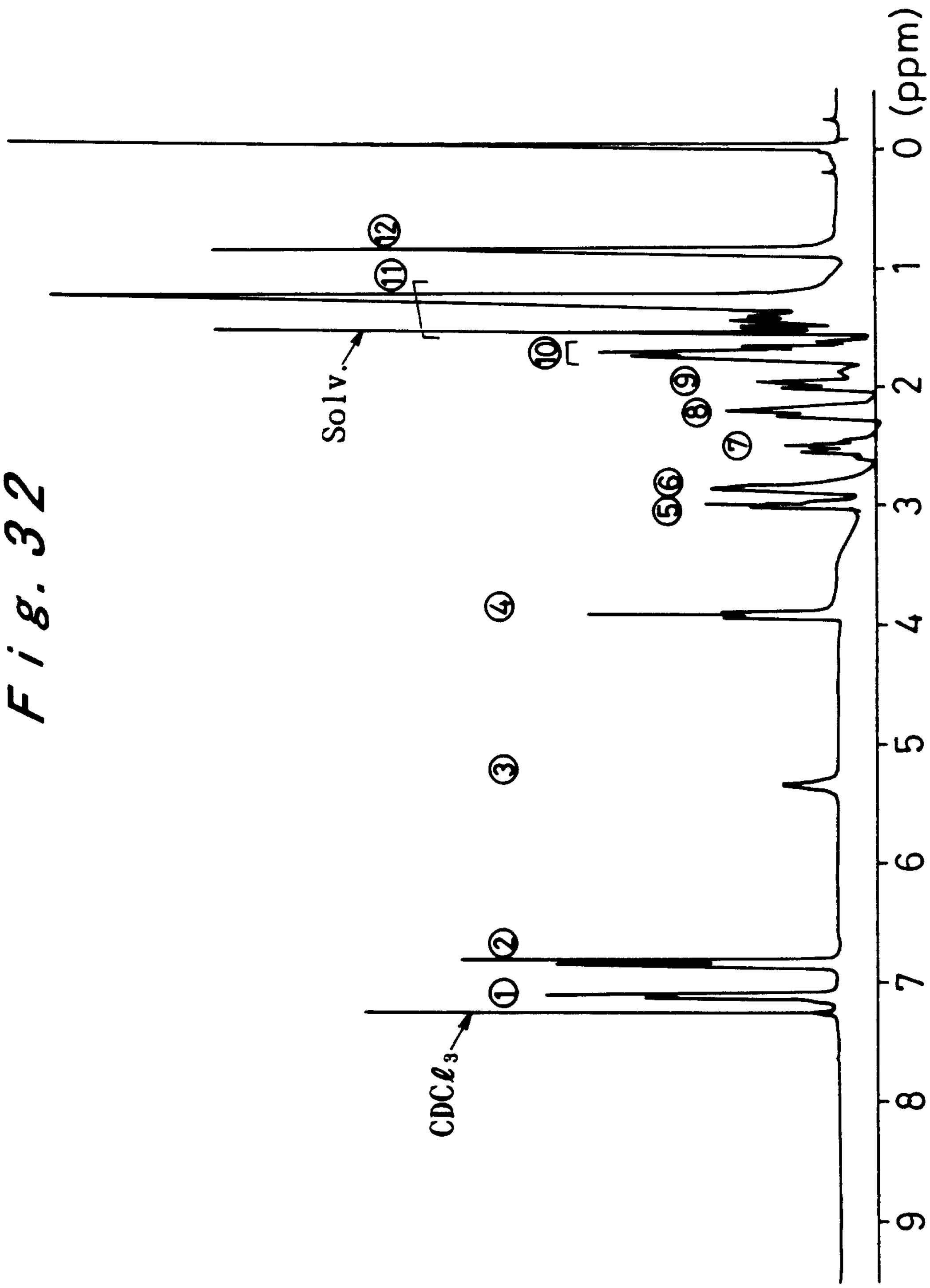

Fig. 33

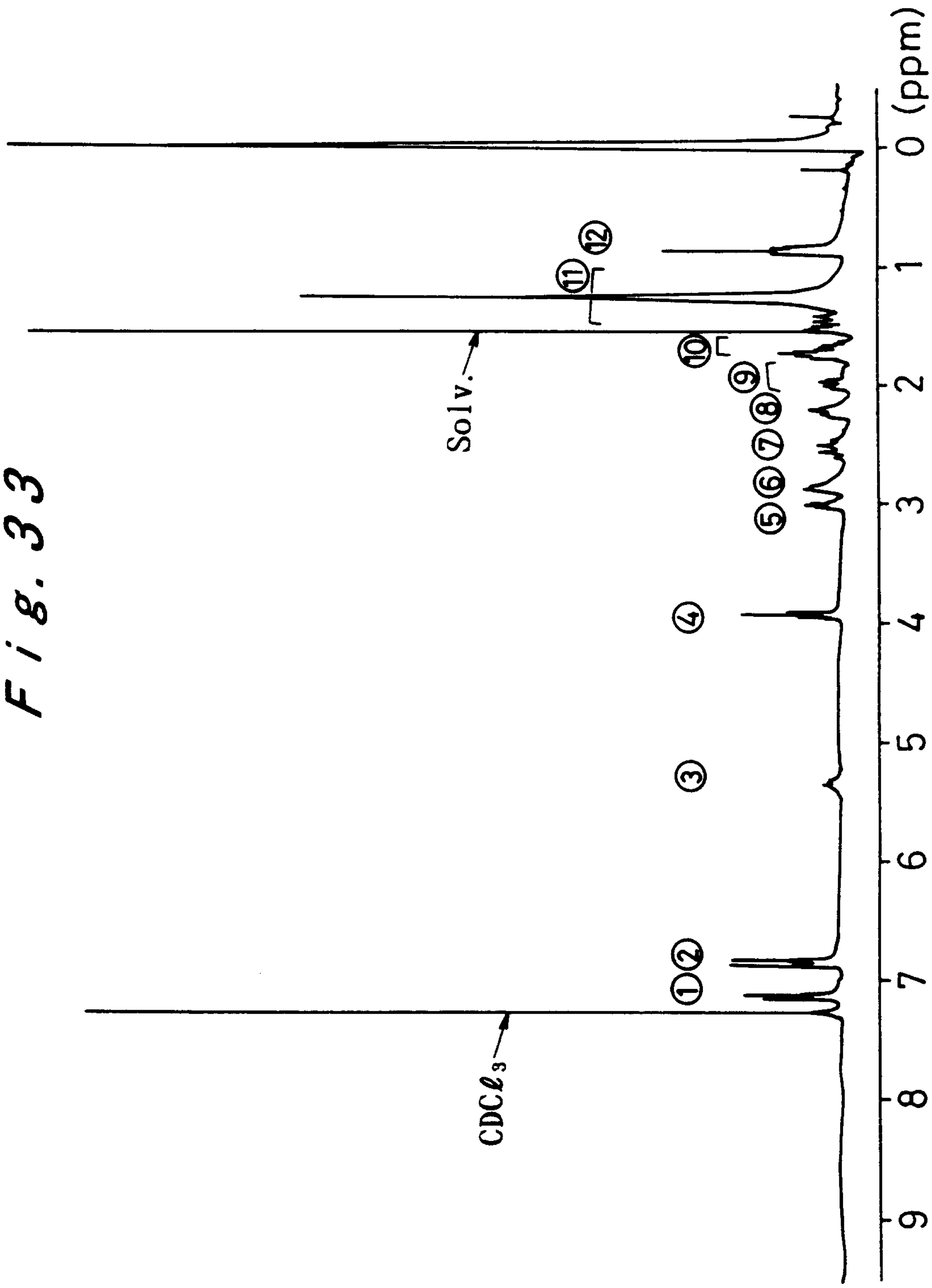

Fig. 34